# EUROPEAN PATENT SPECIFICATION

(11) **EP 3 006 573 B1**
(45) Date of publication and mention of the grant of the patent: **07.03.2018**
(21) Application number: 15181244.3
(22) Date of filing: 01.12.2010
(51) Int. Cl.: C12Q 1/68, C12P 19/34

(54) **METHODS FOR DETERMINING FRACTION OF FETAL NUCLEIC ACIDS IN MATERNAL SAMPLES**
VERFAHREN ZUR BESTIMMUNG DER FRAKTION VON FÖTALEN NUKLEINSÄUREN IN MATERNALEN PROBEN
PROCÉDÉS DE DÉTERMINATION DE FRACTION D'ACIDES NUCLÉIQUES FOETAUX DANS D'ÉCHANTILLONS MATERNELS

(30) Priority: 19.01.2010 US 296358 P; 01.07.2010 US 360837 P; 26.10.2010 US 455849 P; 26.10.2010 US 407017 P
(43) Date of publication of application: 13.04.2016
(62) Divisional of application: 10844163.5
(73) Proprietor: Verinata Health, Inc., Redwood City, CA 94063 (US)
(72) Inventor: RAVA, Richard P., Redwood City, CA 94062 (US); CHUU, Yue-Jen, Cupertino, CA 95014 (US); CHINNAPPA, Manjula, San Mateo, CA 94404 (US); COMSTOCK, David A., San Francisco, CA 94107 (US); HEILEK, Gabrielle, Mountain View, CA 94043 (US); HUNKAPILLER, Michael, San Carlos, CA 94070 (US)
(74) Representative: Chapman, Desmond Mark

(56) References cited:
- WO-A1-2011/057094
- WO-A2-2007/100911
- WO-A2-2007/147074
- US-A1- 2008 050 739
- LI YING ET AL: "Size separation of circulatory DNA in maternal plasma permits ready detection of fetal DNA polymorphisms", CLINICAL CHEMISTRY, AMERICAN ASSOCIATION FOR CLINICAL CHEMISTRY, WASHINGTON, DC, vol. 50, no. 6, 1 June 2004 (2004-06-01), pages 1002-1011, XP002510472, ISSN: 0009-9147, DOI: 10.1373/CLINCHEM.2003.029835
- CHIU R W K ET AL: "Non-invasive prenatal diagnosis by single molecule counting technologies", TRENDS IN GENETICS, ELSEVIER SCIENCE PUBLISHERS B.V. AMSTERDAM, NL, vol. 25, no. 7, 1 July 2009 (2009-07-01), pages 324-331, XP026301457, ISSN: 0168-9525, DOI: 10.1016/J.TIG.2009.05.004 [retrieved on 2009-06-18]
- FAN H C ET AL: "Noninvasive diagnosis of fetal aneuploidy by shotgun sequencing DNA from maternal blood", PROCEEDINGS OF THE NATIONAL ACADEMY OF SCIENCES, NATIONAL ACADEMY OF SCIENCES, US, vol. 105, no. 42, 21 October 2008 (2008-10-21), pages 16266-16271, XP002613056, ISSN: 0027-8424, DOI: 10.1073/PNAS.0808319105 [retrieved on 2008-10-06]
- T. CHU ET AL: "Statistical model for whole genome sequencing and its application to minimally invasive diagnosis of fetal genetic disease", BIOINFORMATICS, vol. 25, no. 10, 23 March 2009 (2009-03-23), pages 1244-1250, XP055018601, ISSN: 1367-4803, DOI: 10.1093/bioinformatics/btp156

### SEQUENCE LISTING

<110> VERINATA HEALTH, INC.
<120> METHODS FOR DETERMINING FRACTION OF FETAL NUCLEIC ACIDS IN MATERNAL SAMPLES
<130> P066852EP
<140> PCT/US2010/058606
   <141> 2010-12-01
<150> 61/455,849
   <151> 2010-10-26
<150> 61/407,017
   <151> 2010-10-26
<150> 61/360,837
   <151> 2010-07-01
<150> 61/296,358
   <151> 2010-01-19
<160> 427
<170> PatentIn version 3.5
<210> 1
   <211> 111
   <212> DNA
   <213> Homo sapiens
<400> 1
   cacatgcaca gccagcaacc ctgtcagcag gagttcccac cagtttcttt ctgagaacat 60
   ctgttcaggt ttctctccat ctctatttac tcaggtcaca ggaccttggg g 111
<210> 2
   <211> 111
   <212> DNA
   <213> Homo sapiens
<400> 2
   cacatgcaca gccagcaacc ctgtcagcag gagttcccac cagtttcttt ctgagaacat 60
   ctgttcaggt ttctctccat ctctgtttac tcaggtcaca ggaccttggg g 111
<210> 3
   <211> 126
   <212> DNA
   <213> Homo sapiens
<400> 3
   tgaggaagtg aggctcagag ggtaagaaac tttgtcacag agctggtggt gagggtggag 60
   attttacact ccctgcctcc cacaccagtt tctccagagt ggaaagactt tcatctcgca 120
   ctggca 126
<210> 4
   <211> 126
   <212> DNA
   <213> Homo sapiens
<400> 4
   tgaggaagtg aggctcagag ggtaagaaac tttgtcacag agctggtggt gagggtggag 60
   attttacact ccctgcctcc cacaccagtt tctccggagt ggaaagactt tcatctcgca 120
   ctggca 126
<210> 5
   <211> 121
   <212> DNA
   <213> Homo sapiens
<400> 5
   gtgccttcag aacctttgag atctgattct atttttaaag cttcttagaa gagagattgc 60
   aaagtgggtt gtttctctag ccagacaggg caggcaaata ggggtggctg gtgggatggg 120
   a 121
<210> 6
   <211> 121
   <212> DNA
   <213> Homo sapiens
<400> 6
   gtgccttcag aacctttgag atctgattct atttttaaag cttcttagaa gagagattgc 60
   aaagtgggtt gtttctctag ccagacaggg caggtaaata ggggtggctg gtgggatggg 120
   a 121
<210> 7
   <211> 111
   <212> DNA
   <213> Homo sapiens
<400> 7
   aggtgtgtct ctcttttgtg aggggagggg tcccttctgg cctagtagag ggcctggcct 60
   gcagtgagca ttcaaatcct caaggaacag ggtggggagg tgggacaaag g 111
<210> 8
   <211> 111
   <212> DNA
   <213> Homo sapiens
<400> 8
   aggtgtgtct ctcttttgtg aggggagggg tcccttctgg cctagtagag ggcctggcct      60
   gcagtgagca ttcaaatcct cgaggaacag ggtggggagg tgggacaaag g              111
<210> 9
   <211> 139
   <212> DNA
   <213> Homo sapiens
<400> 9
   cctcgcctac tgtgctgttt ctaaccatca tgcttttccc tgaatctctt gagtcttttt      60
   ctgctgtgga ctgaaacttg atcctgagat tcacctctag tccctctgag cagcctcctg     120
   gaatactcag ctgggatgg                                                  139
<210> 10
   <211> 139
   <212> DNA
   <213> Homo sapiens
<400> 10
   cctcgcctac tgtgctgttt ctaaccatca tgcttttccc tgaatctctt gagtcttttt      60
   ctgctgtgga ctgaaacttg atcctgagat tcacctctag tccctctggg cagcctcctg     120
   gaatactcag ctgggatgg                                                  139
<210> 11
   <211> 117
   <212> DNA
   <213> Homo sapiens
<400> 11
   aattgcaatg gtgagaggtt gatggtaaaa tcaaacggaa cttgttattt tgtcattctg      60
   atggactgga actgaggatt ttcaatttcc tctccaaccc aagacacttc tcactgg       117
<210> 12
   <211> 117
   <212> DNA
   <213> Homo sapiens
<400> 12
   aattgcaatg gtgagaggtt gatggtaaaa tcaaacggaa cttgttattt tgtcattctg      60
   atggactgga actgaggatt ttcaatttcc tttccaaccc aagacacttc tcactgg       117
<210> 13
   <211> 114
   <212> DNA
   <213> Homo sapiens
<400> 13
   gaaatgcctt ctcaggtaat ggaaggttat ccaaatattt ttcgtaagta tttcaaatag 60
   caatggctcg tctatggtta gtctcacagc cacattctca gaactgctca aacc 114
<210> 14
   <211> 114
   <212> DNA
   <213> Homo sapiens
<400> 14
   gaaatgcctt ctcaggtaat ggaaggttat ccaaatattt ttcgtaagta tttcaaatag 60
   caatggctcg tctatggtta gtctcgcagc cacattctca gaactgctca aacc 114
<210> 15
   <211> 128
   <212> DNA
   <213> Homo sapiens
<400> 15
   acccaaaaca ctggaggggc ctcttctcat tttcggtaga ctgcaagtgt tagccgtcgg 60
   gaccagcttc tgtctggaag ttcgtcaaat tgcagttaag tccaagtatg ccacatagca 120
   gataaggg 128
<210> 16
   <211> 128
   <212> DNA
   <213> Homo sapiens
<400> 16
   acccaaaaca ctggaggggc ctcttctcat tttcggtaga ctgcaagtgt tagccgtcgg 60
   gaccagcttc tgtctggaag ttcgtcaaat tgcagttagg tccaagtatg ccacatagca 120
   gataaggg 128
<210> 17
   <211> 110
   <212> DNA
   <213> Homo sapiens
<400> 17
   gcaccagaat ttaaacaacg ctgacaataa atatgcagtc gatgatgact tcccagagct 60
   ccagaagcaa ctccagcaca cagagaggcg ctgatgtgcc tgtcaggtgc 110
<210> 18
   <211> 110
   <212> DNA
   <213> Homo sapiens
<400> 18
   gcaccagaat ttaaacaacg ctgacaataa atatgcagtc gatgatgact tcccagagct 60
   ccagaagcaa ctccagcaca cggagaggcg ctgatgtgcc tgtcaggtgc 110
<210> 19
   <211> 116
   <212> DNA
   <213> Homo sapiens
<400> 19
   tgactgtata ccccaggtgc acccttgggt catctctatc atagaactta tctcacagag 60
   tataagagct gatttctgtg tctgcctctc acactagact tccacatcct tagtgc 116
<210> 20
   <211> 116
   <212> DNA
   <213> Homo sapiens
<400> 20
   tgactgtata ccccaggtgc acccttgggt catctctatc atagaactta tctcacagag 60
   tataagagct gatttctgtg tctgcctgtc acactagact tccacatcct tagtgc 116
<210> 21
   <211> 110
   <212> DNA
   <213> Homo sapiens
<400> 21
   tgtacgtggt caccagggga cgcctggcgc tgcgagggag gccccgagcc tcgtgccccc 60
   gtgaagcttc agctcccctc cccggctgtc cttgaggctc ttctcacact 110
<210> 22
   <211> 110
   <212> DNA
   <213> Homo sapiens
<400> 22
   tgtacgtggt caccagggga cgcctggcgc tgcgagggag gccccgagcc tcgtgccccc 60
   gtgaagcttc agctcccctc cctggctgtc cttgaggctc ttctcacact 110
<210> 23
   <211> 114
   <212> DNA
   <213> Homo sapiens
<400> 23
   cagtggaccc tgctgcacct ttcctcccct cccatcaacc tcttttgtgc ctccccctcc 60
   gtgtaccacc ttctctgtca ccaaccctgg cctcacaact ctctcctttg ccac 114
<210> 24
   <211> 114
   <212> DNA
   <213> Homo sapiens
<400> 24
   cagtggaccc tgctgcacct ttcctcccct cccatcaacc tcttttgtgc ctccccctcc 60
   gtgtaccacc ttctctgtca ccacccctgg cctcacaact ctctcctttg ccac 114
<210> 25
   <211> 110
   <212> DNA
   <213> Homo sapiens
<400> 25
   cagtggcata gtagtccagg ggctcctcct cagcacctcc agcaccttcc aggaggcagc 60
   agcgcaggca gagaacccgc tggaagaatc ggcggaagtt gtcggagagg 110
<210> 26
   <211> 110
   <212> DNA
   <213> Homo sapiens
<400> 26
   cagtggcata gtagtccagg ggctcctcct cagcacctcc agcaccttcc aggaggcagc 60
   agcgcaggca gagaacccgc tggaaggatc ggcggaagtt gtcggagagg 110
<210> 27
   <211> 129
   <212> DNA
   <213> Homo sapiens
<400> 27
   aggtctgggg gccgctgaat gccaagctgg gaatcttaaa tgttaaggaa caaggtcata 60
   caatgaatgg tgtgatgtaa aagcttggga ggtgatttct gagggtaggt gctgggttta 120
   atgggagga 129
<210> 28
   <211> 129
   <212> DNA
   <213> Homo sapiens
<400> 28
   aggtctgggg gccgctgaat gccaagctgg gaatcttaaa tgttaaggaa caaggtcata 60
   caatgaatgg tgtgatgtaa aagcttggga ggtgattttt gagggtaggt gctgggttta 120
   atgggagga 129
<210> 29
   <211> 107
   <212> DNA
   <213> Homo sapiens
<400> 29
   acggttctgt cctgtagggg agaaaagtcc tcgttgttcc tctgggatgc aacatgagag 60
   agcagcacac tgaggcttta tggattgccc tgccacaagt gaacagg 107
<210> 30
   <211> 107
   <212> DNA
   <213> Homo sapiens
<400> 30
   acggttctgt cctgtagggg agaaaagtcc tcgttgttcc tctgggatgc aacatgagag 60
   agcagcacac tgaggcttta tgggttgccc tgccacaagt gaacagg 107
<210> 31
   <211> 127
   <212> DNA
   <213> Homo sapiens
<400> 31
   gcgcagtcag atgggcgtgc tggcgtctgt cttctctctc tcctgctctc tggcttcatt 60
   tttctctcct tctgtctcac cttctttcgt gtgcctgtgc acacacacgt ttgggacaag 120
   ggctgga 127
<210> 32
   <211> 127
   <212> DNA
   <213> Homo sapiens
<400> 32
   gcgcagtcag atgggcgtgc tggcgtctgt cttctctctc tcctgctctc tggcttcatt        60
   tttctctcct tctgtctcac cttctttcgt gtgcctgtgc atacacacgt ttgggacaag       120
   ggctgga                                                                 127
<210> 33
   <211> 130
   <212> DNA
   <213> Homo sapiens
<400> 33
   gccggacctg cgaaatccca aaatgccaaa cattcccgcc tcacatgatc ccagagagag        60
   gggacccagt gttcccagct tgcagctgag gagcccgagg ttgccgtcag atcagagccc       120
   cagttgcccg                                                              130
<210> 34
   <211> 130
   <212> DNA
   <213> Homo sapiens
<400> 34
   gccggacctg cgaaatccca aaatgccaaa cattcccgcc tcacatgatc ccagagagag        60
   gggacccagt gttcccagct tgcagctgag gagcccgagt ttgccgtcag atcagagccc       120
   cagttgcccg                                                              130
<210> 35
   <211> 121
   <212> DNA
   <213> Homo sapiens
<400> 35
   agcagcctcc ctcgactagc tcacactacg ataaggaaaa ttcatgagct ggtgtccaag        60
   gagggctggg tgactcgtgg ctcagtcagc atcaagattc ctttcgtctt tcccctctgc       120
   c                                                                       121
<210> 36
   <211> 121
   <212> DNA
   <213> Homo sapiens
<400> 36
   agcagcctcc ctcgactagc tcacactacg ataaggaaaa ttcatgagct ggtgtccaag 60
   gagggctggg tgactcgtgg ctcagtcagc gtcaagattc ctttcgtctt tcccctctgc 120
   c 121
<210> 37
   <211> 138
   <212> DNA
   <213> Homo sapiens
<400> 37
   tggcattgcc tgtaatatac atagccatgg ttttttatag gcaatttaag atgaatagct 60
   tctaaactat agataagttt cattacccca ggaagctgaa ctatagctac tttacccaaa 120
   atcattagaa tggtgctt 138
<210> 38
   <211> 138
   <212> DNA
   <213> Homo sapiens
<400> 38
   tggcattgcc tgtaatatac atagccatgg ttttttatag gcaatttaag atgaatagct 60
   tctaaactat agataagttt cattacccca ggaagctgaa ctatagctac tttccccaaa 120
   atcattagaa tggtgctt 138
<210> 39
   <211> 136
   <212> DNA
   <213> Homo sapiens
<400> 39
   atgaagcctt ccaccaactg cctgtatgac tcatctgggg acttctgctc tatactcaaa 60
   gtggcttagt cactgccaat gtatttccat atgagggacg atgattacta aggaaatata 120
   gaaacaacaa ctgatc 136
<210> 40
   <211> 136
   <212> DNA
   <213> Homo sapiens
<400> 40
   atgaagcctt ccaccaactg cctgtatgac tcatctgggg acttctgctc tatactcaaa 60
   gtggcttagt cactgccaat gtatttccat atgagggacg gtgattacta aggaaatata 120
   gaaacaacaa ctgatc 136
<210> 41
   <211> 118
   <212> DNA
   <213> Homo sapiens
<400> 41
   acaacagaat caggtgattg gagaaaagat cacaggccta ggcacccaag gcttgaagga 60
   tgaaagaatg aaagatggac ggaacaaaat taggacctta attctttgtt cagttcag 118
<210> 42
   <211> 118
   <212> DNA
   <213> Homo sapiens
<400> 42
   acaacagaat caggtgattg gagaaaagat cacaggccta ggcacccaag gcttgaagga 60
   tgaaagaatg aaagatggac ggaagaaaat taggacctta attctttgtt cagttcag 118
<210> 43
   <211> 150
   <212> DNA
   <213> Homo sapiens
<400> 43
   ttggggtaaa ttttcattgt catatgtgga atttaaatat accatcatct acaaagaatt 60
   ccacagagtt aaatatctta agttaaacac ttaaaataag tgtttgcgtg atattttgat 120
   gacagataaa cagagtctaa ttcccacccc 150
<210> 44
   <211> 150
   <212> DNA
   <213> Homo sapiens
<400> 44
   ttggggtaaa ttttcattgt catatgtgga atttaaatat accatcatct acaaagaatt 60
   ccacagagtt aaatatctta agttaaacac ttaaaataag tgtttgcgtg atattttgat 120
   gatagataaa cagagtctaa ttcccacccc 150
<210> 45
   <211> 145
   <212> DNA
   <213> Homo sapiens
<400> 45
   tgcaattcaa atcaggaagt atgaccaaaa gacagagatc ttttttggat gatccctagc        60
   ctagcaatgc ctggcagcca tgcaggtgca atgtcaacct taaataatgt attgcaaact       120
   cagagctgac aaacctcgat gttgc                                             145
<210> 46
   <211> 145
   <212> DNA
   <213> Homo sapiens
<400> 46
   tgcaattcaa atcaggaagt atgaccaaaa gacagagatc ttttttggat gatccctagc        60
   ctagcaatgc ctggcagcca tgcaggtgca atgtcaacct taaataatgt attgcaaatt       120
   cagagctgac aaacctcgat gttgc                                             145
<210> 47
   <211> 124
   <212> DNA
   <213> Homo sapiens
<400> 47
   ctgtgctctg cgaatagctg cagaagtaac ttggggaccc aaaataaagc agaatgctaa        60
   tgtcaagtcc tgagaaccaa gccctgggac tctggtgcca tttcggattc tccatgagca       120
   tggt                                                                    124
<210> 48
   <211> 124
   <212> DNA
   <213> Homo sapiens
<400> 48
   ctgtgctctg cgaatagctg cagaagtaac ttggggaccc aaaataaagc agaatgctaa        60
   tgtcaagtcc tgagaaccaa gccctgggac tctggtgcca ttttggattc tccatgagca       120
   tggt                                                                    124
<210> 49
   <211> 118
   <212> DNA
   <213> Homo sapiens
<400> 49
   tttttccagc caactcaagg ccaaaaaaaa tttcttaata tagttattat gcgaggggag        60
   gggaagcaaa ggagcacagg tagtccacag aataagacac aagaaacctc aagctgtg         118
<210> 50
   <211> 118
   <212> DNA
   <213> Homo sapiens
<400> 50
   tttttccagc caactcaagg ccaaaaaaaa tttcttaata tagttattat gcgaggggag 60
   gggaagcaaa ggagcacagg tagtccacag aataggacac aagaaacctc aagctgtg 118
<210> 51
   <211> 110
   <212> DNA
   <213> Homo sapiens
<400> 51
   tcttctcgtc ccctaagcaa acaacatccg cttgcttctg tctgtgtaac cacagtgaat 60
   gggtgtgcac gcttgatggg cctctgagcc cctgttgcac aaaccagaaa 110
<210> 52
   <211> 110
   <212> DNA
   <213> Homo sapiens
<400> 52
   tcttctcgtc ccctaagcaa acaacatccg cttgcttctg tctgtgtaac cacagtgaat 60
   gggtgtgcac gcttggtggg cctctgagcc cctgttgcac aaaccagaaa 110
<210> 53
   <211> 110
   <212> DNA
   <213> Homo sapiens
<400> 53
   cacatggggg cattaagaat cgcccaggga ggaggaggga gaacgcgtgc ttttcacatt 60
   tgcatttgaa ttttcgagtt cccaggatgt gtttttgtgc tcatcgatgt 110
<210> 54
   <211> 110
   <212> DNA
   <213> Homo sapiens
<400> 54
   cacatggggg cattaagaat cgcccaggga ggaggaggga gaacgcgtgc ttttcacatt 60
   tgcatttgaa tttttgagtt cccaggatgt gtttttgtgc tcatcgatgt 110
<210> 55
   <211> 128
   <212> DNA
   <213> Homo sapiens
<400> 55
   gggctctgag gtgtgtgaaa taaaaacaaa tgtccatgtc tgtcctttta tggcattttg 60
   ggactttaca tttcaaacat ttcagacatg tatcacaaca cgaaggaata acagttccag 120
   ggatatct 128
<210> 56
   <211> 128
   <212> DNA
   <213> Homo sapiens
<400> 56
   gggctctgag gtgtgtgaaa taaaaacaaa tgtccatgtc tgtcctttta tggcattttg 60
   ggactttaca tttcaaacat ttcagacatg tatcacaaca cgagggaata acagttccag 120
   ggatatct 128
<210> 57
   <211> 21
   <212> DNA
   <213> Artificial Sequence
<220>
   <223> Description of Artificial Sequence: Synthetic primer
<400> 57
   cacatgcaca gccagcaacc c 21
<210> 58
   <211> 23
   <212> DNA
   <213> Artificial Sequence
<220>
   <223> Description of Artificial Sequence: Synthetic primer
<400> 58
   ccccaaggtc ctgtgacctg agt 23
<210> 59
   <211> 23
   <212> DNA
   <213> Artificial Sequence
<220>
   <223> Description of Artificial Sequence: Synthetic primer
<400> 59
   tgaggaagtg aggctcagag ggt 23
<210> 60
   <211> 24
   <212> DNA
   <213> Artificial Sequence
<220>
   <223> Description of Artificial Sequence: Synthetic primer
<400> 60
   tgccagtgcg agatgaaagt cttt 24
<210> 61
   <211> 27
   <212> DNA
   <213> Artificial Sequence
<220>
   <223> Description of Artificial Sequence: Synthetic primer
<400> 61
   gtgccttcag aacctttgag atctgat 27
<210> 62
   <211> 20
   <212> DNA
   <213> Artificial Sequence
<220>
   <223> Description of Artificial Sequence: Synthetic primer
<400> 62
   tcccatccca ccagccaccc 20
<210> 63
   <211> 25
   <212> DNA
   <213> Artificial Sequence
<220>
   <223> Description of Artificial Sequence: Synthetic primer
<400> 63
   aggtgtgtct ctcttttgtg agggg 25
<210> 64
   <211> 21
   <212> DNA
   <213> Artificial Sequence
<220>
   <223> Description of Artificial Sequence: Synthetic primer
<400> 64
   cctttgtccc acctccccac c 21
<210> 65
   <211> 26
   <212> DNA
   <213> Artificial Sequence
<220>
   <223> Description of Artificial Sequence: Synthetic primer
<400> 65
   cctcgcctac tgtgctgttt ctaacc 26
<210> 66
   <211> 25
   <212> DNA
   <213> Artificial Sequence
<220>
   <223> Description of Artificial Sequence: Synthetic primer
<400> 66
   ccatcccagc tgagtattcc aggag 25
<210> 67
   <211> 26
   <212> DNA
   <213> Artificial Sequence
<220>
   <223> Description of Artificial Sequence: Synthetic primer
<400> 67
   aattgcaatg gtgagaggtt gatggt 26
<210> 68
   <211> 24
   <212> DNA
   <213> Artificial Sequence
<220>
   <223> Description of Artificial Sequence: Synthetic primer
<400> 68
   ccagtgagaa gtgtcttggg ttgg 24
<210> 69
   <211> 27
   <212> DNA
   <213> Artificial Sequence
<220>
   <223> Description of Artificial Sequence: Synthetic primer
<400> 69
   gaaatgcctt ctcaggtaat ggaaggt 27
<210> 70
   <211> 27
   <212> DNA
   <213> Artificial Sequence
<220>
   <223> Description of Artificial Sequence: Synthetic primer
<400> 70
   ggtttgagca gttctgagaa tgtggct 27
<210> 71
   <211> 22
   <212> DNA
   <213> Artificial Sequence
<220>
   <223> Description of Artificial Sequence: Synthetic primer
<400> 71
   acccaaaaca ctggaggggc ct 22
<210> 72
   <211> 27
   <212> DNA
   <213> Artificial Sequence
<220>
   <223> Description of Artificial Sequence: Synthetic primer
<400> 72
   cccttatctg ctatgtggca tacttgg 27
<210> 73
   <211> 27
   <212> DNA
   <213> Artificial Sequence
<220>
   <223> Description of Artificial Sequence: Synthetic primer
<400> 73
   gcaccagaat ttaaacaacg ctgacaa 27
<210> 74
   <211> 22
   <212> DNA
   <213> Artificial Sequence
<220>
   <223> Description of Artificial Sequence: Synthetic primer
<400> 74
   gcacctgaca ggcacatcag cg 22
<210> 75
   <211> 24
   <212> DNA
   <213> Artificial Sequence
<220>
   <223> Description of Artificial Sequence: Synthetic primer
<400> 75
   tgactgtata ccccaggtgc accc 24
<210> 76
   <211> 27
   <212> DNA
   <213> Artificial Sequence
<220>
   <223> Description of Artificial Sequence: Synthetic primer
<400> 76
   gcactaagga tgtggaagtc tagtgtg 27
<210> 77
   <211> 22
   <212> DNA
   <213> Artificial Sequence
<220>
   <223> Description of Artificial Sequence: Synthetic primer
<400> 77
   tgtacgtggt caccagggga cg 22
<210> 78
   <211> 26
   <212> DNA
   <213> Artificial Sequence
<220>
   <223> Description of Artificial Sequence: Synthetic primer
<400> 78
   agtgtgagaa gagcctcaag gacagc 26
<210> 79
   <211> 21
   <212> DNA
   <213> Artificial Sequence
<220>
   <223> Description of Artificial Sequence: Synthetic primer
<400> 79
   cagtggaccc tgctgcacct t 21
<210> 80
   <211> 24
   <212> DNA
   <213> Artificial Sequence
<220>
   <223> Description of Artificial Sequence: Synthetic primer
<400> 80
   gtggcaaagg agagagttgt gagg 24
<210> 81
   <211> 24
   <212> DNA
   <213> Artificial Sequence
<220>
   <223> Description of Artificial Sequence: Synthetic primer
<400> 81
   cagtggcata gtagtccagg ggct 24
<210> 82
   <211> 21
   <212> DNA
   <213> Artificial Sequence
<220>
   <223> Description of Artificial Sequence: Synthetic primer
<400> 82
   cctctccgac aacttccgcc g 21
<210> 83
   <211> 20
   <212> DNA
   <213> Artificial Sequence
<220>
   <223> Description of Artificial Sequence: Synthetic primer
<400> 83
   aggtctgggg gccgctgaat 20
<210> 84
   <211> 23
   <212> DNA
   <213> Artificial Sequence
<220>
   <223> Description of Artificial Sequence: Synthetic primer
<400> 84
   tcctcccatt aaacccagca cct 23
<210> 85
   <211> 23
   <212> DNA
   <213> Artificial Sequence
<220>
   <223> Description of Artificial Sequence: Synthetic primer
<400> 85
   acggttctgt cctgtagggg aga 23
<210> 86
   <211> 22
   <212> DNA
   <213> Artificial Sequence
<220>
   <223> Description of Artificial Sequence: Synthetic primer
<400> 86
   cctgttcact tgtggcaggg ca 22
<210> 87
   <211> 20
   <212> DNA
   <213> Artificial Sequence
<220>
   <223> Description of Artificial Sequence: Synthetic primer
<400> 87
   gcgcagtcag atgggcgtgc 20
<210> 88
   <211> 23
   <212> DNA
   <213> Artificial Sequence
<220>
   <223> Description of Artificial Sequence: Synthetic primer
<400> 88
   tccagccctt gtcccaaacg tgt 23
<210> 89
   <211> 21
   <212> DNA
   <213> Artificial Sequence
<220>
   <223> Description of Artificial Sequence: Synthetic primer
<400> 89
   gccggacctg cgaaatccca a 21
<210> 90
   <211> 21
   <212> DNA
   <213> Artificial Sequence
<220>
   <223> Description of Artificial Sequence: Synthetic primer
<400> 90
   cgggcaactg gggctctgat c 21
<210> 91
   <211> 21
   <212> DNA
   <213> Artificial Sequence
<220>
   <223> Description of Artificial Sequence: Synthetic primer
<400> 91
   agcagcctcc ctcgactagc t 21
<210> 92
   <211> 23
   <212> DNA
   <213> Artificial Sequence
<220>
   <223> Description of Artificial Sequence: Synthetic primer
<400> 92
   ggcagagggg aaagacgaaa gga 23
<210> 93
   <211> 24
   <212> DNA
   <213> Artificial Sequence
<220>
   <223> Description of Artificial Sequence: Synthetic primer
<400> 93
   tggcattgcc tgtaatatac atag 24
<210> 94
   <211> 23
   <212> DNA
   <213> Artificial Sequence
<220>
   <223> Description of Artificial Sequence: Synthetic primer
<400> 94
   aagcaccatt ctaatgattt tgg 23
<210> 95
   <211> 20
   <212> DNA
   <213> Artificial Sequence
<220>
   <223> Description of Artificial Sequence: Synthetic primer
<400> 95
   atgaagcctt ccaccaactg 20
<210> 96
   <211> 27
   <212> DNA
   <213> Artificial Sequence
<220>
   <223> Description of Artificial Sequence: Synthetic primer
<400> 96
   gatcagttgt tgtttctata tttcctt 27
<210> 97
   <211> 22
   <212> DNA
   <213> Artificial Sequence
<220>
   <223> Description of Artificial Sequence: Synthetic primer
<400> 97
   acaacagaat caggtgattg ga 22
<210> 98
   <211> 25
   <212> DNA
   <213> Artificial Sequence
<220>
   <223> Description of Artificial Sequence: Synthetic primer
<400> 98
   ctgaactgaa caaagaatta aggtc 25
<210> 99
   <211> 22
   <212> DNA
   <213> Artificial Sequence
<220>
   <223> Description of Artificial Sequence: Synthetic primer
<400> 99
   ttggggtaaa ttttcattgt ca 22
<210> 100
   <211> 20
   <212> DNA
   <213> Artificial Sequence
<220>
   <223> Description of Artificial Sequence: Synthetic primer
<400> 100
   ggggtgggaa ttagactctg 20
<210> 101
   <211> 23
   <212> DNA
   <213> Artificial Sequence
<220>
   <223> Description of Artificial Sequence: Synthetic primer
<400> 101
   tgcaattcaa atcaggaagt atg 23
<210> 102
   <211> 20
   <212> DNA
   <213> Artificial Sequence
<220>
   <223> Description of Artificial Sequence: Synthetic primer
<400> 102
   gcaacatcga ggtttgtcag 20
<210> 103
   <211> 20
   <212> DNA
   <213> Artificial Sequence
<220>
   <223> Description of Artificial Sequence: Synthetic primer
<400> 103
   ctgtgctctg cgaatagctg 20
<210> 104
   <211> 20
   <212> DNA
   <213> Artificial Sequence
<220>
   <223> Description of Artificial Sequence: Synthetic primer
<400> 104
   accatgctca tggagaatcc 20
<210> 105
   <211> 20
   <212> DNA
   <213> Artificial Sequence
<220>
   <223> Description of Artificial Sequence: Synthetic primer
<400> 105
   tttttccagc caactcaagg 20
<210> 106
   <211> 21
   <212> DNA
   <213> Artificial Sequence
<220>
   <223> Description of Artificial Sequence: Synthetic primer
<400> 106
   cacagcttga ggtttcttgt g 21
<210> 107
   <211> 20
   <212> DNA
   <213> Artificial Sequence
<220>
   <223> Description of Artificial Sequence: Synthetic primer
<400> 107
   tcttctcgtc ccctaagcaa 20
<210> 108
   <211> 20
   <212> DNA
   <213> Artificial Sequence
<220>
   <223> Description of Artificial Sequence: Synthetic primer
<400> 108
   tttctggttt gtgcaacagg 20
<210> 109
   <211> 20
   <212> DNA
   <213> Artificial Sequence
<220>
   <223> Description of Artificial Sequence: Synthetic primer
<400> 109
   cacatggggg cattaagaat 20
<210> 110
   <211> 22
   <212> DNA
   <213> Artificial Sequence
<220>
   <223> Description of Artificial Sequence: Synthetic primer
<400> 110
   acatcgatga gcacaaaaac ac 22
<210> 111
   <211> 20
   <212> DNA
   <213> Artificial Sequence
<220>
   <223> Description of Artificial Sequence: Synthetic primer
<400> 111
   gggctctgag gtgtgtgaaa 20
<210> 112
   <211> 24
   <212> DNA
   <213> Artificial Sequence
<220>
   <223> Description of Artificial Sequence: Synthetic primer
<400> 112
   agatatccct ggaactgtta ttcc 24
<210> 113
   <211> 23
   <212> DNA
   <213> Artificial Sequence
<220>
   <223> Description of Artificial Sequence: Synthetic primer
<400> 113
   acagtaactg ccttcataga tag 23
<210> 114
   <211> 22
   <212> DNA
   <213> Artificial Sequence
<220>
   <223> Description of Artificial Sequence: Synthetic primer
<400> 114
   gtgtcagacc ctgttctaag ta 22
<210> 115
   <211> 26
   <212> DNA
   <213> Artificial Sequence
<220>
   <223> Description of Artificial Sequence: Synthetic primer
<400> 115
   aaataaaatt aggcatattt acaagc 26
<210> 116
   <211> 23
   <212> DNA
   <213> Artificial Sequence
<220>
   <223> Description of Artificial Sequence: Synthetic primer
<400> 116
   gctgagtgat ttgtctgtaa ttg 23
<210> 117
   <211> 21
   <212> DNA
   <213> Artificial Sequence
<220>
   <223> Description of Artificial Sequence: Synthetic primer
<400> 117
   cctgttcctc ccttatttcc c 21
<210> 118
   <211> 21
   <212> DNA
   <213> Artificial Sequence
<220>
   <223> Description of Artificial Sequence: Synthetic primer
<400> 118
   gggaacacag actccatggt g 21
<210> 119
   <211> 22
   <212> DNA
   <213> Artificial Sequence
<220>
   <223> Description of Artificial Sequence: Synthetic primer
<400> 119
   cttagggaac cctcactgaa tg 22
<210> 120
   <211> 22
   <212> DNA
   <213> Artificial Sequence
<220>
   <223> Description of Artificial Sequence: Synthetic primer
<400> 120
   gtccttgtca gcgtttattt gc 22
<210> 121
   <211> 26
   <212> DNA
   <213> Artificial Sequence
<220>
   <223> Description of Artificial Sequence: Synthetic primer
<400> 121
   aataatcagt atgtgacttg gattga 26
<210> 122
   <211> 22
   <212> DNA
   <213> Artificial Sequence
<220>
   <223> Description of Artificial Sequence: Synthetic primer
<400> 122
   ataggatgga tggatagatg ga 22
<210> 123
   <211> 21
   <212> DNA
   <213> Artificial Sequence
<220>
   <223> Description of Artificial Sequence: Synthetic primer
<400> 123
   cagagcaaga ccctgtctca t 21
<210> 124
   <211> 21
   <212> DNA
   <213> Artificial Sequence
<220>
   <223> Description of Artificial Sequence: Synthetic primer
<400> 124
   tcaacagagg cttgcatgta t 21
<210> 125
   <211> 20
   <212> DNA
   <213> Artificial Sequence
<220>
   <223> Description of Artificial Sequence: Synthetic primer
<400> 125
   gggtgatttt cctctttggt 20
<210> 126
   <211> 33
   <212> DNA
   <213> Artificial Sequence
<220>
   <223> Description of Artificial Sequence: Synthetic primer
<400> 126
   aacatttgta tctttatctg tatccttatt tat 33
<210> 127
   <211> 27
   <212> DNA
   <213> Artificial Sequence
<220>
   <223> Description of Artificial Sequence: Synthetic primer
<400> 127
   gaacacttgt catagtttag aacgaac 27
<210> 128
   <211> 20
   <212> DNA
   <213> Artificial Sequence
<220>
   <223> Description of Artificial Sequence: Synthetic primer
<400> 128
   tcattgacag aattgcacca 20
<210> 129
   <211> 27
   <212> DNA
   <213> Artificial Sequence
<220>
   <223> Description of Artificial Sequence: Synthetic primer
<400> 129
   tttgtatttc atgtgtacat tcgtatc 27
<210> 130
   <211> 28
   <212> DNA
   <213> Artificial Sequence
<220>
   <223> Description of Artificial Sequence: Synthetic primer
<400> 130
   acctatcctg tagattattt tcactgtg 28
<210> 131
   <211> 20
   <212> DNA
   <213> Artificial Sequence
<220>
   <223> Description of Artificial Sequence: Synthetic primer
<400> 131
   tctgacccat ctaacgccta 20
<210> 132
   <211> 27
   <212> DNA
   <213> Artificial Sequence
<220>
   <223> Description of Artificial Sequence: Synthetic primer
<400> 132
   cagacagaaa gatagataga tgattga 27
<210> 133
   <211> 21
   <212> DNA
   <213> Artificial Sequence
<220>
   <223> Description of Artificial Sequence: Synthetic primer
<400> 133
   atacagacag acagacaggt g 21
<210> 134
   <211> 23
   <212> DNA
   <213> Artificial Sequence
<220>
   <223> Description of Artificial Sequence: Synthetic primer
<400> 134
   gcatgtatct atcatccatc tct 23
<210> 135
   <211> 21
   <212> DNA
   <213> Artificial Sequence
<220>
   <223> Description of Artificial Sequence: Synthetic primer
<400> 135
   tgagtgacaa attgagacct t 21
<210> 136
   <211> 26
   <212> DNA
   <213> Artificial Sequence
<220>
   <223> Description of Artificial Sequence: Synthetic primer
<400> 136
   gtcttacaat aacagttgct actatt 26
<210> 137
   <211> 18
   <212> DNA
   <213> Artificial Sequence
<220>
   <223> Description of Artificial Sequence: Synthetic primer
<400> 137
   attccccaag tgaattgc 18
<210> 138
   <211> 26
   <212> DNA
   <213> Artificial Sequence
<220>
   <223> Description of Artificial Sequence: Synthetic primer
<400> 138
   ggtagataga ctggatagat agacga 26
<210> 139
   <211> 20
   <212> DNA
   <213> Artificial Sequence
<220>
   <223> Description of Artificial Sequence: Synthetic primer
<400> 139
   tggaaacaga aatggcttgg 20
<210> 140
   <211> 20
   <212> DNA
   <213> Artificial Sequence
<220>
   <223> Description of Artificial Sequence: Synthetic primer
<400> 140
   gattgcagga gggaaggaag 20
<210> 141
   <211> 22
   <212> DNA
   <213> Artificial Sequence
<220>
   <223> Description of Artificial Sequence: Synthetic primer
<400> 141
   gagcaagaca ccatctcaag aa 22
<210> 142
   <211> 30
   <212> DNA
   <213> Artificial Sequence
<220>
   <223> Description of Artificial Sequence: Synthetic primer
<400> 142
   gaaattttac atttatgttt atgattctct 30
<210> 143
   <211> 18
   <212> DNA
   <213> Artificial Sequence
<220>
   <223> Description of Artificial Sequence: Synthetic primer
<400> 143
   ggcgactgag caagactc 18
<210> 144
   <211> 27
   <212> DNA
   <213> Artificial Sequence
<220>
   <223> Description of Artificial Sequence: Synthetic primer
<400> 144
   ggttattaat tgagaaaact ccttaca 27
<210> 145
   <211> 23
   <212> DNA
   <213> Artificial Sequence
<220>
   <223> Description of Artificial Sequence: Synthetic primer
<400> 145
   attttccccg atgatagtag tct 23
<210> 146
   <211> 26
   <212> DNA
   <213> Artificial Sequence
<220>
   <223> Description of Artificial Sequence: Synthetic primer
<400> 146
   gcgaatgtat gattggcaat attttt 26
<210> 147
   <211> 27
   <212> DNA
   <213> Artificial Sequence
<220>
   <223> Description of Artificial Sequence: Synthetic primer
<400> 147
   acatgtatcc cagaacttaa agtaaac 27
<210> 148
   <211> 22
   <212> DNA
   <213> Artificial Sequence
<220>
   <223> Description of Artificial Sequence: Synthetic primer
<400> 148
   gcagaaggga aaattgaagc tg 22
<210> 149
   <211> 22
   <212> DNA
   <213> Artificial Sequence
<220>
   <223> Description of Artificial Sequence: Synthetic primer
<400> 149
   cagagacacc gaaccaataa ga 22
<210> 150
   <211> 27
   <212> DNA
   <213> Artificial Sequence
<220>
   <223> Description of Artificial Sequence: Synthetic primer
<400> 150
   gccacatgaa tcaattccta taataaa 27
<210> 151
   <211> 25
   <212> DNA
   <213> Artificial Sequence
<220>
   <223> Description of Artificial Sequence: Synthetic primer
<400> 151
   gcacatgtac cctaaaactt aaaat 25
<210> 152
   <211> 26
   <212> DNA
   <213> Artificial Sequence
<220>
   <223> Description of Artificial Sequence: Synthetic primer
<400> 152
   gtcaaccaaa actcaacaag tagtaa 26
<210> 153
   <211> 25
   <212> DNA
   <213> Artificial Sequence
<220>
   <223> Description of Artificial Sequence: Synthetic primer
<400> 153
   aagatgaaat tgccatgtaa aaata 25
<210> 154
   <211> 27
   <212> DNA
   <213> Artificial Sequence
<220>
   <223> Description of Artificial Sequence: Synthetic primer
<400> 154
   gtgtgtataa caaaattcct atgatgg 27
<210> 155
   <211> 22
   <212> DNA
   <213> Artificial Sequence
<220>
   <223> Description of Artificial Sequence: Synthetic primer
<400> 155
   gcacccaaaa ctgaatgtca ta 22
<210> 156
   <211> 20
   <212> DNA
   <213> Artificial Sequence
<220>
   <223> Description of Artificial Sequence: Synthetic primer
<400> 156
   ggtgagagtg agaccctgtc 20
<210> 157
   <211> 27
   <212> DNA
   <213> Artificial Sequence
<220>
   <223> Description of Artificial Sequence: Synthetic primer
<400> 157
   tgtaataact ctacgactgt ctgtctg 27
<210> 158
   <211> 21
   <212> DNA
   <213> Artificial Sequence
<220>
   <223> Description of Artificial Sequence: Synthetic primer
<400> 158
   gaataggagg tggatggatg g 21
<210> 159
   <211> 20
   <212> DNA
   <213> Artificial Sequence
<220>
   <223> Description of Artificial Sequence: Synthetic primer
<400> 159
   gagcgagacc ctgtctcaag 20
<210> 160
   <211> 24
   <212> DNA
   <213> Artificial Sequence
<220>
   <223> Description of Artificial Sequence: Synthetic primer
<400> 160
   ggaaaagaca taggatagca attt 24
<210> 161
   <211> 21
   <212> DNA
   <213> Artificial Sequence
<220>
   <223> Description of Artificial Sequence: Synthetic primer
<400> 161
   tctggattga tctgtctgtc c 21
<210> 162
   <211> 26
   <212> DNA
   <213> Artificial Sequence
<220>
   <223> Description of Artificial Sequence: Synthetic primer
<400> 162
   gaattaaata ccatctgagc actgaa 26
<210> 163
   <211> 27
   <212> DNA
   <213> Artificial Sequence
<220>
   <223> Description of Artificial Sequence: Synthetic primer
<400> 163
   tgttataatg cattgagttt tattctg 27
<210> 164
   <211> 28
   <212> DNA
   <213> Artificial Sequence
<220>
   <223> Description of Artificial Sequence: Synthetic primer
<400> 164
   gcctgtctca aaaataaaga gatagaca 28
<210> 165
   <211> 25
   <212> DNA
   <213> Artificial Sequence
<220>
   <223> Description of Artificial Sequence: Synthetic primer
<400> 165
   ttaatgaatt gaacaaatga gtgag 25
<210> 166
   <211> 25
   <212> DNA
   <213> Artificial Sequence
<220>
   <223> Description of Artificial Sequence: Synthetic primer
<400> 166
   gcaactctgg ttgtattgtc ttcat 25
<210> 167
   <211> 21
   <212> DNA
   <213> Artificial Sequence
<220>
   <223> Description of Artificial Sequence: Synthetic primer
<400> 167
   caaagcgaga ctctgtctca a 21
<210> 168
   <211> 27
   <212> DNA
   <213> Artificial Sequence
<220>
   <223> Description of Artificial Sequence: Synthetic primer
<400> 168
   gaaaatgcta tcctctttgg tataaat 27
<210> 169
   <211> 27
   <212> DNA
   <213> Artificial Sequence
<220>
   <223> Description of Artificial Sequence: Synthetic primer
<400> 169
   gggtatttca agataactgt agatagg 27
<210> 170
   <211> 24
   <212> DNA
   <213> Artificial Sequence
<220>
   <223> Description of Artificial Sequence: Synthetic primer
<400> 170
   gcttctgaaa gcttctagtt tacc 24
<210> 171
   <211> 19
   <212> DNA
   <213> Artificial Sequence
<220>
   <223> Description of Artificial Sequence: Synthetic primer
<400> 171
   tccacatcct caccaacac 19
<210> 172
   <211> 21
   <212> DNA
   <213> Artificial Sequence
<220>
   <223> Description of Artificial Sequence: Synthetic primer
<400> 172
   gcctaggaag gctactgtca a 21
<210> 173
   <211> 18
   <212> DNA
   <213> Artificial Sequence
<220>
   <223> Description of Artificial Sequence: Synthetic primer
<400> 173
   ccacccgtcc atttaggc 18
<210> 174
   <211> 27
   <212> DNA
   <213> Artificial Sequence
<220>
   <223> Description of Artificial Sequence: Synthetic primer
<400> 174
   gtgaaaaagt agatataatg gttggtg 27
<210> 175
   <211> 25
   <212> DNA
   <213> Artificial Sequence
<220>
   <223> Description of Artificial Sequence: Synthetic primer
<400> 175
   ggttttccaa gagatagacc aatta 25
<210> 176
   <211> 27
   <212> DNA
   <213> Artificial Sequence
<220>
   <223> Description of Artificial Sequence: Synthetic primer
<400> 176
   gtcctctcat aaatccctac tcatatc 27
<210> 177
   <211> 27
   <212> DNA
   <213> Artificial Sequence
<220>
   <223> Description of Artificial Sequence: Synthetic primer
<400> 177
   ctgttggtac ataataggta ggtaggt 27
<210> 178
   <211> 19
   <212> DNA
   <213> Artificial Sequence
<220>
   <223> Description of Artificial Sequence: Synthetic primer
<400> 178
   gtcgtgggcc ccataaatc 19
<210> 179
   <211> 27
   <212> DNA
   <213> Artificial Sequence
<220>
   <223> Description of Artificial Sequence: Synthetic primer
<400> 179
   aaggtacata acagttcaat agaaagc 27
<210> 180
   <211> 26
   <212> DNA
   <213> Artificial Sequence
<220>
   <223> Description of Artificial Sequence: Synthetic primer
<400> 180
   gtgaaatgac tgaaaaatag taacca 26
<210> 181
   <211> 24
   <212> DNA
   <213> Artificial Sequence
<220>
   <223> Description of Artificial Sequence: Synthetic primer
<400> 181
   ctaggagatc atgtgggtat gatt 24
<210> 182
   <211> 24
   <212> DNA
   <213> Artificial Sequence
<220>
   <223> Description of Artificial Sequence: Synthetic primer
<400> 182
   gcagtgaata aatgaacgaa tgga 24
<210> 183
   <211> 21
   <212> DNA
   <213> Artificial Sequence
<220>
   <223> Description of Artificial Sequence: Synthetic primer
<400> 183
   cccaaaatta cttgagccaa t 21
<210> 184
   <211> 24
   <212> DNA
   <213> Artificial Sequence
<220>
   <223> Description of Artificial Sequence: Synthetic primer
<400> 184
   gagacaaaat gaagaaacag acag 24
<210> 185
   <211> 25
   <212> DNA
   <213> Artificial Sequence
<220>
   <223> Description of Artificial Sequence: Synthetic primer
<400> 185
   tctttgctct catgaataga tcagt 25
<210> 186
   <211> 24
   <212> DNA
   <213> Artificial Sequence
<220>
   <223> Description of Artificial Sequence: Synthetic primer
<400> 186
   gtttgtgata atgaacccac tcag 24
<210> 187
   <211> 27
   <212> DNA
   <213> Artificial Sequence
<220>
   <223> Description of Artificial Sequence: Synthetic primer
<400> 187
   tgaacacaga tgttaagtgt gtatatg 27
<210> 188
   <211> 23
   <212> DNA
   <213> Artificial Sequence
<220>
   <223> Description of Artificial Sequence: Synthetic primer
<400> 188
   gtctgaggtg gacagttatg aaa 23
<210> 189
   <211> 23
   <212> DNA
   <213> Artificial Sequence
<220>
   <223> Description of Artificial Sequence: Synthetic primer
<400> 189
   ctgtggctca tctatgaaaa ctt 23
<210> 190
   <211> 22
   <212> DNA
   <213> Artificial Sequence
<220>
   <223> Description of Artificial Sequence: Synthetic primer
<400> 190
   gaagtggctg tggtgttatg at 22
<210> 191
   <211> 24
   <212> DNA
   <213> Artificial Sequence
<220>
   <223> Description of Artificial Sequence: Synthetic primer
<400> 191
   ttctgttggt atagagcagt gttt 24
<210> 192
   <211> 20
   <212> DNA
   <213> Artificial Sequence
<220>
   <223> Description of Artificial Sequence: Synthetic primer
<400> 192
   gtgacaggaa ggacggaatg 20
<210> 193
   <211> 27
   <212> DNA
   <213> Artificial Sequence
<220>
   <223> Description of Artificial Sequence: Synthetic primer
<400> 193
   catgaggttt gcaaatacta tcttaac 27
<210> 194
   <211> 24
   <212> DNA
   <213> Artificial Sequence
<220>
   <223> Description of Artificial Sequence: Synthetic primer
<400> 194
   gttttaattt tctccaaatc tcca 24
<210> 195
   <211> 27
   <212> DNA
   <213> Artificial Sequence
<220>
   <223> Description of Artificial Sequence: Synthetic primer
<400> 195
   tcttagccta gatagatact tgcttcc 27
<210> 196
   <211> 23
   <212> DNA
   <213> Artificial Sequence
<220>
   <223> Description of Artificial Sequence: Synthetic primer
<400> 196
   gtcaaccttt gaggctatag gaa 23
<210> 197
   <211> 19
   <212> DNA
   <213> Artificial Sequence
<220>
   <223> Description of Artificial Sequence: Synthetic primer
<400> 197
   tcctggaaac aaaagtatt 19
<210> 198
   <211> 21
   <212> DNA
   <213> Artificial Sequence
<220>
   <223> Description of Artificial Sequence: Synthetic primer
<400> 198
   aaccttacaa caaagctaga a 21
<210> 199
   <211> 20
   <212> DNA
   <213> Artificial Sequence
<220>
   <223> Description of Artificial Sequence: Synthetic primer
<400> 199
   actaagcctt ggggatccag 20
<210> 200
   <211> 21
   <212> DNA
   <213> Artificial Sequence
<220>
   <223> Description of Artificial Sequence: Synthetic primer
<400> 200
   tgctgtggaa atactaaaag g 21
<210> 201
   <211> 20
   <212> DNA
   <213> Artificial Sequence
<220>
   <223> Description of Artificial Sequence: Synthetic primer
<400> 201
   ctccagaggt aatcctgtga 20
<210> 202
   <211> 21
   <212> DNA
   <213> Artificial Sequence
<220>
   <223> Description of Artificial Sequence: Synthetic primer
<400> 202
   tggtgtgaga tggtatctag g 21
<210> 203
   <211> 22
   <212> DNA
   <213> Artificial Sequence
<220>
   <223> Description of Artificial Sequence: Synthetic primer
<400> 203
   gtataatcca tgaatcttgt tt 22
<210> 204
   <211> 22
   <212> DNA
   <213> Artificial Sequence
<220>
   <223> Description of Artificial Sequence: Synthetic primer
<400> 204
   ttcaaattgt atataagaga gt 22
<210> 205
   <211> 20
   <212> DNA
   <213> Artificial Sequence
<220>
   <223> Description of Artificial Sequence: Synthetic primer
<400> 205
   gcaggaaagt tatttttaat 20
<210> 206
   <211> 21
   <212> DNA
   <213> Artificial Sequence
<220>
   <223> Description of Artificial Sequence: Synthetic primer
<400> 206
   tgcttgagaa agctaacact t 21
<210> 207
   <211> 20
   <212> DNA
   <213> Artificial Sequence
<220>
   <223> Description of Artificial Sequence: Synthetic primer
<400> 207
   cagtgtttgg aaattgtctg 20
<210> 208
   <211> 20
   <212> DNA
   <213> Artificial Sequence
<220>
   <223> Description of Artificial Sequence: Synthetic primer
<400> 208
   ggcactggga gattattgta 20
<210> 209
   <211> 20
   <212> DNA
   <213> Artificial Sequence
<220>
   <223> Description of Artificial Sequence: Synthetic primer
<400> 209
   tcctgttgtt aagtacacat 20
<210> 210
   <211> 18
   <212> DNA
   <213> Artificial Sequence
<220>
   <223> Description of Artificial Sequence: Synthetic primer
<400> 210
   gggccgtaat tacttttg 18
<210> 211
   <211> 21
   <212> DNA
   <213> Artificial Sequence
<220>
   <223> Description of Artificial Sequence: Synthetic primer
<400> 211
   actcagtagg cactttgtgt c 21
<210> 212
   <211> 18
   <212> DNA
   <213> Artificial Sequence
<220>
   <223> Description of Artificial Sequence: Synthetic primer
<400> 212
   tcttccacca caccaatc 18
<210> 213
   <211> 19
   <212> DNA
   <213> Artificial Sequence
<220>
   <223> Description of Artificial Sequence: Synthetic primer
<400> 213
   tggcttttca aaggtaaaa 19
<210> 214
   <211> 21
   <212> DNA
   <213> Artificial Sequence
<220>
   <223> Description of Artificial Sequence: Synthetic primer
<400> 214
   gcaacgttaa catctgaatt t 21
<210> 215
<400> 215
   000
<210> 216
   <211> 22
   <212> DNA
   <213> Artificial Sequence
<220>
   <223> Description of Artificial Sequence: Synthetic primer
<400> 216
   attttatatg tcatgatcta ag 22
<210> 217
   <211> 19
   <212> DNA
   <213> Artificial Sequence
<220>
   <223> Description of Artificial Sequence: Synthetic primer
<400> 217
   agagattaca ggtgtgagc 19
<210> 218
   <211> 19
   <212> DNA
   <213> Artificial Sequence
<220>
   <223> Description of Artificial Sequence: Synthetic primer
<400> 218
   atgatcctca actgcctct 19
<210> 219
   <211> 20
   <212> DNA
   <213> Artificial Sequence
<220>
   <223> Description of Artificial Sequence: Synthetic primer
<400> 219
   tgaaactcaa aagagaaaag 20
<210> 220
   <211> 20
   <212> DNA
   <213> Artificial Sequence
<220>
   <223> Description of Artificial Sequence: Synthetic primer
<400> 220
   acagatttct acttaaaatt 20
<210> 221
   <211> 20
   <212> DNA
   <213> Artificial Sequence
<220>
   <223> Description of Artificial Sequence: Synthetic primer
<400> 221
   tgaaactcaa aagagaaaag 20
<210> 222
   <211> 20
   <212> DNA
   <213> Artificial Sequence
<220>
   <223> Description of Artificial Sequence: Synthetic primer
<400> 222
   acagatttct acttaaaatt 20
<210> 223
   <211> 20
   <212> DNA
   <213> Artificial Sequence
<220>
   <223> Description of Artificial Sequence: Synthetic primer
<400> 223
   gcaaaggggt actctatgta 20
<210> 224
   <211> 20
   <212> DNA
   <213> Artificial Sequence
<220>
   <223> Description of Artificial Sequence: Synthetic primer
<400> 224
   tatcgggtca tcttgttaaa 20
<210> 225
   <211> 22
   <212> DNA
   <213> Artificial Sequence
<220>
   <223> Description of Artificial Sequence: Synthetic primer
<400> 225
   tctaacaaag ctctgtccaa aa 22
<210> 226
   <211> 21
   <212> DNA
   <213> Artificial Sequence
<220>
   <223> Description of Artificial Sequence: Synthetic primer
<400> 226
   ccacactgaa taactggaac a 21
<210> 227
   <211> 21
   <212> DNA
   <213> Artificial Sequence
<220>
   <223> Description of Artificial Sequence: Synthetic primer
<400> 227
   gcaagcaagc tctctacctt c 21
<210> 228
   <211> 22
   <212> DNA
   <213> Artificial Sequence
<220>
   <223> Description of Artificial Sequence: Synthetic primer
<400> 228
   tgttcttcca aaattcacat gc 22
<210> 229
   <211> 21
   <212> DNA
   <213> Artificial Sequence
<220>
   <223> Description of Artificial Sequence: Synthetic primer
<400> 229
   atttcactat tccttcattt t 21
<210> 230
   <211> 22
   <212> DNA
   <213> Artificial Sequence
<220>
   <223> Description of Artificial Sequence: Synthetic primer
<400> 230
   taattgttgc acactaaatt ac 22
<210> 231
   <211> 21
   <212> DNA
   <213> Artificial Sequence
<220>
   <223> Description of Artificial Sequence: Synthetic primer
<400> 231
   aaaaagccac agaaatcagt c 21
<210> 232
   <211> 22
   <212> DNA
   <213> Artificial Sequence
<220>
   <223> Description of Artificial Sequence: Synthetic primer
<400> 232
   ttcttatatc tcactgggca tt 22
<210> 233
   <211> 22
   <212> DNA
   <213> Artificial Sequence
<220>
   <223> Description of Artificial Sequence: Synthetic primer
<400> 233
   ggatggtaga agagaagaaa gg 22
<210> 234
   <211> 22
   <212> DNA
   <213> Artificial Sequence
<220>
   <223> Description of Artificial Sequence: Synthetic primer
<400> 234
   ggatggtaga agagaagaaa gg 22
<210> 235
   <211> 20
   <212> DNA
   <213> Artificial Sequence
<220>
   <223> Description of Artificial Sequence: Synthetic primer
<400> 235
   tgcaaagatg cagaaccaac 20
<210> 236
   <211> 22
   <212> DNA
   <213> Artificial Sequence
<220>
   <223> Description of Artificial Sequence: Synthetic primer
<400> 236
   ttttgttcct tgtcctggct ga 22
<210> 237
   <211> 20
   <212> DNA
   <213> Artificial Sequence
<220>
   <223> Description of Artificial Sequence: Synthetic primer
<400> 237
   tgcaaagatg cagaaccaac 20
<210> 238
   <211> 21
   <212> DNA
   <213> Artificial Sequence
<220>
   <223> Description of Artificial Sequence: Synthetic primer
<400> 238
   gcctccagct ctatccaagt t 21
<210> 239
   <211> 22
   <212> DNA
   <213> Artificial Sequence
<220>
   <223> Description of Artificial Sequence: Synthetic primer
<400> 239
   ccttaatatc ttcccatgtc ca 22
<210> 240
   <211> 22
   <212> DNA
   <213> Artificial Sequence
<220>
   <223> Description of Artificial Sequence: Synthetic primer
<400> 240
   attgttagtg cctcttctgc tt 22
<210> 241
   <211> 20
   <212> DNA
   <213> Artificial Sequence
<220>
   <223> Description of Artificial Sequence: Synthetic primer
<400> 241
   gagaagtgag gtcagcagct 20
<210> 242
   <211> 22
   <212> DNA
   <213> Artificial Sequence
<220>
   <223> Description of Artificial Sequence: Synthetic primer
<400> 242
   tttctaaatt tccattgaac ag 22
<210> 243
   <211> 20
   <212> DNA
   <213> Artificial Sequence
<220>
   <223> Description of Artificial Sequence: Synthetic primer
<400> 243
   gaaattggca atctgattct 20
<210> 244
   <211> 21
   <212> DNA
   <213> Artificial Sequence
<220>
   <223> Description of Artificial Sequence: Synthetic primer
<400> 244
   caacttgtcc tttattgatg t 21
<210> 245
   <211> 22
   <212> DNA
   <213> Artificial Sequence
<220>
   <223> Description of Artificial Sequence: Synthetic primer
<400> 245
   ctatgttgat aaaacattga aa 22
<210> 246
   <211> 20
   <212> DNA
   <213> Artificial Sequence
<220>
   <223> Description of Artificial Sequence: Synthetic primer
<400> 246
   gcctgtctgg aatatagttt 20
<210> 247
   <211> 22
   <212> DNA
   <213> Artificial Sequence
<220>
   <223> Description of Artificial Sequence: Synthetic primer
<400> 247
   cagggcatat aatctaagct gt 22
<210> 248
   <211> 21
   <212> DNA
   <213> Artificial Sequence
<220>
   <223> Description of Artificial Sequence: Synthetic primer
<400> 248
   caatgactct gagttgagca c 21
<210> 249
   <211> 18
   <212> DNA
   <213> Artificial Sequence
<220>
   <223> Description of Artificial Sequence: Synthetic primer
<400> 249
   actctctccc tcccctct 18
<210> 250
   <211> 20
   <212> DNA
   <213> Artificial Sequence
<220>
   <223> Description of Artificial Sequence: Synthetic primer
<400> 250
   tatggcccca aaactattct 20
<210> 251
   <211> 20
   <212> DNA
   <213> Artificial Sequence
<220>
   <223> Description of Artificial Sequence: Synthetic primer
<400> 251
   acaagtactg ggcagattga 20
<210> 252
   <211> 20
   <212> DNA
   <213> Artificial Sequence
<220>
   <223> Description of Artificial Sequence: Synthetic primer
<400> 252
   gccaggttta gctttcaagt 20
<210> 253
   <211> 22
   <212> DNA
   <213> Artificial Sequence
<220>
   <223> Description of Artificial Sequence: Synthetic primer
<400> 253
   ttttatatca ggagaaacac tg 22
<210> 254
   <211> 21
   <212> DNA
   <213> Artificial Sequence
<220>
   <223> Description of Artificial Sequence: Synthetic primer
<400> 254
   ccagaatttt ggaggtttaa t 21
<210> 255
   <211> 22
   <212> DNA
   <213> Artificial Sequence
<220>
   <223> Description of Artificial Sequence: Synthetic primer
<400> 255
   tgtcattcct cctttatctc ca 22
<210> 256
   <211> 20
   <212> DNA
   <213> Artificial Sequence
<220>
   <223> Description of Artificial Sequence: Synthetic primer
<400> 256
   ttcttttgcc tctcccaaag 20
<210> 257
   <211> 20
   <212> DNA
   <213> Artificial Sequence
<220>
   <223> Description of Artificial Sequence: Synthetic primer
<400> 257
   accctggcac agtgttgact 20
<210> 258
   <211> 20
   <212> DNA
   <213> Artificial Sequence
<220>
   <223> Description of Artificial Sequence: Synthetic primer
<400> 258
   tgggcctgag ttgagaagat 20
<210> 259
   <211> 21
   <212> DNA
   <213> Artificial Sequence
<220>
   <223> Description of Artificial Sequence: Synthetic primer
<400> 259
   aatttgtaag tatgtgcaac g 21
<210> 260
   <211> 20
   <212> DNA
   <213> Artificial Sequence
<220>
   <223> Description of Artificial Sequence: Synthetic primer
<400> 260
   tttttcccat ttccaactct 20
<210> 261
   <211> 20
   <212> DNA
   <213> Artificial Sequence
<220>
   <223> Description of Artificial Sequence: Synthetic primer
<400> 261
   aaaagatgag acaggcaggt 20
<210> 262
   <211> 20
   <212> DNA
   <213> Artificial Sequence
<220>
   <223> Description of Artificial Sequence: Synthetic primer
<400> 262
   acccctgtga atctcaaaat 20
<210> 263
   <211> 21
   <212> DNA
   <213> Artificial Sequence
<220>
   <223> Description of Artificial Sequence: Synthetic primer
<400> 263
   gcacttgctt ctattgtttg t 21
<210> 264
   <211> 20
   <212> DNA
   <213> Artificial Sequence
<220>
   <223> Description of Artificial Sequence: Synthetic primer
<400> 264
   cccttcctct cttccattct 20
<210> 265
   <211> 13
   <212> DNA
   <213> Artificial Sequence
<220>
   <223> Description of Artificial Sequence: Synthetic primer
<400> 265
   agcactgcag gta 13
<210> 266
   <211> 21
   <212> DNA
   <213> Artificial Sequence
<220>
   <223> Description of Artificial Sequence: Synthetic primer
<400> 266
   acagatacca aagaactgca a 21
<210> 267
   <211> 20
   <212> DNA
   <213> Artificial Sequence
<220>
   <223> Description of Artificial Sequence: Synthetic primer
<400> 267
   tggacacctt tcaacttaga 20
<210> 268
   <211> 22
   <212> DNA
   <213> Artificial Sequence
<220>
   <223> Description of Artificial Sequence: Synthetic primer
<400> 268
   gaacagtaat gttgaacttt tt 22
<210> 269
   <211> 21
   <212> DNA
   <213> Artificial Sequence
<220>
   <223> Description of Artificial Sequence: Synthetic primer
<400> 269
   tcttgcaaaa agcttagcac a 21
<210> 270
   <211> 21
   <212> DNA
   <213> Artificial Sequence
<220>
   <223> Description of Artificial Sequence: Synthetic primer
<400> 270
   aaaaagatct caaagggtcc a 21
<210> 271
   <211> 20
   <212> DNA
   <213> Artificial Sequence
<220>
   <223> Description of Artificial Sequence: Synthetic primer
<400> 271
   gcttttgctg aacatcaagt 20
<210> 272
   <211> 19
   <212> DNA
   <213> Artificial Sequence
<220>
   <223> Description of Artificial Sequence: Synthetic primer
<400> 272
   ccttccagca gcatagtct 19
<210> 273
   <211> 18
   <212> DNA
   <213> Artificial Sequence
<220>
   <223> Description of Artificial Sequence: Synthetic primer
<400> 273
   aaatccagga tgtgcagt 18
<210> 274
   <211> 19
   <212> DNA
   <213> Artificial Sequence
<220>
   <223> Description of Artificial Sequence: Synthetic primer
<400> 274
   atgatgaggt cagtggtgt 19
<210> 275
   <211> 22
   <212> DNA
   <213> Artificial Sequence
<220>
   <223> Description of Artificial Sequence: Synthetic primer
<400> 275
   catcacagat catagtaaat gg 22
<210> 276
   <211> 21
   <212> DNA
   <213> Artificial Sequence
<220>
   <223> Description of Artificial Sequence: Synthetic primer
<400> 276
   aattattatt ttgcaggcaa t 21
<210> 277
   <211> 20
   <212> DNA
   <213> Artificial Sequence
<220>
   <223> Description of Artificial Sequence: Synthetic primer
<400> 277
   catgaggcaa acacctttcc 20
<210> 278
   <211> 22
   <212> DNA
   <213> Artificial Sequence
<220>
   <223> Description of Artificial Sequence: Synthetic primer
<400> 278
   gctggactca ggataaagaa ca 22
<210> 279
   <211> 20
   <212> DNA
   <213> Artificial Sequence
<220>
   <223> Description of Artificial Sequence: Synthetic primer
<400> 279
   tggaagcctg agctgactaa 20
<210> 280
   <211> 20
   <212> DNA
   <213> Artificial Sequence
<220>
   <223> Description of Artificial Sequence: Synthetic primer
<400> 280
   ccttcttttc ccccagaatc 20
<210> 281
   <211> 21
   <212> DNA
   <213> Artificial Sequence
<220>
   <223> Description of Artificial Sequence: Synthetic primer
<400> 281
   taggagaaca gaagatcaga g 21
<210> 282
   <211> 22
   <212> DNA
   <213> Artificial Sequence
<220>
   <223> Description of Artificial Sequence: Synthetic primer
<400> 282
   aaagactatt gctaaatgct tg 22
<210> 283
   <211> 20
   <212> DNA
   <213> Artificial Sequence
<220>
   <223> Description of Artificial Sequence: Synthetic primer
<400> 283
   taagcgtagg gctgtgtgtg 20
<210> 284
   <211> 21
   <212> DNA
   <213> Artificial Sequence
<220>
   <223> Description of Artificial Sequence: Synthetic primer
<400> 284
   ggacggatag actccagaag g 21
<210> 285
   <211> 23
   <212> DNA
   <213> Artificial Sequence
<220>
   <223> Description of Artificial Sequence: Synthetic primer
<400> 285
   gaatgacctt ggcactttta tca 23
<210> 286
   <211> 27
   <212> DNA
   <213> Artificial Sequence
<220>
   <223> Description of Artificial Sequence: Synthetic primer
<400> 286
   aaggatagag atatacagat gaatgga 27
<210> 287
   <211> 18
   <212> DNA
   <213> Artificial Sequence
<220>
   <223> Description of Artificial Sequence: Synthetic primer
<400> 287
   catgcaccgc gcaaatac 18
<210> 288
   <211> 19
   <212> DNA
   <213> Artificial Sequence
<220>
   <223> Description of Artificial Sequence: Synthetic primer
<400> 288
   atgcctcacc cacaaacac 19
<210> 289
   <211> 20
   <212> DNA
   <213> Artificial Sequence
<220>
   <223> Description of Artificial Sequence: Synthetic primer
<400> 289
   tccaagccct tctcactcac 20
<210> 290
   <211> 20
   <212> DNA
   <213> Artificial Sequence
<220>
   <223> Description of Artificial Sequence: Synthetic primer
<400> 290
   ctgggacggt gacattttct 20
<210> 291
   <211> 21
   <212> DNA
   <213> Artificial Sequence
<220>
   <223> Description of Artificial Sequence: Synthetic primer
<400> 291
   cccaggaaga gtggaaagat t 21
<210> 292
   <211> 21
   <212> DNA
   <213> Artificial Sequence
<220>
   <223> Description of Artificial Sequence: Synthetic primer
<400> 292
   ttagcttgca tgtacctgtg t 21
<210> 293
   <211> 20
   <212> DNA
   <213> Artificial Sequence
<220>
   <223> Description of Artificial Sequence: Synthetic primer
<400> 293
   agctagatgg ggtgaatttt 20
<210> 294
   <211> 18
   <212> DNA
   <213> Artificial Sequence
<220>
   <223> Description of Artificial Sequence: Synthetic primer
<400> 294
   tgggctgagg ggagattc 18
<210> 295
   <211> 22
   <212> DNA
   <213> Artificial Sequence
<220>
   <223> Description of Artificial Sequence: Synthetic primer
<400> 295
   atcaagctaa ttaatgttat ct 22
<210> 296
   <211> 20
   <212> DNA
   <213> Artificial Sequence
<220>
   <223> Description of Artificial Sequence: Synthetic primer
<400> 296
   aatgaataag gtcctcagag 20
<210> 297
   <211> 21
   <212> DNA
   <213> Artificial Sequence
<220>
   <223> Description of Artificial Sequence: Synthetic primer
<400> 297
   tttaatctga tcattgccct a 21
<210> 298
   <211> 18
   <212> DNA
   <213> Artificial Sequence
<220>
   <223> Description of Artificial Sequence: Synthetic primer
<400> 298
   agctgtgggt gaccttga 18
<210> 299
   <211> 20
   <212> DNA
   <213> Artificial Sequence
<220>
   <223> Description of Artificial Sequence: Synthetic primer
<400> 299
   tgtcccacca ttgtgtatta 20
<210> 300
   <211> 20
   <212> DNA
   <213> Artificial Sequence
<220>
   <223> Description of Artificial Sequence: Synthetic primer
<400> 300
   tcagacttga agtccaggat 20
<210> 301
   <211> 20
   <212> DNA
   <213> Artificial Sequence
<220>
   <223> Description of Artificial Sequence: Synthetic primer
<400> 301
   gcttcagggg tgttagtttt 20
<210> 302
   <211> 20
   <212> DNA
   <213> Artificial Sequence
<220>
   <223> Description of Artificial Sequence: Synthetic primer
<400> 302
   ctttgtgaaa agtcgtccag 20
<210> 303
   <211> 18
   <212> DNA
   <213> Artificial Sequence
<220>
   <223> Description of Artificial Sequence: Synthetic primer
<400> 303
   ccatcatgga aagcatgg 18
<210> 304
   <211> 20
   <212> DNA
   <213> Artificial Sequence
<220>
   <223> Description of Artificial Sequence: Synthetic primer
<400> 304
   tcatctccat gactgcacta 20
<210> 305
   <211> 20
   <212> DNA
   <213> Artificial Sequence
<220>
   <223> Description of Artificial Sequence: Synthetic primer
<400> 305
   gagatgacgg agtagctcat 20
<210> 306
   <211> 18
   <212> DNA
   <213> Artificial Sequence
<220>
   <223> Description of Artificial Sequence: Synthetic primer
<400> 306
   cccagctgca ctgtctac 18
<210> 307
   <211> 20
   <212> DNA
   <213> Artificial Sequence
<220>
   <223> Description of Artificial Sequence: Synthetic primer
<400> 307
   tcttgttcca atcacaggac 20
<210> 308
   <211> 20
   <212> DNA
   <213> Artificial Sequence
<220>
   <223> Description of Artificial Sequence: Synthetic primer
<400> 308
   atgctgttag ctgaagctct 20
<210> 309
   <211> 20
   <212> DNA
   <213> Artificial Sequence
<220>
   <223> Description of Artificial Sequence: Synthetic primer
<400> 309
   tgaaagctcc taaagcagag 20
<210> 310
   <211> 20
   <212> DNA
   <213> Artificial Sequence
<220>
   <223> Description of Artificial Sequence: Synthetic primer
<400> 310
   ttgaagagat gtgctatcat 20
<210> 311
   <211> 54
   <212> DNA
   <213> Artificial Sequence
<220>
   <223> Description of Artificial Sequence: Synthetic primer
<400> 311
   gccgcctgca gcccgcgccc cccgtgcccc cgccccgccg ccggcccggg cgcc 54
<210> 312
   <211> 601
   <212> DNA
   <213> Homo sapiens
<400> 312
   catagtgaca ggtatatgcc caactaactg tggaaaacag ttctttcttt caaccttact       60
   catcaccctc acggtctgtt tatgaggctc tcctccacca gccagaaagg atgacgtgcc      120
   atacctgcaa aacttataca gcatcaacag aatgaatctt tccaacaagc cgaaacattg      180
   agtattgtgg cacagaatat gccccaccca ttactcaatc tagatatcct tttattccac      240
   cgtctcatga ttttcttttt cctggaaaac aaaagtattt ctttcatagc ccagctagca      300
   ygataaatca gcgagtcaga attctagctt tgttgtaagg ttttgcgaat atctgatcct      360
   cttattttgt acttttctat ttcctaggca aatctgagta tttcacccag ttttccttaa      420
   ctaggcattg aaaactcagt ttttttctta caaaccttca tgtcttcctg ctcatttgca      480
   cagtcttatc ttgcacctcc tataaaatgg agaaacttga cattaaaacg taatttttat      540
   tacattttga gggattccca gagaattttt ccccaatctc cttaggtagg gacttcttta 600
   c 601
<210> 313
   <211> 601
   <212> DNA
   <213> Homo sapiens
<400> 313
   gtgggaacta tagtaaagaa gtccctacct aaggagattg gggaaaaatt ctctgggaat 60
   ccctcaaaat gtaataaaaa ttacgtttta atgtcaagtt tctccatttt ataggaggtg 120
   caagataaga ctgtgcaaat gagcaggaag acatgaaggt ttgtaagaaa aaaactgagt 180
   tttcaatgcc tagttaagga aaactgggtg aaatactcag atttgcctag gaaatagaaa 240
   agtacaaaat aagaggatca gatattcgca aaaccttaca acaaagctag aattctgact 300
   ygctgattta tcgtgctagc tgggctatga aagaaatact tttgttttcc aggaaaaaga 360
   aaatcatgag acggtggaat aaaaggatat ctagattgag taatgggtgg ggcatattct 420
   gtgccacaat actcaatgtt tcggcttgtt ggaaagattc attctgttga tgctgtataa 480
   gttttgcagg tatggcacgt catcctttct ggctggtgga ggagagcctc ataaacagac 540
   cgtgagggtg atgagtaagg ttgaaagaaa gaactgtttt ccacagttag ttgggcatat 600
   a 601
<210> 314
   <211> 650
   <212> DNA
   <213> Homo sapiens
<400> 314
   tttattggtc ctgactggta caaatactga taaaaaggat tttaagatca tattcatact        60
   tttggggaat gagagccaca attaattaac aatgtctgcc atgagattgg atgcaagagt       120
   atggcactca tactattcct acttctgtct aattacacta tttgtttctg tgtgcaaaaa       180
   tctttggtag gtggtggatg tgcccaagac acagggaaga aaaagaagta aacagggaag       240
   tacaacacag actctgaaat ggggcatcat ggaagacgga gctttgtcgt cttggtcttt       300
   gctgtatatt cacttcctac aacagtgcta aataccttgt ggatgcttaa atatattaaa       360
   tgaatgcata aatgaaaaga gtaaataaag agtgtatatg aaagtatgta gataaaattc       420
   ttcactaagc cttggggatc cagctgcttm aggactaaga ccgtatctag ctccttttag       480
   tatttccaca gcatgccatg gagatacatg tttctgatta tatatgatac atggaaatta       540
   tatgttgttg aatgagtgat tgagtaaatg tgtactaggg cagctaatca taaatatttc       600
   tactattgct aaaatgactg gatttatcca ttccttctga gagtttatac                  650
<210> 315
   <211> 626
   <212> DNA
   <213> Homo sapiens
<400> 315
   ctgcttaagg actaagaccr tatctagctc cttttagtat ttccacagca tgccatggag        60
   atacatgttt ctgattatat atgatacatg gaaattatat gttgttgaat gagtgattga       120
   gtaaatgtgt actagggcag ctaatcataa atatttctac tattgctaaa atgactggat       180
   ttatccattc cttctgagag tttatactga ttgcttatat tgtatcaaat accgtaactg       240
   agggcaatgt ttactcaaac taatagcacc attcaaattt atgcaaacaa taacactata       300
   tctttaaaat gttttcacta aaagctgcat aaagagtgta ttcaacaaca atagaataat       360
   tttacaatct tttttcttgc ttaatggcca tttgtgcctt ctgacatgct gctagccatt       420
   caaaggtcac actaccttga agttgaagat caagacaaat gattagactc ataaaagaca       480
   aatcacgtct ttctggacag gtgattatta ataattaatt agcatttaaa catgtattat       540
   ttaagttctt tttaagttat aaagtctttg atttgctaaa cagtttaaat aatgaataaa       600
   acataaaata ataatagtta ccattt                                            626
<210> 316
   <211> 1113
   <212> DNA
   <213> Homo sapiens
<400> 316
   caagagctgc atctcactcc aatttttctt ctccctataa ccttatctag attcccagtt 60
   gagggaaccg atgacctaat tcctctcagt ttaaatgcaa cacaggagca aattccaaat 120
   atctatgctg gtcttgctgg gattgcagaa ccccagggtg gttatcctcc tccagaggta 180
   atcctgtgat cagcactaac rccacatacc agccctttca tcagcttgtt ggagaagcat 240
   ctttacttcc caccaagcag tgacctagat accatctcac accagttaga atcaggatca 300
   ttaaaaagtc aagaaaaaac agatgctgaa gaggatgtgg agaaatagga atgcttttac 360
   actgttagtg ggaatgtaaa ttagttcaac cattgtcaaa gacagtgtgg cgatccctca 420
   cagatctaga accagaaata ccatttgacc cagcaatccc attactgggt ctatacccaa 480
   aggattataa attactctac tataaagaca catgcacaca tatgtttatt gcagcaccat 540
   tcacaatagc aaagaattgc aaccaaccct aatgcccatc aatgacagac tggataaaga 600
   aaatctggca catatacacc atggaatact acgcagccat aaaaaaggat gagtttatgt 660
   cctttacagg gacatggatg aagctggaaa ccatcattct cagcaaacta acacaggaac 720
   agaaaaccaa acacatgttc tcactcacaa gtgggagttg aacaatgaga acacatggac 780
   acagggaggg gaacatcaca caccactgct tgtcaggggg tggggggcta ggggaaggat 840
   agcattagga gaaataccta atgtagatga agggttgatg ggtgcagcaa accaccatgg 900
   catgtgtata cctgtgtaac aaacctccat gttctgcacg tgtatcccag aacttaaagt 960
   acaatacaaa aaaaaaaaaa agtgtaatcc agtttacatt ttcaaggtca aagtgggtac 1020
   aatgctatct atcttgggct aagaagagaa aaggaaaaat tcttgcttta aatcttagaa 1080
   gtctggtttt tttccctgtt ttgtacccca tcc 1113
<210> 317
   <211> 1113
   <212> DNA
   <213> Homo sapiens
<400> 317
   ttcccagttg agggaaccga tgacctaatt cctctcagtt taaatgcaac acaggagcaa 60
   attccaaata tctatgctgg tcttgctggg attgcagaac cccagggtgg ttatcctcct 120
   ccagaggtaa tcctgtgatc agcactaacg ccacatacca gccctttcat cagcttgttg 180
   gagaagcatc tttacttccc rccaagcagt gacctagata ccatctcaca ccagttagaa 240
   tcaggatcat taaaaagtca agaaaaaaca gatgctgaag aggatgtgga gaaataggaa 300
   tgcttttaca ctgttagtgg gaatgtaaat tagttcaacc attgtcaaag acagtgtggc 360
   gatccctcac agatctagaa ccagaaatac catttgaccc agcaatccca ttactgggtc 420
   tatacccaaa ggattataaa ttactctact ataaagacac atgcacacat atgtttattg 480
   cagcaccatt cacaatagca aagaattgca accaacccta atgcccatca atgacagact 540
   ggataaagaa aatctggcac atatacacca tggaatacta cgcagccata aaaaaggatg 600
   agtttatgtc ctttacaggg acatggatga agctggaaac catcattctc agcaaactaa 660
   cacaggaaca gaaaaccaaa cacatgttct cactcacaag tgggagttga acaatgagaa 720
   cacatggaca cagggagggg aacatcacac accactgctt gtcagggggt ggggggctag 780
   gggaaggata gcattaggag aaatacctaa tgtagatgaa gggttgatgg gtgcagcaaa 840
   ccaccatggc atgtgtatac ctgtgtaaca aacctccatg ttctgcacgt gtatcccaga 900
   acttaaagta caatacaaaa aaaaaaaaaa gtgtaatcca gtttacattt tcaaggtcaa 960
   agtgggtaca atgctatcta tcttgggcta agaagagaaa aggaaaaatt cttgctttaa 1020
   atcttagaag tctggttttt ttccctgttt tgtaccccat cctcttggtc tctctagata 1080
   tatttaagac tcacatagga cttgtctttt cta 1113
<210> 318
   <211> 1001
   <212> DNA
   <213> Homo sapiens
<400> 318
   tcatcaacta aatagttgat gaggggaaat tgttctgtat atgttcatac ttcagctaat 60
   caattaaaaa tgatgaaata ataagattac cattttgcaa acccctaatg caatgttgga 120
   tccaggcaat gatcatcaat ggccactaaa atcacacaaa aggagataac cagaatatgt 180
   gctttgtgat ggaagcatta aatacaacta atgagatatt gtttataaga aagaaaggaa 240
   gcaagaaagc aatcacacca agctctgtat ctagctacca catttaagga aaaaaagaga 300
   cagaagagca tgttaaatgt taccaagaag atacagtcag tcggaaaaaa tacagacaag 360
   aaaatacaga gcaaaacaac ccagcttctt cagcaaatca atataaaaaa attttaagaa 420
   agagttaaag tataaactga gagacttcag aaacatatta tccaagtata atccatgaat 480
   cttgtttaaa tatagatcaa rtaaaccact ataccaaaaa catcaaaaga caactgggta 540
   aattttttaa atgactagct atttgatgtt aaggaagtaa tgttactctc ttatatacaa 600
   tttgaaataa tctagcgagg agcagcaaat gtgcggctat gaggaagaaa cacaattggc 660
   cattcttgaa tcattagctg gatggtggct atatgggggt agattttact actctctaat 720
   tttacatata tttaaaatgt tccataataa attgttgagt tatcaaaaga aatatttcta 780
   tataatagct aaaattattt ataaaagtta gtggtctcat aactttattt atttatttac 840
   ttattttgag accgagtctc cctctgttat gcaggctgga gtgcagtggc tccatctcgg 900
   ctcactgcaa acttcacctc ctggattgaa gcgattctcc tgcctcagcc cccccgagta 960
   gctgggatta caggcttgca cccccacgcc cagctaattt t 1001
<210> 319
   <211> 601
   <212> DNA
   <213> Homo sapiens
<400> 319
   agctaccaca tttaaggaaa aaaagagaca gaagagcatg ttaaatgtta ccaagaagat 60
   acagtcagtc ggaaaaaata cagacaagaa aatacagagc aaaacaaccc agcttcttca 120
   gcaaatcaat ataaaaaaat tttaagaaag agttaaagta taaactgaga gacttcagaa 180
   acatattatc caagtataat ccatgaatct tgtttaaata tagatcaaat aaaccactat 240
   accaaaaaca tcaaaagaca actgggtaaa ttttttaaat gactagctat ttgatgttaa 300
   rgaagtaatg ttactctctt atatacaatt tgaaataatc tagcgaggag cagcaaatgt 360
   gcggctatga ggaagaaaca caattggcca ttcttgaatc attagctgga tggtggctat 420
   atgggggtag attttactac tctctaattt tacatatatt taaaatgttc cataataaat 480
   tgttgagtta tcaaaagaaa tatttctata taatagctaa aattatttat aaaagttagt 540
   ggtctcataa ctttatttat ttatttactt attttgagac cgagtctccc tctgttatgc 600
   a 601
<210> 320
   <211> 601
   <212> DNA
   <213> Homo sapiens
<400> 320
   ccaactgatc taattagata aacttagtca atatatttga atcccacatt ccagcagcta 60
   ttttctccat ttgcttttat tgctgtttgt ggtgagtttg atatataatt ttaaggtgtt 120
   aacatcccta acttatgtat gggtacagct cataaatacg aacctgtgtc atgcaactca 180
   tatatgactg tgttcaaaat aatgtgtatt agactgtaaa acgattttaa tattttaaat 240
   aactttcctg catttgtcgg tttcagcagg aaagttattt ttaataactt ccctgtattt 300
   sttggtttca gtattaatta atctcattaa tgctaaactt tgtgatccta ggttaaaaaa 360
   catattcaag atagcttcag aatgtttggt atacaaatag gtctggctaa atataagtgt 420
   tagctttctc aagcatctaa atgctggcgg gcttttaaaa aaccagggct ttaaggagaa 480
   aacacctgct ctgtggtttt gtagcagata tgaagtattc aaatttctta ataaatagaa 540
   aaagaaatat ataacagaaa caggttgcac ttgtctttct cattaagcag gtggttagta 600
   c 601
<210> 321
   <211> 501
   <212> DNA
   <213> Homo sapiens
<400> 321
   agctcataaa tacgaacctg tgtcatgcaa ctcatatatg actgtgttca aaataatgtg 60
   tattagactg taaaacgatt ttaatatttt aaataacttt cctgcatttg tcggtttcag 120
   caggaaagtt atttttaata acttccctgt atttgttggt ttcagtatta attaatctca 180
   ttaatgctaa actttgtgat cctaggttaa aaaacatatt caagatagct tcagaatgtt 240
   tggtatacaa rtaggtctgg ctaaatataa gtgttagctt tctcaagcat ctaaatgctg 300
   gcgggctttt aaaaaaccag ggctttaagg agaaaacacc tgctctgtgg ttttgtagca 360
   gatatgaagt attcaaattt cttaataaat agaaaaagaa atatataaca gaaacaggtt 420
   gcacttgtct ttctcattaa gcaggtggtt agtaccatta tttgcattct catagcctta 480
   atatacattt tccttctcta g 501
<210> 322
   <211> 601
   <212> DNA
   <213> Homo sapiens
<400> 322
   ttttgagttt ctactttagt gtcttagtgc tttctcgata tgggagaatt catgtcctcc      60
   attcagaagt atgcactaag taagaggtat catgtctggt tcttgattag gtactaatct     120
   tgaaatatta tcctacaata ggttagagca cgtatatctc ctgataatat attgaatatg     180
   atagatttaa ataattggtt aactaaatac taaagcaaat tgctgcacgt atcatttatt     240
   attcattgtg tagaaagtgc ctgactcagt gtttggaaat tgtctgactt ttcctcatat     300
   rtagtgtggt ttcatgttat tgtatataag acctgacatg aactctgttt acaataatct     360
   cccagtgcca taaagaccat aataaataat ataaccaatt ggtttcttta tgctgtcatt     420
   tattagggca tatggcatta gtggaggatt accttgtatt acccatagtg cttagagtat     480
   gaatcacaca tgcaccttga aggaaaagag gtgcaatgta ataagaaacc agatattgaa     540
   aatgcaagtt ttgttatgtt attctgggta tgttaacctt tattcctgcc ctccatatgc     600
   a                                                                     601
<210> 323
   <211> 501
   <212> DNA
   <213> Homo sapiens
<400> 323
   aagaggtatc atgtctggtt cttgattagg tactaatctt gaaatactat cctacagtag      60
   gttagagcac gtatatctcc tgataatata ttgaatatga tagatttaaa taattggtta     120
   actaaatact aaagcaaatt gctgcacgta tcatttatta ttcattgtgt agaaagtgcc     180
   tgactcagtg tttggaaatt gtctgacttt tcctcatata tagtgtggtt tcatgttatt     240
   gtatataaga mctgacatga accctgttta caataatctc ccagtgccat aaagaccata     300
   ataaataata taaccaattg gtttctttat gctgtcattt attagggcat atggcattag     360
   tggaggatta ccttgtatta cccatagtgc ttagagtatg aatcacacat gcaccttgaa     420
   ggaaaagagg tgcaatgtaa taagaaacca gatattgaaa atgcaagttt tgttatgtta     480
   ttctgggtat gttaaccttt a                                               501
<210> 324
   <211> 854
   <212> DNA
   <213> Homo sapiens
<400> 324
   tttcagcact gagagccaga gtggaattgt ctccttcatt gccactgcct tcacgttttg 60
   tgtgtcgtat ctgttttgtg atcactgaga cccaagaacc cccgacttgc cgacatacta 120
   tgtggccccg agagaggact tgagctctct gggtttcatc attaccatca attaaataaa 180
   caggacagta gcttcttcct tggattgtta atttaaggct ctggataata catgtaaccg 240
   ccttatgata gagcagaatt gtaagtaggc tcatggtaga atcgttcaat gacatttccc 300
   tttcctttgg gagaaacaga aattcacagg tctaattctt ttcctattaa tagttcctgr 360
   ccattattcc agaactgtcc taaaggaatt ctttctcctt aaggacacca cctcccagga 420
   gggtatttaa agatttgcac aggccgggca cggtggctca tgcttgtaat cccagcagtt 480
   tgggaggcca aggcgggtgg atcacttgtg ctcaggggtt caagaccggc ctggccaaca 540
   tggtgaaacc ctatctctac taaaaacaca aaagttagct gggcctggct atgcatgcct 600
   gtaattccag ctactcggga ggctgaggct ggagaatagc ttgaaccagg gaggtggaga 660
   taacagtgag ctgagatgcc actatgacac tccagcctgg gtgacagagc aagactctct 720
   ctcaaaaaaa aaaaaagatt tttatagtcc agtattcaac gttcatagta cacctttctt 780
   atcctagtaa atcttctttt atcaaggtat atgatcccat atagtagtta actcttactc 840
   ttactttatg acaa 854
<210> 325
   <211> 501
   <212> DNA
   <213> Homo sapiens
<400> 325
   aaatacttac tattaaatat gagaaactgt ggtgtttatc ggtaagatcc acgaaggaag 60
   aagttttaaa gaaaaatact ttaaccgtgg aaaaaaaaaa ctttaatgtc tattatcgaa 120
   taggggccgt aattactttt gcaaaataaa aaaacaaaca agactagcta tagtgtaaat 180
   gtaatctgta tgctttttaa tgaaacaatt aagtaggttg cccatttaca attagcctga 240
   ttttctcctg ygtggtatta tgtgtactta acaacaggac ccagtggaaa ttcactcatt 300
   taacaaagtc tgcctacatg gtttcaaata tgggcctaac ttgaaaattc agtcataatt 360
   aaatctaagg actaaaacaa atctgtataa aaagattctg ctaaataagg gaaaattcaa 420
   gtctagggct acattctgaa agatattgaa gtagaacctc tgcagcaaga ctaggcttgg 480
   aaagtgcggg gaggagggaa a 501
<210> 326
   <211> 601
   <212> DNA
   <213> Homo sapiens
<400> 326
   ccacatcaga aacatgagga aattctacat ggtaaaaaca gcaacaacca aaaaatactt      60
   aaagtcaaca aaccaggaaa agacatctct gaatatagga atgccaaacc tttaacacaa     120
   taaaacacag attatatttc agaaggctat attatatgtg tataccaaca tcaatatgtc     180
   cagagtagct gcacagagtt ccatatttta gtctttataa gttcccctcc tcaccctact     240
   cagtaggcac tttgtgtcta gaaacttctg tgtcaacagt tttccctctc tctggaattc     300
   mtcaggacag aagtgattgg tgtggtggaa gagggttgtg ctaagagtga agttatatga     360
   aagtaggatg gaggttagca agtagttaaa gtccagaaag gcaataaggt gttaaggaag     420
   aacttttcca ttttacaggt ctgagcaagc aggaaatcaa ctctacaaac tttgaaactt     480
   ggtaaatatg aaaacattct caataccatt tgtcatttaa taaatacaaa ttatactatt     540
   ttactgcttg catctagaag tttgtcaaag atctcgtctt aattattcat tgtgtcggcg     600
   a                                                                     601
<210> 327
   <211> 601
   <212> DNA
   <213> Homo sapiens
<400> 327
   gacgagatct ttgacaaact tctagatgca agcagtaaaa tagtataatt tgtatttatt      60
   aaatgacaaa tggtattgag aatgttttca tatttaccaa gtttcaaagt ttgtagagtt     120
   gatttcctgc ttgctcagac ctgtaaaatg gaaaagttct tccttaacac cttattgcct     180
   ttctggactt taactacttg ctaacctcca tcctactttc atataacttc actcttagca     240
   caaccctctt ccaccacacc aatcacttct gtcctgatga attccagaga gagggaaaac     300
   ygttgacaca gaagtttcta gacacaaagt gcctactgag tagggtgagg aggggaactt     360
   ataaagacta aaatatggaa ctctgtgcag ctactctgga catattgatg ttggtataca     420
   catataatat agccttctga aatataatct gtgttttatt gtgttaaagg tttggcattc     480
   ctatattcag agatgtcttt tcctggtttg ttgactttaa gtattttttg gttgttgctg     540
   tttttaccat gtagaatttc ctcatgtttc tgatgtggaa agtataagaa tatcagccag     600
   a                                                                     601
<210> 328
   <211> 811
   <212> DNA
   <213> Homo sapiens
<400> 328
   taaataatct ctaattagta taatgggtgt tcttagtgca gtgggtactt ttaaagtgct        60
   ttgtggcttt tgatgaaaat tgtcttagta tttaaaactt tttcttaccc aattttttgt      120
   tcccatcgaa ttagcaatgc tgtaaagaaa ggcatcttat tccatttttt gttgctataa       180
   aggaatactt gaggctgggt aatttataaa gatgaaaagt ttatttggct cgcaattctg       240
   gatggctgga aggttaagta ctgggccaca gcatctggtg ggggcctcga gctgcttcta        300
   gtcataatgg aaggtgaagg gtgtaaagat catgtgacaa gggaggaaag aagagaagga       360
   aggaggtgct ggttctttct atcaaccaat tcgcaagaga actaatagag aaagaactca       420
   cttagccctg tgggaacaca ttaatctatt cataagggat ctggctgtat gatacaaaca       480
   cctcccatta ggccccacct ccaaattgta tcccattggg gatcaaattt caaaaagaga       540
   tttggaagga acaaacaaac catatctaag ccatagtaaa aggaatggct tttcaaaggt       600
   aaaatttact ragtgtatta atattttacc aatttccagc caggagagta tgaatgttgc       660
   attattacat tgctttgaaa caaagcatta gtcttaattc agaagtttaa attcagatgt       720
   taacgttgca tatttaataa tgcacaacca gtactaaaat cctcattgaa atgacaaata       780
   attttatttc gaatccctta tagaggttca c                                      811
<210> 329
   <211> 811
   <212> DNA
   <213> Homo sapiens
<400> 329
   tgtcttagta tttaaaactt tttcttaccc aattttttgt tcccatcgaa ttagcaatgc        60
   tgtaaagaaa ggcatcttat tccatttttt gttgctataa aggaatactt gaggctgggt       120
   aatttataaa gatgaaaagt ttatttggct cgcaattctg gatggctgga aggttaagta       180
   ctgggccaca gcatctggtg ggggcctcga gctgcttcta gtcataatgg aaggtgaagg       240
   gtgtaaagat catgtgacaa gggaggaaag aagagaagga aggaggtgct ggttctttct       300
   atcaaccaat tcgcaagaga actaatagag aaagaactca cttagccctg tgggaacaca       360
   ttaatctatt cataagggat ctggctgtat gatacaaaca cctcccatta ggccccacct       420
   ccaaattgta tcccattggg gatcaaattt caaaaagaga tttggaagga acaaacaaac       480
   catatctaag ccatagtaaa aggaatggct tttcaaaggt aaaatttact aagtgtatta       540
   atattttacc aatttccagc caggagagta tgaatgttgc attattacat tgctttgaaa       600
   caaagcatta ktcttaattc agaagtttaa attcagatgt taacgttgca tatttaataa       660
   tgcacaacca gtactaaaat cctcattgaa atgacaaata attttatttc gaatccctta       720
   tagaggttca caatgtttta acaatgtagt tttgactaaa tagaagtagt caaaacctgt       780
   cagattggaa atagtattta taaaacataa a                                      811
<210> 330
   <211> 601
   <212> DNA
   <213> Homo sapiens
<400> 330
   gctcatcaat tttgacttaa gaaaattcta gcaacattta tagattttgc caaaattcag        60
   cttcttccca aatcaatcta taagaaggct cttccttaaa cataattttt atatctatga       120
   actgcactag catttactat atatttttat cactctcacc attactggat aataaataaa       180
   agctcattaa aagagttaac aaaacatatt tattttaggc atcctgaaaa aaagattcaa       240
   ttttattatc atttctacaa taagtattga agaaaggaga atttaaatta cttcatatac       300
   stgataaagg aaaacatatg caaggcaaat aaacatctta gatcatgaca tataaaataa       360
   tagattatta ctaaagatta aaatactttc ttaagaatta aagcaattct aaaagcaata       420
   gtaaataaca ttctttctag tgatcagaca ctggatacta tgtttgagat agacagtgaa       480
   ttgggaatgt tgttttacag aagctcctac cttgcaagga caggcaagtt taaatgtcag       540
   ctagaaaact atcttgagtt ttcagtaatg taagattttc ctattcaatt tcacacttta       600
   a                                                                       601
<210> 331
   <211> 601
   <212> DNA
   <213> Homo sapiens
<400> 331
   agaaaattct agcaacattt atagattttg ccaaaattca gcttcttccc aaatcaatct        60
   ataagaaggc tcttccttaa acataatttt tatatctatg aactgcacta gcatttacta       120
   tatattttta tcactctcac cattactgga taataaataa aagctcatta aaagagttaa       180
   caaaacatat ttattttagg catcctgaaa aaaagattca attttattat catttctaca       240
   ataagtattg aagaaaggag aatttaaatt acttcatata cctgataaag gaaaacatat       300
   rcaaggcaaa taaacatctt agatcatgac atataaaata atagattatt actaaagatt       360
   aaaatacttt cttaagaatt aaagcaattc taaaagcaat agtaaataac attctttcta       420
   gtgatcagac actggatact atgtttgaga tagacagtga attgggaatg ttgttttaca       480
   gaagctccta ccttgcaagg acaggcaagt ttaaatgtca gctagaaaac tatcttgagt       540
   tttcagtaat gtaagatttt cctattcaat ttcacacttt aaattttata tatatataaa       600
   a                                                                       601
<210> 332
   <211> 1110
   <212> DNA
   <213> Homo sapiens
<400> 332
   tgtagaagtt cttatcactt cctggccttt tggctaagat caagtgtgaa atgtagaagt        60
   tcctctaagc tttacttccc tcaaaaacta gttttatctt gtcagcagga ttcacttaaa       120
   aagacaaatt cagattatga atttttttct tttttacagg gtctgctctg ttgcccaggc       180
   tggagtgcag aggcacaatc tcggctcact gcagcctccg cctcctgggt tcaagcaatt       240
   ctcttgcctc agcctcccga gtaactggga ttacaggcat gtgccaccac ccagctaatt       300
   tttgtatttt tagtagagat ggggtttcac cacattggtc aggctggtct cgaactgctg       360
   gcctcaagtg atccacttgc ctcggcctcc caaagtgcag agattacagg tgtgagccac       420
   cgtgcccagc ctcataaccg tttcaactac tttttcactt gacaagcaga tgtgaagtta       480
   acaaagtcac ccatatttga aataaagata gtatattcct ggggyaggca gaggcagttg 540
   aggatcatga aataactatg ttggcatagt tatttaggtg ttgatactgt tattatgcca 600
   ttgaaagtta aacagagaac cctctgggta catgttttat accaatgcac actatcttat 660
   tagtccctct cataatgtgc agtcatcatt actgttacgg gttgaggtgt ccccatcctc 720
   tatgggacac ctctatgttg aagtctcaga ttccctagaa tctcagaatg tgaccttgtt 780
   tggaaacaga tttgctacag acgcaattag ttgagatgcg cttatatggg taggtcctaa 840
   ttcagtgact ggtgtcctta aaaaaatgga aatgtacaca cggtggtaga catgcataga 900
   gggaagagag atggagaaaa tggtcaccta caagccaaag acaggggtct ggagcagatc 960
   cttccctcac agccctcaga aggaaccaat cttgccaata ccttgatttt ggacttccac 1020
   ctccagaact ataacacatt tctgttcttc aagcaatttg tagccatttg ttacagctaa 1080
   tacaatcaca catagaaatg acttgtaaat 1110
<210> 333
   <211> 691
   <212> DNA
   <213> Homo sapiens
<400> 333
   taaaacatgt acccagaggg ttctctgttt aactttcaat ggcataataa cagtatcaac 60
   acctaaataa ctatgccaac atagttattt catgatcctc aactgcctct gcctacccca 120
   ggaatatact atctttattt caaatatggg tgactttgtt aacttcacat ctgcttgtca 180
   agtgaaaaag tagttgaaac rgttatgagg ctgggcacgg tggctcacac ctgtaatctc 240
   tgcactttgg gaggccgagg caagtggatc acttgaggcc agcagttcga gaccagcctg 300
   accaatgtgg tgaaacccca tctctactaa aaatacaaaa attagctggg tggtggcaca 360
   tgcctgtaat cccagttact cgggaggctg aggcaagaga attgcttgaa cccaggaggc 420
   ggaggctgca gtgagccgag attgtgcctc tgcactccag cctgggcaac agagcagacc 480
   ctgtaaaaaa gaaaaaaatt cataatctga atttgtcttt ttaagtgaat cctgctgaca 540
   agataaaact agtttttgag ggaagtaaag cttagaggaa cttctacatt tcacacttga 600
   tcttagccaa aaggccagga agtgataaga acttctacat tttaagttat tcacaagata 660
   actattaatg aacctgaaat agtttgtaaa g 691
<210> 334
   <211> 640
   <212> DNA
   <213> Homo sapiens
<400> 334
   aaaccttttt cctgttttac tattactaaa ggtggcacaa cagcaacctc aacaactttg        60
   caccatgcca acactgatgt ttacacccag cacagcattt ttggtctcta tttttattct        120
   cctctgaatg taatgaggat tcctagatgg ctagccaatt cgaatattta aggcaactga        180
   aagttagaat gtttctgaaa catagtgttg ttgccagaga gtacgaaagt tttcaagaat        240
   atcgggcaat tctgaaagta caaagaagcc agattaaatg aaataacact ggcgaagttt        300
   tagcaaggtg actctcatat aatgatcatt atcattacca cagttaaaag aaaagagttg        360
   tttatgaaag gccatgtgtc tgcaatgaaa ctcaaaagag aaaagttaac aggtgcaara        420
   ggtagtttta ttataaaagg agggtaggca acaagaatat gtttaatttt tcttcctttt        480
   catgagtaag gacaagagtt tcatatatgt gaatattttt atttaatttt aagtagaaat        540
   ctgtttttaa aatatgggta tatgcttatt tgtgtaagtg taagaaacag aagtaagtac        600
   agcaaaccag aaataggcca aacactcctg agcataattt                              640
<210> 335
   <211> 919
   <212> DNA
   <213> Homo sapiens
<400> 335
   tacacccagc acagcatttt tggtctctat ttttattctc ctctgaatgt aatgaggatt        60
   cctagatggc tagccaattc gaatatttaa ggcaactgaa agttagaatg tttctgaaac       120
   atagtgttgt tgccagagag tacgaaagtt ttcaagaata tcgggcaatt ctgaaagtac       180
   aaagaagcca gattaaatga aataacactg gcgaagtttt agcaaggtga ctctcatata       240
   atgatcatta tcattaccac agttaaaaga aaagagttgt ttatgaaagg ccatgtgtct       300
   gcaatgaaac tcaaaagaga aaagttaaca ggtgcaaaag gtagttttat tataaaagga       360
   gggtaggcaa caagaatatg tttaattttt cttccttttc atgagtaagg acaagagtkt       420
   catatatgtg aatattttta tttaatttta agtagaaatc tgtttttaaa atatgggtat       480
   atgcttattt gtgtaagtgt aagaaacaga agtaagtaca gcaaaccaga aataggccaa       540
   acactcctga gcataatttt acttggtaga ttattcctga aacttaagga atcatctttg       600
   aactcttttc ctcacttgac ttccaggatt caccatgcac ttgtgatttt cctttcattt       660
   cactctccgt tcctcctcag tctttttttc tcccccaggt cttttttgtt catcttaaac      720
   tctaaatttt agaatatccc aggggtctgc cttcggcctt ctcttttata tctacactgg       780
   cctcatacat aatcttaacc aagtcattat tttaaatacc tacaatatac tgaaaacttc       840
   taaatttgta ttttaattct tgacttcttc catacagtct agatttgtat gtccataggc       900
   tgacatcatt ggctgatac                                                    919
<210> 336
   <211> 1001
   <212> DNA
   <213> Homo sapiens
<400> 336
   ttactaaata ttctccaaca aatatatact tagtatatac tattagtgat gcatgctttc 60
   aaatatttgg actatatcaa tgaatgaaac aaaaaattat ttgcccttaa ggagcttaga 120
   ttctaacaga tggattcaga tgatttttat gccttatttc gtaggtttaa aagagcaatg 180
   gggaaaaggg aagaagagag ggattgaaaa tattgagaag gttgggagac ttagcaattt 240
   taagtaaggt agtgagggta ggttttattg gcaaagtgat ttttcagcag agactgggaa 300
   agatgaacgt ggtatcctgg aggaaagcct cccaggcaga gttaagctgc taacaaaagt 360
   gcccttaggc tggagtgggc ttgtttgatt aaggaacaaa gaggtcagca tggttgcact 420
   agagagaaaa aatcagatgg cgtaaggaga tgaaatcaga aagatacgag gctaggcaaa 480
   ggggtactct atgtaatgaa yatgacctgg cagtactgac atctcctgag ggactgttag 540
   aagtgcagac tcttgtatct tttctcaagt ctatgaaatc tagacttcat tttaacaaga 600
   tgacccgata tttacataca cattaaagtt ccagaagcac tgatataaca cattgtaaga 660
   tcgcacagga cttcaattct ttttctggtt tttagaggca gtcctttggg gtgttttgtg 720
   tagagtataa tgacctgaaa tatctaggat cactctagct actatcttga ggaaagagtg 780
   caataaggcg gaacagttca gaggcaatgg tggtcttcta aatgaaagac acacagcact 840
   caaaccaggc agttgaggag ggatgggaag aagttgtcaa attctagaca tattttaaag 900
   gtagtgtcca gagaatttcc ttagatgcgt aggaacatgg aggataggac atagggtgga 960
   aataaacgaa ataaagaaac tgaagctgat tctgacattt t 1001
<210> 337
   <211> 1576
   <212> DNA
   <213> Homo sapiens
<400> 337
   atacctttta agtgacatcc tagtgaatct ccatttgtca cgagacctca agctttccag 60
   ttctggcaca aagtgattac tcataccatc acttcaaaat gatgattatc ttcatttatt 120
   ttagttatat tgaacaaaat atacatttaa aaaatctaat tactaaatat tctccaacaa 180
   atatatactt agtatatact attagtgatg catgctttca aatatttgga ctatatcaat 240
   gaatgaaaca aaaaattatt tgcccttaag gagcttagat tctaacagat ggattcagat 300
   gatttttatg ccttatttcg taggtttaaa agagcaatgg ggaaaaggga agaagagagg 360
   gattgaaaat attgagaagg ttgggagact tagcaatttt aagtaaggta gtgagggtag 420
   gttttattgg caaagtgatt tttcagcaga gactgggaaa gatgaacgtg gtatcctgga 480
   ggaaagcctc ccaggcagag ttaagctgct aacaaaagtg cccttaggct ggagtgggct 540
   tgtttgatta aggaacaaag aggtcagcat ggttgcacta gagagaaaaa atcagatggc 600
   gtaaggagat gaaatcagaa agatacgagg ctaggcaaag gggtactcta tgtaatgaac 660
   atgacctggc agtactgaca tctcctgagg gactgttaga agtgcagact cttgtatctt 720
   ttctcaartc tatgaaatct agacttcatt ttaacaagat gacccgatat ttacatacac 780
   attaaagttc cagaagcact gatataacac attgtaagat cgcacaggac ttcaattctt 840
   tttctggttt ttagaggcag tcctttgggg tgttttgtgt agagtataat gacctgaaat 900
   atctaggatc actctagcta ctatcttgag gaaagagtgc aataaggcgg aacagttcag 960
   aggcaatggt ggtcttctaa atgaaagaca cacagcactc aaaccaggca gttgaggagg 1020
   gatgggaaga agttgtcaaa ttctagacat attttaaagg tagtgtccag agaatttcct 1080
   tagatgcgta ggaacatgga ggataggaca tagggtggaa ataaacgaaa taaagaaact 1140
   gaagctgatt ctgacatttt agacctaaaa tctcaactaa aagttgccaa gatgggaaaa 1200
   actaggtgca tcttgtttgg tgagtggaaa tcagccttgt gaattaagac ttaaactgat 1260
   gtctttaatc ccgtagaaat accatgaagg cagtagaaga tggctaaaga gaggtctaga 1320
   ctgtaggtac aaatttaaaa gtcacttgca tttggatgct taaagtcagg atattgtgaa 1380
   gtcaacagag gaataaataa atgcagagag gggaaagaaa aggcccatag actgagccat 1440
   tgtctggttt atttacatat tagtatatat tttcttaaag atgtttgcta tataataatg 1500
   agttacctaa agtgtgactt ttctaaattt atggggaatt ttctacattg tgttatggca 1560
   ctactaaaaa taataa 1576
<210> 338
   <211> 1275
   <212> DNA
   <213> Homo sapiens
<400> 338
   gtaaaactaa ttataattaa aatcaaaata tttactgaac ctacttactc ctataatttg 60
   cgttgctggt taaaacccag ctataaaaat tttgatcaaa aatttttatt ttgtaaatga 120
   tctgacacag cataaatgtt aatcacattt ctttatttta tttgcagatt aatttgagta 180
   atttgaaaaa ttattaatgt tacttaatta ctctcaacac cttacagtgt ctcctgtaag 240
   cactattggt gatactgaat ttaagttaca tttaacaact atcagaaaat agtttttaaa 300
   gtaaaaatta tgatttggag tttaccaact aaatcttgtt agctttcact gcctctattg 360
   agaagagcag cagttcttat cttcctcctt tttcttcttt aattaacaag agattatttg 420
   tatcatagcc ataaaatcag ttcaggtatt acatgaacga cacccctgac tgcaatggtg 480
   tagtttattg tattagtcca ttttcatgct gctgataaag acatacataa gactgggtaa 540
   tttataaaga aatagaagtt taacggactc acagttccat gtggctgggg aagcctcaca 600
   atcatgatcg aaggcaaaag gcacatctta catggcaaca ggcaagagag aatgagagcc 660
   aagtgaaagg agaaacccct tataaaacct tcagacctca tgagacttat tcactaccac 720
   aagaacagta tgtgagaaac agtcccatga tccagttatc tcccactggg tccctcccac 780
   cacacaaggg aattatggga actgcaattc aagatgaaat gtgggtggaa gcacaacgga 840
   actatatcat gatcaaagca ttattgtttt ctctgataag ctgatctaga aagtgctgct 900
   tgtgatcagc tttggtgacc atgatcagtg aaatggttaa ggaaatctac agattttgta 960
   ggtttgtgcc ttgacagacg accggtatct gtttctcttt tcatgatgaa gtatctaaca 1020
   aagctctgtc caaaattttg aatttctcgt taaawgcatc atgattatag aacagaggtt 1080
   acaatcaatt attcagtcac acaatcactc tcatcagtca ttaaggtgca tacctggtgt 1140
   tccagttatt cagtgtggta taacaaacta cctggaactt aatggcttga aatagtcacc 1200
   attacattat gattgtccat tctctgcatc aataattagg atttggcaaa gagggaatgg 1260
   tttgtttaca gacag 1275
<210> 339
   <211> 1275
   <212> DNA
   <213> Homo sapiens
<400> 339
   gtaaaactaa ttataattaa aatcaaaata tttactgaac ctacttactc ctataatttg      60
   cgttgctggt taaaacccag ctataaaaat tttgatcaaa aatttttatt ttgtaaatga     120
   tctgacacag cataaatgtt aatcacattt ctttatttta tttgcagatt aatttgagta     180
   atttgaaaaa ttattaatgt tacttaatta ctctcaacac cttacagtgt ctcctgtaag     240
   cactattggt gatactgaat ttaagttaca tttaacaact atcagaaaat agtttttaaa     300
   gtaaaaatta tgatttggag tttaccaact aaatcttgtt agctttcact gcctctattg     360
   agaagagcag cagttcttat cttcctcctt tttcttcttt aattaacaag agattatttg     420
   tatcatagcc ataaaatcag ttcaggtatt acatgaacga cacccctgac tgcaatggtg     480
   tagtttattg tattagtcca ttttcatgct gctgataaag acatacataa gactgggtaa     540
   tttataaaga aatagaagtt taacggactc acagttccat gtggctgggg aagcctcaca     600
   atcatgatcg aaggcaaaag gcacatctta catggcaaca ggcaagagag aatgagagcc     660
   aagtgaaagg agaaacccct tataaaacct tcagacctca tgagacttat tcactaccac     720
   aagaacagta tgtgagaaac agtcccatga tccagttatc tcccactggg tccctcccac     780
   cacacaaggg aattatggga actgcaattc aagatgaaat gtgggtggaa gcacaacgga     840
   actatatcat gatcaaagca ttattgtttt ctctgataag ctgatctaga aagtgctgct     900
   tgtgatcagc tttggtgacc atgatcagtg aaatggttaa ggaaatctac agattttgta     960
   ggtttgtgcc ttgacagacg accggtatct gtttctcttt tcatgatgaa gtatctaaca    1020
   aagctctgtc caaaattttg aatttctcgt taaatgcatc atgattatag aacagaggtt    1080
   acaatcaatt attcagtcac acaatcactc tcatcagtca ttaaggtgcr tacctggtgt    1140
   tccagttatt cagtgtggta taacaaacta cctggaactt aatggcttga aatagtcacc    1200
   attacattat gattgtccat tctctgcatc aataattagg atttggcaaa gagggaatgg    1260
   tttgtttaca gacag                                                     1275
<210> 340
   <211> 1001
   <212> DNA
   <213> Homo sapiens
<400> 340
   gaaacaaaaa attgcttttt atatattgat atttttgcac ggatttctta ggattttcta 60
   tgtacatgac catgtcatct gcaaatgaaa tagttttatt tctttatcaa tccggatgaa 120
   tttattaaaa ttatcttgcc taatttccca aatagggcct ccatgttgaa cataagtggt 180
   ggcaagggtg atctgttgct aatctcagtg gatgatattc agtgttttac aatgatcttc 240
   gacagctctg gctgttaaat tatcatagtc tgtatggcct aaacaaacaa aatacttatg 300
   attatggggg aggctgggat atccaagatc aagttgctgg caggtctagc aacctgccac 360
   tgggaagccc tgcttcccag ttttcagatg gccaccttct tatagtatct tcaccaaaga 420
   tagggcagag agagcaagca agctctctac cttctcatat aagggcacta atcccaccat 480
   gaaggcgcca ctgtcatgac stgattatgt cacaaagacc ccggggcaaa tattaccact 540
   gtgaggagta cagttttagc atgtgaattt tggaagaaca caaacattta gtacagagtg 600
   actattaagt atgttattaa ctatggagtt tttgtaggca ttttttaaca cattgagaaa 660
   gtttcctcta ttcctacttt tgttgagaag tttttatgat gacaaggcat tacattttat 720
   ccaatgactt ttctgtgtgt attgagatga ctgatttgtt ctgccaattt aaatccattg 780
   ttgattctct ctaggatttt ttttatttca gttattaaat ttttcaacag gagaattact 840
   gtcttgttct tttttttgta atttctgtcc ccttactggt attccatatt taataaggca 900
   tcataatagt actcttcttt agtttcttaa agatggtttt ctttagtttt taacatattt 960
   atgtctattt agaagtcttt gttaagtctg acatctgagc t 1001
<210> 341
   <211> 1001
   <212> DNA
   <213> Homo sapiens
<400> 341
   ggatttctta ggattttcta tgtacatgac catgtcatct gcaaatgaaa tagttttatt 60
   tctttatcaa tccggatgaa tttattaaaa ttatcttgcc taatttccca aatagggcct 120
   ccatgttgaa cataagtggt ggcaagggtg atctgttgct aatctcagtg gatgatattc 180
   agtgttttac aatgatcttc gacagctctg gctgttaaat tatcatagtc tgtatggcct 240
   aaacaaacaa aatacttatg attatggggg aggctgggat atccaagatc aagttgctgg 300
   caggtctagc aacctgccac tgggaagccc tgcttcccag ttttcagatg gccaccttct 360
   tatagtatct tcaccaaaga tagggcagag agagcaagca agctctctac cttctcatat 420
   aagggcacta atcccaccat gaaggcgcca ctgtcatgac ctgattatgt cacaaagacc 480
   ccggggcaaa tattaccact stgaggagta cagttttagc atgtgaattt tggaagaaca 540
   caaacattta gtacagagtg actattaagt atgttattaa ctatggagtt tttgtaggca 600
   ttttttaaca cattgagaaa gtttcctcta ttcctacttt tgttgagaag tttttatgat 660
   gacaaggcat tacattttat ccaatgactt ttctgtgtgt attgagatga ctgatttgtt 720
   ctgccaattt aaatccattg ttgattctct ctaggatttt ttttatttca gttattaaat 780
   ttttcaacag gagaattact gtcttgttct tttttttgta atttctgtcc ccttactggt 840
   attccatatt taataaggca tcataatagt actcttcttt agtttcttaa agatggtttt 900
   ctttagtttt taacatattt atgtctattt agaagtcttt gttaagtctg acatctgagc 960
   tctctcaaag tttctgctga tttttttttt cctatgtttg g 1001
<210> 342
   <211> 701
   <212> DNA
   <213> Homo sapiens
<400> 342
   ggaaaccctg gcctcttgat cacactttcc tggagtttag tcccctctgc aatatgtacc 60
   tgggagtcat aagaaatgcc agttacaaaa acttcctgta cagatatcct agcactcaac 120
   tggaaaccgg ggagagtcac aattctgtct ttccagccat atgtaactga aatggagatc 180
   ttttcaccct gagccagggg tgatgggaaa gggagctggt catggctcaa tgtttagcct 240
   tttcttggtc ttcaagattt catagacatt cttaaataca tgtttctttc aatgaagttt 300
   gcccttagga caattcacag ctacattagg tactttttaa ataatacttt tgaccatccg 360
   tggttatttc attgaagaaa atctatagag cacctcagcc atcattccag aagtgactat 420
   cctcctcagt aatggttctt attctaattt taaatatcat tgatgtagaa cattctattt 480
   cactattcct tcattttatt rttatgggaa attatataca gttctccaga tttttaaagc 540
   cttgctaaca tgttttaagt cacacaaata ttcttctgtg ggaaaatgac agtaatttag 600
   tgtgcaacaa ttatatagaa ctatttttca aacttataaa cgaagtgaaa ttctaaataa 660
   aatcatttat caaacacaaa aatttgagcc agaataagga a 701
<210> 343
   <211> 701
   <212> DNA
   <213> Homo sapiens
<400> 343
   aatgccagtt acaaaaactt cctgtacaga tatcctagca ctcaactgga aaccggggag      60
   agtcacaatt ctgtctttcc agccatatgt aactgaaatg gagatctttt caccctgagc     120
   caggggtgat gggaaaggga gctggtcatg gctcaatgtt tagccttttc ttggtcttca     180
   agatttcata gacattctta aatacatgtt tctttcaatg aagtttgccc ttaggacaat     240
   tcacagctac attaggtact ttttaaataa tacttttgac catccgtggt tatttcattg     300
   aagaaaatct atagagcacc tcagccatca ttccagaagt gactatcctc ctcagtaatg     360
   gttcttattc taattttaaa tatcattgat gtagaacatt ctatttcact attccttcat     420
   tttattatta tgggaaatta tatacagttc tccagatttt taaagccttg ctaacatgtt     480
   ttaagtcaca caaatattct yctgtgggaa aatgacagta atttagtgtg caacaattat     540
   atagaactat ttttcaaact tataaacgaa gtgaaattct aaataaaatc atttatcaaa     600
   cacaaaaatt tgagccagaa taaggaatgt aaattacaat ttaaacacag attataaact     660
   atcttacttt taaaatgtta aaattcctaa cttgtttgaa a                         701
<210> 344
   <211> 768
   <212> DNA
   <213> Homo sapiens
<400> 344
   ctaaaatcta ccattatatg atatccttcc caatacataa attaaaaaaa aaaacactgt 60
   agaggaaaaa gcaatatttt gaaatgatat gcttttcttt gtttgtcttc aaacaattac 120
   atcttcatca taatggttgt attagtctgt ttttacactg ctataaagaa ttgcctgaga 180
   ctgagtaaca tataaagaaa aaagttttaa ttgaccacag tttcacaggc ttaataggaa 240
   gcatgactgg gaaacttaga atcatggcag aagaggaagg ggaagcaagg atcttcttca 300
   catggtagca ggagagagag cacaaagggg gacacgctac acactttcaa acaacgagat 360
   ctcctgagaa ctctatcggg agaacagcaa gagggaagtt cacccctatg attcaatcag 420
   ctcccaccgg gcttctcccc tgacacatga ggaattacaa ttggatgaga gatttgggtg 480
   gggacacaca gacaaaccat atcaactgtc atggacttaa acaattgtct ttgaattgtc 540
   ttttttcata cttttatttg catctttyca ctaaaaagat gacacaaagt aatcctagtt 600
   tacatttttt accatgtaat tccatattac tttttcctga aagttactta tttttaaatc 660
   tcaaagctct tcatacttat ggtttgatct gcacttacaa ctggatctca gaaagattga 720
   attctcccat cataccaagt tcatgtctct cactcttaat atttgttc 768
<210> 345
   <211> 701
   <212> DNA
   <213> Homo sapiens
<400> 345
   aaatgatatg cttttctttg tttgtcttca aacaattaca tcttcatcat aatggttgta 60
   ttagtctgtt tttacactgc tataaagaat tgcctgagac tgagtaacat ataaagaaaa 120
   aagttttaat tgaccacagt ttcacaggct taataggaag catgactggg aaacttagaa 180
   tcatggcaga agaggaaggg gaagcaagga tcttcttcac atggtagcag gagagagagc 240
   acaaaggggg acacgctaca cactttcaaa caacgagatc tcctgagaac tctatcggga 300
   gaacagcaag agggaagttc acccctatga ttcaatcagc tcccaccggg cttctcccct 360
   gacacatgag gaattacaat tggatgagag atttgggtgg ggacacacag acaaaccata 420
   tcaactgtca tggacttaaa caattgtctt tgaattgtct tttttcatac ttttatttgc 480
   atcttttcac taaaaagatg rcacaaagta atcctagttt acatttttta ccatgtaatt 540
   ccatattact ttttcctgaa agttacttat ttttaaatct caaagctctt catacttatg 600
   gtttgatctg cacttacaac tggatctcag aaagattgaa ttctcccatc ataccaagtt 660
   catgtctctc actcttaata tttgttccca agacaacaat t 701
<210> 346
   <211> 6758
   <212> DNA
   <213> Homo sapiens
<400> 346
   agagtgggcc attgttctga ctagtctggg gctccccaaa gaactggtat ctgtctcacc      60
   tgactcagaa caatgataag gctgtagatc tttttggaag tctatgaaaa caggcacaat     120
   gaaggcagca tgttagagat ataattccac aggaagatgc caggtaaaac aaaagagaaa     180
   aagcaggaac aagctgatta ggaaatttgt gatgactaaa agtatataca caagcccaaa     240
   taagatactc caaagatgtt tgataggttc tagatctcta gatatactgc tcaatgaaag     300
   tgtccccctg aacaaagcca gtctgcaaag actgggtgag atgatttttt ttaaatgtca     360
   agtctcagca acaacaaaaa tgacaagaca tgcacagaag caagaaaata taacacaatc     420
   aaagaaaaaa aagccacaga aatcagtcct agagaaaacy gatctatgag ctgcctgaca     480
   ataattataa aataactatc ataaaaatgc ccagtgagat ataagaaaac acagacaact     540
   aaatgaatca ggaaaatgat gcatgaacaa aatgggcata tcaacagaga tggaaatgac     600
   aaagataaac aaacagaaat tttggagctt aaaaatacag taagtaaagt gaataattca     660
   ctaaaaatat tcaatagcag actagatcag gcagaagaaa atatcaatga acttgaagac     720
   agatcatcaa gtcagaggaa caacagcaac aaaaaagaat gaaaaaagtg aagacagcct     780
   aagggactta ggagtcagta ccaaggaaat caatatatac gttatagatg tatcagaaga     840
   aaaagggaga aaaatgaaaa gaaagcatat ttgaaaaaat aatagctgaa gaattctcaa 900
   tttcaaagag agaaattgat atacaaattc aagaagttca aaagactcta gccataataa 960
   atctaaagag actcacacta agacatatta tcatcaaact gtcaaaatca aagacaaaga 1020
   attgtgaaat ctgccaagga aaagtgactc atcacacata agagatataa cataagattg 1080
   tcacaggatt tctgaacaga cactttgcag gtcagaggga agtagggtga catattccag 1140
   gtgctgaaag aagaaaacac cctgccaacc aagaatatgg catccagaaa aactttccta 1200
   gaagaatgaa ggagaaattt agactttccc aaataaacaa aagctgaggg agttcattac 1260
   taccagacct gctctgcaaa atgctaaaga gaaaccttca ggtgaaacaa aaagatgcta 1320
   gacagtaaca caaaaccact cataaataac ttcttcagta aaaataatac atcgacaaat 1380
   atggtaacct gtattaatac tggtgcacaa attcactttc aaattttata aataagaatt 1440
   taaaggatga aaacatctaa aactaactat aaatctatat aatgaatata caatatataa 1500
   aaaaatttgt gatcacaata acataaaatg ggggaggtag agctgtatag gggtagagct 1560
   tttgtatgca attgaaatta ccatcagttt aaactgaact gttataacat taagatgttt 1620
   tatgtaattg caatggtaac tatattctat agaatatatt aaaaagaaaa agaaaatagg 1680
   aagggaatca aagcatgtcc ttgtaaaaaa gtcaatgaaa gcaaaagaaa ggcagaaaga 1740
   gtgaaaagga ggaataaaaa gttataagac ataaaaaaaa tgaaaatagt aatagtcctg 1800
   ccatatcagt aattacatta aatataaatg gattaaactc cctaatcaaa tcatagattg 1860
   gtttgcaaga actaacttta caattaaaga cacacagctg acggtgaagg gagaaaaaaa 1920
   acttccatgc agtgaccaaa atagaggagg gtggctgtat tactgtcaga caaaataaaa 1980
   tttaagtcaa aaactgttac aagagtaaaa gaagggcatt atacagttaa aaaagtaaat 2040
   tcgccaggca gacacaacaa ttataaatat caatacataa aaataagagc tcctaaatat 2100
   atgcagcaaa cagacataat tgaagaaaga aataaatagc taaaatggta gaagacttta 2160
   atacccccac ttacaataat gtataaaata acaagacaga atgtaaataa aaatgtagag 2220
   aatttgagca acactgtaga ccaattggac ctaataaata tactcagaat aatccatcca 2280
   accaaagcag aaacagaata tacattcttt tcaagtacac atttgacatt ctctgggatt 2340
   aactacatgt tatgcaacaa acaagtctca acaatgttta aaagtctgat attacacaaa 2400
   gtattgtttc tgatgacgat ggaaagaacc tagaagccaa tagcaaaaag aaaatagaaa 2460
   atccacacat atgtggaaat taaactacat gcaattaagc aaagggccaa agaagaagaa 2520
   gaaaaaagaa aacaccgtga aacaaataaa aacaaaaata cagcatatga aaatgcatgg 2580
   gatgcagcaa aagtgatggt aagagaaatg tttatagtta taaatgcaaa ccttaaaaaa 2640
   gaagaaagaa aacaaaaata ctcaaattaa caactttaca agtcaagaag gtagagaaaa 2700
   aagaacaaac tataccaaaa gctaacacag aaagaaaaga ataaagatta aaaacaaaaa 2760
   caatttaaaa aatagcagaa ctaaaagttg gttctttgaa aagatcaaca gaattgacaa 2820
   tttcttagct acattaagaa aaatacaaga ctcaaataac acaaatcagt ggtgaaaggg 2880
   ggtattataa ctgatgccac agaaatacaa aaggatcata agggactact acaaattgta 2940
   tgacaacaaa ttgagtaacc taggatacct tgataaattc caaaaaatgc acaatatact 3000
   gaatcatgaa tacatgaccc ttataaatca agactaaatc ataaagaaat agaaaatatc 3060
   aacagaccaa taattagtaa ggagaataaa ctagtaatca gaaacctccc aacaaagaaa 3120
   agcttaggac caaatggctt tactggagaa ttctaccaac cattaaaagg ataattaaga 3180
   ccaatcttcc tcaaactttt aaaacaaatg ttaaagagga ggaaactctt tcaatctcat 3240
   tcataaggtc agcattatcc ttataccaaa accagacaaa gacactatta aaaaaactta 3300
   gaccaatatc cctgatgaat ttcgatgcaa gaatcctcag caaaatacta tcaaacaatt 3360
   caacagcata cttaaatgat tatatgctgt aatcaagatg catttattct ttgaatgcaa 3420
   gtgtaattca acacataaaa ttcaatcaat gtaatacacc acattaacag aatgagagac 3480
   aaaaaccaca taattatatc aactgatgca gaaaaaaatc tgacacagtt caacaccttt 3540
   tgtgataaaa acactcaaca aactaggaaa agaaggaaac aactttaaca catcatatgc 3600
   tcactgatga aaatctacaa gttctttata aaagatcagg aacaagacaa taatctgcat 3660
   tgttaccact tctattatac gtagtattgg aagttctaat cagagcaaat taggcaagaa 3720
   aaataaataa aaggcatcca aagtggaaag gaagtaaaat aatctctttt tacagatgat 3780
   ataaccttag aattagaaaa tcctaaaaat ttcacatacc aagaaaaagc gtgttaaaat 3840
   taataagtaa attcagcaag ttgactgata caaaatcaac acagaaagct cagttgtgtg 3900
   tctgtgtgtc tcatacacta acaatgaaca atctgaaaag gagattaaga aaacaatttc 3960
   atttacaata gcatcaggaa aaaaaataaa tacttaggaa caaacttaac caaggggttg 4020
   gaattcctgt atactgaaaa ctacaaatat tgccaaaaga aaataaagga gacacaaata 4080
   agtgatatgt ttttaatatg tccacccaaa gtgatcttca gattcaatga aatccctatc 4140
   aaagttataa tggcattttt ctgcaggaat gtaaaaattt atcctaaaat tcatatagaa 4200
   tctctaggta ccctgagggc caaacaattt tgagaaaaaa aaaagaacaa aattggagga 4260
   ctcacacttc cagattacaa gaatatttac aaattacata tttacaaaaa aaattacaaa 4320
   gccacaataa tcaaaacaac gtgggatttg cataaaggca gatatataga ccagtggaat 4380
   agtattgaga gtccagaaat aaacccttag gtatatcatc aaatgacatt tgacaaagtg 4440
   ctggtaccac tcaatgggaa tgggacaatt tgttcaacaa atagagcaaa gaaaactaaa 4500
   catccatgtg caaaagaata aatctggacc cttatattac actatagaca aaattaattc 4560
   aaaatggatt aaagatctaa atgaaagatc taaaactata aaactcctag gagaaaacag 4620
   aggaaaaatt tcatgctaat ttggcaacat tttgtgatgt gacaccaaaa gcagagtcaa 4680
   taaaagcaaa aattagacag atggaaatcc atcatagttt ataacttttg gtcattaaag 4740
   aacagtcaac agagtgaaaa ggcaatctat aaaatggggg aaaaacagaa aatatgtgca 4800
   aatcacagat atctgatagg ggattcatat ccagaataaa taaagaactc ctatatctca 4860
   acaacaaaaa atctaatcca atcaaaaaat gggccaaggg agtgaagata catttctcca 4920
   aagatgttat acaaatggcc aggaagcata tgaaaagatg ttcaatgtca ctaatcatca 4980
   gagaaatgca aatcaaaacc acagtgcaat atcacttcac attcattaga atggcttctg 5040
   tcatgaacaa cagaaaataa caagtgttga tgagtgtgta gagaaattga gacctttata 5100
   taattttggc agaaattcaa aatggtgcaa ccactataaa aaatgatatg gaggtcctca 5160
   aaaaattaaa aatagaacta ccatatgatc cacaatccca cctctgggta catattcaaa 5220
   agaattgaaa gcagggtgtt gaagatatat ttgcacactc tttatagcag cactgttcac 5280
   aatagccaag agatgaaagt aacccaaagg ttcatgaagc aatgaataaa caaaatatat 5340
   tatgtacata gagtaaaata ctgtgcagct ttaaagagaa aggaaatctt atactatgct 5400
   acaacatgaa tggaacttta gggcattata gtaagtaaaa taagccagtt ttttttaaag 5460
   gacaaataaa cactatacga ttctacttaa gtatttaatg ttgtcaaatt tataaatata 5520
   gaatgtagaa tagtggttac cctgagctgg gggaaagggg caaaggggaa ttgttatttt 5580
   aatgggtata gtttcagttc tgcaaaatga aaaggttctg gaaatctgtt tcacaatgtt 5640
   gtaaatataa ttactctgaa attgtacact taaaaatggt taagatgaca aatagagttg 5700
   tgatgtcttc ttttgttatt atatagaaaa actttttcat atgataatag tctttgtttt 5760
   taagctgact ttgctgatat taatataatc cttccatttt tctttaaaat gctatatgct 5820
   ttcacataat tttgctttac gttgatgtat ttatacataa ggtgggtttc ttatagatac 5880
   cacgttgtgt gtctttttta tctaagttga tagacttgcc ttttgttagg gtatttaaat 5940
   aatttatatt taatgtaatt attgatatag ttgagtgtgt tgatttttgt tttctatttg 6000
   ctccatctgt tgttggttct cattattcct ctgtttctac cttcttttgt actaattatt 6060
   atattttatt atttttcatc tcaactgttg gcttattagc cacattgctt ttaaaatttt 6120
   taatgattgc tctagggttt ataataaaca aaatgttagc attttctacc atcaaatatt 6180
   tttacactat tcatgtatac ttcaatttct ttcttcccat cctttgaact atatcttcat 6240
   acattttact ctacatttgt tataactcag tgctttgaaa gtcaattatt tttgtctttg 6300
   acagtcaatg atttttaaag agtttaacag tgaaaaaaaa tggctttcat ctttttccat 6360
   tagatttcat actccttctg cctgaagaat ttcttttaat agaccttgta ctgcgggtct 6420
   caggcaagaa attctctcag cctttgttgg tttgaaaaac tgcttattac acctttgttt 6480
   ttgaaagata ttttcactag gtatagaagt ctgggttgac agttctcatt gtttgtcaca     6540
   gcatttttaa gatgcccatt caattgtctt gtcttgtata attttggatt agtctggtgt     6600
   atttcttacc tttgttcctc tctgtgcaat gcttcaacca tcccacttca ggctgccttt     6660
   aagatgtttt cttttccctt aatctttagt ttttagctgg ttgacagtga cgcatctaag     6720
   tgtagtgtat gaggttgctt ttattgtcac tgttgttg                             6758
<210> 347
   <211> 6758
   <212> DNA
   <213> Homo sapiens
<400> 347
   agagtgggcc attgttctga ctagtctggg gctccccaaa gaactggtat ctgtctcacc 60
   tgactcagaa caatgataag gctgtagatc tttttggaag tctatgaaaa caggcacaat 120
   gaaggcagca tgttagagat ataattccac aggaagatgc caggtaaaac aaaagagaaa 180
   aagcaggaac aagctgatta ggaaatttgt gatgactaaa agtatataca caagcccaaa 240
   taagatactc caaagatgtt tgataggttc tagatctcta gatatactgc tcaatgaaag 300
   tgtccccctg aacaaagcca gtctgcaaag actgggtgag atgatttttt ttaaatgtca 360
   agtctcagca acaacaaaaa tgacaagaca tgcacagaag caagaaaata taacacaatc 420
   aaagaaaaaa aagccacaga aatcagtcct agagaaaact gatctatgag ctgcctgama 480
   ataattataa aataactatc ataaaaatgc ccagtgagat ataagaaaac acagacaact 540
   aaatgaatca ggaaaatgat gcatgaacaa aatgggcata tcaacagaga tggaaatgac 600
   aaagataaac aaacagaaat tttggagctt aaaaatacag taagtaaagt gaataattca 660
   ctaaaaatat tcaatagcag actagatcag gcagaagaaa atatcaatga acttgaagac 720
   agatcatcaa gtcagaggaa caacagcaac aaaaaagaat gaaaaaagtg aagacagcct 780
   aagggactta ggagtcagta ccaaggaaat caatatatac gttatagatg tatcagaaga 840
   aaaagggaga aaaatgaaaa gaaagcatat ttgaaaaaat aatagctgaa gaattctcaa 900
   tttcaaagag agaaattgat atacaaattc aagaagttca aaagactcta gccataataa 960
   atctaaagag actcacacta agacatatta tcatcaaact gtcaaaatca aagacaaaga 1020
   attgtgaaat ctgccaagga aaagtgactc atcacacata agagatataa cataagattg 1080
   tcacaggatt tctgaacaga cactttgcag gtcagaggga agtagggtga catattccag 1140
   gtgctgaaag aagaaaacac cctgccaacc aagaatatgg catccagaaa aactttccta 1200
   gaagaatgaa ggagaaattt agactttccc aaataaacaa aagctgaggg agttcattac 1260
   taccagacct gctctgcaaa atgctaaaga gaaaccttca ggtgaaacaa aaagatgcta 1320
   gacagtaaca caaaaccact cataaataac ttcttcagta aaaataatac atcgacaaat 1380
   atggtaacct gtattaatac tggtgcacaa attcactttc aaattttata aataagaatt 1440
   taaaggatga aaacatctaa aactaactat aaatctatat aatgaatata caatatataa 1500
   aaaaatttgt gatcacaata acataaaatg ggggaggtag agctgtatag gggtagagct 1560
   tttgtatgca attgaaatta ccatcagttt aaactgaact gttataacat taagatgttt 1620
   tatgtaattg caatggtaac tatattctat agaatatatt aaaaagaaaa agaaaatagg 1680
   aagggaatca aagcatgtcc ttgtaaaaaa gtcaatgaaa gcaaaagaaa ggcagaaaga 1740
   gtgaaaagga ggaataaaaa gttataagac ataaaaaaaa tgaaaatagt aatagtcctg 1800
   ccatatcagt aattacatta aatataaatg gattaaactc cctaatcaaa tcatagattg 1860
   gtttgcaaga actaacttta caattaaaga cacacagctg acggtgaagg gagaaaaaaa 1920
   acttccatgc agtgaccaaa atagaggagg gtggctgtat tactgtcaga caaaataaaa 1980
   tttaagtcaa aaactgttac aagagtaaaa gaagggcatt atacagttaa aaaagtaaat 2040
   tcgccaggca gacacaacaa ttataaatat caatacataa aaataagagc tcctaaatat 2100
   atgcagcaaa cagacataat tgaagaaaga aataaatagc taaaatggta gaagacttta 2160
   atacccccac ttacaataat gtataaaata acaagacaga atgtaaataa aaatgtagag 2220
   aatttgagca acactgtaga ccaattggac ctaataaata tactcagaat aatccatcca 2280
   accaaagcag aaacagaata tacattcttt tcaagtacac atttgacatt ctctgggatt 2340
   aactacatgt tatgcaacaa acaagtctca acaatgttta aaagtctgat attacacaaa 2400
   gtattgtttc tgatgacgat ggaaagaacc tagaagccaa tagcaaaaag aaaatagaaa 2460
   atccacacat atgtggaaat taaactacat gcaattaagc aaagggccaa agaagaagaa 2520
   gaaaaaagaa aacaccgtga aacaaataaa aacaaaaata cagcatatga aaatgcatgg 2580
   gatgcagcaa aagtgatggt aagagaaatg tttatagtta taaatgcaaa ccttaaaaaa 2640
   gaagaaagaa aacaaaaata ctcaaattaa caactttaca agtcaagaag gtagagaaaa 2700
   aagaacaaac tataccaaaa gctaacacag aaagaaaaga ataaagatta aaaacaaaaa 2760
   caatttaaaa aatagcagaa ctaaaagttg gttctttgaa aagatcaaca gaattgacaa 2820
   tttcttagct acattaagaa aaatacaaga ctcaaataac acaaatcagt ggtgaaaggg 2880
   ggtattataa ctgatgccac agaaatacaa aaggatcata agggactact acaaattgta 2940
   tgacaacaaa ttgagtaacc taggatacct tgataaattc caaaaaatgc acaatatact 3000
   gaatcatgaa tacatgaccc ttataaatca agactaaatc ataaagaaat agaaaatatc 3060
   aacagaccaa taattagtaa ggagaataaa ctagtaatca gaaacctccc aacaaagaaa 3120
   agcttaggac caaatggctt tactggagaa ttctaccaac cattaaaagg ataattaaga 3180
   ccaatcttcc tcaaactttt aaaacaaatg ttaaagagga ggaaactctt tcaatctcat 3240
   tcataaggtc agcattatcc ttataccaaa accagacaaa gacactatta aaaaaactta 3300
   gaccaatatc cctgatgaat ttcgatgcaa gaatcctcag caaaatacta tcaaacaatt 3360
   caacagcata cttaaatgat tatatgctgt aatcaagatg catttattct ttgaatgcaa 3420
   gtgtaattca acacataaaa ttcaatcaat gtaatacacc acattaacag aatgagagac 3480
   aaaaaccaca taattatatc aactgatgca gaaaaaaatc tgacacagtt caacaccttt 3540
   tgtgataaaa acactcaaca aactaggaaa agaaggaaac aactttaaca catcatatgc 3600
   tcactgatga aaatctacaa gttctttata aaagatcagg aacaagacaa taatctgcat 3660
   tgttaccact tctattatac gtagtattgg aagttctaat cagagcaaat taggcaagaa 3720
   aaataaataa aaggcatcca aagtggaaag gaagtaaaat aatctctttt tacagatgat 3780
   ataaccttag aattagaaaa tcctaaaaat ttcacatacc aagaaaaagc gtgttaaaat 3840
   taataagtaa attcagcaag ttgactgata caaaatcaac acagaaagct cagttgtgtg 3900
   tctgtgtgtc tcatacacta acaatgaaca atctgaaaag gagattaaga aaacaatttc 3960
   atttacaata gcatcaggaa aaaaaataaa tacttaggaa caaacttaac caaggggttg 4020
   gaattcctgt atactgaaaa ctacaaatat tgccaaaaga aaataaagga gacacaaata 4080
   agtgatatgt ttttaatatg tccacccaaa gtgatcttca gattcaatga aatccctatc 4140
   aaagttataa tggcattttt ctgcaggaat gtaaaaattt atcctaaaat tcatatagaa 4200
   tctctaggta ccctgagggc caaacaattt tgagaaaaaa aaaagaacaa aattggagga 4260
   ctcacacttc cagattacaa gaatatttac aaattacata tttacaaaaa aaattacaaa 4320
   gccacaataa tcaaaacaac gtgggatttg cataaaggca gatatataga ccagtggaat 4380
   agtattgaga gtccagaaat aaacccttag gtatatcatc aaatgacatt tgacaaagtg 4440
   ctggtaccac tcaatgggaa tgggacaatt tgttcaacaa atagagcaaa gaaaactaaa 4500
   catccatgtg caaaagaata aatctggacc cttatattac actatagaca aaattaattc 4560
   aaaatggatt aaagatctaa atgaaagatc taaaactata aaactcctag gagaaaacag 4620
   aggaaaaatt tcatgctaat ttggcaacat tttgtgatgt gacaccaaaa gcagagtcaa 4680
   taaaagcaaa aattagacag atggaaatcc atcatagttt ataacttttg gtcattaaag 4740
   aacagtcaac agagtgaaaa ggcaatctat aaaatggggg aaaaacagaa aatatgtgca 4800
   aatcacagat atctgatagg ggattcatat ccagaataaa taaagaactc ctatatctca 4860
   acaacaaaaa atctaatcca atcaaaaaat gggccaaggg agtgaagata catttctcca 4920
   aagatgttat acaaatggcc aggaagcata tgaaaagatg ttcaatgtca ctaatcatca 4980
   gagaaatgca aatcaaaacc acagtgcaat atcacttcac attcattaga atggcttctg 5040
   tcatgaacaa cagaaaataa caagtgttga tgagtgtgta gagaaattga gacctttata 5100
   taattttggc agaaattcaa aatggtgcaa ccactataaa aaatgatatg gaggtcctca 5160
   aaaaattaaa aatagaacta ccatatgatc cacaatccca cctctgggta catattcaaa 5220
   agaattgaaa gcagggtgtt gaagatatat ttgcacactc tttatagcag cactgttcac 5280
   aatagccaag agatgaaagt aacccaaagg ttcatgaagc aatgaataaa caaaatatat 5340
   tatgtacata gagtaaaata ctgtgcagct ttaaagagaa aggaaatctt atactatgct 5400
   acaacatgaa tggaacttta gggcattata gtaagtaaaa taagccagtt ttttttaaag 5460
   gacaaataaa cactatacga ttctacttaa gtatttaatg ttgtcaaatt tataaatata 5520
   gaatgtagaa tagtggttac cctgagctgg gggaaagggg caaaggggaa ttgttatttt 5580
   aatgggtata gtttcagttc tgcaaaatga aaaggttctg gaaatctgtt tcacaatgtt 5640
   gtaaatataa ttactctgaa attgtacact taaaaatggt taagatgaca aatagagttg 5700
   tgatgtcttc ttttgttatt atatagaaaa actttttcat atgataatag tctttgtttt 5760
   taagctgact ttgctgatat taatataatc cttccatttt tctttaaaat gctatatgct 5820
   ttcacataat tttgctttac gttgatgtat ttatacataa ggtgggtttc ttatagatac 5880
   cacgttgtgt gtctttttta tctaagttga tagacttgcc ttttgttagg gtatttaaat 5940
   aatttatatt taatgtaatt attgatatag ttgagtgtgt tgatttttgt tttctatttg 6000
   ctccatctgt tgttggttct cattattcct ctgtttctac cttcttttgt actaattatt 6060
   atattttatt atttttcatc tcaactgttg gcttattagc cacattgctt ttaaaatttt 6120
   taatgattgc tctagggttt ataataaaca aaatgttagc attttctacc atcaaatatt 6180
   tttacactat tcatgtatac ttcaatttct ttcttcccat cctttgaact atatcttcat 6240
   acattttact ctacatttgt tataactcag tgctttgaaa gtcaattatt tttgtctttg 6300
   acagtcaatg atttttaaag agtttaacag tgaaaaaaaa tggctttcat ctttttccat 6360
   tagatttcat actccttctg cctgaagaat ttcttttaat agaccttgta ctgcgggtct 6420
   caggcaagaa attctctcag cctttgttgg tttgaaaaac tgcttattac acctttgttt 6480
   ttgaaagata ttttcactag gtatagaagt ctgggttgac agttctcatt gtttgtcaca 6540
   gcatttttaa gatgcccatt caattgtctt gtcttgtata attttggatt agtctggtgt 6600
   atttcttacc tttgttcctc tctgtgcaat gcttcaacca tcccacttca ggctgccttt 6660
   aagatgtttt cttttccctt aatctttagt ttttagctgg ttgacagtga cgcatctaag 6720
   tgtagtgtat gaggttgctt ttattgtcac tgttgttg 6758
<210> 348
   <211> 501
   <212> DNA
   <213> Homo sapiens
<400> 348
   gaccatgtta tgacatttta gtgcttgcta agcagtaaat actgacttac tttcctgcta 60
   cactcttcag agcagaaaga gaaatctaca aaaagggcaa tgtagttggg atccaccaca 120
   gccttgagac tgggccatgt ttctacagct tacccacatt ttacccccac tttctctgag 180
   aaacaatgca aactggagaa caaggtcaga gaagttatct tggatggtag aagagaagaa 240
   aggagaagaa rggataagca gaaaatcaaa aagggcataa aaaaattact ggggaaaata 300
   attcttagtc actcaccatt tcttatgttt gtgaaaacag aaacgaggag caagtgttgt 360
   tgtaagaatt gttcttgccc ctccccctcc accacccaca tctgtcaagc tatccctgtt 420
   tcactgtttc ctctgcactc tctattaact tctttgtcct cctcttttct tttcctacag 480
   caaagacttt ttgtcatgtt t 501
<210> 349
   <211> 501
   <212> DNA
   <213> Homo sapiens
<400> 349
   tgacttactt tcctgctaca ctcttcagag cagaaagaga aatctacaaa aagggcaatg 60
   tagttgggat ccaccacagc cttgagactg ggccatgttt ctacagctta cccacatttt 120
   acccccactt tctctgagaa acaatgcaaa ctggagaaca aggtcagaga agttatcttg 180
   gatggtagaa gagaagaaag gagaagaaag gataagcaga aaatcaaaaa gggcataaaa 240
   aaattactgg rgaaaataat tcttagtcac tcaccatttc ttatgtttgt gaaaacagaa 300
   acgaggagca agtgttgttg taagaattgt tcttgcccct ccccctccac cacccacatc 360
   tgtcaagcta tccctgtttc actgtttcct ctgcactctc tattaacttc tttgtcctcc 420
   tcttttcttt tcctacagca aagacttttt gtcatgtttt gtttcttttt ctattgtttc 480
   tttccctttt ctaatccttg a 501
<210> 350
   <211> 1148
   <212> DNA
   <213> Homo sapiens
<400> 350
   tatgagattt aatgttaaga aataaaatgt aggatctaaa acgtaatcta tagcataatc      60
   tcaaaaatgg tttagaaatg acataataat acagacattt gtgggtggta ggattatgca     120
   tatttttata tatttttaaa tatatttttc aaaagcttcc tataaagaat gtaattcttt     180
   cccaattcca aatctagctt aaacataatt ttacaaaaat tattctctca gaatgtaaac     240
   tagtaccacc tctatggaaa acattatgga gatttcctaa agagttaaaa gtagatctac     300
   catttgatcc agcaatctta atactgggta tctacccgga ggaaaagaag tcattgtatg     360
   aaaaagacac ttgtacacat atgtttacag gaccacaatt cacaaatgca aagatgcaga     420
   accaacctaa gtggccastg actaatgaga ggataaagaa gatgtggcat atatatatca     480
   gggactacta ctcagccatt acaaggaaca aaataatgtc ttttgcaaca acttggatag     540
   agctggaggc cattattcta agtaaagtaa ttcaggaatt ggaaaaccaa aaaccgtatg     600
   ttctctctta taagtgggaa ctaagttagg aataagcaaa ggcacacaga gggacatatt     660
   ggactttaga gactcacgag gaggagggta ataggggact agggattaaa agaaaaacta     720
   gacattaggt acaaggtacc ctacttaagt gcactaaaat ctcagaattc accactacgt     780
   aattcaacta agtaacaaga aaccacttgt accccaaaag ctactgaaat aaaaattatt     840
   ctctcaaaaa ttttaagccc taaacttcag ttcctattgt ttatatttac taagaaaaac     900
   aacagaaaac actgttttaa aaatggtgga tttttttaag gttaaaggta tataagacag      960
   ctgcctaagg aaacgcagat acccctgtac cttgttgttg ttgttgtttt tcactttttt   1020
   aaaaaacata gagatgggat ctccttatgc tgcccaggct tgtctcaaac tcctgagctc    1080
   aagcaatcct ctgacctcag actctcaaag ttttgggact acaggcgaca gtcaccatgc    1140
   cagccaat                                                             1148
<210> 351
   <211> 1148
   <212> DNA
   <213> Homo sapiens
<400> 351
   tatgagattt aatgttaaga aataaaatgt aggatctaaa acgtaatcta tagcataatc      60
   tcaaaaatgg tttagaaatg acataataat acagacattt gtgggtggta ggattatgca     120
   tatttttata tatttttaaa tatatttttc aaaagcttcc tataaagaat gtaattcttt     180
   cccaattcca aatctagctt aaacataatt ttacaaaaat tattctctca gaatgtaaac     240
   tagtaccacc tctatggaaa acattatgga gatttcctaa agagttaaaa gtagatctac     300
   catttgatcc agcaatctta atactgggta tctacccgga ggaaaagaag tcattgtatg     360
   aaaaagacac ttgtacacat atgtttacag gaccacaatt cacaaatgca aagatgcaga     420
   accaacctaa gtggccactg actaatgaga ggataaagaa gatgtggcat atatayatca     480
   gggactacta ctcagccatt acaaggaaca aaataatgtc ttttgcaaca acttggatag     540
   agctggaggc cattattcta agtaaagtaa ttcaggaatt ggaaaaccaa aaaccgtatg     600
   ttctctctta taagtgggaa ctaagttagg aataagcaaa ggcacacaga gggacatatt     660
   ggactttaga gactcacgag gaggagggta ataggggact agggattaaa agaaaaacta     720
   gacattaggt acaaggtacc ctacttaagt gcactaaaat ctcagaattc accactacgt     780
   aattcaacta agtaacaaga aaccacttgt accccaaaag ctactgaaat aaaaattatt     840
   ctctcaaaaa ttttaagccc taaacttcag ttcctattgt ttatatttac taagaaaaac     900
   aacagaaaac actgttttaa aaatggtgga tttttttaag gttaaaggta tataagacag      960
   ctgcctaagg aaacgcagat acccctgtac cttgttgttg ttgttgtttt tcactttttt    1020
   aaaaaacata gagatgggat ctccttatgc tgcccaggct tgtctcaaac tcctgagctc    1080
   aagcaatcct ctgacctcag actctcaaag ttttgggact acaggcgaca gtcaccatgc    1140
   cagccaat                                                             1148
<210> 352
   <211> 1148
   <212> DNA
   <213> Homo sapiens
<400> 352
   tatgagattt aatgttaaga aataaaatgt aggatctaaa acgtaatcta tagcataatc      60
   tcaaaaatgg tttagaaatg acataataat acagacattt gtgggtggta ggattatgca     120
   tatttttata tatttttaaa tatatttttc aaaagcttcc tataaagaat gtaattcttt     180
   cccaattcca aatctagctt aaacataatt ttacaaaaat tattctctca gaatgtaaac     240
   tagtaccacc tctatggaaa acattatgga gatttcctaa agagttaaaa gtagatctac     300
   catttgatcc agcaatctta atactgggta tctacccgga ggaaaagaag tcattgtatg     360
   aaaaagacac ttgtacacat atgtttacag gaccacaatt cacaaatgca aagatgcaga     420
   accaacctaa gtggccactg actaatgaga ggataaagaa gatgtggcat atatayatca     480
   gggactacta ctcagccatt acaaggaaca aaataatgtc ttttgcaaca acttggatag     540
   agctggaggc cattattcta agtaaagtaa ttcaggaatt ggaaaaccaa aaaccgtatg     600
   ttctctctta taagtgggaa ctaagttagg aataagcaaa ggcacacaga gggacatatt     660
   ggactttaga gactcacgag gaggagggta ataggggact agggattaaa agaaaaacta     720
   gacattaggt acaaggtacc ctacttaagt gcactaaaat ctcagaattc accactacgt     780
   aattcaacta agtaacaaga aaccacttgt accccaaaag ctactgaaat aaaaattatt     840
   ctctcaaaaa ttttaagccc taaacttcag ttcctattgt ttatatttac taagaaaaac     900
   aacagaaaac actgttttaa aaatggtgga tttttttaag gttaaaggta tataagacag      960
   ctgcctaagg aaacgcagat acccctgtac cttgttgttg ttgttgtttt tcactttttt    1020
   aaaaaacata gagatgggat ctccttatgc tgcccaggct tgtctcaaac tcctgagctc    1080
   aagcaatcct ctgacctcag actctcaaag ttttgggact acaggcgaca gtcaccatgc    1140
   cagccaat                                                             1148
<210> 353
   <211> 1148
   <212> DNA
   <213> Homo sapiens
<400> 353
   tatgagattt aatgttaaga aataaaatgt aggatctaaa acgtaatcta tagcataatc      60
   tcaaaaatgg tttagaaatg acataataat acagacattt gtgggtggta ggattatgca     120
   tatttttata tatttttaaa tatatttttc aaaagcttcc tataaagaat gtaattcttt     180
   cccaattcca aatctagctt aaacataatt ttacaaaaat tattctctca gaatgtaaac     240
   tagtaccacc tctatggaaa acattatgga gatttcctaa agagttaaaa gtagatctac     300
   catttgatcc agcaatctta atactgggta tctacccgga ggaaaagaag tcattgtatg     360
   aaaaagacac ttgtacacat atgtttacag gaccacaatt cacaaatgca aagatgcaga     420
   accaacctaa gtggccactg actaatgaga ggataaagaa gatgtggcat atatatatca     480
   gggactactr ctcagccatt acaaggaaca aaataatgtc ttttgcaaca acttggatag     540
   agctggaggc cattattcta agtaaagtaa ttcaggaatt ggaaaaccaa aaaccgtatg     600
   ttctctctta taagtgggaa ctaagttagg aataagcaaa ggcacacaga gggacatatt     660
   ggactttaga gactcacgag gaggagggta ataggggact agggattaaa agaaaaacta     720
   gacattaggt acaaggtacc ctacttaagt gcactaaaat ctcagaattc accactacgt     780
   aattcaacta agtaacaaga aaccacttgt accccaaaag ctactgaaat aaaaattatt     840
   ctctcaaaaa ttttaagccc taaacttcag ttcctattgt ttatatttac taagaaaaac     900
   aacagaaaac actgttttaa aaatggtgga tttttttaag gttaaaggta tataagacag     960
   ctgcctaagg aaacgcagat acccctgtac cttgttgttg ttgttgtttt tcactttttt    1020
   aaaaaacata gagatgggat ctccttatgc tgcccaggct tgtctcaaac tcctgagctc    1080
   aagcaatcct ctgacctcag actctcaaag ttttgggact acaggcgaca gtcaccatgc    1140
   cagccaat                                                             1148
<210> 354
   <211> 611
   <212> DNA
   <213> Homo sapiens
<400> 354
   caaaacctca accttccaga taagtctaag ggtgagaact tcacacaaga tgaataagaa 60
   ccaatttctt ccagggcgat gttgaacctg gaaatgaaag ccaatctctc ttggaaggcc 120
   tggtttgtag aaatgtcagt ctttgtttca agctgtggga gaatgagaag caagacttta 180
   gggaaagagg aataaaatag atgtgcagaa ataacagagt gagaaagtct tcagggtgtc 240
   gctagcccta attgcaggca tccctgaatc ctagaccttg gattgcaaga gactccttaa 300
   tatcttccca tgtccacatt tgcttcacat agtttgaatg tggcttctat tatatacaga 360
   tacaagattc aaatccaacc tctaygatga ctggtcttgt gaataagcag aagaggcact 420
   aacaatatga cgtgagggat tcagggaaga gcactttctt gagcacatat cttccctggt 480
   ctgccagctg tagtttatga aattccacaa tgaggatgaa atggaatcac catttacaga 540
   gtactctcca gatgtctaac cctaagctag gtaccttcaa aatattatct agtttagata 600
   atcaaccctt t 611
<210> 355
   <211> 601
   <212> DNA
   <213> Homo sapiens
<400> 355
   ttctctagtc caaagggttg attatctaaa ctagataata ttttgaaggt acctagctta 60
   gggttagaca tctggagagt actctgtaaa tggtgattcc atttcatcct cattgtggaa 120
   tttcataaac tacagctggc agaccaggga agatatgtgc tcaagaaagt gctcttccct 180
   gaatccctca cgtcatattg ttagtgcctc ttctgcttat tcacaagacc agtcatcata 240
   gaggttggat ttgaatcttg tatctgtata taatagaagc cacattcaaa ctatgtgaag 300
   yaaatgtgga catgggaaga tattaaggag tctcttgcaa tccaaggtct aggattcagg 360
   gatgcctgca attagggcta gcgacaccct gaagactttc tcactctgtt atttctgcac 420
   atctatttta ttcctctttc cctaaagtct tgcttctcat tctcccacag cttgaaacaa 480
   agactgacat ttctacaaac caggccttcc aagagagatt ggctttcatt tccaggttca 540
   acatcgccct ggaagaaatt ggttcttatt catcttgtgt gaagttctca cccttagact 600
   t 601
<210> 356
   <211> 527
   <212> DNA
   <213> Homo sapiens
<400> 356
   gctctagaat atggcattcc agaagtggga tgctacaaat agtctcattg agagtcaact 60
   tgcacaatgt atcgtcctac ccttacatca atttctgaaa caacttctct ttgcacttcc 120
   cctatagtta catgcataat aaattctgac aactcttatg aagtcatgga ataactttct 180
   tcttatgttt cctatcaatg tcattagccc tttatcttgt ttgagtttcc atcagcaatg 240
   ttttcaagtc ccaagatcat tcatgtatcc acaagcaatg atacgccaga tttggacaaa 300
   taatactgaa tactatctta ttttcactgc catgatcaag gcagtgtgga ttgctgccaa 360
   gtccaagaga agtgaggtca gcagctgcaa gccacctccg tcatttagaa aagcttcatg 420
   atgtagtgtg tcgtttcgat gtgacactgt ctcacagagt taaaatgatg tgmaaggaac 480
   tgttcaatgg aaatttagaa atttctcttt ttctcaattt tagtgta 527
<210> 357
   <211> 601
   <212> DNA
   <213> Homo sapiens
<400> 357
   gaacaagatt ttcctgcttt taaaaatact acattaaagc tgaaaattta ggccaaaatt 60
   ttcaagtggt aatagttaca ggcaattcat ctttctggtc agaaaagggt gttactgcag 120
   ctatttctgc ctgaaactgg gtggcactac tacttttttt tttttttttt taactgagca 180
   gacattttcc ttacactaaa attgagaaaa agagaaattt ctaaatttcc attgaacagt 240
   tccttgcaca tcattttaac tctgtgagac agtgtcacat cgaaacgaca cactacatca 300
   ygaagctttt ctaaatgacg gaggtggctt gcagctgctg acctcacttc tcttggactt 360
   ggcagcaatc cacactgcct tgatcatggc agtgaaaata agatagtatt cagtattatt 420
   tgtccaaatc tggcgtatca ttgcttgtgg atacatgaat gatcttggga cttgaaaaca 480
   ttgctgatgg aaactcaaac aagataaagg gctaatgaca ttgataggaa acataagaag 540
   aaagttattc catgacttca taagagttgt cagaatttat tatgcatgta actacagggg 600
   a 601
<210> 358
   <211> 1001
   <212> DNA
   <213> Homo sapiens
<400> 358
   gcttaatacc tgagtgatgg aatattctgt tcaacaaacc cctctgacat aggtttgcct 60
   atataataaa cctgttcatg tactcctgaa cctaaaagtt taaaaaagat tatgtagaaa 120
   acccaaagga atctataaaa agtctactag agctagagtg attttaacaa gatttcaata 180
   cacaaattca aatgtctttc tatatattaa tgacaatcaa caataaaatt ttaaaacatt 240
   attaaagtat aatgaaaata tcaactgttt agggagaaat gtaacaagaa tggtgaagga 300
   cctatacact aaaaagcttc aatatgttgt tgagattaac tgaagaaggt ctaaatagat 360
   ttttttttca tgtctcggaa gacttaatat gtgaagatac caattcttcc ccaaatgatc 420
   aacaggtgaa atgcaatccc aatcaaaatc ccagcaatta ttttaagggg gaaattggca 480
   atctgattct aaaattcata yggaaaaaaa caatggagtt agaataacta aaacaagtcc 540
   gaaaaagaaa aagaaatgga ggactaatgc tacctgattt caagtcttat cgtataaatc 600
   tacatcaata aaggacaagt tggtattggg ttaaagatag ataaatacat cagtggaata 660
   gaatattgaa tccagaataa atccacacat atatggataa aaataccaga caattcagtg 720
   gagatggttt tgtttttaca acaaatgtta ctggaacaaa ttgatatatg tattagtcag 780
   atatggctgc cataacaaag aaccacaaac aggtggttta aataatggaa ataaatttcc 840
   tcagaattct ggagtatgga agcccaagat caagttgctg ggaggattcg tttcttctga 900
   gtgtctcttt ttttgatgac agatgactat cttttaccaa tgtcttcact tggttttccc 960
   tctgtgtgtg cctaggtcct attctccaat tcctataagg a 1001
<210> 359
   <211> 1001
   <212> DNA
   <213> Homo sapiens
<400> 359
   ctaaaagttt aaaaaagatt atgtagaaaa cccaaaggaa tctataaaaa gtctactaga 60
   gctagagtga ttttaacaag atttcaatac acaaattcaa atgtctttct atatattaat 120
   gacaatcaac aataaaattt taaaacatta ttaaagtata atgaaaatat caactgttta 180
   gggagaaatg taacaagaat ggtgaaggac ctatacacta aaaagcttca atatgttgtt 240
   gagattaact gaagaaggtc taaatagatt tttttttcat gtctcggaag acttaatatg 300
   tgaagatacc aattcttccc caaatgatca acaggtgaaa tgcaatccca atcaaaatcc 360
   cagcaattat tttaaggggg aaattggcaa tctgattcta aaattcatat ggaaaaaaac 420
   aatggagtta gaataactaa aacaagtccg aaaaagaaaa agaaatggag gactaatgct 480
   acctgatttc aagtcttatc rtataaatct acatcaataa aggacaagtt ggtattgggt 540
   taaagataga taaatacatc agtggaatag aatattgaat ccagaataaa tccacacata 600
   tatggataaa aataccagac aattcagtgg agatggtttt gtttttacaa caaatgttac 660
   tggaacaaat tgatatatgt attagtcaga tatggctgcc ataacaaaga accacaaaca 720
   ggtggtttaa ataatggaaa taaatttcct cagaattctg gagtatggaa gcccaagatc 780
   aagttgctgg gaggattcgt ttcttctgag tgtctctttt tttgatgaca gatgactatc 840
   ttttaccaat gtcttcactt ggttttccct ctgtgtgtgc ctaggtccta ttctccaatt 900
   cctataagga aaccagtcat attggattag ggcccactct aatggcccca ttttacttgc 960
   attatctctt taaagacact atctccagat gtagccacac t 1001
<210> 360
   <211> 1058
   <212> DNA
   <213> Homo sapiens
<400> 360
   catgattagc tatgctactt tccactgctc ttagtatact gagaggcagc ataagtaaaa 60
   ctaaaatatc tgaagatagc aatagactat ttaaagtaga agaagtatgc tatttttgtt 120
   ttgttttcat ttcgaaggaa atatgcaaag gtttattgag tatttcagct tctcttacag 180
   taggtttttt ttggattctt tctgtgtttg tctatgttga taaaacattg aaatgccata 240
   tagctcaaag gtcattcact taagaaatct aagtactgat aacatcttag ccccgattct 300
   tcataggcat tgttaagcct attataattt tggtwcagag agaaggtaaa ctatattcca 360
   gacaggcata taaagcaatt tctcctataa ttggagttca cgaaaaattc acatatttct 420
   ttttaatagt aactctcaca gcaagaacat atgtttgtaa ataatacatc acagaatctt 480
   attggcagac aaggaaattc ctaaaatatt ttttactgcc acatcaatta agatatataa 540
   aataccttat atagaagatg tttgcaccca ggccaaacaa atcaaacaag aatagaagca 600
   ctgacagtct tatttcaaaa ttggtttaac ttgtatttac aggatattgt agtaccttat 660
   aaagttgatt gctgattggc cgtcttttac agaattctgt cagattgtta ttatttcttg 720
   taaagattga ttcaaacaaa taaaaattgt caggattgga tatgtcctat agtgaggtgt 780
   agttatgtca catgagattt ttaattacaa agaaatggaa aataaaatga gaatagaatt 840
   gagactcccc tgtcacctca caaatatgtt gaaatacaat gaaatttcca aagatgttaa 900
   agcatataaa gttgaataat tcttattatg tattaaactt acagaaattt aatttcttta 960
   ctttataaga ggtagtgaaa atataaaatt aattatgaag acagagtagt cttagtcaga 1020
   catggcccta taaagcatat tcccattcgt tacatcaa 1058
<210> 361
   <211> 1058
   <212> DNA
   <213> Homo sapiens
<400> 361
   catgattagc tatgctactt tccactgctc ttagtatact gagaggcagc ataagtaaaa      60
   ctaaaatatc tgaagatagc aatagactat ttaaagtaga agaagtatgc tatttttgtt     120
   ttgttttcat ttcgaaggaa atatgcaaag gtttattgag tatttcagct tctcttacag     180
   taggtttttt ttggattctt tctgtgtttg tctatgttga taaaacattg aaatgccaya     240
   tagctcaaag gtcattcact taagaaatct aagtactgat aacatcttag ccccgattct     300
   tcataggcat tgttaagcct attataattt tggtacagag agaaggtaaa ctatattcca     360
   gacaggcata taaagcaatt tctcctataa ttggagttca cgaaaaattc acatatttct     420
   ttttaatagt aactctcaca gcaagaacat atgtttgtaa ataatacatc acagaatctt     480
   attggcagac aaggaaattc ctaaaatatt ttttactgcc acatcaatta agatatataa     540
   aataccttat atagaagatg tttgcaccca ggccaaacaa atcaaacaag aatagaagca     600
   ctgacagtct tatttcaaaa ttggtttaac ttgtatttac aggatattgt agtaccttat     660
   aaagttgatt gctgattggc cgtcttttac agaattctgt cagattgtta ttatttcttg     720
   taaagattga ttcaaacaaa taaaaattgt caggattgga tatgtcctat agtgaggtgt     780
   agttatgtca catgagattt ttaattacaa agaaatggaa aataaaatga gaatagaatt     840
   gagactcccc tgtcacctca caaatatgtt gaaatacaat gaaatttcca aagatgttaa     900
   agcatataaa gttgaataat tcttattatg tattaaactt acagaaattt aatttcttta     960
   ctttataaga ggtagtgaaa atataaaatt aattatgaag acagagtagt cttagtcaga    1020
   catggcccta taaagcatat tcccattcgt tacatcaa                            1058
<210> 362
   <211> 956
   <212> DNA
   <213> Homo sapiens
<400> 362
   aaaacaagga acaaacaaac aaaaatgtta caaccgaaca acagactttt gagtcatgtt 60
   tcaggccaag aggtgatgag ttactgtagt tgcttgagct ggttggtgaa atattacctg 120
   gcaacaaaac tgaaatagaa ggtggcttag taaaatgcag attcagaatg agtgccttaa 180
   ggttaaggca tataagacca aactgatttt ctttttcacg aggtcttcag gtaaggccat 240
   tgtagaagat accttgtttg cgaacttcag taaattactt cacttgtctc atattttcat 300
   tttcaggatg gaggcttgag attgaattgt agtgcaatta ggtaaatttt tacccatttt 360
   aaatataata ttaaaatatt aattataaat taccttattt gaatctggaa taatatttat 420
   tgcagggcat ataatctaag ctgtaaacgt cctgtyagaa gacaacatat tcatcttgct 480
   aaggtataag ctatatgact ggcactgtgc tcaactcaga gtcattgaat gaacagtatt 540
   tatttaatct atgaatgaga gcacttcaag tatacagaaa gatatctcaa aagattcagc 600
   cttacattgc tcataacttc aatgacttag atgaaaacct cctgaacatt tttatcagtt 660
   gtataggtac cccaaatcat aagggaatgt ttatcaatta gatgatgaaa tggggatgca 720
   actacatcat ggcaggctaa agcaatagaa tgactttgac aagaggaaat tacatagagg 780
   cacctgagtc tcctaaacca atttcaaagg tatgagaggg gggtgatata aataaatagt 840
   tgatagatga aaaaactcag aagttatagt tgacagcaat tttaatataa tatgaaaaat 900
   gtggttggac ttttagggaa aaaaacctaa taaaatctaa tggaaattag tggtcc 956
<210> 363
   <211> 956
   <212> DNA
   <213> Homo sapiens
<400> 363
   caaccgaaca acagactttt gagtcatgtt tcaggccaag aggtgatgag ttactgtagt 60
   tgcttgagct ggttggtgaa atattacctg gcaacaaaac tgaaatagaa ggtggcttag 120
   taaaatgcag attcagaatg agtgccttaa ggttaaggca tataagacca aactgatttt 180
   ctttttcacg aggtcttcag gtaaggccat tgtagaagat accttgtttg cgaacttcag 240
   taaattactt cacttgtctc atattttcat tttcaggatg gaggcttgag attgaattgt 300
   agtgcaatta ggtaaatttt tacccatttt aaatataata ttaaaatatt aattataaat 360
   taccttattt gaatctggaa taatatttat tgcagggcat ataatctaag ctgtaaacgt 420
   cctgtcagaa gacaacatat tcatcttgct aaggtrtaag ctatatgact ggcactgtgc 480
   tcaactcaga gtcattgaat gaacagtatt tatttaatct atgaatgaga gcacttcaag 540
   tatacagaaa gatatctcaa aagattcagc cttacattgc tcataacttc aatgacttag 600
   atgaaaacct cctgaacatt tttatcagtt gtataggtac cccaaatcat aagggaatgt 660
   ttatcaatta gatgatgaaa tggggatgca actacatcat ggcaggctaa agcaatagaa 720
   tgactttgac aagaggaaat tacatagagg cacctgagtc tcctaaacca atttcaaagg 780
   tatgagaggg gggtgatata aataaatagt tgatagatga aaaaactcag aagttatagt 840
   tgacagcaat tttaatataa tatgaaaaat gtggttggac ttttagggaa aaaaacctaa 900
   taaaatctaa tggaaattag tggtccactc atttctccac ctaggatgtt aaaaat 956
<210> 364
   <211> 601
   <212> DNA
   <213> Homo sapiens
<400> 364
   gtaaaacaca tagatcgctg tatccttgtt cagtaagcta caacatactc gtatctcctg 60
   aaatcctggg cttaaatcga ggtctcaaag gctttgtttt gttttgttgt atggttgtat 120
   ggtgagtgtg tgtgtgtgtg tgtgtgtgtg tgtttattct cctgaaattc tcctcctcac 180
   ttgacttaag ctaaaagata aacgtcctct tcctttcagc cacagatggt gatggataaa 240
   ttgaatgtca ttcacattat tcccttaaaa taaactctct ccctcccctc tcccgtctca 300
   wccttgtccc tttctttata taatgggtaa tgcgttaatg tcagcagaat agttttgggg 360
   ccataatggc aagtatcacg tggatggttt agcattgttt ttagaatgct gtgaatttgg 420
   gtatatgtga gttttgggga aagttttgca actatatgtt tgttaattaa atgaggacta 480
   taaagtaata taaaattatg tttctggaac atattttgga agctataaag tcatctgtat 540
   ttattatcca cagacataat gtcattgttc aggtcctgca accttcttat aatcaacata 600
   c 601
<210> 365
   <211> 601
   <212> DNA
   <213> Homo sapiens
<400> 365
   agtaagctac aacatactcg tatctcctga aatcctgggc ttaaatcgag gtctcaaagg 60
   ctttgttttg ttttgttgta tggttgtatg gtgagtgtgt gtgtgtgtgt gtgtgtgtgt 120
   gtttattctc ctgaaattct cctcctcact tgacttaagc taaaagataa acgtcctctt 180
   cctttcagcc acagatggtg atggataaat tgaatgtcat tcacattatt cccttaaaat 240
   aaactctctc cctcccctct cccgtctcat ccttgtccct ttctttatat aatgggtaat 300
   kcgttaatgt cagcagaata gttttggggc cataatggca agtatcacgt ggatggttta 360
   gcattgtttt tagaatgctg tgaatttggg tatatgtgag ttttggggaa agttttgcaa 420
   ctatatgttt gttaattaaa tgaggactat aaagtaatat aaaattatgt ttctggaaca 480
   tattttggaa gctataaagt catctgtatt tattatccac agacataatg tcattgttca 540
   ggtcctgcaa ccttcttata atcaacatac gtgggcccag ggattttatg tatcttcgcc 600
   t 601
<210> 366
   <211> 1079
   <212> DNA
   <213> Homo sapiens
<400> 366
   gaatttatgg tctgatggag aagggaatca ttaaagttct atgtagtgag atatccccaa      60
   ggggtgtatt aggcttacca ccactggaat ctggatagat gaagacagag tggcagggaa     120
   gtcgtattaa ggttctgttt ctgctgggag ccacaggtcc tcaggaagca acaagtactg     180
   ggcagattga tactgtagct rggctctagc tctatacctc tagaataaag gttacaaact     240
   agcaacttga aagctaaacc tggcccacag atatgtttta tttggctctt acactgtttt     300
   aaaaaatatt accaacattt aaaactggga agttttatga aaaaacccag acttctggat     360
   tctgttgaaa aaaaaaatca gaagatctgg caatactgag ctgacattcc tatatgacaa     420
   caattggctg gatctatgca gcttctctcc aaaaagcaaa gaatgtgttc ttgcttaaca     480
   cagtccccac cactccctca tattctccaa tcctggacct gagcgtcatt tgctatgtat     540
   cgccatttgc catgaagttt tacactctac agaaatataa tttttttgta gaagactatg     600
   ctttaatcaa gatcaggata atataaagtg agatctgaaa gtggaaaaaa gataaatgtc     660
   caacaatgat agactggatt aagaaaatgt ggcacatata caccgtggag tactatgcag     720
   ccaaaaaaaa cgatgagttc atgtcctttg tagggacatg gatgaagctg gaaaccacca     780
   ttctcagcaa actatcgcaa ggacaaaaaa ccaaacgccg catgttctca ctcataggtg     840
   ggaattgaac aatgagaaca cttgggcaca ggaaggggaa catcacacac cgggccctgt     900
   tgtggggtgg ggggaggagg gagggatagc atttggagat atacctaatg ttaaatgact     960
   agtttctggg tgcagcacac catcatggca catgtataca tatgtaacta acctgcacat    1020
   tgtgcacatg taccctaaaa cttaaagtat aatttttaaa aaaagatatt ttcttatct     1079
<210> 367
   <211> 501
   <212> DNA
   <213> Homo sapiens
<400> 367
   ataaattttc tcttccctca agaatttatg gtctgatgga gaagggaatc attaaagttc      60
   tatgtagtga gatatcccca aggggtgtat taggcttacc accactggaa tctggataga     120
   tgaagacaga gtggcaggga agtcgtatta aggttctgtt tctgctggga gccacaggtc     180
   ctcaggaagc aacaagtact gggcagattg atactgtagc tgggctctag ctctatacct     240
   ctagaataaa kgttacaaac tagcaacttg aaagctaaac ctggcccaca gatatgtttt     300
   atttggctct tacactgttt taaaaaatat taccaacatt taaaactggg aagttttatg     360
   aaaaaaccca gacttctgga ttctgttgaa aaaaaaaatc agaagatctg gcaatactga     420
   gctgacattc ctatatgaca acaattggct ggatctatgc agcttctctc caaaaagcaa     480
   agaatgtgtt cttgcttaac a                                               501
<210> 368
   <211> 1001
   <212> DNA
   <213> Homo sapiens
<400> 368
   tgaagaagcc gcctggcttc ttgtttcttc tcatagcaaa atgcaatgag aaagagataa      60
   tttgagaaaa gaaccgttta aacaaaaaga aaccaagaca taatgatttt ggaaattctc     120
   agtttattca gactgcaaaa gatattaaaa taaagaaact cagtaacagg gatagataat     180
   ctaaagaaaa agcctaggac acggctgtag taaccttctg tttttatacc tcagcaattt     240
   gctaatgcct caaaaagatc aaaagtactc aaatataaag ggctctttga agagattaga     300
   tttcctcaat caaaccaaag agcatcgagg aagcttaagg ttactgtccc tcacatatct     360
   cagcagaagg caaaaataga agactgatta tctaagaaag atctctgaaa gagtctcata     420
   ttatggagtg aacccctgtg gcatacatgg gagacccact tggttcttga gaattttata    480
   tcaggagaaa cactgtcagt ytgtattgaa aggaacagag aaaatacgaa attaaagaag     540
   actattaaac ctccaaaatt ctggcaggaa agaagcttac acagctactc agttgcaaag     600
   atctgccact tttcatatac atgaaaggac tcagaggagg aagccacagg tttagaagga     660
   aaagctaaaa gcaacatcgt attagtcttg gatctaggaa cctaatttct ctagcagaat     720
   ctagaaatgg cttgggacaa gtgattgttt ttttacctag gattttctcc ctcttgaaaa     780
   caggactgtc tgtaactatt atcctatgcc tgccctacca tcatatttca gaaacaggta     840
   acttatgttt tcactttcaa agattcacaa taaagagaaa ttgtacctca gaatggatta     900
   taccagagct ttcctcatgc ataaattaaa taatttaggt tatgtgattt gaagcttttg     960
   agtgggtgag gtgacatttt ggatgctgag ttggtgccgt a                        1001
<210> 369
   <211> 1001
   <212> DNA
   <213> Homo sapiens
<400> 369
   tcttctcata gcaaaatgca atgagaaaga gataatttga gaaaagaacc gtttaaacaa 60
   aaagaaacca agacataatg attttggaaa ttctcagttt attcagactg caaaagatat 120
   taaaataaag aaactcagta acagggatag ataatctaaa gaaaaagcct aggacacggc 180
   tgtagtaacc ttctgttttt atacctcagc aatttgctaa tgcctcaaaa agatcaaaag 240
   tactcaaata taaagggctc tttgaagaga ttagatttcc tcaatcaaac caaagagcat 300
   cgaggaagct taaggttact gtccctcaca tatctcagca gaaggcaaaa atagaagact 360
   gattatctaa gaaagatctc tgaaagagtc tcatattatg gagtgaaccc ctgtggcata 420
   catgggagac ccacttggtt cttgagaatt ttatatcagg agaaacactg tcagtctgta 480
   ttgaaaggaa cagagaaaat rcgaaattaa agaagactat taaacctcca aaattctggc 540
   aggaaagaag cttacacagc tactcagttg caaagatctg ccacttttca tatacatgaa 600
   aggactcaga ggaggaagcc acaggtttag aaggaaaagc taaaagcaac atcgtattag 660
   tcttggatct aggaacctaa tttctctagc agaatctaga aatggcttgg gacaagtgat 720
   tgttttttta cctaggattt tctccctctt gaaaacagga ctgtctgtaa ctattatcct 780
   atgcctgccc taccatcata tttcagaaac aggtaactta tgttttcact ttcaaagatt 840
   cacaataaag agaaattgta cctcagaatg gattatacca gagctttcct catgcataaa 900
   ttaaataatt taggttatgt gatttgaagc ttttgagtgg gtgaggtgac attttggatg 960
   ctgagttggt gccgtagtga gtccagaatt ctgcggaact t 1001
<210> 370
   <211> 601
   <212> DNA
   <213> Homo sapiens
<400> 370
   ctctagactc ctcctgtatt ttaatttagc cactttttta gggcctacaa ttttagatct 60
   ccacagggct cttgaaactt cttgaacctc atcagtaaca tgtccattag tggcatgacc 120
   caagagttct agaacatcta ttcagcaagt gtgtatctgg taagtgaata ttccttctat 180
   gtgttccctt ttgcatcaaa ctacacactg tcattcctcc tttatctcca aaagcttgaa 240
   aattcctcac ttgtatctca ttctttctct cttagaaaac tgatcacctc tgatgaatta 300
   raacggaatg accaagcttt gggagaggca aaagaatctc ggtgttaaag actcagagtt 360
   taagaagcaa caaaaagatt atacagatgt gaatatgtga ccttcctcca ccagggcatg 420
   ttgccttgga gtaagataat ctaagcacac acttcatagc ctgagaacaa ttttggaagt 480
   ctttgcttta tggatattta cataaagcaa atatggatat ttacctaaag gctggaccaa 540
   ggcctaattc ctctagagcc ccttgatcat gaacaccatt cctgtcatga ttcttaaggt 600
   c 601
<210> 371
   <211> 601
   <212> DNA
   <213> Homo sapiens
<400> 371
   acaagctcca gccatggacg caattccttc tagaagcaaa atttatctct agactcctcc 60
   tgtattttaa tttagccact tttttagggc ctacaatttt agatctccac agggctcttg 120
   aaacttcttg aacctcatca gtaacatgtc cattagtggc atgacccaag agttctagaa 180
   catctattca gcaagtgtgt atctggtaag tgaatattcc ttctatgtgt tcccttttgc 240
   atcaaactac acactgtcat tcctccttta tctccaaaag cttgaaaatt cctcacttgt 300
   rtctcattct ttctctctta gaaaactgat cacctctgat gaattagaac ggaatgacca 360
   agctttggga gaggcaaaag aatctcggtg ttaaagactc agagtttaag aagcaacaaa 420
   aagattatac agatgtgaat atgtgacctt cctccaccag ggcatgttgc cttggagtaa 480
   gataatctaa gcacacactt catagcctga gaacaatttt ggaagtcttt gctttatgga 540
   tatttacata aagcaaatat ggatatttac ctaaaggctg gaccaaggcc taattcctct 600
   a 601
<210> 372
   <211> 701
   <212> DNA
   <213> Homo sapiens
<400> 372
   gaagatgcac tctaatgttt tttcccagaa gctctgtagg tttagctttt acctttctgg 60
   gtttgttttg ttttgttttt tgagatggag tcccactcgt gtcacccagg ctggagtaca 120
   atggtgcaat ctcggttcac tgcaacctcc acctcccggg ttcaagcaat tcccctgtct 180
   ccacctctcg agtagctggg atgggaggcg cctgccacca tacctggcta attttcatat 240
   ttttagtaaa gatagggttt caccatgtta gccaggctgg tctcgaactc ctgacctcaa 300
   gtgatccacc cgcctcagct tcccaaagtg ctgggattac aggcgtgagc cactgcgccc 360
   agccctagct ttttggtcta tgattcctcc caaattaatt tctgtgaacc attaccttaa 420
   gatgttgaga tttaatgtcc agaatctcat ttgttcacct ttgaaaatta agaaaccctg 480
   gcacagtgtt gactggagcc wcttacctta atagaaaata aagctcacat atatccataa 540
   tgaaaagcag agaccagcac aaccatagtc acctgacagt tttaaaatcc aaggccagga 600
   tcttctcaac tcaggcccac tcacttactc cacaacatac ttcttctttc ctcagcatct 660
   actacttgtg ctgggacctt ggtcttccca ttgttcatgt c 701
<210> 373
   <211> 701
   <212> DNA
   <213> Homo sapiens
<400> 373
   agatggagtc ccactcgtgt cacccaggct ggagtacaat ggtgcaatct cggttcactg 60
   caacctccac ctcccgggtt caagcaattc ccctgtctcc acctctcgag tagctgggat 120
   gggaggcgcc tgccaccata cctggctaat tttcatattt ttagtaaaga tagggtttca 180
   ccatgttagc caggctggtc tcgaactcct gacctcaagt gatccacccg cctcagcttc 240
   ccaaagtgct gggattacag gcgtgagcca ctgcgcccag ccctagcttt ttggtctatg 300
   attcctccca aattaatttc tgtgaaccat taccttaaga tgttgagatt taatgtccag 360
   aatctcattt gttcaccttt gaaaattaag aaaccctggc acagtgttga ctggagccac 420
   ttaccttaat agaaaataaa gctcacatat atccataatg aaaagcagag accagcacaa 480
   ccatagtcac ctgacagttt waaaatccaa ggccaggatc ttctcaactc aggcccactc 540
   acttactcca caacatactt cttctttcct cagcatctac tacttgtgct gggaccttgg 600
   tcttcccatt gttcatgtca ttcttttcct cacagttccc attcttttct ccctgaaata 660
   aagaaatttc aaaatatacc atgtttcatg aaaaagacaa a 701
<210> 374
   <211> 701
   <212> DNA
   <213> Homo sapiens
<400> 374
   gatttccacc ctcaggtgat ggggatggtt gaacatccaa cacctgaaac aggacagacg      60
   atattgacag tacttgttag ttgcatataa tcacagacca gtggaaacag atgaaccaca     120
   cagggccaca gcggggtttc actggggaac agagtgaaca atcaggaggt gtgggaggca     180
   ggtttagtag tttaaagagg ttgaggtgtc cccctggatc ccatgggagg atcacattgg     240
   ctcatttgaa ttatcatacg gactggcagg gaactgaaat cttctactca gggataagca     300
   gaaactgtcc ctggtttcct tgataaaaag ggttgtttga taggggacct tatccatggg     360
   aggaaagtga ggagggaaat ttgtggctaa gccattcaag gccctcccag ttttactaga     420
   tgtcaaggca gcacacgtaa tattgggact taattttagc cacataacta ataaatttgt     480
   aagtatgtgc aacggctcac rcttgcttcc agaatggcac ctaaaaaaca gatttacctc     540
   tccccaaatt cagatatgga attaaatgta atgtcaggaa aattgtctaa gagttggaaa     600
   tgggaaaaaa atgttctttt ggtggagtta tggactccag aggttatcag attctattga     660
   ataacgtact tttgattgta tttgtaacaa ttaggctatt t                         701
<210> 375
   <211> 1001
   <212> DNA
   <213> Homo sapiens
<400> 375
   gcatataatc acagaccagt ggaaacagat gaaccacaca gggccacagc ggggtttcac 60
   tggggaacag agtgaacaat caggaggtgt gggaggcagg tttagtagtt taaagaggtt 120
   gaggtgtccc cctggatccc atgggaggat cacattggct catttgaatt atcatacgga 180
   ctggcaggga actgaaatct tctactcagg gataagcaga aactgtccct ggtttccttg 240
   ataaaaaggg ttgtttgata ggggacctta tccatgggag gaaagtgagg agggaaattt 300
   gtggctaagc cattcaaggc cctcccagtt ttactagatg tcaaggcagc acacgtaata 360
   ttgggactta attttagcca cataactaat aaatttgtaa gtatgtgcaa cggctcacac 420
   ttgcttccag aatggcacct aaaaaacaga tttacctctc cccaaattca gatatggaat 480
   taaatgtaat gtcaggaaaa ytgtctaaga gttggaaatg ggaaaaaaat gttcttttgg 540
   tggagttatg gactccagag gttatcagat tctattgaat aacgtacttt tgattgtatt 600
   tgtaacaatt aggctatttg tgaactcggt aggggtagaa atcgagttgt agaaaatgga 660
   tggtaatgca agtgattttt gaccatatca atgcaaatga attctgttgg tagaaatatt 720
   catttccaca ctgtagatga ccctaaacat atgtcattac attatatttt attgccttat 780
   agactattaa ccaattttga atcatacagt agcaaattta tttcagcatt cttgtgtgta 840
   tgtgtttata tatacacgtg catatgtatt taagatatat aattgtatat tcttcaaatt 900
   cttctttgaa caggtttgaa cctcttatta gtttcctcat taaggaattt aataagacct 960
   ttaatgcatg tttgtatttt catgagagtc attattttac c 1001
<210> 376
   <211> 695
   <212> DNA
   <213> Homo sapiens
<400> 376
   tgctccttca ttagtgcaat ggaacagcaa atcaggatac tttcacagtt ctcttaagtg 60
   agcctagaag tggggagctg cttgttcaca aacttgaagc ctgaatatgt taatattctt 120
   tcagtggccg gacgcggtgg ctcatgcctg taatcccaac actttgggag gccgaggtag 180
   gcagatcaac ctgaagtcag gagttcgagg ccagcctggc caacatggtg aaaccccacc 240
   tgttggtctg tactaaaaat agaaaaatta gctgggcatg gtggcgcatg cctgtaatcc 300
   cagctactca ggaggctgtg gcagaagaat cgcctgcacc tgggaggcag aggttgcttt 360
   gagttgatat cgtgtcactg cactccagcc tgggcaacag agtgagatcc tttcagaaac 420
   ctgctgtctg tatttggata caattaaaaa aaaaaaaaag atgagacagg caggtgcgaa 480
   agaaataaaa gtcamaactg atccagttgg gaaactcaga attgacagtt acgtgtcctt 540
   tcatttattg atattttgag attcacaggg gtttaaactt tatttttcca agactgaata 600
   gttcccacct cccttccata tataaaattt gagtagctgg ggagatttaa aagaggctcc 660
   ccataaactc agaagttaaa agagacaagg gtccc 695
<210> 377
   <211> 601
   <212> DNA
   <213> Homo sapiens
<400> 377
   aaccccacct gttggtctgt actaaaaata gaaaaattag ctgggcatgg tggcgcatgc 60
   ctgtaatccc agctactcag gaggctgtgg cagaagaatc gcctgcacct gggaggcaga 120
   ggttgctttg agttgatatc gtgtcactgc actccagcct gggcaacaga gtgagatcct 180
   ttcagaaacc tgctgtctgt atttggatac aattaaaaaa aaaaaaaaga tgagacaggc 240
   aggtgcgaaa gaaataaaag tcacaactga tccagttggg aaactcagaa ttgacagtta 300
   sgtgtccttt catttattga tattttgaga ttcacagggg tttaaacttt attcttccaa 360
   gactgaatag ttcccacctc ccttccatat ataaaatttg agtagctggg gagatttaaa 420
   agaggctccc cataaactca gaagttaaaa gagacaaggg tcccagtaaa tacaaaatga 480
   ttggggttga ggaggcagat tttctgtcct cagtgaagtt tgttggttgg ttggttggtt 540
   ggttggttaa ttggttggtt tttgagtcag ggtctcactt tgtcacccaa gctggagtgc 600
   a 601
<210> 378
   <211> 663
   <212> DNA
   <213> Homo sapiens
<400> 378
   tgtagcaaca ggagggatga gacccaaagg tctgaaaagc cagtatttta agaagtcttg      60
   gaaaatgtgg aggttgaaaa atctaacagg agtgcttgct tcagcagcaa tttagagtag     120
   attagcatgg cctctgcgcc aggatgacat gcacattcct aaaagtgttc cgtgttttaa     180
   aaaaaagaga gagacagaat ctaaggggat gtgtacattt gctagagcta ctataacaaa     240
   gtaccagagg cagggtcact tcaacaacag aaatttattt ctcacagttc tggaggctag     300
   acgtccaaga ttaaggtgtt gactgggttg aattcagccc ataacaggaa ataaggagtt     360
   aaataaagca cttgcttcta ttgtttgtac ctaaacttaa cagaayacag taagtaacaa     420
   gtcattggga tgcagaaaag aaaaaagaga gtgaaggaag gagagaaggt gaagggagaa     480
   tggaagagag gaagggaggg aggaaagaaa agtttgatga atgattgcag tctaaactgg     540
   ttcaaacaag agatcttgtt taattaagga attcatccca tctctgccta ttaggaggag     600
   gaaaaagtct aaaatagaag atggtgaaag ttggatgacc ccaggcatta aggccattca     660
   tct                                                                   663
<210> 379
   <211> 662
   <212> DNA
   <213> Homo sapiens
<400> 379
   ttaagaagtc ttggaaaatg tggaggttga aaaatctaac aggagtgctt gcttcagcag      60
   caatttagag tagattagca tggcctctgc gccaggatga catgcacatt cctaaaagtg     120
   ttccgtgttt taaaaaaaag agagagacag aatctaaggg gatgtgtaca tttgctagag     180
   ctactataac aaagtaccag aggcagggtc acttcaacaa cagaaattta tttctcacag     240
   ttctggaggc tagacgtcca agattaaggt gttgactggg ttgaattcag cccataacag     300
   gaaataagga gttaaataaa gcacttgctt ctattgtttg tacctaaact taacagaaca     360
   cagtaagtaa caagtcattg ggatgcagaa aagaaaaaag agagtgaagg aaggagaraa     420
   ggtgaaggga gaatggaaga gaggaaggga gggaggaaag aaaagtttga tgaatgattg     480
   cagtctaaac tggttcaaac aagagatctt gtttaattaa ggaattcatc ccatctctgc     540
   ctattaggag gaggaaaaag tctaaaatag aagatggtga aagttggatg accccaggca     600
   ttaaggccat tcatctttaa ctgttatgct tggatcatgc aaatgtgtct ggtagctaca     660
   ag                                                                    662
<210> 380
   <211> 615
   <212> DNA
   <213> Homo sapiens
<400> 380
   ttccatacat tccttccaca ccattgccct taacctttca aattcctgct taaaactaat     60
   cccattttta tggctgacct caccctgtat caaaaactcc gacatccctt tacgacagag    120
   agcacaaact agtggtccaa aatgtcatgg gggtcttctc agagttgttt tttcaatcag    180
   gaaatttcac ataaaaatat ggatttctga tttctctttt aaaaacagaa aaacgagcca    240
   ccagtgggag cactgcaggt atctgtgtga gaccygtact tcacaactcc tgctttccct    300
   ccataaagta gcttgcattt tccacattga ctttgcagtt ctttggtatc tgtattggtt    360
   ttaagataat ttctactata tcacatatct cctcacagta caaagatatc attttctttc    420
   ccttttcttt ttaaaaaatt tgtattttta atttttgtgg gtacacagta gatatttatg    480
   gggcatatga ggtattttat aggcatataa tatgtactag ggtaagtggg gtattcatca    540
   cctcaagcat ttatcctttc tttgtgtaaa atatagcatt ttctgaacac tatgaatact    600
   taagtacaag gatca                                                     615
<210> 381
   <211> 994
   <212> DNA
   <213> Homo sapiens
<400> 381
   tcaaagtgta acaaatttcc tttcctcata aactagcaga cattctatcc cctcattatt 60
   gtaacacatt tctaatatct ttctcaaatt gtcttcctgt attacaatgc actcaccttg 120
   gcttagaatg tctgagacaa gaaaatctat tcaccattcc cacagatgac tccctcactc 180
   tcctcccaag tcttccatac attccttcca caccattgcc cttaaccttt caaattcctg 240
   cttaaaacta atcccatttt tatggctgac ctcaccctgt atcaaaaact ccgacatccc 300
   tttacgacag agagcacaaa ctagtggtcc aaaatgtcat gggggtcttc tcagagttgt 360
   tttttcaatc aggaaatttc acataaaaat atggatttct gatttctctt ttaaaaacag 420
   aaaaacgagc caccagtggg agcactgcag gtatctgtgt gagacctgta cttcacaact 480
   cctgctttcc ctccataaag yagcttgcat tttccacatt gactttgcag ttctttggta 540
   tctgtattgg ttttaagata atttctacta tatcacatat ctcctcacag tacaaagata 600
   tcattttctt tccctttctt ttttaaaaaa tttgtatttt taatttttgt gggtacacag 660
   tagatattta tggggcatat gaggtatttt ataggcatat aatatgtact agggtaagtg 720
   gggtattcat cacctcaagc atttatcctt tctttgtgta aaatatagca ttttctgaac 780
   actatgaata cttaagtaca aggatcaagt cataggattt ggaattgatt tttaaaatat 840
   gttgaccaaa gtgctcttat catcaaactt aacatcacta atgaaggatg aacatcccaa 900
   atctgaaaat ccaaaatcca aaatgctcca taatctaaaa cttgttgagc accaacatga 960
   tgcttaaagg aaatgctcct ggagcatttc agat 994
<210> 382
   <211> 1001
   <212> DNA
   <213> Homo sapiens
<400> 382
   ctatgagaaa tatttttaaa gtggttagga acaattcata gcactgacat gttatcagta 60
   aaaatagaag aaaataaatt aatattatga aatattaatt atatttcatt aattatgtaa 120
   tatgaattat gttttagctc aaatatttcc caagggacaa ttaagtaaat gaaaaataca 180
   cacagattaa aataataaat agagaaggag atattaatga ggtacaaaaa gaaaaaatac 240
   atgtaatcac atgaaatgct attatttgaa agattaacaa aacttgtaaa ctacctgcta 300
   acttgatcaa agaaaaaaat cgagaaacca tatgcgcaat taatagtaag agggaaataa 360
   acattgaaac agaagacatt tgaaatacca tataagactg ggtttcagag ctctatgtac 420
   gtaaattgat aatgtcctgg agaagtgcag atgaccaaaa tggacacctt tcaacttaga 480
   aatcataaac agattcattt ycttaaagtt aatgaaaaga attaacagac cctcctcaaa 540
   aaagacatat atgcggccta caatcatatg aaaaaaagtt caacattact gttcattaga 600
   gaaatgcaaa tcaaaaccac aatgagatac catctcacac cagtcagaat ggctattatt 660
   aagaagtcaa aaaataaaag atgctggcga ggttgtggag aaaaaagaat gcttttatac 720
   acttggtggg aatgtaaatt agttcagtca ttgtggaaga ctttgatgat tcctagaaga 780
   cctaaataca gaactactat ttgacccaac aatcccatta ctgggtatat actcaaatga 840
   ctataaatca ttctattata aagacacatg catggatatg ttcattacag cactatgcac 900
   aatagcaaag acttggaatc aacatgaatg tccatcaatg atagactaga taaagaaaat 960
   gtggtacaca tataccatgg aatactatgc agccataaaa a 1001
<210> 383
   <211> 1001
   <212> DNA
   <213> Homo sapiens
<400> 383
   tcagtaaaaa tagaagaaaa taaattaata ttatgaaata ttaattatat ttcattaatt 60
   atgtaatatg aattatgttt tagctcaaat atttcccaag ggacaattaa gtaaatgaaa 120
   aatacacaca gattaaaata ataaatagag aaggagatat taatgaggta caaaaagaaa 180
   aaatacatgt aatcacatga aatgctatta tttgaaagat taacaaaact tgtaaactac 240
   ctgctaactt gatcaaagaa aaaaatcgag aaaccatatg cgcaattaat agtaagaggg 300
   aaataaacat tgaaacagaa gacatttgaa ataccatata agactgggtt tcagagctct 360
   atgtacgtaa attgataatg tcctggagaa gtgcagatga ccaaaatgga cacctttcaa 420
   cttagaaatc ataaacagat tcatttcctt aaagttaatg aaaagaatta acagaccctc 480
   ctcaaaaaag acatatatgc rgcctacaat catatgaaaa aaagttcaac attactgttc 540
   attagagaaa tgcaaatcaa aaccacaatg agataccatc tcacaccagt cagaatggct 600
   attattaaga agtcaaaaaa taaaagatgc tggcgaggtt gtggagaaaa aagaatgctt 660
   ttatacactt ggtgggaatg taaattagtt cagtcattgt ggaagacttt gatgattcct 720
   agaagaccta aatacagaac tactatttga cccaacaatc ccattactgg gtatatactc 780
   aaatgactat aaatcattct attataaaga cacatgcatg gatatgttca ttacagcact 840
   atgcacaata gcaaagactt ggaatcaaca tgaatgtcca tcaatgatag actagataaa 900
   gaaaatgtgg tacacatata ccatggaata ctatgcagcc ataaaaatga aggagatcat 960
   gccctttgca gggacacgaa tagaggtgga ggccattatc c 1001
<210> 384
   <211> 501
   <212> DNA
   <213> Homo sapiens
<400> 384
   agttgcttga aagcaaagtt ctcgcagtag ctctctatct agaaggaggc attttattta 60
   tgtaaggaag tcacctaaaa gaaaattcat ttgttatggt gtggctttaa gagttactta 120
   cttttaatgg aatcccccag ataataataa attctgaaaa aaaaaaatca gaatcatggc 180
   atgttaaaac tggatacatt cctagaaata gatggaaact gctcttgcaa aaagcttagc 240
   acatgttaaa rcattttaga aacaatttgc caaagtttat ttagtctagt gatttcgaca 300
   ggttaaatgg accctttgag atcttttttc ctcaagtaca aaggctcact tgcttaatga 360
   acacagtccc agaaaagcag ggggctgaac cttggctcta ccatcttacc taagattcta 420
   gagttagcaa agggtttcca caagcccaaa ttattatgtt taatcttttc aattatctgt 480
   gaagcattag gttggtgcaa a 501
<210> 385
   <211> 501
   <212> DNA
   <213> Homo sapiens
<400> 385
   gaggcatttt atttatgtaa ggaagtcacc taaaagaaaa ttcatttgtt atggtgtggc      60
   tttaagagtt acttactttt aatggaatcc cccagataat aataaattct gaaaaaaaaa     120
   aatcagaatc atggcatgtt aaaactggat acattcctag aaatagatgg aaactgctct     180
   tgcaaaaagc ttagcacatg ttaaagcatt ttagaaacaa tttgccaaag tttatttagt     240
   ctagtgattt ygacaggtta aatggaccct ttgagatctt ttttcctcaa gtacaaaggc     300
   tcacttgctt aatgaacaca gtcccagaaa agcagggggc tgaaccttgg ctctaccatc     360
   ttacctaaga ttctagagtt agcaaagggt ttccacaagc ccaaattatt atgtttaatc     420
   ttttcaatta tctgtgaagc attaggttgg tgcaaaagta actgcaggtt ttgacattaa     480
   aactggcaaa aactgcaata a                                               501
<210> 386
   <211> 703
   <212> DNA
   <213> Homo sapiens
<400> 386
   gacaccagtt agcatattgt cgcgggggag aggggtggga aaggcgagag aacagcatgt      60
   ggtccagagg ccatacccag atggaggctg cagtcagctc cccagtcaaa ggcaaagccc     120
   aagtcaaagc catgcttccc tcttgcccac ctgctccaat gccacccaca gagagtgcgc     180
   cacagctcac aggatgcagg tctggttgaa tcttaacaat aactttgtaa gggaggtgtc     240
   attagctcca ttctcctggc aggaggatga ggctcaaggc agctaaaggc ttttgctgaa     300
   catcaagtgg tgagccagga ctcaawgcca gatcttcttg tttccctgtt aggtgtatgt     360
   agcacaactg gtatctgcag actatgctgc tggaagggct agccgtcact gttatcacag     420
   cgactgctgc ctgagatatg ccaggtactg ctgcaagaag tttacaaata taagctcact     480
   tgatcttcat aacatactac ctaggtacaa tcattatatt tatttgacag atacagagac     540
   agaggggaca cagaaaggat tagtaacttg ccccaaacca cacagccagc aaggtgtaag     600
   tgagcacctg cagtctagat gagacaccac tcaaaacgtc atttttctgg cagccccgtg     660
   cagttaccac agtggtcacc ccagtggtca gctaaaggcc aag                       703
<210> 387
   <211> 704
   <212> DNA
   <213> Homo sapiens
<400> 387
   gcatattgtc gcgggggaga ggggtgggaa aggcgagaga acagcatgtg gtccagaggc 60
   catacccaga tggaggctgc agtcagctcc ccagtcaaag gcaaagccca agtcaaagcc 120
   atgcttccct cttgcccacc tgctccaatg ccacccacag agagtgcgcc acagctcaca 180
   ggatgcaggt ctggttgaat cttaacaata actttgtaag ggaggtgtca ttagctccat 240
   tctcctggca ggaggatgag gctcaaggca gctaaaggct tttgctgaac atcaagtggt 300
   gagccaggac tcaatgccag atcttcttgt ttccctgtta ggtgtwtgta gcacaactgg 360
   tatctgcaga ctatgctgct ggaagggcta gccgtcactg ttatcacagc gactgctgcc 420
   tgagatatgc caggtactgc tgcaagaagt ttacaaatat aagctcactt gatcttcata 480
   acatactacc taggtacaat cattatattt atttgacaga tacagagaca gaggggacac 540
   agaaaggatt agtaacttgc cccaaaccac acagccagca aggtgtaagt gagcacctgc 600
   agtctagatg agacaccact caaaacgtca tttttctggc agccccgtgc agttaccaca 660
   gtggtcaccc cagtggtcag ctaaaggcca agcccaccgt ttct 704
<210> 388
   <211> 975
   <212> DNA
   <213> Homo sapiens
<400> 388
   gacttaagac aagggggtct taatttgatt atttttttct gttttatatg atttctatga 60
   aaactacaac aaaataaagt taattctatt taagtgactt tttaatgaat tgcctttgtt 120
   agaaaaaaaa ttaagtgttt ttgtctcact ctgtcaccca ggctggagca cagtggtgtg 180
   atcatggctt actgcagcca tgacctcccg ggctcaggtg atcctcccac ctcagcttcc 240
   caaatagatg ggactacagt tgtgtgccac aacgcctggc taatttttgt atttttttgt 300
   agagacaggg tctcaccagg ttgcccaggc tgatcttgaa ctccttggct caagcgatcc 360
   acccacctca gcctccctga gtgctgggat tacaggcatg agccagcgca cccagccaga 420
   attacatttt tttaaatggt actgtcctag aaaatccagg atgtgcagtg atcaygtatg 480
   aatgcatgga cctgcacaca caggagtgaa caaaagaccc acccctgcca ggtcaccact 540
   catatctcac cccagcccac gctagctcac actcctcccc acacaccact gacctcatca 600
   ttgctaggta cccacttgac ttctcaacag gttcaagaca attggccttc ctcgtctctt 660
   ctagaaacac cctcttttct gggctttgtg taacacctgg tctttctccc ctctctggcc 720
   acttctcagc ttttcttttt ctttctttct tttttttttt tttttttttg ccacttcctc 780
   ttcctctaca tcaagcttgt ccaacccaca gcccaggaca gctttgaatg cagcctaaca 840
   caaattcgta agctttctta aaacattatg agatgtgtgt gtgtgtgtgt gtgtgtgtgt 900
   gtgtgtgtgt gtgtgtgtgt gtttagctca tcagctatcg ttattgttag tgtattttat 960
   gtgtggccca agaca 975
<210> 389
   <211> 976
   <212> DNA
   <213> Homo sapiens
<400> 389
   gactttttaa tgaattgcct ttgttagaaa aaaaattaag tgtttttgtc tcactctgtc 60
   acccaggctg gagcacagtg gtgtgatcat ggcttactgc agccatgacc tcccgggctc 120
   aggtgatcct cccacctcag cttcccaaat agatgggact acagttgtgt gccacaacgc 180
   ctggctaatt tttgtatttt tttgtagaga cagggtctca ccaggttgcc caggctgatc 240
   ttgaactcct tggctcaagc gatccaccca cctcagcctc cctgagtgct gggattacag 300
   gcatgagcca gcgcacccag ccagaattac atttttttaa atggtactgt cctagaaaat 360
   ccaggatgtg cagtgatcac gtatgaatgc atggacctgc acacacagga gtgaacaaaa 420
   gacccacccc tgccaggtca ccactcatat ctcaccccag cccacgctag ctcacrctcc 480
   tccccacaca ccactgacct catcattgct aggtacccac ttgacttctc aacaggttca 540
   agacaattgg ccttcctcgt ctcttctaga aacaccctct tttctgggct ttgtgtaaca 600
   cctggtcttt ctcccctctc tggccacttc tcagcttttc tttttctttc tttctttttt 660
   tttttttttt ttttgccact tcctcttcct ctacatcaag cttgtccaac ccacagccca 720
   ggacagcttt gaatgcagcc taacacaaat tcgtaagctt tcttaaaaca ttatgagatg 780
   tgtgtgtgtg tgtgtgtgtg tgtgtgtgtg tgtgtgtgtg tgtgtgttta gctcatcagc 840
   tatcgttatt gttagtgtat tttatgtgtg gcccaagaca tttcttcttc cagtgtggcc 900
   cagggaagcc aaaagattgg acacccctgc tctacaacat ctcaatatag gcctttttca 960
   tgtttcattc tagatt 976
<210> 390
   <211> 801
   <212> DNA
   <213> Homo sapiens
<400> 390
   atccagacgg tgcccatact ccctgctctg tctagatggt gtccacattc cctgctccgt 60
   ctagactgtg cccatattcg ctgctggctg caaatgcgag gagttgacag cagcctcccc 120
   tttacaaggc aggaggtgcc actgttcgcc attgtctcca cctagggctt cacttgcttt 180
   ctatctgcag acatcagagg gacccacatc tctctgttct gacacgctgt gtgttgatgg 240
   cagagtttaa ttatccacat gcaatcttac tttccttatt cccaagtccg tggggctgcc 300
   tcatcaaagc attgtaagaa ctgataacca tcttctagaa gtatcatagt gatattaaga 360
   acacacatca cagatcatag taaatggctt taatttttta rcgaaatctc actactgcaa 420
   atgcattgtt gtcctagcta atgaatgcat agagtattgc ctgcaaaata ataattgaga 480
   ttctattttt aagaagctta gaacagtaca tggtgcatag caaagactct gtgtatgtga 540
   agccagattt taaaatatgg taacaagtgt ctgaaaatat gtggctcaat ttgtctcccg 600
   gttacttttc cctctccccc tttaaaatgt agaggaagga gaagaagaga taagaggttt 660
   gtgagtgaag acaagggccc tttaaggcct gggaagacta acgccatagg gatctccctc 720
   tgccttaaaa ggcacaggaa tcttagtggg gaaaaagaag tggtgataaa tagccagtcc 780
   gtgtgcctgg aatatcaaag t 801
<210> 391
   <211> 801
   <212> DNA
   <213> Homo sapiens
<400> 391
   ccctgctccg tctagactgt gcccatattc gctgctggct gcaaatgcga ggagttgaca 60
   gcagcctccc ctttacaagg caggaggtgc cactgttcgc cattgtctcc acctagggct 120
   tcacttgctt tctatctgca gacatcagag ggacccacat ctctctgttc tgacacgctg 180
   tgtgttgatg gcagagttta attatccaca tgcaatctta ctttccttat tcccaagtcc 240
   gtggggctgc ctcatcaaag cattgtaaga actgataacc atcttctaga agtatcatag 300
   tgatattaag aacacacatc acagatcata gtaaatggct ttaatttttt agcgaaatct 360
   cactactgca aatgcattgt tgtcctagct aatgaatgca yagagtattg cctgcaaaat 420
   aataattgag attctatttt taagaagctt agaacagtac atggtgcata gcaaagactc 480
   tgtgtatgtg aagccagatt ttaaaatatg gtaacaagtg tctgaaaata tgtggctcaa 540
   tttgtctccc ggttactttt ccctctcccc ctttaaaatg tagaggaagg agaagaagag 600
   ataagaggtt tgtgagtgaa gacaagggcc ctttaaggcc tgggaagact aacgccatag 660
   ggatctccct ctgccttaaa aggcacagga atcttagtgg ggaaaaagaa gtggtgataa 720
   atagccagtc cgtgtgcctg gaatatcaaa gtcagtgcgt gccagggatc acactgcggg 780
   tcacgtgcac tctgggtctc t 801
<210> 392
   <211> 601
   <212> DNA
   <213> Homo sapiens
<400> 392
   ttggcctggg gctgattcct ccaaagcaat gtgtctcttc gcagagtctc ttagagctgc 60
   aaggcagtat gggatcatca gagaggatgc taggaagctt cagaaatgga ggtcctggta 120
   gaaagggtcc tttggcgtgg cctctgaaga gtccaaatgt gggacaagac cctccgaaag 180
   cggtggcctg gggagccaca ggtggggcag ccagcacgga agagggtggc tttgctacca 240
   ttgggaaaac ttatcctcca catcctcatg aggcaaacac ctttcctacc ttaccgctcc 300
   ycagtggcct ccctgttgcc ttcttattca agactaagac cctctagaat gttctttatc 360
   ctgagtccag ctgattgtct atactaatat cagtacgggg tgtagatgag gacaaccagt 420
   gtgcctggct gccaggcacc ccctccccaa accccaggag tttctggaac attccaactc 480
   tgcttgaggg tatccatgca gcatctacta ctgtgagcag gtggtctgat ctgtggaaaa 540
   cttctatgat tcacctgagg gtaactgccc tttgtgattt gaaagaatga tgctaacaga 600
   a 601
<210> 393
   <211> 601
   <212> DNA
   <213> Homo sapiens
<400> 393
   gcagagtctc ttagagctgc aaggcagtat gggatcatca gagaggatgc taggaagctt 60
   cagaaatgga ggtcctggta gaaagggtcc tttggcgtgg cctctgaaga gtccaaatgt 120
   gggacaagac cctccgaaag cggtggcctg gggagccaca ggtggggcag ccagcacgga 180
   agagggtggc tttgctacca ttgggaaaac ttatcctcca catcctcatg aggcaaacac 240
   ctttcctacc ttaccgctcc tcagtggcct ccctgttgcc ttcttattca agactaagac 300
   yctctagaat gttctttatc ctgagtccag ctgattgtct atactaatat cagtacgggg 360
   tgtagatgag gacaaccagt gtgcctggct gccaggcacc ccctccccaa accccaggag 420
   tttctggaac attccaactc tgcttgaggg tatccatgca gcatctacta ctgtgagcag 480
   gtggtctgat ctgtggaaaa cttctatgat tcacctgagg gtaactgccc tttgtgattt 540
   gaaagaatga tgctaacaga aagtgttgtc atttctgaac ttttctgaac tctgcagcga 600
   g 601
<210> 394
   <211> 1001
   <212> DNA
   <213> Homo sapiens
<400> 394
   agatttggat ggggacacaa aaccaaacca tatcataggt taaattgtgt ctcccacccc      60
   aaaaatgtgt atgttgaagt cctaaccttc agtactcaga atgtgacatt atttggaaat     120
   agggtcattg cagatggagt tagttaagat gaggtcatta ggatgagtcc ctaatccaat     180
   atgactggtg ctcttacaaa aaggggaagt ttggacacag agccatgcac atgggtggga     240
   agaatcccaa atgaacggat aggcagaggg ttggagagat gcatcaacaa ggaacaccaa     300
   agattgccag caacccccag aagctggggg agaggcctgg aacagattct ccctcacagc     360
   ctgagaggaa ccaagctggc tgacaccttg atctcaggtt accggccttg agaactgaga     420
   gaccctgggt ttctgttgtt taagcctctc agggtgcagc actttattat ggaagcctga     480
   gctgactaat acaggtgtct ytatatctca ctgagggaaa gtgacaggaa agtaagaacc     540
   atttatgtcc aagagtccag aggagtcaac cagattctgg gggaaaagaa ggtacaatgc     600
   tggcctctcc atgcagccta gtccccaaca cttgtagggc ccagggcaag atctaaagca     660
   ctctctcacc tatgcatcta tatgctgtaa ctcagataaa caaactatta aataatatat     720
   gtgtcttgcc tctcaatctg acaattacac ctttataata gcaacatagg aaaataacta     780
   aaactatggt ttttaggcaa ccaaatacca gcaaaatgta ataattccta ttattagata     840
   tgtttaagtg ttctgctggt gggtcagcat ctttggtaga gtcataaaat taaaatgtac     900
   ataattaatt aaatattata tgtttattcc ctaacattta tttctgtcat ttcttttttc     960
   tttttttcag acagtctcac tcttttgccc aggccggagt g                         1001
<210> 395
   <211> 1001
   <212> DNA
   <213> Homo sapiens
<400> 395
   ttgtgtctcc caccccaaaa atgtgtatgt tgaagtccta accttcagta ctcagaatgt      60
   gacattattt ggaaataggg tcattgcaga tggagttagt taagatgagg tcattaggat     120
   gagtccctaa tccaatatga ctggtgctct tacaaaaagg ggaagtttgg acacagagcc     180
   atgcacatgg gtgggaagaa tcccaaatga acggataggc agagggttgg agagatgcat     240
   caacaaggaa caccaaagat tgccagcaac ccccagaagc tgggggagag gcctggaaca 300
   gattctccct cacagcctga gaggaaccaa gctggctgac accttgatct caggttaccg 360
   gccttgagaa ctgagagacc ctgggtttct gttgtttaag cctctcaggg tgcagcactt 420
   tattatggaa gcctgagctg actaatacag gtgtctctat atctcactga gggaaagtga 480
   caggaaagta agaaccattt rtgtccaaga gtccagagga gtcaaccaga ttctggggga 540
   aaagaaggta caatgctggc ctctccatgc agcctagtcc ccaacacttg tagggcccag 600
   ggcaagatct aaagcactct ctcacctatg catctatatg ctgtaactca gataaacaaa 660
   ctattaaata atatatgtgt cttgcctctc aatctgacaa ttacaccttt ataatagcaa 720
   cataggaaaa taactaaaac tatggttttt aggcaaccaa ataccagcaa aatgtaataa 780
   ttcctattat tagatatgtt taagtgttct gctggtgggt cagcatcttt ggtagagtca 840
   taaaattaaa atgtacataa ttaattaaat attatatgtt tattccctaa catttatttc 900
   tgtcatttct tttttctttt tttcagacag tctcactctt ttgcccaggc cggagtgcag 960
   tggcgtgatc tcagctcact gcaacctccg cctcccaggt t 1001
<210> 396
   <211> 1218
   <212> DNA
   <213> Homo sapiens
<400> 396
   gataaagaaa ggtcatcctc aatttcaatt tactttatat attctttgag aggtaaccgt      60
   gtcttatctc cccccaaaat tccttttaaa aggaaatttc caaagatgct ctattctgtg     120
   aataaagcat tgtgccacag ccgagaggat ccagcaatga acatgagatt gcccttgatt     180
   cataaggtct acaagctagt aaggatagag aacactttaa aataaaaaaa aatagttttt     240
   ggtatattta tattgtgtat ttggtataat tgagttttct acattctcat atatgtattt     300
   catattttga agaatatgca gaaaataatc aagcttccaa ataaacattt ttttttaaga     360
   actgcacaag tgagaattta ggagaacaga agatcagagg gctgcacrgg ctaaactaga     420
   caatgagccc atgcaagtaa gttaagagga gaagcgggta agtatgcacc tgctttgtct     480
   aggtgaccag caagcattta gcaatagtct tttcaaaaca acagctcctt atattgtcaa     540
   atctcaagaa gtaatattta tggttaaaaa aatctcagac ccaacagaaa atccatgagg     600
   gagatggttt tggaaacgca gaattttcag ctatgatatc cttttataaa caagcagata     660
   ctttccccaa atataattca atgcctcagt ctacctcctg ctgaaaccac taacaccacc     720
   actaaagctc gactatatgg gaaaatttag gtgtcacttt caaaatatgt cctagcataa     780
   aggcaattaa aaaatgtaaa gcaccaaaga tgcaagagag acataaatga ataaaatatc     840
   tggcacgaaa gttttcaaaa gcttgggaat ctgattcaaa aaaaaataaa atcagccaag     900
   cagtgttagt aagttagcca atcaggtttc aagaaggcag aaagacaaaa tcaacatcac     960
   cagcatttga caccgctact gggggaaaaa agggggatgg agttcgttta tggccttttt    1020
   aaaaatgcca ttacttggac aagagtcata acagagaagc actgcttatt tcagttctgt    1080
   taactgtaaa tatcagagcc aacacccaga aaaagttcac cattagccaa ttggttttgc    1140
   ctggccaatt ggagatggta ataggcctgc tatggatgac attctttctg atataagttg    1200
   tttcttgctt tttctccc                                                  1218
<210> 397
   <211> 1218
   <212> DNA
   <213> Homo sapiens
<400> 397
   gataaagaaa ggtcatcctc aatttcaatt tactttatat attctttgag aggtaaccgt      60
   gtcttatctc cccccaaaat tccttttaaa aggaaatttc caaagatgct ctattctgtg     120
   aataaagcat tgtgccacag ccgagaggat ccagcaatga acatgagatt gcccttgatt     180
   cataaggtct acaagctagt aaggatagag aacactttaa aataaaaaaa aatagttttt     240
   ggtatattta tattgtgtat ttggtataat tgagttttct acattctcat atatgtattt     300
   catattttga agaatatgca gaaaataatc aagcttccaa ataaacattt ttttttaaga     360
   actgcacaag tgagaattta ggagaacaga agatcagagg gctgcacggg ctaaactaga     420
   caatgagccc atgcaagtaa gttaagagga gaagcgggta agtatgcacc tgctttgtct     480
   aggwgaccag caagcattta gcaatagtct tttcaaaaca acagctcctt atattgtcaa     540
   atctcaagaa gtaatattta tggttaaaaa aatctcagac ccaacagaaa atccatgagg     600
   gagatggttt tggaaacgca gaattttcag ctatgatatc cttttataaa caagcagata     660
   ctttccccaa atataattca atgcctcagt ctacctcctg ctgaaaccac taacaccacc     720
   actaaagctc gactatatgg gaaaatttag gtgtcacttt caaaatatgt cctagcataa     780
   aggcaattaa aaaatgtaaa gcaccaaaga tgcaagagag acataaatga ataaaatatc     840
   tggcacgaaa gttttcaaaa gcttgggaat ctgattcaaa aaaaaataaa atcagccaag     900
   cagtgttagt aagttagcca atcaggtttc aagaaggcag aaagacaaaa tcaacatcac     960
   cagcatttga caccgctact gggggaaaaa agggggatgg agttcgttta tggccttttt    1020
   aaaaatgcca ttacttggac aagagtcata acagagaagc actgcttatt tcagttctgt    1080
   taactgtaaa tatcagagcc aacacccaga aaaagttcac cattagccaa ttggttttgc    1140
   ctggccaatt ggagatggta ataggcctgc tatggatgac attctttctg atataagttg    1200
   tttcttgctt tttctccc                                                   1218
<210> 398
   <211> 1072
   <212> DNA
   <213> Homo sapiens
<400> 398
   cacttaaaag ctctggaaac ctacgagatt atctttaaaa tcgtggggac caaatggctg      60
   gccaaggact tgtttctgta caggtgcgat tgcttctctg ctgtgttcct ttttattacc     120
   caagtaaccg gtatttcagc tcacaagatg agaaaatgac aaacaggcaa aataagcgta     180
   gggctgtgtg tgcaacagtt watcataaag ccatcaccag gagacgtcac tgggcgcctt     240
   ctggagtcta tccgtcctaa ctttgctttc tttctttttt tttttaaatt taagttctag     300
   ggtacatatg cacaacgtgc aggtttgtca cacatgtata catgtgccat gttggtgtgc     360
   tgcacccatt aactcgtcat ttacattagg tgtatctcct agtgctatcc ctccccactc     420
   ccccgacccc atgacaggcc ccagtgtgtg atgttcccct tcctgtgtcc aagtgttctc     480
   attgttcaat ccccacctat gagtgagaac atgccatgtt tggttttttg tccttgcgat     540
   agtttgctga gaatgatggt ttccagcttc atccatgtcc ctacaaagga catgaactca     600
   tcctttttta tggctacata gtattccatg gtgtatatgt gccacatttt cttaatccag     660
   tctatcatcg atggacattt gggttggttc caagtctttg ctattgtgac tagtgttgca     720
   ataaatatac gtgtggatgt gtctttatag cagtttgatt tataatcctt tgggtatata     780
   cccagtaacg ggatggctgg gtcaaatggt atttctagtt ctagatcctt gaggaatcgc     840
   cacactgact tccacaatgg ttgaactagt taacagtccc accaacagtg tgaaagtgtt     900
   cctatttctc cacatcctct ccagcacccc attttgactt tgctataagg gaactttagc     960
   atctgaacgt gcggacagct tcattgctgg cttgttacgt aacagtgttt tgtgaccatc    1020
   tcatgtcata cccacacatc gaaaccagca gtttaaatgg ccagctgttt gc            1072
<210> 399
   <211> 1072
   <212> DNA
   <213> Homo sapiens
<400> 399
   agattatctt taaaatcgtg gggaccaaat ggctggccaa ggacttgttt ctgtacaggt      60
   gcgattgctt ctctgctgtg ttccttttta ttacccaagt aaccggtatt tcagctcaca     120
   agatgagaaa atgacaaaca ggcaaaataa gcgtagggct gtgtgtgcaa cagtttatca     180
   taaagccatc accaggagac rtcactgggc gccttctgga gtctatccgt cctaactttg     240
   ctttctttct tttttttttt aaatttaagt tctagggtac atatgcacaa cgtgcaggtt     300
   tgtcacacat gtatacatgt gccatgttgg tgtgctgcac ccattaactc gtcatttaca     360
   ttaggtgtat ctcctagtgc tatccctccc cactcccccg accccatgac aggccccagt     420
   gtgtgatgtt ccccttcctg tgtccaagtg ttctcattgt tcaatcccca cctatgagtg 480
   agaacatgcc atgtttggtt ttttgtcctt gcgatagttt gctgagaatg atggtttcca 540
   gcttcatcca tgtccctaca aaggacatga actcatcctt ttttatggct acatagtatt 600
   ccatggtgta tatgtgccac attttcttaa tccagtctat catcgatgga catttgggtt 660
   ggttccaagt ctttgctatt gtgactagtg ttgcaataaa tatacgtgtg gatgtgtctt 720
   tatagcagtt tgatttataa tcctttgggt atatacccag taacgggatg gctgggtcaa 780
   atggtatttc tagttctaga tccttgagga atcgccacac tgacttccac aatggttgaa 840
   ctagttaaca gtcccaccaa cagtgtgaaa gtgttcctat ttctccacat cctctccagc 900
   accccatttt gactttgcta taagggaact ttagcatctg aacgtgcgga cagcttcatt 960
   gctggcttgt tacgtaacag tgttttgtga ccatctcatg tcatacccac acatcgaaac 1020
   cagcagttta aatggccagc tgtttgcttg tgaaaactcc cctcggctgg ct 1072
<210> 400
   <211> 948
   <212> DNA
   <213> Homo sapiens
<400> 400
   aaattttctt tgctgaagtg tcttttcaaa tttttgcctt ttaaaaaaat tgagttgtct 60
   taatattgag tcgtaaggtt ctttatatat tctggctata tgtcctttgt cagatatatg 120
   tcttgcaaat attttctccc agtctgtggc ttaccttttc catttttaaa ctgtgtttta 180
   taaaaaaaag aagttttttt agatcaaagt ccattttaat cattttttct tttatagttc 240
   atgctttttg tgtctcattt aagaaatctt tccctactcc aatgtcacaa atatattctc 300
   tgagaagctt aacagttttt gcaactaaat ttaggtctat gatccgtttt gacttaattt 360
   ttccatatgg tgtcatgtaa cagttgagat ttttttccta tgcaggcaga tattcaatgg 420
   ttcaagtacc atttattgaa atggctatct tttctccact gaatgacctt ggcactttta 480
   tcaaacatca actggccaca yacaggtgag tctacttctg gacacttacc ctgttccatt 540
   catctgtata tctctatcct tacaccaaca cgcatagtct tgaatactag ggcaagttaa 600
   ttttaagatg tctcctggat atgtaaaaat tatatctgag ttgaactaca gtttatttat 660
   atatccaggc agcaaataaa tgtgagaatc tggaggtgag ggaagagatc agagatacca 720
   ccttggaaac catcaattta gagatgattc ttaaggcagg ggactaaggg acactctgta 780
   ggacacagac atagagaagg gaaggggctg cggcctgaac accccacctg catgctcact 840
   cacatacttt cgtcggcctg tgttaacgaa gtgctgggtc tccccagcct ctctcatctg 900
   taagcagtgc caacaacgtc caacacagtt ccatccaatt tggatctg 948
<210> 401
   <211> 920
   <212> DNA
   <213> Homo sapiens
<400> 401
   aatttttgcc ttttaaaaaa attgagttgt cttaatattg agtcgtaagg ttctttatat      60
   attctggcta tatgtccttt gtcagatata tgtcttgcaa atattttctc ccagtctgtg     120
   gcttaccttt tccattttta aactgtgttt tataaaaaaa agaagttttt ttagatcaaa     180
   gtccatttta atcatttttt cttttatagt tcatgctttt tgtgtctcat ttaagaaatc     240
   tttccctact ccaatgtcac aaatatattc tctgagaagc ttaacagttt ttgcaactaa     300
   atttaggtct atgatccgtt ttgacttaat ttttccatat ggtgtcatgt aacagttgag     360
   atttttttcc tatgcaggca gatattcaat ggttcaagta ccatttattg aaatggctat     420
   cttttctcca ctgaatgacc ttggcacttt tatcaaacat caactggcca cacacaggtg     480
   agtctacttc tggacactta ycctgttcca ttcatctgta tatctctatc cttacaccaa     540
   cacgcatagt cttgaatact agggcaagtt aattttaaga tgtctcctgg atatgtaaaa     600
   attatatctg agttgaacta cagtttattt atatatccag gcagcaaata aatgtgagaa     660
   tctggaggtg agggaagaga tcagagatac caccttggaa accatcaatt tagagatgat     720
   tcttaaggca ggggactaag ggacactctg taggacacag acatagagaa gggaaggggc     780
   tgcggcctga acaccccacc tgcatgctca ctcacatact ttcgtcggcc tgtgttaacg     840
   aagtgctggg tctccccagc ctctctcatc tgtaagcagt gccaacaacg tccaacacag     900
   ttccatccaa tttggatctg                                                 920
<210> 402
   <211> 701
   <212> DNA
   <213> Homo sapiens
<400> 402
   tgtgctgctt ccattccata ggcacctgat cctaagtgtt aaccaatccc agaactctcc 60
   ccttatttct tgctgcatgt tttgaattga tgtgataaac aatgtgattc gagcgtctta 120
   actcagccta tgagcctctc tattctgtga ctgctggaat aggctgcttg gccatgttct 180
   tggaagctac caccatatca rggtaatttc ccacacaaca ttccagcccc tgctttcccc 240
   tctggcctta tctagggcca ttccccaact caggtgaatg cagactccaa atgtactgag 300
   ctgtgtgcag gggccaggtg caaatgcttt ctgtgcatct gcacatgctg ttctacctgg 360
   gaagtccttt cctcctttca cctattttta ccttaaacct cagacatcat ctaccctgga 420
   aagtccttcc tgacctcacg catctaagta ggtccccccc ataatcccta tccatgcctt 480
   ctatagtact taacatggtg acctttaatt gttcatttac ttagctctct gctctcccac 540
   actgtgaact ccttacaaac agggaatgtc atctctgaat gaatctttca tctccatgta 600
   acacatgcct ccaaccctac ctagcacaca atctggcata taacaggcac tcaataaacc 660
   ttcaatgaat gccttgatca agtacaagga acataagcaa a 701
<210> 403
   <211> 701
   <212> DNA
   <213> Homo sapiens
<400> 403
   ttaaccaatc ccagaactct ccccttattt cttgctgcat gttttgaatt gatgtgataa 60
   acaatgtgat tcgagcgtct taactcagcc tatgagcctc tctattctgt gactgctgga 120
   ataggctgct tggccatgtt cttggaagct accaccatat cagggtaatt tcccacacaa 180
   cattccagcc cctgctttcc yctctggcct tatctagggc cattccccaa ctcaggtgaa 240
   tgcagactcc aaatgtactg agctgtgtgc aggggccagg tgcaaatgct ttctgtgcat 300
   ctgcacatgc tgttctacct gggaagtcct ttcctccttt cacctatttt taccttaaac 360
   ctcagacatc atctaccctg gaaagtcctt cctgacctca cgcatctaag taggtccccc 420
   ccataatccc tatccatgcc ttctatagta cttaacatgg tgacctttaa ttgttcattt 480
   acttagctct ctgctctccc acactgtgaa ctccttacaa acagggaatg tcatctctga 540
   atgaatcttt catctccatg taacacatgc ctccaaccct acctagcaca caatctggca 600
   tataacaggc actcaataaa ccttcaatga atgccttgat caagtacaag gaacataagc 660
   aaatttcctg tggaaaaaaa gaattgtatt aagttctttg g 701
<210> 404
   <211> 601
   <212> DNA
   <213> Homo sapiens
<400> 404
   atgttcactt acacatcttt ctttcactta attgaatcct ttatttttgt cttagaatct      60
   tctgaatatt gaaaacagag aactatactg gaagaacata gtgtattaag actcatggag     120
   agggagatgt gatactgtgt cactgaggtc gttccagtca taggagaaat gttaccactg     180
   gattgaggtc tggtacattt taaaagatga tttaattcta tgatatgtgt tcaacttgca     240
   ctaggatagt ttttactttc acctttgttc catgcaccgc gcaaatacct gggaaccctt     300
   rttgcccaac tcaagagcca gagtcctctg tcatcatttt gcctctctcc taagtgacag     360
   gactgagtgc agacttggtg tttgtgggtg aggcatgtgg actgacaggc aggcttcagt     420
   ttatttagcg agtgtgagcc ctggcaggaa gattctcttt ctctgcttgc caggttgagg     480
   aggcctcatt aagcagtttg aacttgtggt tttggcgtgt ctagtcctgg tgcaggtggc       540
   ttggtatcct cacaggcatt tctttggcct cacccttggg gtgactgttc acttgtgttt       600
   g                                                                     601
<210> 405
   <211> 601
   <212> DNA
   <213> Homo sapiens
<400> 405
   tcttctgaat attgaaaaca gagaactata ctggaagaac atagtgtatt aagactcatg      60
   gagagggaga tgtgatactg tgtcactgag gtcgttccag tcataggaga aatgttacca     120
   ctggattgag gtctggtaca ttttaaaaga tgatttaatt ctatgatatg tgttcaactt     180
   gcactaggat agtttttact ttcacctttg ttccatgcac cgcgcaaata cctgggaacc     240
   cttgttgccc aactcaagag ccagagtcct ctgtcatcat tttgcctctc tcctaagtga     300
   saggactgag tgcagacttg gtgtttgtgg gtgaggcatg tggactgaca ggcaggcttc     360
   agtttattta gcgagtgtga gccctggcag gaagattctc tttctctgct tgccaggttg     420
   aggaggcctc attaagcagt ttgaacttgt ggttttggcg tgtctagtcc tggtgcaggt     480
   ggcttggtat cctcacaggc atttctttgg cctcaccctt ggggtgactg ttcacttgtg     540
   tttgagcggc tgggactcag taggttcact ggagtaggta tttctttaga gccactggcg     600
   g                                                                     601
<210> 406
   <211> 701
   <212> DNA
   <213> Homo sapiens
<400> 406
   cagctccttg gcaagcctgc tccttcccca gcaaatggaa acaccattct gaacacctgg 60
   gcattgtctc tgatgtccct tttcatctcc ctactctcac acaatccagc tgcctctctg 120
   ccttccacgg atattaagaa cgtccaccat ctcctgagtc caagcccttc tcactcacct 180
   ctttcttgaa ctaatttctt yctgtttttt tccagtcctc ccttctgttc atgtctctcc 240
   tctgcacact tccattttct ggttcagaaa atgtcaccgt cccagtcaca cttgccttat 300
   ggctgttgtg tcataaatac agttgacact tgaacaacat gggtttgaac tgcatggatt 360
   cacttataca catatttttt caatacaaat atatttaaaa attttggaga tttgcaacaa 420
   tttgaaaaaa cttgcagatg aacagcatag catagaaata ttgaaaaatt aagaaaaagg 480
   tatgtcatga atgcataaaa catatgcaga tactagtcta ttttaacctt tactgccata 540
   aaatatacac aaatctatta taaaaggtta aagtttatca aagcttatgc acacaaacac 600
   ttatagacca tatagggagc cattcagtag agagaaatgt aagcgaacgt aaaggtgtgc 660
   tatttaatca caactgcata cacactgtac cactgcacta a 701
<210> 407
   <211> 701
   <212> DNA
   <213> Homo sapiens
<400> 407
   gggcattgtc tctgatgtcc cttttcatct ccctactctc acacaatcca gctgcctctc 60
   tgccttccac ggatattaag aacgtccacc atctcctgag tccaagccct tctcactcac 120
   ctctttcttg aactaatttc tttctgtttt tttccagtcc tcccttctgt tcatgtctct 180
   cctctgcaca cttccatttt stggttcaga aaatgtcacc gtcccagtca cacttgcctt 240
   atggctgttg tgtcataaat acagttgaca cttgaacaac atgggtttga actgcatgga 300
   ttcacttata cacatatttt ttcaatacaa atatatttaa aaattttgga gatttgcaac 360
   aatttgaaaa aacttgcaga tgaacagcat agcatagaaa tattgaaaaa ttaagaaaaa 420
   ggtatgtcat gaatgcataa aacatatgca gatactagtc tattttaacc tttactgcca 480
   taaaatatac acaaatctat tataaaaggt taaagtttat caaagcttat gcacacaaac 540
   acttatagac catataggga gccattcagt agagagaaat gtaagcgaac gtaaaggtgt 600
   gctatttaat cacaactgca tacacactgt accactgcac taatttcaga gccacctcct 660
   gttgtgattg tggtgagccc aagtgttgtg aggatctgct t 701
<210> 408
   <211> 501
   <212> DNA
   <213> Homo sapiens
<400> 408
   caggtagggt aagcaaatga acacaaattc aaactcggaa ttcaaaacca gcctctgtgt      60
   attcctgagg accatactgt ctgctaagtg tagagaaagg cacatcctgg ttcaacagca     120
   gagaaagcaa acaggaggca ctttctgtga gtcatctcca ccacagggcc ctctcttttg     180
   tgatccagcg atacttgttc acagtcaaag cccaggaaga gtggaaagat taacctttgt     240
   gagccaaacc rtgtgacact tgattacttg acagaactaa tccttctgtc ctgatgacag     300
   aaattcaact acacaggtac atgcaagcta atatctgttg taatgcctcc cagtttctct     360
   ggagaattcc ttagtttcct ggacatctct gaaatgcaaa gttttggcaa cgagtctctg     420
   aattaacctc tgaaaatctc acccagccaa gatggccttc ttgagaagac tgaagaacat     480
   ggttggtttc aggctgagct g                                               501
<210> 409
   <211> 604
   <212> DNA
   <213> Homo sapiens
<400> 409
   cactttctgt gagtcatctc caccacaggg ccctctcttt tgtgatccag cgatacttgt      60
   tcacagtcaa agcccaggaa gagtggaaag attaaccttt gtgagccaaa ccgtgtgaca     120
   cttgattact tgacagaact aatccttctg tcctgatgac agaamttcaa ctacacaggt     180
   acatgcaagc taatatctgt tgtaatgcct cccagtttct ctggagaatt ccttagtttc     240
   ctggacatct ctgaaatgca aagttttggc aacgagtctc tgaattaacc tctgaaaatc     300
   tcacccagcc aagatggcct tcttgagaag actgaagaac atggttggtt tcaggctgag     360
   ctggaagtgg tttacctccc aggagaggtt ccccacagtg gtgtttaagg catggggtgg     420
   accaacacca ggaagactca gacatcacac cacccacctt caactcagtc acatccacct     480
   acattttctg aaaacaaaag gcagtctccc caaaaagcac tgagactctt gtgtaggtaa     540
   tctgagcaga caccaacttc ccagggcttc cttttatcca ggagagcttg gctgttcttt     600
   ttaa                                                                  604
<210> 410
   <211> 701
   <212> DNA
   <213> Homo sapiens
<400> 410
   ctccttccgc catggttgta agtttcctga cgcctcccag tcatgcttcc cgtacagcct 60
   gcagaactgc gagtcaatga aatccctttt ctccacaaat tacccagtct caggtagttc 120
   cttacagcag cgtgggaaca gactcaagag ctgaagcaag caaggccgtt agcaaggagc 180
   gggctgggga gagcactcca ggcagaggga acagccaggg ccagggcctt gagacagacg 240
   tgagccagga tatctgagga acagcagaga agccagtgtg gccgcagcta aatgaggaac 300
   aatgtgtgag ttccctgggg cggccaaaac aaacaccacg gacgggggcc ttcaaccaca 360
   gacaccgatt tcctcacagc tctggaggcg aaaagtccaa gaaaactgca cggagtatct 420
   atgaggccct gatggagacc tgacctggtc cacacccatg gcctggcaag ctagatgggg 480
   tgaattttca cctgccacag ycgcaagtca aagccaccgg cttctctctt ctccctccca 540
   ttgctcctga cagccagggt taatattttg cctcatgtaa acagggaggc atccacccga 600
   gaatctcccc tcagcccaca taagctctgc agagagggct gtgttgctcc agttcccacc 660
   tggacatgag cactttgaag ggcagcttcc ctcccggggt c 701
<210> 411
   <211> 612
   <212> DNA
   <213> Homo sapiens
<400> 411
   gggctgggga gagcactcca ggcagaggga acagccaggg ccagggcctt gagacagacg 60
   tgagccagga tatctgagga acagcagaga agccagtgtg gccgcagcta aatgaggaac 120
   aatgtgtgag ttccctgggg cggccaaaac aaacaccacg gacgggggcc ttcaaccaca 180
   gacaccgatt tcctcacagc tctggaggcg aaaagtccaa gaaaactgca cggagtatct 240
   atgaggccct gatggagacc tgacctggtc cacacccatg gcctggcaag ctagatgggg 300
   tgaattttca cctgccacag tcgcaagtca aagccaccgg cttctctctt ctccctccca 360
   ttgctcctga cagccagggt taatattttg cctcatgtaa acagggaggc ayccacccga 420
   gaatctcccc tcagcccaca taagctctgc agagagggct gtgttgctcc agttcccacc 480
   tggacatgag cactttgaag ggcagcttcc ctcccggggt ctggctgagc tcagggtagg 540
   cgtcagtctg catggattgg atggaggaag gctgtgcgtg gcaggagatg acactgccct 600
   tgggctgtgt gg 612
<210> 412
   <211> 1001
   <212> DNA
   <213> Homo sapiens
<400> 412
   ttggggaagg aagcactggg gggaaggaag cactgggctt gggacagggc tgggcgctgc      60
   ctcttcactg gaccatgaca aggttgttac ctcaccaagg agaggtgcaa aaagcttagg     120
   ggcttggatt tctagatttc agtgccaact atgccactta ctggctttat ccttggggaa     180
   tttatctact ctgtgaccct cagttttttt atcttaatta ttaatacata cctcataatg     240
   tgactgtgag gattcactta ataatatatg gaaaaccata gaatagtgcc cagcatctag     300
   gaagtgccac agcccccttc agaagctagt gaaacctgca gaccactttt cagagtgata      360
   ttattatttt tttctaggtt tactgagtta taattgaaaa aataaaaatg gaatatagat     420
   gtacaacatg aagctctgat gcatatatcc attgtgaaat gatgaccaca atcaagctaa     480
   ttaatgttat ctatcacttc wcatagttca accttttttt gtggtgagag tactgaagat      540
   ctactctctt agcaattttc aaatctaaaa tacattatta ttaacacagt cactgtgccg     600
   tacgttagct ctgaggacct tattcatttt atacctaaaa gtctgtatcc tttaaccaac     660
   ctctcctaat ttcccactgt catccctact gccacctctg gtaaccagcc ttctgctctg     720
   tttctgagtc caaccttctt agattccaca tatgagtgag atcatgctgt gcagtgtttg     780
   tttttctgtg tctggcttgc tttcacttag cataatgtcc tccaggtcca cccatgttgt     840
   tgcaaatggc agaatcttct tcttgttaaa gactgaataa tatccctgtg tgtgcgtgca     900
   tgtgtgtgtg tgtttgtgtg tgtgtgtgta tcacattttc ttcatccatt catccatcaa     960
   tggacactaa gcactaaggt tgattccgta tcttggctat t                        1001
<210> 413
   <211> 2480
   <212> DNA
   <213> Homo sapiens
<400> 413
   aacattactt ggggaaggaa gcactggggg gaaggaagca ctgggcttgg gacagggctg 60
   ggcgctgcct cttcactgga ccatgacaag gttgttacct caccaaggag aggtgcaaaa 120
   agcttagggg cttggatttc tagatttcag tgccaactat gccacttact ggctttatcc 180
   ttggggaatt tatctactct gtgaccctca gtttttttat cttaattatt aatacatacc 240
   tcataatgtg actgtgagga ttcacttaat aatatatgga aaaccataga atagtgccca 300
   gcatctagga agtgccacag cccccttcag aagctagtga aacctgcaga ccacttttca 360
   gagtgatatt attatttttt tctaggttta ctgagttata attgaaaaaa taaaaatgga 420
   atatagatgt acaacatgaa gctctgatgc atatatccat tgtgaaatga tgaccacaat 480
   caagctaatt aatgttatct atcacttcac atagttcaac ctttttttgt ggtgagagta 540
   ctgaagatct actctcttag caattttcaa atctaaaata cattattatt aacacagtca 600
   ctgtgccrta cgttagctct gaggacctta ttcattttat acctaaaagt ctgtatcctt 660
   taaccaacct ctcctaattt cccactgtca tccctactgc cacctctggt aaccagcctt 720
   ctgctctgtt tctgagtcca accttcttag attccacata tgagtgagat catgctgtgc 780
   agtgtttgtt tttctgtgtc tggcttgctt tcacttagca taatgtcctc caggtccacc 840
   catgttgttg caaatggcag aatcttcttc ttgttaaaga ctgaataata tccctgtgtg 900
   tgcgtgcatg tgtgtgtgtg tttgtgtgtg tgtgtgtatc acattttctt catccattca 960
   tccatcaatg gacactaagc actaaggttg attccgtatc ttggctattg tgaataatgc 1020
   tgcaataaac atatgagtcc agatacctct tcaagatact gatttcattt cctttaaata 1080
   tatgcccaga agtgggattg ctggatcata tggtagttct atatttagta tcttgaggaa 1140
   tttccatact gtttttcata atgattgtag caatctatat tcccatcaac agtgtacaag 1200
   ggttccattt tctacatggc cttaccaacg tttgttatca cttatctttt tgataataga 1260
   tattctagca ggtgtgaggt ggtatctcat tgtggtttta atttgcattt tcctgatgat 1320
   tagtggtgta gagcatcttt tcatattccc attggtaatt cgtatatctt cctttgagaa 1380
   atatttattc agatcttttg cccattgtta gctgagttat atgtgagttg gttttggttt 1440
   gttgttgttt tttgtttttg ctattgagct gagttccttg tatattttgg atattaaatc 1500
   cttctcagct gtatggttga cagatacatt cttgcattct gtaagttgca tctgtaggtt 1560
   gcaacagagt ctctttactc tgttgattgc ttgctttact gtgtgaaagc ttttttagct 1620
   tgatgtaatt gtgtttgtct atttttgctt ttgttgcttg tacttttagt gtcatatcca 1680
   aaaagttatt gcccagacca gtgtcatccc ctatgttttc ttctagtaat tttaaagttt 1740
   caggtcttat gtctatgtct ttaatccatt ttgagttaat ttttgtgtag ggtttaagat 1800
   aagaatccaa ttttattttt attttttgta tatggatatc caatttcccc aacaccattt 1860
   attgaaaatt ctatcctttc tttgttgtgt attaacatca gaataatatt tttaaataca 1920
   taaaattcag aagatgacaa aggaaaccaa ttacattgaa atgcatacag agttataatt 1980
   ctgaaagagc aatatatgtg cctctttgta aacacatcat atatcaaact gcagtgaccg 2040
   ttctaacaac tattgcaatt tcaaagtcat gttgagtagg aggagtactt tgagattctg 2100
   aaacaacgtt cttgtgctat gaaatatcca tgattttgat tggtgatggt atcccaggtc 2160
   ttgttaatgc tgctgtaatc tgttgcttcc attccatagt tgaataaaat gcttgatatc 2220
   tgttggaaat tagtaaaaat aaaaacgtat ttttttccat ccaagttcat tctcagaccc 2280
   tgaagagtca cttctctgga ttctgcagca aagttcccag ctggggcagc aagatttagg 2340
   caattgaaaa gaacatacac cttgttctca gtggcaaacc acatggaaag ctttaaatgt 2400
   cagagaagaa ttctgccatt ttgctgactt ttttgtagtt ctcctaataa acaagtgtta 2460
   agtgacaagc ttttcagagg 2480
<210> 414
   <211> 601
   <212> DNA
   <213> Homo sapiens
<400> 414
   cccccccccc ccccgcagat ctcaggtggg catttttgaa cttaactaga taacaaaaca 60
   cagctaagac aagtcctttt ctccagcaaa gatggcaatg ctctaataac tctgagcata 120
   ttaaagattc tccaagactc tagcctctgc tgcaaaaaca catacaaata cctactacta 180
   ctgctgctgt gatgatgatg atgacagcaa tagtgagaat attttaaata tgccaggcac 240
   ggtggcaact gctttccaaa tattatcata tttaatctga tcattgccct atgaggtagg 300
   ragtattctg attcccattt tataaataag gaacccgagg cttagagagc atcggtgact 360
   tgttcaaggt cacccacagc tgtcaagtga cagaacttcg ataaaaatcc agactccttt 420
   aatggagtat ggagggaggt cagaaaacat aggaagtaag ggattgtgat tgacaatgtg 480
   tccttgcaaa gggacaggtt aagagacaca agggcagctg tctgaggtgt gccattcacc 540
   agcttcagga gagaagtggc aggctacctc cagctatcca gccctatcca gccaaggaag 600
   c 601
<210> 415
   <211> 601
   <212> DNA
   <213> Homo sapiens
<400> 415
   caaaacacag ctaagacaag tccttttctc cagcaaagat ggcaatgctc taataactct      60
   gagcatatta aagattctcc aagactctag cctctgctgc aaaaacacat acaaatacct     120
   actactactg ctgctgtgat gatgatgatg acagcaatag tgagaatatt ttaaatatgc     180
   caggcacggt ggcaactgct ttccaaatat tatcatattt aatctgatca ttgccctatg     240
   aggtagggag tattctgatt cccattttat aaataaggaa cccgaggctt agagagcatc     300
   rgtgacttgt tcaaggtcac ccacagctgt caagtgacag aacttcgata aaaatccaga     360
   ctcctttaat ggagtatgga gggaggtcag aaaacatagg aagtaaggga ttgtgattga     420
   caatgtgtcc ttgcaaaggg acaggttaag agacacaagg gcagctgtct gaggtgtgcc     480
   attcaccagc ttcaggagag aagtggcagg ctacctccag ctatccagcc ctatccagcc     540
   aaggaagctt gggagacatg ttagttcccg ccttcatttc catcagcaac ctcaaagcca     600
   c                                                                     601
<210> 416
   <211> 5823
   <212> DNA
   <213> Homo sapiens
<400> 416
   tatttcaggc tttcttcttt ctatggataa gaaagctcct caggtggcaa caaaggccat      60
   ttctttggaa gcaggcatgg catgtgacga aaaaaagaca tctcagaaaa gagccaagaa     120
   taagactgga gagccactgt cagagaacag aaactgggct taatcaagga acatctcttg     180
   ttcccagagt aggaggctgg caatattttc tcactgaaat ttcagaattg ttatggacca     240
   gtgactgctc tatgtgttca atttgttccc ttttcaaatg gaagcattta ttgcagacga     300
   cctgcctctg tcccaccatt gtgtattagg tttgtagagy gtagacaact tgccttttta     360
   gtttgtaggt ttctgtatca agagaagatg tgtgtgggcc taacctagat tacaggatcc     420
   tggacttcaa gtctgatata atgactggat gagactttga ctgtcctaga attgggatga     480
   acatattttg ccggtgggag ggcgtgagta attgcggtta gagggcagac tgtccctcac     540
   acctattcct tttcatggtg ccttcccaaa ctgcctctgg aggtggccac acaaatggct     600
   ttggccattg tgaccatggg aaacttgatg cagaggctgg aaaaagcact tgcatgtttc     660
   tgtctcctct cttgttcctc tacaatcaca agaaatgtct aggcaggtct gagcaggccc     720
   aggctcatct gccatggaag aagaatggca catggaagag ggtcacattg tcccaaccaa     780
   gacgatccta gaccagccag gccccagttc atggttcaag acacatgaac atagttgcac     840
   gaaccaagat tagttgtgta tggcccagac tagcagcagc acccatccaa cctacagact     900
   ctgagaaata aatactagtt gtcttaagct tccaagtttc agtgtgagca ttaggtagta     960
   acagttaatg aataagacag ataatcattt tatctgtctg gatacttata caatgatttc    1020
   tattttttat tgatacataa tattttacat attgctgggg tacatgtgac attttgctac    1080
   atacatagaa tgtgtaatga tccagtcagg atatctgagg tgtccatcac tttgagaatt    1140
   tctcacttct gtgtgttggg aacaattcaa gtcgtctctt ctagttattt taaaatatac 1200
   aatacattgt taactgtagt cttttttatt gaatgacagg acttgtacct tttatctaac 1260
   tgtatgtttg tatctattaa gctagttctc tttatccctg ccccctccta cccactcact 1320
   cttcccaacc tctaacatgt atcatcctat tctatatctc catgagatca acttctttag 1380
   ctcccacata tgagcaaaaa catatgatgt ttgtctttct gtgcccggtt tatttcactt 1440
   atgacctcca tttccatcca tgttactata aatgacagga tttcattctt tttgtggcca 1500
   aacagtattt cattgtgtat atatactaca ttttctttat ccattcatcc attgatgaac 1560
   acttacgttg attccatatc tttgctattg tgaatggtgc tgcaataaac atgcacgtgc 1620
   agttatccct ttgatacact gatttatttt cctttggata aatacccagt agtgagattg 1680
   ctggatcata cggtagttct acttttagtt tttgagacat ttccatactt ttccagtgtt 1740
   tgtattaatt tacattccca tcaacaatgt ataagatttc cctttcctcc acatcctcac 1800
   cagcatctgc tattttttgt ctttttaata atagtcattc taactggggt gagaggatat 1860
   ctcgctatgg ttttgatttg catttccctg atatttaatg atattgagca tttcttcata 1920
   taacctattg gccatttgtg tgtctttttt tttttttttt ttttttttga gaattgtcta 1980
   ctcatttttg gctttttaaa agatttattt tttgttgttg ttgagtttag tgcatatcct 2040
   ggatattagt ctcttatctg atgaagagtt tgccaatatt ttctcccatt caacaggttg 2100
   tctcttcatt ctgttgactg tttcctttgc tgtgcagaag cactttatat acagtcccat 2160
   ttgtctattt tttagtagtc tatgcattta aggtctcagc cacaaaatct ttgcctagac 2220
   cagtcctaaa gtgtttcccc tatattttct tctagtagtt ttattgtttc atgtcttata 2280
   tttaagtcta taatccattg tgagttgatt tttgtatatg gtgagatagg ggccttgttt 2340
   cattcctctg catatagata tttaattttc tcagcaccat ttattgaagg tgtccttccc 2400
   tattgtatgt tcttggtgcc tttgtcaaaa ttcagttggc tataaatatg tgaatttatt 2460
   tctgggttct ctatgtggtt ccattagtct atgtgtctat ttttatacca atatcatgct 2520
   gttttgatta ccatagcctt gtaatatatt ttgaagtcag gtagtgtgat gcctccagct 2580
   ttgttctttt tgctcaggat tgctgtgcat actctggctt tttggttaca tacaaatttc 2640
   aggatttttg tatttctgtg aaaaatggca ttagtatttt gataggaatt gcactggatc 2700
   tgtatattgt cctggacaac atggtcattt taacaatatt aattcttcta atctatgagt 2760
   atgagacgtc ttcccacttg tttgtgtcct cttcaatttc tttcattggt gtttcataat 2820
   ctcccttcta caggcctttc acctccttgg ttaaattaat tcctaggtat ttttttgtag 2880
   ctactgtaaa tgggactgcc ttctttctca gctagttcat ttttggtgca tagaaaccct 2940
   atttttgtat gttcattttc tatcctgcaa cattaccaaa tttgcttatc agctttaagt 3000
   gtgtattttg ctttgcttgt agagtcttct ggtttctcta aatgtaagac gatgtcatct 3060
   gcaaacgggg acaatttgac ttcctcttaa aaatctgtat gccttttatt cctttctctt 3120
   gcctgattgc tctggctcta cctccagtac tatactgaat aaaagtggta aaagtgagca 3180
   tccttccttg tcttgctcta gttcttagag gaaatacttt cagtttttcc ccactcagta 3240
   tgatgttagc tgtgggtcat atatagcctt tattatgtta agatatgttt cttctgtacc 3300
   tggtttgttg acagcttttt atcataaaag gatgtagaat tttatcaaat gttttttctg 3360
   catctgttga gataatcata tggtttttgt cattccttct actgttgtga tgtatcatgt 3420
   ttattgattt gtgtatgtta aaccatcctt gtgtccttgg tataaattat acttggtcat 3480
   ggtgtattat ctttttggca tcctgtcgaa ttgtttgcta gctttttgtt ttgttctttt 3540
   tgagaatttt tatgtctagg ttccttagaa acactggcct gtagttctct ttttgtgtgt 3600
   gtgtccttgt ctagtttggt gtcagggaaa tggtggtctt gtagaatgag ttgttttttc 3660
   tttgattttt ttgcaagagt ttgaggagaa tgggtattag ttcttcttta tgtggttggt 3720
   caaattggca gtgaattcat tcagtcatga gcttttcttt ttttgggagg gttctcatta 3780
   ctgagttaat cacactgctc attactgatc tgttcagatt ttctatttct tctggaatct 3840
   cagtagttgt atgtttccag caatttatcc atttcctcta ggttttctag tttggtagta 3900
   tatagctatt cataatagtc tctgatgatc ttttgtattt ctgtgatatc agttgtaatg 3960
   tctttttcat ttcctatttt atttgggtct tttcttgttt agtctagcaa ggggtttatc 4020
   tattttatct ttttgaagaa ccaacttttt gtttcattga ccctttctac gtctttagtc 4080
   tttatttcat ttagatttgc tctgaacttt actatgtctt tccttctaat tttgggtttg 4140
   gtttgttctt ttctagttcc ttgaggtgca tcattgaatt gtttctttga tatctatcta 4200
   ctcatttgat gtaggtgttt attgctatac actctcccct cctagagctc cttttgttgt 4260
   gtcccatagg tcttggtatg ttgtttctat tttcatttgt ttcaaacatt ttatttccat 4320
   attaattttt atcattcagg aggagcatat tatttaattc ccatgtattt gtatagtttc 4380
   caaagttcct cttatttcta tttttactcc attgtggtct gagaagatac ttcatatgat 4440
   ttcaattttt aaaaatttgt caagacttgt tttttgtcct aacatatggt ctatcctgga 4500
   gaatgttcca tgtgctgatg agaaaaatgt gtactcagca gttgttgagt aacatgttct 4560
   acaaatatct gttagatcca tttggtctaa agtctagttt aaatccaatg agtttttgtt 4620
   aattttgtct agatgatcat gatctgagac tgaggtgaag tccccaacaa ttatcgtgtt 4680
   ggagtctacc tctcttttta aatctagaaa tatttgcttt ataaatccgg gtggtctagt 4740
   gttgggtgca tatatttagt tgttatttcc tcattagatt gatctcttta ctattatata 4800
   ataactgttt actgcttctg gcataaagtc tgttttatgt aagtacagcc attcctgctt 4860
   gagtttagta ccatgttgac aaagggatgc atagagagtt ggtaaagcat gatttctggg 4920
   tgtctgtgtg aaggtgtttt gagaagagtt tagcatgagt ctgtggagtg agtgggaaga 4980
   ttctccctca atgtcagcag gaaccatcca tccactgggg gcccaggtag aaaaagatga 5040
   agaaatggtg aattctctct ctctcctgga gctgggtcac ccttcttctg cccttgaaca 5100
   ggacatcaca actccaggct ctccagcctt tggactccaa gactgacacc agtgcccctc 5160
   cccaattacc ccaggccctc aggcctttgg cctaggattg agacttacac catcagcttc 5220
   cctggttctg aggcttctgg acttgcactg ggccatacta ccagcatccc agggtctcca 5280
   gcttgcagag agcctgttgt gggacttttc agcctccata atcaagtaag ccaatttccc 5340
   tggtatctat atagatatac aatcatgttt tgcttaccag cctgaaaaat gtatcgctag 5400
   atgagtctgt cattgcataa acatcatagt gtacttacac aaacctagat tctatagcct 5460
   actacacacc tagtctataa acatgtacag catgttactg tactgaatat tgtaggcaac 5520
   tgtaacacaa tggtgaatat ttgcatattt aaacatatct tatcattaaa aagatacagt 5580
   aaacataagg tataaaagac aaaaaccggc acacctatat agggcactta ccataaatgc 5640
   agcttgcagg actagaagtc actcagggtg agtcagtgag cgaacgtgaa ggcctaggtt 5700
   attactgtcc actacggtag actttatcaa cactgtacac aggctacact aaatttattt 5760
   tttaaaaatt tgctctccaa taataaatta atcttcgcat cctttttttg ttgttcactg 5820
   tgg 5823
<210> 417
   <211> 5823
   <212> DNA
   <213> Homo sapiens
<400> 417
   tatttcaggc tttcttcttt ctatggataa gaaagctcct caggtggcaa caaaggccat        60
   ttctttggaa gcaggcatgg catgtgacga aaaaaagaca tctcagaaaa gagccaagaa       120
   taagactgga gagccactgt cagagaacag aaactgggct taatcaagga acatctcttg       180
   ttcccagagt aggaggctgg caatattttc tcactgaaat ttcagaattg ttatggacca       240
   gtgactgctc tatgtgttca atttgttccc ttttcaaatg gaagcattta ttgcagacga       300
   cctgcctctg tcccaccatt gtgtattagg tttgtagagt gtagacaact tgccttttta       360
   gtttgtaggt ttctgtatca agagaagatg tgtgtrggcc taacctagat tacaggatcc       420
   tggacttcaa gtctgatata atgactggat gagactttga ctgtcctaga attgggatga       480
   acatattttg ccggtgggag ggcgtgagta attgcggtta gagggcagac tgtccctcac       540
   acctattcct tttcatggtg ccttcccaaa ctgcctctgg aggtggccac acaaatggct       600
   ttggccattg tgaccatggg aaacttgatg cagaggctgg aaaaagcact tgcatgtttc       660
   tgtctcctct cttgttcctc tacaatcaca agaaatgtct aggcaggtct gagcaggccc       720
   aggctcatct gccatggaag aagaatggca catggaagag ggtcacattg tcccaaccaa      780
   gacgatccta gaccagccag gccccagttc atggttcaag acacatgaac atagttgcac      840
   gaaccaagat tagttgtgta tggcccagac tagcagcagc acccatccaa cctacagact      900
   ctgagaaata aatactagtt gtcttaagct tccaagtttc agtgtgagca ttaggtagta      960
   acagttaatg aataagacag ataatcattt tatctgtctg gatacttata caatgatttc     1020
   tattttttat tgatacataa tattttacat attgctgggg tacatgtgac attttgctac     1080
   atacatagaa tgtgtaatga tccagtcagg atatctgagg tgtccatcac tttgagaatt     1140
   tctcacttct gtgtgttggg aacaattcaa gtcgtctctt ctagttattt taaaatatac     1200
   aatacattgt taactgtagt cttttttatt gaatgacagg acttgtacct tttatctaac     1260
   tgtatgtttg tatctattaa gctagttctc tttatccctg ccccctccta cccactcact     1320
   cttcccaacc tctaacatgt atcatcctat tctatatctc catgagatca acttctttag     1380
   ctcccacata tgagcaaaaa catatgatgt ttgtctttct gtgcccggtt tatttcactt     1440
   atgacctcca tttccatcca tgttactata aatgacagga tttcattctt tttgtggcca     1500
   aacagtattt cattgtgtat atatactaca ttttctttat ccattcatcc attgatgaac     1560
   acttacgttg attccatatc tttgctattg tgaatggtgc tgcaataaac atgcacgtgc     1620
   agttatccct ttgatacact gatttatttt cctttggata aatacccagt agtgagattg     1680
   ctggatcata cggtagttct acttttagtt tttgagacat ttccatactt ttccagtgtt     1740
   tgtattaatt tacattccca tcaacaatgt ataagatttc cctttcctcc acatcctcac     1800
   cagcatctgc tattttttgt ctttttaata atagtcattc taactggggt gagaggatat     1860
   ctcgctatgg ttttgatttg catttccctg atatttaatg atattgagca tttcttcata     1920
   taacctattg gccatttgtg tgtctttttt tttttttttt ttttttttga gaattgtcta     1980
   ctcatttttg gctttttaaa agatttattt tttgttgttg ttgagtttag tgcatatcct     2040
   ggatattagt ctcttatctg atgaagagtt tgccaatatt ttctcccatt caacaggttg     2100
   tctcttcatt ctgttgactg tttcctttgc tgtgcagaag cactttatat acagtcccat     2160
   ttgtctattt tttagtagtc tatgcattta aggtctcagc cacaaaatct ttgcctagac     2220
   cagtcctaaa gtgtttcccc tatattttct tctagtagtt ttattgtttc atgtcttata     2280
   tttaagtcta taatccattg tgagttgatt tttgtatatg gtgagatagg ggccttgttt     2340
   cattcctctg catatagata tttaattttc tcagcaccat ttattgaagg tgtccttccc     2400
   tattgtatgt tcttggtgcc tttgtcaaaa ttcagttggc tataaatatg tgaatttatt     2460
   tctgggttct ctatgtggtt ccattagtct atgtgtctat ttttatacca atatcatgct     2520
   gttttgatta ccatagcctt gtaatatatt ttgaagtcag gtagtgtgat gcctccagct     2580
   ttgttctttt tgctcaggat tgctgtgcat actctggctt tttggttaca tacaaatttc 2640
   aggatttttg tatttctgtg aaaaatggca ttagtatttt gataggaatt gcactggatc 2700
   tgtatattgt cctggacaac atggtcattt taacaatatt aattcttcta atctatgagt 2760
   atgagacgtc ttcccacttg tttgtgtcct cttcaatttc tttcattggt gtttcataat 2820
   ctcccttcta caggcctttc acctccttgg ttaaattaat tcctaggtat ttttttgtag 2880
   ctactgtaaa tgggactgcc ttctttctca gctagttcat ttttggtgca tagaaaccct 2940
   atttttgtat gttcattttc tatcctgcaa cattaccaaa tttgcttatc agctttaagt 3000
   gtgtattttg ctttgcttgt agagtcttct ggtttctcta aatgtaagac gatgtcatct 3060
   gcaaacgggg acaatttgac ttcctcttaa aaatctgtat gccttttatt cctttctctt 3120
   gcctgattgc tctggctcta cctccagtac tatactgaat aaaagtggta aaagtgagca 3180
   tccttccttg tcttgctcta gttcttagag gaaatacttt cagtttttcc ccactcagta 3240
   tgatgttagc tgtgggtcat atatagcctt tattatgtta agatatgttt cttctgtacc 3300
   tggtttgttg acagcttttt atcataaaag gatgtagaat tttatcaaat gttttttctg 3360
   catctgttga gataatcata tggtttttgt cattccttct actgttgtga tgtatcatgt 3420
   ttattgattt gtgtatgtta aaccatcctt gtgtccttgg tataaattat acttggtcat 3480
   ggtgtattat ctttttggca tcctgtcgaa ttgtttgcta gctttttgtt ttgttctttt 3540
   tgagaatttt tatgtctagg ttccttagaa acactggcct gtagttctct ttttgtgtgt 3600
   gtgtccttgt ctagtttggt gtcagggaaa tggtggtctt gtagaatgag ttgttttttc 3660
   tttgattttt ttgcaagagt ttgaggagaa tgggtattag ttcttcttta tgtggttggt 3720
   caaattggca gtgaattcat tcagtcatga gcttttcttt ttttgggagg gttctcatta 3780
   ctgagttaat cacactgctc attactgatc tgttcagatt ttctatttct tctggaatct 3840
   cagtagttgt atgtttccag caatttatcc atttcctcta ggttttctag tttggtagta 3900
   tatagctatt cataatagtc tctgatgatc ttttgtattt ctgtgatatc agttgtaatg 3960
   tctttttcat ttcctatttt atttgggtct tttcttgttt agtctagcaa ggggtttatc 4020
   tattttatct ttttgaagaa ccaacttttt gtttcattga ccctttctac gtctttagtc 4080
   tttatttcat ttagatttgc tctgaacttt actatgtctt tccttctaat tttgggtttg 4140
   gtttgttctt ttctagttcc ttgaggtgca tcattgaatt gtttctttga tatctatcta 4200
   ctcatttgat gtaggtgttt attgctatac actctcccct cctagagctc cttttgttgt 4260
   gtcccatagg tcttggtatg ttgtttctat tttcatttgt ttcaaacatt ttatttccat 4320
   attaattttt atcattcagg aggagcatat tatttaattc ccatgtattt gtatagtttc 4380
   caaagttcct cttatttcta tttttactcc attgtggtct gagaagatac ttcatatgat 4440
   ttcaattttt aaaaatttgt caagacttgt tttttgtcct aacatatggt ctatcctgga 4500
   gaatgttcca tgtgctgatg agaaaaatgt gtactcagca gttgttgagt aacatgttct 4560
   acaaatatct gttagatcca tttggtctaa agtctagttt aaatccaatg agtttttgtt 4620
   aattttgtct agatgatcat gatctgagac tgaggtgaag tccccaacaa ttatcgtgtt 4680
   ggagtctacc tctcttttta aatctagaaa tatttgcttt ataaatccgg gtggtctagt 4740
   gttgggtgca tatatttagt tgttatttcc tcattagatt gatctcttta ctattatata 4800
   ataactgttt actgcttctg gcataaagtc tgttttatgt aagtacagcc attcctgctt 4860
   gagtttagta ccatgttgac aaagggatgc atagagagtt ggtaaagcat gatttctggg 4920
   tgtctgtgtg aaggtgtttt gagaagagtt tagcatgagt ctgtggagtg agtgggaaga 4980
   ttctccctca atgtcagcag gaaccatcca tccactgggg gcccaggtag aaaaagatga 5040
   agaaatggtg aattctctct ctctcctgga gctgggtcac ccttcttctg cccttgaaca 5100
   ggacatcaca actccaggct ctccagcctt tggactccaa gactgacacc agtgcccctc 5160
   cccaattacc ccaggccctc aggcctttgg cctaggattg agacttacac catcagcttc 5220
   cctggttctg aggcttctgg acttgcactg ggccatacta ccagcatccc agggtctcca 5280
   gcttgcagag agcctgttgt gggacttttc agcctccata atcaagtaag ccaatttccc 5340
   tggtatctat atagatatac aatcatgttt tgcttaccag cctgaaaaat gtatcgctag 5400
   atgagtctgt cattgcataa acatcatagt gtacttacac aaacctagat tctatagcct 5460
   actacacacc tagtctataa acatgtacag catgttactg tactgaatat tgtaggcaac 5520
   tgtaacacaa tggtgaatat ttgcatattt aaacatatct tatcattaaa aagatacagt 5580
   aaacataagg tataaaagac aaaaaccggc acacctatat agggcactta ccataaatgc 5640
   agcttgcagg actagaagtc actcagggtg agtcagtgag cgaacgtgaa ggcctaggtt 5700
   attactgtcc actacggtag actttatcaa cactgtacac aggctacact aaatttattt 5760
   tttaaaaatt tgctctccaa taataaatta atcttcgcat cctttttttg ttgttcactg 5820
   tgg 5823
<210> 418
   <211> 707
   <212> DNA
   <213> Homo sapiens
<400> 418
   aacggtgtca gctggagtga actcctgtgt gtgcaaggcc tgggtctcct ggtcagacta      60
   ctttctatgg gaaaggcata gtgtatagtc tatatactat acataggggt gctgggagga     120
   actggggttt tcacagccag ctttggtttt cattaggttt gtttagtttc cattgcttca     180
   ggggtgttag ttttgtgttc mcaactagat tataaactcc tcttgcattc ctgatggcag     240
   tgacttgaag gcatttattt gaagaataat agacatacag aaaggggcgc atgtcataaa     300
   ggtacagctg gacgactttt cacaaagtga gcacatttgt atgatcgatg ttgagaccaa     360
   gagcattcag tggacaactc ctttccagtt actccacccc actcccagtg accatcattc     420
   tgacttctaa ctgtgtagac atgttttgct tgttttgtac tttacaaaca tatctactct     480
   attttaggtg gctagacaat gtgttttaca atgctggcca tgacagtgtt tgaaagaata     540
   aaatggaatc aaatagaatg ggcagtatca gagtgtgttg cctgcctaag aaatgttttg     600
   tgacattttg gctttgggtc tatttacaca ttaaatctaa gagcaccaga atgtggtgtc     660
   aaaatgtgtt tggggatgaa gatattctaa agtcctgtag taagcaa                   707
<210> 419
   <211> 712
   <212> DNA
   <213> Homo sapiens
<400> 419
   cagactactt tctatgggaa aggcatagtg tatagtctat atactataca taggggtgct      60
   gggaggaact ggggttttca cagccagctt tggttttcat taggtttgtt tagtttccat     120
   tgcttcaggg gtgttagttt tgtgttccca actagattat aaactcctct tgcattcctg     180
   atggcagtga cttgaaggca tttatttgaa gaataataga catacagaaa ggggcrcatg     240
   tcataaaggt acagctggac gacttttcac aaagtgagca catttgtatg atcgatgttg     300
   agaccaagag cattcagtgg acaactcctt tccagttact ccaccccact cccagtgacc     360
   atcattctga cttctaactg tgtagacatg ttttgcttgt tttgtacttt acaaacatat     420
   ctactctatt ttaggtggct agacaatgtg ttttacaatg ctggccatga cagtgtttga     480
   aagaataaaa tggaatcaaa tagaatgggc agtatcagag tgtgttgcct gcctaagaaa     540
   tgttttgtga cattttggct ttgggtctat ttacacatta aatctaagag caccagaatg     600
   tggtgtcaaa atgtgtttgg ggatgaagat attctaaagt cctgtagtaa gcaatgcaaa     660
   acgttctgga ggtgtttatt aaacatttgt ttgtagaatg gagaggaaga ca              712
<210> 420
   <211> 1210
   <212> DNA
   <213> Homo sapiens
<400> 420
   aaagcagcac tgctctgcat tcagccttgc tacgtctcct tcagatgggc gcactagata      60
   ctgagtgatg atcatgcctt gtctaggatc tcaccaagac agttcatgaa agagacagtg     120
   cagctcatgg aggagatggt gcagctcaca gagaggatgg tgccatcatg gaaagcatgg     180
   ggcagtcatg gagatgacgg rgtagctcat ggagaatata atgccatcat ggaaggcata     240
   gtgcagtcat ggagatgatg gtgcagctca tggagaagat ggtgccatca tggaaggcat     300
   ggtgcaatca tggagtagac agtgcagctg ggccaagatt ctccctgact aagctcttct     360
   caggcacctc tgagccgtcg tcttaactag gcctccagct tggcttgtga aaactgcaga     420
   ctctcagcac aaatgatttg cctcctacat taagagactt aaataaacac ttgcatggct     480
   gtgtttattt aaacagctca aggctgtgtc cctgggatga caatgactcc agcccctaaa     540
   attcctgctt gtgaaagctc attgctgaca gaaggatcta ccatttgttc cagccaacac     600
   ctggtggcag gcagataggc cctgagcccc atttaagagc agttccttta gaaagcttgc     660
   aattgtaaat cttttctctg cccatttgag atgtaaatct tctaccacct agaactgtct     720
   tctcaaggac ctgtgagctg actcactgaa atgcaaacat tcagggagat aactccactc     780
   ctgtccccat gcgacggcga ggccctgact ttggtgggca ccttgctctt atttgcacca     840
   ccacctcctg tcctaaagac atgagacgtt tgtctctcct ctggataagt gcctattaac     900
   caacccaggt gtcctggtca catgaaccag tccagcctag cacctggcac tgcctttccc     960
   tcagcacact ccagtctgta aaagtctcct tatggttgtt ttggcaaagt tgagcttagt    1020
   taatgctaga ccccttctct actgcaatag ttactgctga ataaagtcta tccttaccac    1080
   tttaactagt gttgggcttt gtttctcttt cataagctca tggagaagac aatgcagttc    1140
   catcaagttt ctggctctta cactgctaac agtcagctct ggggtccctg agagggacag    1200
   actcacacca                                                           1210
<210> 421
   <211> 1194
   <212> DNA
   <213> Homo sapiens
<400> 421
   gcattcagcc ttgctacgtc tccttcagat gggcgcacta gatactgagt gatgatcatg      60
   ccttgtctag gatctcacca agacagttca tgaaagagac agtgcagctc atggaggaga     120
   tggtgcagct cacagagagg atggtgccat catggaaagc atggggcagt catggagatg     180
   acggagtagc tcatggagaa kataatgcca tcatggaagg catagtgcag tcatggagat     240
   gatggtgcag ctcatggaga agatggtgcc atcatggaag gcatggtgca atcatggagt     300
   agacagtgca gctgggccaa gattctccct gactaagctc ttctcaggca cctctgagcc     360
   gtcgtcttaa ctaggcctcc agcttggctt gtgaaaactg cagactctca gcacaaatga     420
   tttgcctcct acattaagag acttaaataa acacttgcat ggctgtgttt atttaaacag     480
   ctcaaggctg tgtccctggg atgacaatga ctccagcccc taaaattcct gcttgtgaaa     540
   gctcattgct gacagaagga tctaccattt gttccagcca acacctggtg gcaggcagat     600
   aggccctgag ccccatttaa gagcagttcc tttagaaagc ttgcaattgt aaatcttttc     660
   tctgcccatt tgagatgtaa atcttctacc acctagaact gtcttctcaa ggacctgtga     720
   gctgactcac tgaaatgcaa acattcaggg agataactcc actcctgtcc ccatgcgacg     780
   gcgaggccct gactttggtg ggcaccttgc tcttatttgc accaccacct cctgtcctaa     840
   agacatgaga cgtttgtctc tcctctggat aagtgcctat taaccaaccc aggtgtcctg     900
   gtcacatgaa ccagtccagc ctagcacctg gcactgcctt tccctcagca cactccagtc     960
   tgtaaaagtc tccttatggt tgttttggca aagttgagct tagttaatgc tagacccctt    1020
   ctctactgca atagttactg ctgaataaag tctatcctta ccactttaac tagtgttggg    1080
   ctttgtttct ctttcataag ctcatggaga agacaatgca gttccatcaa gtttctggct    1140
   cttacactgc taacagtcag ctctggggtc cctgagaggg acagactcac acca          1194
<210> 422
   <211> 1194
   <212> DNA
   <213> Homo sapiens
<400> 422
   gcattcagcc ttgctacgtc tccttcagat gggcgcacta gatactgagt gatgatcatg 60
   ccttgtctag gatctcacca agacagttca tgaaagagac agtgcagctc atggaggaga 120
   tggtgcagct cacagagagg atggtgccat catggaaagc atggggcagt catggagatg 180
   acggagtagc tcatggagaa kataatgcca tcatggaagg catagtgcag tcatggagat 240
   gatggtgcag ctcatggaga agatggtgcc atcatggaag gcatggtgca atcatggagt 300
   agacagtgca gctgggccaa gattctccct gactaagctc ttctcaggca cctctgagcc 360
   gtcgtcttaa ctaggcctcc agcttggctt gtgaaaactg cagactctca gcacaaatga 420
   tttgcctcct acattaagag acttaaataa acacttgcat ggctgtgttt atttaaacag 480
   ctcaaggctg tgtccctggg atgacaatga ctccagcccc taaaattcct gcttgtgaaa 540
   gctcattgct gacagaagga tctaccattt gttccagcca acacctggtg gcaggcagat 600
   aggccctgag ccccatttaa gagcagttcc tttagaaagc ttgcaattgt aaatcttttc 660
   tctgcccatt tgagatgtaa atcttctacc acctagaact gtcttctcaa ggacctgtga 720
   gctgactcac tgaaatgcaa acattcaggg agataactcc actcctgtcc ccatgcgacg 780
   gcgaggccct gactttggtg ggcaccttgc tcttatttgc accaccacct cctgtcctaa 840
   agacatgaga cgtttgtctc tcctctggat aagtgcctat taaccaaccc aggtgtcctg 900
   gtcacatgaa ccagtccagc ctagcacctg gcactgcctt tccctcagca cactccagtc 960
   tgtaaaagtc tccttatggt tgttttggca aagttgagct tagttaatgc tagacccctt 1020
   ctctactgca atagttactg ctgaataaag tctatcctta ccactttaac tagtgttggg 1080
   ctttgtttct ctttcataag ctcatggaga agacaatgca gttccatcaa gtttctggct 1140
   cttacactgc taacagtcag ctctggggtc cctgagaggg acagactcac acca 1194
<210> 423
   <211> 1118
   <212> DNA
   <213> Homo sapiens
<400> 423
   accaagacag ttcatgaaag agacagtgca gctcatggag gagatggtgc agctcacaga 60
   gaggatggtg ccatcatgga aagcatgggg cagtcatgga gatgacggag tagctcatgg 120
   agaagataat gccatcatgg aaggcatagt gcagtcatgg agatgatggt gcagctcatg 180
   gagaagatgg tgccatcatg raaggcatgg tgcaatcatg gagtagacag tgcagctggg 240
   ccaagattct ccctgactaa gctcttctca ggcacctctg agccgtcgtc ttaactaggc 300
   ctccagcttg gcttgtgaaa actgcagact ctcagcacaa atgatttgcc tcctacatta 360
   agagacttaa ataaacactt gcatggctgt gtttatttaa acagctcaag gctgtgtccc 420
   tgggatgaca atgactccag cccctaaaat tcctgcttgt gaaagctcat tgctgacaga 480
   aggatctacc atttgttcca gccaacacct ggtggcaggc agataggccc tgagccccat 540
   ttaagagcag ttcctttaga aagcttgcaa ttgtaaatct tttctctgcc catttgagat 600
   gtaaatcttc taccacctag aactgtcttc tcaaggacct gtgagctgac tcactgaaat 660
   gcaaacattc agggagataa ctccactcct gtccccatgc gacggcgagg ccctgacttt 720
   ggtgggcacc ttgctcttat ttgcaccacc acctcctgtc ctaaagacat gagacgtttg 780
   tctctcctct ggataagtgc ctattaacca acccaggtgt cctggtcaca tgaaccagtc 840
   cagcctagca cctggcactg cctttccctc agcacactcc agtctgtaaa agtctcctta 900
   tggttgtttt ggcaaagttg agcttagtta atgctagacc ccttctctac tgcaatagtt 960
   actgctgaat aaagtctatc cttaccactt taactagtgt tgggctttgt ttctctttca 1020
   taagctcatg gagaagacaa tgcagttcca tcaagtttct ggctcttaca ctgctaacag 1080
   tcagctctgg ggtccctgag agggacagac tcacacca 1118
<210> 424
   <211> 601
   <212> DNA
   <213> Homo sapiens
<400> 424
   gtaggggcac tgtctatact ggctgcactc tggccagtgc tgtcccaacg ctgacccctc 60
   tggaagctaa tctggcttat aatgaggatg ctttctttag aggggactct ccatgcacag 120
   cagaaaatcc caatggagtg gttcttccct atgtccccaa gggactggga atattctttc 180
   agtaacaatg gcccattggg ggaagaagga tgaaagtggg gtgagagacg tgaaatttgg 240
   agaggtccct caaagattgt gatgtgcctc tcttgttcca atcacaggac aggggtataa 300
   yggctttcct ttgaaacacg gggatgaatt taactattca cttcccaggt agattcatca 360
   gggtctagag cttcagctaa cagcatgagg aagattccaa atgtgccccc atcagcatag 420
   gaactgggta tgttgagtct atggtctcat aaaaccagaa gaaggacaag ggattgtggc 480
   tccaggcttg ggagcacctt ttccttacca tgggctacag tatttattta gggtaaagga 540
   aggaaactcc tgaggtgcta tggggtgcca gcaatttgga gcatcagtaa ttcaatgtcc 600
   c 601
<210> 425
   <211> 601
   <212> DNA
   <213> Homo sapiens
<400> 425
   acgctgaccc ctctggaagc taatctggct tataatgagg atgctttctt tagaggggac 60
   tctccatgca cagcagaaaa tcccaatgga gtggttcttc cctatgtccc caagggactg 120
   ggaatattct ttcagtaaca atggcccatt gggggaagaa ggatgaaagt ggggtgagag 180
   acgtgaaatt tggagaggtc cctcaaagat tgtgatgtgc ctctcttgtt ccaatcacag 240
   gacaggggta taacggcttt cctttgaaac acggggatga atttaactat tcacttccca 300
   rgtagattca tcagggtcta gagcttcagc taacagcatg aggaagattc caaatgtgcc 360
   cccatcagca taggaactgg gtatgttgag tctatggtct cataaaacca gaagaaggac 420
   aagggattgt ggctccaggc ttgggagcac cttttcctta ccatgggcta cagtatttat 480
   ttagggtaaa ggaaggaaac tcctgaggtg ctatggggtg ccagcaattt ggagcatcag 540
   taattcaatg tcccttcagc catgtgtatt caactcctgc tgtgggtgtg gacttggtgc 600
   a 601
<210> 426
   <211> 701
   <212> DNA
   <213> Homo sapiens
<400> 426
   ttcctgggca tcgtcatatt ctgtaaaaca aggaagctca gcccagtgtg ttctaacatg        60
   acctcctttc tacatcctta ggtgttgtta tgcgtgaatc acgtcccccc aaaagacatg       120
   ttcatgtcct aacccccagg acctcagaat gtgtgatctg gtttggaaat aaggtcatca       180
   cagatgaaat tagctaagac aaggtcatat tggaataggg ttggccctta atccactgtg       240
   actggtgtcc ttttaagaag aggacacaga cacaggaggg gagagggcca tgggatgatg       300
   caggtggaga ctggagtgct acagctgcaa gcaaatacat ttctgtgctg tgaagccacc       360
   catttggtgg tactacgtta aaacagctct aggaaattaa tacagatgtt gcctgtattt       420
   ttgtttctca tattactact cattgtttta atgatgactg ttttattcat taagttgaaa       480
   gctcctaaag cagagggacc rtatttttat gtcccaactc tccttaaggc cttgcctatg       540
   atagcacatc tcttcaatag aattgtccta actttaacag agacaacttg ggttatttaa       600
   tatggagaac aaagggttaa gctggtgcca gatgggtttc attttctcta aatctggaac       660
   caaaggcagc aagtctatgg ggtggacgga gttcttagct c                           701
<210> 427
   <211> 701
   <212> DNA
   <213> Homo sapiens
<400> 427
   caaggaagct cagcccagtg tgttctaaca tgacctcctt tctacatcct taggtgttgt        60
   tatgcgtgaa tcacgtcccc ccaaaagaca tgttcatgtc ctaaccccca ggacctcaga       120
   atgtgtgatc tggtttggaa ataaggtcat cacagatgaa attagctaag acaaggtcat       180
   attggaatag ggttggccct taatccactg tgactggtgt ccttttaaga agaggacaca       240
   gacacaggag gggagagggc catgggatga tgcaggtgga gactggagtg ctacagctgc       300
   aagcaaatac atttctgtgc tgtgaagcca cccatttggt ggtactacgt taaaacagct       360
   ctaggaaatt aatacagatg ttgcctgtat ttttgtttct catattacta ctcattgttt       420
   taatgatgac tgttttattc attaagttga aagctcctaa agcagaggga ccatattttt       480
   atgtcccaac tctccttaag sccttgccta tgatagcaca tctcttcaat agaattgtcc       540
   taactttaac agagacaact tgggttattt aatatggaga acaaagggtt aagctggtgc       600
   cagatgggtt tcattttctc taaatctgga accaaaggca gcaagtctat ggggtggacg       660
   gagttcttag ctcaaccctt tggtgaggta agaagaagga t                           701

## Description

### FIELD OF THE INVENTION

The present invention relates to methods for determining the fraction of cell-free fetal nucleic acid circulating in a maternal sample.

### BACKGROUND OF THE INVENTION

Invasive prenatal tests are potentially harmful to the mother and to the fetus. Therefore, there is a need for the development of noninvasive prenatal tests. Maternal blood can contain fetal cells (see e.g., U.S. Patent Application Publication No. 20080070792) and cell-free fetal DNA (see e.g., Huang et al. (2008), Methods in Molecular Biology, 444:203-208). While circulating fetal cells present an attractive target for non invasive prenatal diagnostics, particularly for the diagnosis of fetal sex and chromosomal abnormalities by simple karyotyping, the scarcity of intact fetal cells in the maternal circulation (around one cell per ml of maternal blood), low efficiency of enrichment (Bianchi et al., Am J Hum Genet 61:822-829 [1997]) and difficulties with chromosomal analysis associated with abnormally dense nuclei in some cells (Babochkina et al., Haematologica 90:740-745 [2005]), have favored research on cell-free DNA.

The establishment of the concentrations of cell-free fetal DNA (cfDNA) in maternal plasma in healthy pregnant women has formed the platform on which fetal DNA abnormalities in pregnancy-associated disorders can be studied. The finding of a gradual increase in fetal DNA concentration in maternal serum as gestation progresses has been shown to precede complications associated with preterm labor. A five-fold increase in fetal DNA concentration has also been found in the serum obtained from women affected by preeclampsia. Other pregnancy-related disorders that have been linked to an elevated concentration of cfDNA include hyperemesis gravidarum (severe morning sickness), invasive placentation (in which the placenta contacts the maternal bloodstream), intrauterine growth restriction, feto-maternal haemorrhage and polyhydramnios. (Wright C.F. and Burton H., Human Reproduction Update 15(1):139-151 [2009]).

Quantitative analysis of cell free DNA by real-time PCR strategies has also indicated that the concentrations of circulatory fetal DNA are increased in pregnancies with fetal aneuploidies, most notably trisomy 21 (Lo et al., Clin Chem 45:1747-1751 [1999]). However, the fraction of fetal DNA in maternal cell-free plasma DNA is usually determined by comparing the amount of fetal-specific locus (such as the SRY locus on chromosome Y in male pregnancies) to that of a locus on any autosome that is common to both the mother and the fetus by using quantitative real-time PCR (Dahllan et al., Lancet 369:474-481 [2007]; Li et al., Clin Chem 1002-1011 [2004]; Fan et al., Proc Natl Acad Sci 105:16266-16271 [2008]).

Thus, there is a need for additional methods that would enable the determination of the fraction of fetal nucleic acid in both male and female pregnancies.

The method of the invention fulfills the need in providing the means to determine fetal fraction that is independent of the gender of the fetus. The method can be applied for determining simultaneously the presence or absence of a chromosomal aneuploidy or other copy number variation, and may be used in conjunction with nay known methods that are used to determine aneuploidies in maternal sample.

### SUMMARY OF THE INVENTION

The invention is as defined in the claims.

In one embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic nucleic acids are located on a plurality of different chromosomes. In one embodiment, the plurality of polymorphic nucleic acids can be located on chromosomes 1-22. In another embodiment, the plurality of polymorphic nucleic acids can be located on different autosomes other than chromosomes 13, 18 and 21. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22 For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The massively parallel sequencing is sequencing-by-synthesis with reversible dye terminators. Alternatively, the massively parallel sequencing is sequencing-by-ligation or single molecule sequencing. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs101733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs101733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs101733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs101733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The maternal sample is selected from blood, plasma, serum, urine and saliva. Preferably, the maternal sample is a plasma sample.

In one embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs111088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, the disclosure provides a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs119037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method is a fetal gender-independent method.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids are located on a plurality of different chromosomes other than chromosomes 13, 18, 21, X or Y. The method further comprises preamplifying the mixture of fetal and maternal nucleic acids. The method is a fetal gender-independent method.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises resolving the size of the STRs using capillary electrophoresis. The method is a fetal gender-independent method.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises preamplifying the mixture of fetal and maternal nucleic acids, and resolving the size of the STRs using capillary electrophoresis. The method is a fetal gender-independent method.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids can be located on a plurality of different chromosomes. For example, the plurality of polymorphic nucleic acids are located on a plurality of different chromosomes other than chromosomes 13, 18, 21, X or Y. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA. The method is a fetal gender-independent method.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises preamplifying the mixture of fetal and maternal nucleic acids. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA. The method is a fetal gender-independent method.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids are located on a plurality of different chromosomes other than chromosomes 13, 18, 21, X or Y. The method further comprises resolving the size of the STRs using capillary electrophoresis. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA.

In another embodiment, the disclosure provides a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises preamplifying the mixture of fetal and maternal nucleic acids, and resolving the size of the STRs using capillary electrophoresis. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in said mixture. The set of primers does not amplify a sequence on the Y chromosome.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in said mixture. The polymorphic nucleic acid comprises an STR, a SNP and/or a tandem SNP.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in said mixture. The polymorphic nucleic acid comprises an STR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113; and/or a SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, rs530022; and/or a tandem SNP rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392-rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; andrs2837381-rs4816672.

In another embodiment, the disclosure provides a composition for determining the fraction of fetal cfDNA in a maternal sample by massively parallel sequencing the fetal and maternal cfDNA in the maternal sample, *e.g.* a plasma sample, wherein the composition comprises at least one set of primers for amplifying at least one polymorphic nucleic acid in said mixture.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in said mixture. The polymorphic nucleic acid comprises an STR, a SNP and/or a tandem SNP.

In another embodiment, the disclosure provides a composition for determining the fraction of fetal cfDNA in a maternal sample by massively parallel sequencing the fetal and maternal cfDNA in the maternal sample, *e.g.* a plasma sample, wherein the composition comprises at least one set of primers for amplifying at least one polymorphic nucleic acid in said mixture. The polymorphic nucleic acid comprises an STR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113; a SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022; and/or a tandem SNP rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in said mixture. The set of primers does not amplify a sequence on the Y chromosome.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in the mixture. The set of primers does not amplify a sequence on the Y chromosome. The polymorphic nucleic acid comprises an STR, a SNP and/or a tandem SNP.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in said mixture. The polymorphic nucleic acid comprises an STR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113; a SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022; and/or a tandem SNP rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

In another embodiment, the disclosure provides a composition for determining the fraction of fetal cfDNA in a maternal sample by massively parallel sequencing the fetal and maternal cfDNA in the maternal sample, *e.g.* a plasma sample, wherein the composition comprises at least one set of primers for amplifying at least one polymorphic nucleic acid in said mixture.

In another embodiment, the disclosure provides a composition for determining the fraction of fetal cfDNA in a maternal sample, *e.g.* a plasma sample, wherein the composition comprises at least one set of primers for amplifying at least one polymorphic nucleic acid in said mixture. The set of primers does not amplify a sequence on the Y chromosome. The polymorphic nucleic acid comprises an STR, a SNP and/or a tandem SNP.

In another embodiment, the disclosure provides a composition for determining the fraction of fetal cfDNA in a maternal sample by massively parallel sequencing the fetal and maternal cfDNA in the maternal sample, *e.g.* a plasma sample, wherein the composition comprises at least one set of primers for amplifying at least one polymorphic nucleic acid in said mixture. The polymorphic nucleic acid comprises an STR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113; a SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022; and/or a tandem SNP rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

### BRIEF DESCRIPTION OF THE DRAWINGS

**Figure 1** is a flowchart of a method **100** for determining the fetal fraction in a maternal test sample comprising a mixture of fetal and maternal nucleic acids using massively parallel sequencing methods or size separation of polymorphic nucleic acid sequences.
**Figure 2** is a bar diagram showing the identification of fetal and maternal polymorphic sequences (SNPs) used to determine fetal fraction in a test sample. The total number of sequence reads (Y-axis) mapped to the SNP sequences identified by rs numbers (X-axis), and the relative level of fetal nucleic acids (*) are shown.
**Figure 3** is a flowchart outlining alternative embodiments of the method for determining fetal fraction by massively parallel sequencing shown in **Figure 1****.**
**Figure 4** illustrates STR markers used in the AmpFℓSTR® Identifiler® PCR Amplification Kit.
**Figure 5** illustrates STR markers used in the AmpFℓSTR® MiniFiler® PCR Amplification Kit.
**Figure 6** illustrates the correlation of fetal fraction determined by massively parallel sequencing size separation of polymorphic sequences comprising SNPs and STRs.
**Figure 7** illustrates an embodiment of use of fetal fraction for determining cutoff thresholds for aneuploidy detection.

### DETAILED DESCRIPTION OF THE INVENTION

The invention provides methods for determining the fraction of fetal nucleic acids in a maternal sample comprising a mixture of fetal and maternal nucleic acids. The fraction of fetal nucleic acids can be used in determining the presence or absence of fetal aneuploidy. The invention is as set out in the appended claims.

Unless otherwise indicated, the practice of the present invention involves conventional techniques commonly used in molecular biology, microbiology, protein purification, protein engineering, protein and DNA sequencing, and recombinant DNA fields, which are within the skill of the art. Such techniques are known to those of skill in the art and are described in numerous standard texts and reference works.

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### DEFINITIONS

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

The term "portion" when used in reference to the amount of sequence information of fetal and maternal nucleic acid molecules in a biological sample herein refers to the amount of sequence information of fetal and maternal nucleic acid molecules in a biological sample that in sum amount to less than the sequence information of <1 human genome.

The terms "polynucleotide", "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next, include sequences of any form of nucleic acid, including, but not limited to RNA, DNA and cfDNA molecules. The term "polynucleotide" includes, without limitation, single- and doublestranded polynucleotide.

The term "copy number variation" herein refers to variation in the number of copies of a nucleic acid sequence that is 1 kb or larger present in a test sample in comparison with the copy number of the nucleic acid sequence present in a qualified sample. A "copy number variant" refers to the 1 kb or larger sequence of nucleic acid in which copy-number differences are found by comparison of a sequence of interest in test sample with that present in a qualified sample. Copy number variants/variations include deletions, including microdeletions, insertions, including microinsertions, duplications, multiplications, inversions, translocations and complex multi-site variants. CNV encompass chromosomal aneuploidies and partial aneuplodies.

As used herein, the term "fetal fraction" is used interchangeably with "fraction of fetal nucleic acid", which refers to the fraction of fetal nucleic acid in a sample comprising fetal and maternal nucleic acid. Similarly, the term "minor fraction" or "minor component" herein refers to the lesser fraction of the total genetic material that is present in a sample containing genetic material derived from separate sources *e.g.* individuals.

As used herein the term "allele" refers to one specific form of a genetic sequence (such as a gene) within a cell, a sample, an individual or within a population, the specific form differing from other forms of the same gene in the sequence of at least one, and frequently more than one, variant sites within the sequence of the gene. The sequences at these variant sites that differ between different alleles are termed "variants", "polymorphisms", or "mutations". In general, polymorphism is used to refer to variants that have a frequency of at least 1% in a population, while the term mutation is generally used for variants that occur at a frequency of less than 1 % in a population. In diploid organisms such as humans, at each autosomal specific chromosomal location or "locus" an individual possesses two alleles, a first inherited from one parent and a second inherited from the other parent, for example one from the mother and one from the father. An individual is "heterozygous" at a locus if it has two different alleles at the locus. An individual is "homozygous" at a locus if it has two identical alleles at that locus.

The term "enrich" herein refers to the process of amplifying polymorphic target nucleic acids contained in a portion of a maternal sample, and combining the amplified product with the remainder of the maternal sample from which the portion was removed.

As used herein, the term "genotyping" refers to the determination of the genetic information an individual carries at one or more positions in the genome. For example, genotyping may comprise the determination of which allele or alleles an individual carries for a single SNP or the determination of which allele or alleles an individual carries for a plurality of SNPs. For example, a particular nucleotide in a genome may be a T in some individuals and a C in other individuals. Those individuals who have a T at the position have the T allele and those who have a C have the C allele. In a diploid organism the individual will have two copies of the sequence containing the polymorphic position so the individual may have a T allele and a C allele or alternatively two copies of the T allele or two copies of the C allele. Those individuals who have two copies of the C allele are homozygous for the C allele, those individuals who have two copies of the T allele are homozygous for the T allele, and those individuals who have one copy of each allele are heterozygous. The alleles are often referred to as the A allele, often the major allele, and the B allele, often the minor allele. The genotypes may be AA (homozygous A), BB (homozygous B) or AB (heterozygous). Genotyping methods generally provide for identification of the sample as AA, BB or AB.

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multi-chromosomal genome and from one genome to another. In the case of the human genome, the entire DNA mass of a given chromosome is usually greater than about 100,000,000 bp. For example, the size of the entire human genome is about 3.times.10.sup.9 bp. The largest chromosome, chromosome no. 1, contains about 2.4.times.10.sup.8 by while the smallest chromosome, chromosome no. 22, contains about 5.3.times.10.sup.7 bp.

The term "aneuploidy" herein refers to the occurrence of one or more extra or missing chromosomes.

As used herein the term "chromosomal region" is a portion of a chromosome. The actual physical size or extent of any individual chromosomal region can vary greatly. The term "region" is not necessarily definitive of a particular one or more genes because a region need not take into specific account the particular coding segments (exons) of an individual gene.

As used herein the term "genetic marker" refers to a sequence of DNA that has a specific location on a chromosome that can be measured in a laboratory. The term "genetic marker" can also be used to refer to, e.g., a cDNA and/or an mRNA encoded by a genomic sequence, as well as to that genomic sequence. To be useful, a marker needs to have two or more alleles or variants. Markers can be either direct, that is, located within the gene or locus of interest (i.e., candidate gene), or indirect, that is closely linked with the gene or locus of interest (presumably due to a location which is proximate to, but not inside the gene or locus of interest). Moreover, markers can also include sequences which either do or do not modify the amino acid sequence of a gene.

As used herein, the term "maternal sample" refers to a biological sample obtained from a pregnant subject, and comprises a mixture of fetal and maternal nucleic acids. A "pregnant subject" is not limited to a human being, but may also include other organisms including but not limited to mammals, plants, bacteria or cells derived from any of the above.

The term "whole genome amplification" or "WGA" as used herein generally refers to a method for amplification of a limited DNA sample in a non-specific manner, in order to generate a new sample that is indistinguishable from the original but with a higher DNA concentration. The ideal whole genome amplification technique would amplify a sample up to a microgram level while maintaining the original sequence representation. The DNA of the sample may include an entire genome or a portion thereof. Degenerate oligonucleotide-primed PCR (DOP), primer extension PCR technique (PEP) including modified improved primer extension preamplification (mIPEP), and multiple displacement amplification (MDA), are examples of whole genome amplification methods.

The term "short tandem repeat" or "STR" as used herein refers to a class of polymorphisms that occurs when a pattern of two or more nucleotides are repeated and the repeated sequences are directly adjacent to each other. The pattern can range in length from 2 to 10 base pairs (bp) (for example (CATG)n in a genomic region) and is typically in the non-coding intron region. By examining several STR loci and counting how many repeats of a specific STR sequence there are at a given locus, it is possible to create a unique genetic profile of an individual.

The term "primer," as used herein refers to an isolated oligonucleotide which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, use of the method, and the parameters used for primer design, as disclosed herein.

The term "primer pair" or "primer set" refers to a set of primers including a 5' "upstream primer" or "forward primer" that hybridizes with the complement of the 5' end of the DNA sequence to be amplified and a 3' "downstream primer" or "reverse primer" that hybridizes with the 3' end of the sequence to be amplified. As will be recognized by those of skill in the art, the terms "upstream" and "downstream" or "forward" and "reverse" are not intended to be limiting, but rather provide illustrative orientation in particular embodiments. A primer pair is said to be "unique" if it can be employed to specifically amplify a particular target nucleotide sequence in a given amplification mixture.

A "polymorphic marker" or "polymorphic site" is a locus at which nucleotide sequence divergence occurs. The locus may be as small as one base pair. Illustrative markers have at least two alleles, each occurring at frequency of greater than 1%, and more typically greater than 10% or 20% of a selected population. A polymorphic site may be as small as one base pair. Polymorphic markers include restriction fragment length polymorphism (RFLPs), variable number of tandem repeats (VNTR's), hypervariable regions, minisatellites, dinucleotide repeats, trinucleotide repeats, tetranucleotide repeats, simple sequence repeats, deletions, and insertion elements such as Alu. The first identified allelic form is arbitrarily designated as the reference form and other allelic forms are designated as alternative or variant alleles. The allelic form occurring most frequently in a selected population is sometimes referred to as the wild type form. Diploid organisms may be homozygous or heterozygous for allelic forms. A diallelic polymorphism has two forms. A triallelic polymorphism has three forms. A polymorphism between two nucleic acids can occur naturally, or be caused by exposure to or contact with chemicals, enzymes, or other agents, or exposure to agents that cause damage to nucleic acids, for example, ultraviolet radiation, mutagens or carcinogens. The terms "polymorphic locus" and "polymorphic site" are herein used interchangeably.

The terms "polymorphic target nucleic acid", "polymorphic sequence", "polymorphic target nucleic acid sequence" and "polymorphic nucleic acid" are used interchangeably herein to refer to a nucleic acid sequence *e.g.* a DNA sequence, that comprises one or more polymorphic sites *e.g* one SNP or a tandem SNP. Polymorphic sequences according to the present technology can be used to specifically differentiate between maternal and non-maternal alleles in the maternal sample comprising a mixture of fetal and maternal nucleic acids.

A "single nucleotide polymorphism" (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A SNP usually arises due to substitution of one nucleotide for another at the polymorphic site. A transition is the replacement of one purine by another purine or one pyrimidine by another pyrimidine. A transversion is the replacement of a purine by a pyrimidine or vice versa. SNPs can also arise from a deletion of a nucleotide or an insertion of a nucleotide relative to a reference allele. Single nucleotide polymorphisms (SNPs) are positions at which two alternative bases occur at appreciable frequency (>1%) in the human population, and are the most common type of human genetic variation.

As used herein, the term "short tandem repeat" or "STR" as used herein refers to a class of polymorphisms that occurs when a pattern of two or more nucleotides are repeated and the repeated sequences are directly adjacent to each other. The pattern can range in length from 2 to 10 base pairs (bp) (for example (CATG)n in a genomic region) and is typically in the non-coding intron region. By examining several STR loci and counting how many repeats of a specific STR sequence there are at a given locus, it is possible to create a unique genetic profile of an individual.

As used herein, the term "miniSTR" herein refers to tandem repeat of four or more base pairs that spans less than about 300 base pairs, less than about 250 base airs, less than about 200 base pairs, less than about 150 base pairs, less than about 100 base pairs, less than about 50 base pairs, or less than about 25 base pairs. "miniSTRs" are STRs that are amplifiable from cfDNA templates.

The term "tandem SNPs" herein refers to two or more SNPs that are present within a polymorphic target nucleic acid sequence.

The terms "plurality of polymorphic target nucleic acids", "polymorphic nucleic acids" and "polymorphic sequences" are used interchangeably herein and refer to a number of nucleic acid sequences each comprising at least one polymorphic site *e.g.* one SNP, such that at least 1, 2, 3, 4, 5, 6, 7, 8, 9, 10, 15, 20, 25, 30, or 40 or more different polymorphic sites are amplified from the polymorphic target nucleic acids to identify and/or quantify fetal alleles present in maternal samples comprising fetal and maternal nucleic acids.

As used herein, the term "substantially cell free" encompasses preparations of the desired sample from which components that are normally associated with it are removed. For example, a plasma sample is rendered essentially cell free by removing blood cells *e.g.* red cells, that are normally associated with it. In some embodiments, substantially free samples are processed to remove cells that would otherwise contribute to the desired genetic material that is to be tested for an abnormality

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multi-chromosomal genome and from one genome to another. In the case of the human genome, the entire DNA mass of a given chromosome is usually greater than about 100,000,000 bp. For example, the size of the entire human genome is about 3.times.10.sup.9 bp. The largest chromosome, chromosome no. 1, contains about 2.4.times.10.sup.8 by while the smallest chromosome, chromosome no. 22, contains about 5.3.times.10.sup.7 bp.

The term "oligonucleotide" is used to refer to a nucleic acid that is relatively short, generally shorter than 200 nucleotides, more particularly, shorter than 100 nucleotides, most particularly, shorter than 50 nucleotides. Typically, oligonucleotides are single-stranded DNA molecules.

The term "primer" refers to an oligonucleotide that is capable of hybridizing (also termed "annealing") with a nucleic acid and serving as an initiation site for nucleotide (RNA or DNA) polymerization under appropriate conditions (i.e., in the presence of four different nucleoside triphosphates and an agent for polymerization, such as DNA or RNA polymerase or reverse transcriptase) in an appropriate buffer and at a suitable temperature. The appropriate length of a primer depends on the intended use of the primer, but primers are typically at least 7 nucleotides long and, more typically range from 10 to 30 nucleotides, or even more typically from 15 to 30 nucleotides, in length. Other primers can be somewhat longer, e.g., 30 to 50 nucleotides long.

The term "allele call" as used herein, refers to successful characterization of an allele by a given analysis method. If the analysis provides successful characterization of both alleles of a gene locus of a DNA sample, it is said that two allele calls are made. If one allele is characterized while the other allele is not characterized, it is said that one allele call is made. If neither of the two alleles is successfully characterized, no allele calls are made.

The term "allele" as used herein, is any one of a number of viable DNA codings occupying a given locus (position) on a chromosome. Usually alleles are DNA (deoxyribonucleic acid) sequences that code for a gene, but sometimes the term is used to refer to a non-gene sequence. An individual's genotype for that gene is the set of alleles it happens to possess. In a diploid organism, one that has two copies of each chromosome, two alleles make up the individual's genotype.

The term "reaction mixture" as used herein refers to a mixture containing sufficient components to carry out an amplification reaction.

The term "sequence tag density" herein refers to the number of sequence reads that are mapped to a reference genome sequence *e.g.* the sequence tag density for chromosome 21 is the number of sequence reads generated by the sequencing method that are mapped to chromosome 21 of the reference genome. The term "sequence tag density ratio" herein refers to the ratio of the number of sequence tags that are mapped to a chromosome of the reference genome *e.g.* chromosome 21, to the length of the reference genome chromosome 21.

The terms "threshold value" and "qualified threshold value" herein refer to any number that is calculated using a qualifying data set and serves as a limit of diagnosis of a copy number variation *e.g.* an aneuploidy, in an organism. If a threshold is exceeded a subject can be diagnosed with a copy number variation *e.g.* trisomy 21.

The term "read" refers to a DNA sequence of sufficient length (*e.g.,* at least about 30 bp) that can be used to identify a larger sequence or region, e.g.that can be aligned and specifically assigned to a chromosome or genomic region or gene.

The term "sequence tag" is herein used interchangeably with the term "mapped sequence tag" to refer to a sequence read that has been specifically assigned *i.e.* mapped, to a larger sequence *e.g.* a reference genome, by alignment. Mapped sequence tags are uniquely mapped to a reference genome *i.e.* they are assigned to a single location to the reference genome. Tags that can be mapped to more than one location on a reference genome *i.e.* tags that do not map uniquely, are not included in the analysis.

The terms "aligned", "alignment", or "aligning" refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysis pipeline. The matching of a sequence read in aligning can be a 100% sequence match or less than 100% (non-perfect match).

The term "reference genome" refers to any particular known genome sequence, whether partial or complete, of any organism or virus which may be used to reference identified sequences from a subject. For example, a reference genome used for human subjects as well as many other organisms is found at the National Center for Biotechnology Information at www.ncbi.nlm.nih.gov. A "genome" refers to the complete genetic information of an organism or virus, expressed in nucleic acid sequences.

The term "artificial target sequences genome" herein refers to a grouping of known sequences that encompass alleles of known polymorphic sites. For example, a "SNP reference genome" is an artificial target sequences genome comprising a grouping of sequences that encompass alleles of known SNPs.

The term "clinically-relevant sequence" herein refers to a nucleic acid sequence that is known or is suspected to be associated or implicated with a genetic or disease condition. Determining the absence or presence of a clinically-relevant sequence can be useful in determining a diagnosis or confirming a diagnosis of a medical condition, or providing a prognosis for the development of a disease.

The term "mixed sample" herein refers to a sample containing a mixture of nucleic acids, which are derived from different genomes.

The term "original maternal sample" herein refers to a biological sample obtained from a pregnant subject *e.g.* a woman, who serves as the source from which a portion is removed to amplify polymorphic target nucleic acids. The "original sample" can be any sample obtained from a pregnant subject, and the processed fractions thereof *e.g.* a purified cfDNA sample extracted from a maternal plasma sample. The term "original maternal sample" herein refers to a biological sample obtained from a pregnant subject *e.g.* a woman, who serves as the source from which a portion is removed to amplify polymorphic target nucleic acids. The "original sample" can be any sample obtained from a pregnant subject, and the processed fractions thereof *e.g.* a purified cfDNA sample extracted from a maternal plasma sample.

The term "biological fluid" herein refers to a liquid taken from a biological source and includes, for example, blood, serum, plasma, sputum, lavage fluid, cerebrospinal fluid, urine, semen, sweat, tears, saliva, and the like. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The terms "maternal nucleic acids" and "fetal nucleic acids" herein refer to the nucleic acids of a pregnant female subject and the nucleic acids of the fetus being carried by the pregnant female, respectively.

The term "corresponding to" herein refers to a nucleic acid sequence *e.g.* a gene or a chromosome, that is present in the genome of different subjects, and which does not necessarily have the same sequence in all genomes, but serves to provide the identity rather than the genetic information of a sequence of interest *e.g.* a gene or chromosome.

The term "group of chromosomes" herein refers to two or more chromosomes.

The term "subject" herein refers to a human subject as well as a non-human subject such as a mammal.

### DESCRIPTION

The methods described herein enable the determination of the fraction of the minor fetal nucleic acid component in a sample comprising a mixture of fetal and maternal nucleic acids. In particular, the method enables the determination of the fraction of cfDNA contributed by a fetus to the mixture of fetal and maternal cfDNA in a maternal sample *e.g.* a plasma sample. The difference between the maternal and fetal fraction is determined by the relative contribution of a polymorphic allele derived from the fetal genome to the contribution of the corresponding polymorphic allele derived from the maternal genome. Polymorphic sequences can be used in conjunction with clinically-relevant diagnostic tests as a positive control for the presence of cfDNA in order to highlight false-negative or false-positive results stemming from low levels of cfDNA below the identification limit. The methods described are independent of the gender of the fetus, and are useful across a range of gestational ages.

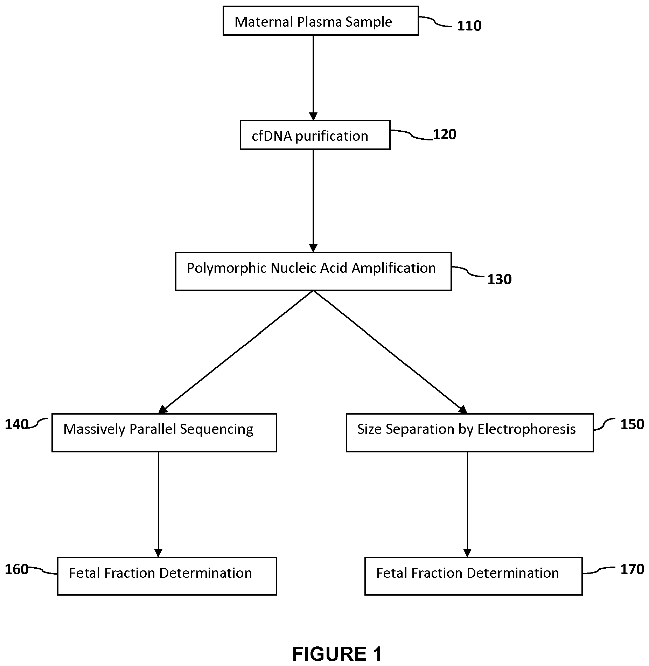

**Figure 1** provides a flow diagram of an embodiment **100** for determining the fraction of fetal nucleic acids in a maternal biological sample by massively parallel sequencing of PCR-amplified polymorphic target nucleic acids. In step **110** a maternal sample comprising a mixture of fetal and maternal nucleic acids is obtained from a subject. The sample is a maternal sample that is obtained from a pregnant female, for example a pregnant woman. Other maternal samples can be from mammals, for example, cow, horse, dog, or cat. If the subject is a human, the sample can be taken in the first or second trimester of pregnancy. Any maternal biological sample can be used a source of fetal and maternal nucleic acids which are contained in cells or that are "cell-free". In some embodiments, it is advantageous to obtain a maternal sample that comprises cell-free nucleic acids *e.g.* cfDNA. Preferably, the maternal biological sample is a biological fluid sample. A biological fluid includes, as non-limiting examples, blood, plasma, serum, sweat, tears, sputum, urine, sputum, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples. In some embodiments, the biological fluid sample is a sample that is easily obtainable by non-invasive procedures *e.g.* blood, plasma, serum, sweat, tears, sputum, urine, sputum, ear flow, and saliva. In some embodiments, the biological sample is a peripheral blood sample, or the plasma and/or the serum fractions thereof. In another embodiment, the sample is a mixture of two or more biological samples *e.g.* a biological sample can comprise two or more of a biological fluid samples. As used herein, the terms "blood," "plasma" and "serum" expressly encompass fractions or processed portions thereof. In some embodiments, the biological sample is processed to obtain a sample fraction *e.g.* plasma, that contains the mixture of fetal and maternal nucleic acids. A sample that can be used to determine the genotype of one or more fetal alleles can be any sample that contains fetal cells or fetal nucleic acid. For example, maternal serum or plasma sample comprising fetal and maternal cell-free nucleic acids (e.g., DNA or RNA) can be used to determine the genotypes of fetal alleles. In one embodiment, the sample can comprise a fetal cell, e.g., a fetal nucleated red blood cell or a trophoblast.

In step **120,** the mixture of fetal and maternal nucleic acids is further processed from the sample fraction *e.g.* plasma, to obtain a sample comprising a purified mixture of fetal and maternal nucleic acids *e.g.* cfDNA. Cell-free nucleic acids, including cell-free DNA, can be obtained by various methods known in the art from biological samples including but not limited to plasma, serum and urine (Fan et al., Proc Natl Acad Sci 105:16266-16271 [2008]; Koide et al., Prenatal Diagnosis 25:604-607 [2005]; Chen et al., Nature Med. 2: 1033-1035 [1996]; Lo et al., Lancet 350: 485-487 [1997). To separate cfDNA from cells, fractionation, centrifugation (e.g., density gradient centrifugation), DNA-specific precipitation, or high-throughput cell sorting and/or separation methods can be used. Examples of methods for processing fluid samples have been previously disclosed, e.g., U.S. Patent Application Nos. 20050282293, 20050224351, and 20050065735. Commercially available kits for manual and automated separation of cfDNA are available (Roche Diagnostics, Indianapolis, IN, Qiagen,Valencia, CA, Macherey-Nagel, Duren, DE). In some instances, it can be advantageous to fragment the nucleic acid molecules in the nucleic acid sample. Fragmentation can be random, or it can be specific, as achieved, for example, using restriction endonuclease digestion. Methods for random fragmentation are well known in the art, and include, for example, limited DNAse digestion, alkali treatment and physical shearing. In one embodiment, sample nucleic acids are obtained as cfDNA, which is not subjected to fragmentation. In other embodiments, the sample nucleic acids are obtained as genomic DNA, which is subjected to fragmentation into fragments of approximately 500 or more base pairs, and to which NGS methods can be readily applied.

In step **130,** a portion of the purified mixture of fetal and maternal cfDNA is used for amplifying a plurality of polymorphic target nucleic acids each comprising a polymorphic site. In some embodiments, the target nucleic acids each comprise a SNP. In other embodiments, each of the target nucleic acids comprises a pair of tandem SNPs. In yet other embodiments, each the target nucleic acids comprises an STR. Polymorphic sites that are contained in the target nucleic acids include without limitation single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs) Short Tandem Repeats (STRs), restriction fragment length polymorphism (RFLP), or a polymorphism comprising any other change of sequence in a chromosome. In some embodiments, the polymorphic sites that are encompassed by the method of the invention are located on autosomal chromosomes, thereby enabling the determination of fetal fraction independently of sex of the fetus. Polymorphisms associated with chromosomes other than chromosome 13, 18, 21 and Y can also be used in the methods described herein.

Polymorphisms can be indicative, informative, or both. Indicative polymorphisms indicate the presence of fetal cell-free DNA in a maternal sample. For example, the more there is of a particular genetic sequence, *e.g.* a SNP, the more a method will translate its presence into a particular color intensity, density of color, or some other property which is detectable and measurable and indicative of the presence, absence, and quantity of a particular fragment of DNA and/or particular polymorphism *e.g.* SNP of the embryo. Informative polymorphisms yield information about the fetus - for example, the presence or absence of a disease, genetic abnormality, or any other biological information such as the stage of gestation or gender. With regard to the present invention, the methods are not conducted using all possible SNPs in a genome, but use those which are said to be "informative". "Informative SNPs" in this instance are those which identify differences in the sequence of the mother and the fetus. Any polymorphic site that can be encompassed by the reads generated by the sequencing methods described herein can be used to determine the fetal fraction.

In one embodiment, a portion of the mixture of fetal and maternal nucleic acids in the sample *e.g.* cfDNA, is used as template for amplifying target nucleic acids that comprise at least one SNP. In some embodiments, each target nucleic acid comprises a single *i.e.* one SNP. Target nucleic acid sequences comprising SNPs are available from publicly accessible databases including, but not limited to Human SNP Database at world wide web address wi.mit.edu, NCBI dbSNP Home Page at world wide web address ncbi.nlm.nih.gov, world wide web address lifesciences.perkinelmer.com, Applied Biosystems by Life Technologies™ (Carlsbad, CA) at world wide web address appliedbiosystems.com, Celera Human SNP database at world wide web address celera.com, the SNP Database of the Genome Analysis Group (GAN) at world wide web address gan.iarc.fr. In one embodiment, the SNPs chosen for enriching the fetal and maternal cfDNA are selected from the group of 92 individual identification SNPs (IISNPs) described by Pakstis *el al.* (Pakstis et el. Hum Genet 127:315-324 [2010]), which have been shown to have a very small variation in frequency across populations (Fₛₜ <0.06), and to be highly informative around the world having an average heterozygosity ≥0.4. SNPs that are encompassed by the method of the invention include linked and unlinked SNPs. Other useful SNPs applicable or useful for the methods described herein are disclosed in U.S. Pat. Application Nos. 20080070792, 20090280492, 20080113358, 20080026390, 20080050739, 20080220422, and 20080138809. Each target nucleic acid comprises at least one polymorphic site *e.g.* a single SNP, that differs from that present on another target nucleic acid to generate a panel of polymorphic sites *e.g.* SNPs, that contain a sufficient number of polymorphic sites of which at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more are informative. For example, a panel of SNPs can be configured to comprise at least one informative SNP. In one embodiment, the SNPs that are targeted for amplification are selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In one embodiment, the panel of SNPs comprises at least 3, at least 5, at least 10, at least 13, at least 15, at least 20, at least 25, at least 30 or more SNPs. In one embodiment, the panel of SNPs comprises rs560681, rs1109037, rs9866013, rs13182883, rs13218440, rs7041158, rs740598, rs10773760, rs4530059, rs7205345, rs8078417, rs576261, and rs2567608. The polymorphic nucleic acids comprising the SNPs can be amplified using exemplary primer pairs provided in Example 3, and disclosed as SEQ ID NOs:57-112.

In other embodiments, each target nucleic acid comprises two or more SNPs *i.e.* each target nucleic acid comprises tandem SNPs. Preferably, each target nucleic acid comprises two tandem SNPs. The tandem SNPs are analyzed as a single unit as short haplotypes, and are provided herein as sets of two SNPs. To identify suitable tandem SNP sequences, the International HapMap Consortium database can be searched (The International HapMap Project, Nature 426:789-796 [2003]). The database is available on the world wide web at hapmap.org. In one embodiment, tandem SNPs that are targeted for amplification are selected from the following sets of tandem pairs of SNPS rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The polymorphic nucleic acids comprising the tandem SNPs can be amplified using primer pairs that amplify polymorphic sequences comprising the tandem SNPs. Examples of primer pairs that can be used to amplify the tandem SNPs disclosed herein are SEQ ID NOs:197-310 as provided in Example 8.

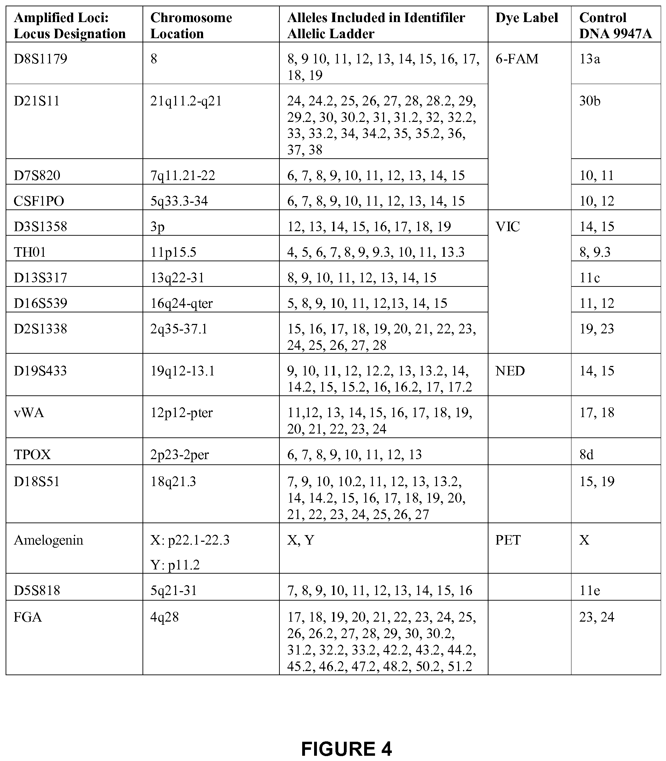

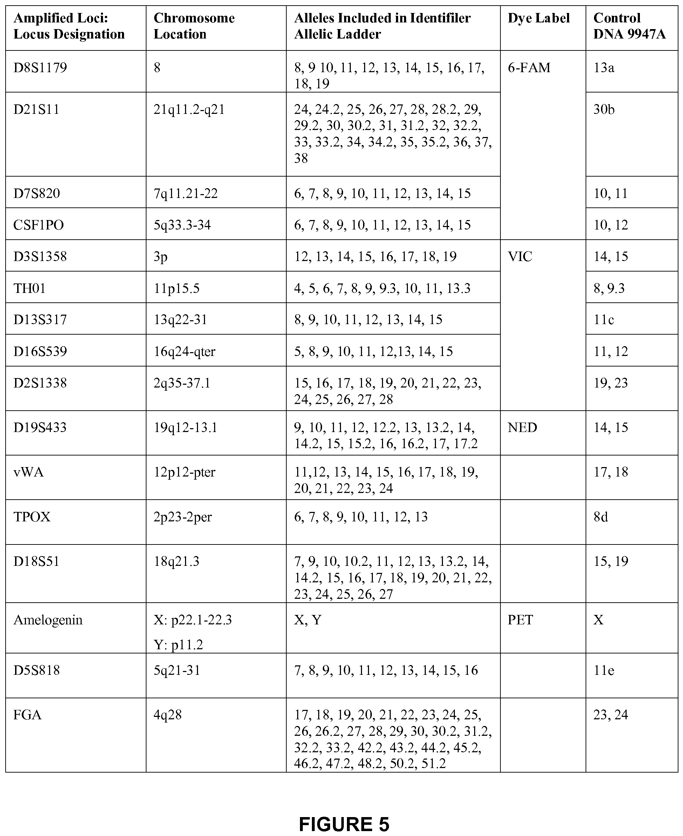

In one embodiment, a portion of the mixture of fetal and maternal nucleic acids in the sample *e.g.* cfDNA, is used as template for amplifying target nucleic acids that comprise at least one STR. In some embodiments, each target nucleic acid comprises a single *i.e.* one STR. STR loci are found on almost every chromosome in the genome and may be amplified using a variety of polymerase chain reaction (PCR) primers. Tetranucleotide repeats have been preferred among forensic scientists due to their fidelity in PCR amplification, although some tri- and pentanucleotide repeats are also in use. A comprehensive listing of references, facts and sequence information on STRs, published PCR primers, common multiplex systems, and related population data are compiled in STRBase, which may be accessed via the World Wide Web at ibm4.carb.nist.gov:8800/dna/home.htm. Sequence information from GenBank® (http://www2.ncbi.nlm.nih.gov/cgi-bin/genbank) for commonly used STR loci is also accessible through STRBase. Commercial kits available for the analysis of STR loci generally provide all of the necessary reaction components and controls required for amplification. STR multiplex systems allow the simultaneous amplification of multiple nonoverlapping loci in a single reaction, substantially increasing throughput. With multicolor fluorescent detection, even overlapping loci can be multiplexed. The polymorphic nature of tandem repeated DNA sequences that are widespread throughout the human genome have made them important genetic markers for gene mapping studies, linkage analysis, and human identity testing. Because of the high polymorphism of STRs, most individuals will be heterozygous *i.e.* most people will possess two alleles (versions) of each-one inherited from each parent-with a different number of repeats. The PCR products comprising the STRs can be separated and detected using manual, semi-automated or automated methods. Semi-automated systems are gel-based and combine electrophoresis, detection and analysis into one unit. On a semiautomated system, gel assembly and sample loading are still manual processes; however, once samples are loaded onto the gel, electrophoresis, detection and analysis proceed automatically. Data collection occurs in "real time" as fluorescently labeled fragments migrate past the detector at a fixed point and can be viewed as they are collected. As the name implies, capillary electrophoresis is carried out in a microcapillary tube rather than between glass plates. Once samples, gel polymer and buffer are loaded onto the instrument, the capillary is filled with gel polymer and the sample is loaded automatically. Therefore, the non-maternally inherited fetal STR sequence will differ in the number of repeats from the maternal sequence. Amplification of these STR sequences can result in one or two major amplification products corresponding to the maternal alleles (and the maternally inherited fetal allele) and one minor product corresponding to the non-maternally inherited fetal allele. This technique was first reported in 2000 (Pertl et al., Human Genetics 106:45-49 [2002]) and has subsequently been developed using simultaneous identification of multiple different STR regions using real-time PCR (Liu et al., Acta Obset Gyn Scand 86:535-541 [2007]). Various sized PCR amplicons have been used to discern the respective size distributions of circulating fetal and maternal DNA species, and have shown that the fetal DNA molecules in the plasma of pregnant women are generally shorter than maternal DNA molecules (Chan et al., Clin Chem 50:8892 [2004]). Size fractionation of circulating fetal DNA has confirmed that the average length of circulating fetal DNA fragments is <300 bp, while maternal DNA has been estimated to be between about 0.5 and 1 Kb (Li et al., Clin Chem, 50: 1002-1011 [2004]). The disclosure provides a method for determining the fraction of fetal nucleic acid in a maternal sample comprising determining the amount of copies of at least one fetal and one maternal allele at a polymorphic miniSTR site, which can be amplified to generate amplicons that are of lengths about the size of the circulating fetal DNA fragments *e.g.* less than about 250 base pairs. In one embodiment, the fetal fraction can be determined by a method that comprises sequencing at least a portion of amplified polymorphic target nucleic acids each comprising a miniSTR. Fetal and maternal alleles at an informative STR site are discerned by their different lengths *i.e.* number of repeats, and the fetal fraction can be calculated as a percent ratio of the amount of fetal maternal alleles at that site. The method can use one or a combination of any number of informative miniSTRs to determine the fraction of fetal nucleic acid. For example, any one or a combination of any number of miniSTRs, for example the miniSTRs disclosed in Table 7 and Figures 4 and 5, can be used. In one embodiment, the fraction of fetal nucleic acid in a maternal sample is performed using a method that includes determining the number of copies of the maternal and fetal nucleic acid present in the maternal sample by amplifying at least one autosomal miniSTR chosen from CSF1PO, FGA, TH01, TPOX. vWA, D3S1358,D5S818, D7S820, D8S1179, D13S317, D16S539, D18S51, D21S11, Penta D, Penta E, D2S1338, D1S1677, D2S441, D4S2364, D10S1248, D14S1434, D22S1045, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D5S2500, D4S2408, D4S2364, D3S4529, D3S3053, D2S1776, D2S441, D1S1677, D1S1627, and D1GATA113. In another embodiment, the at least one autosomal miniSTR is the group of miniSTRs CSF1PO, FGA, D13S317, D16S539, D18S51, D2S1338, D21S11, D2S1338 and D7S820. In one embodiment, the method comprises determining the number of copies of at least one fetal and at least one maternal allele at least at one polymorphic miniSTR that is amplified to generate amplicons that are less than about 300 bp, less than about 250 bp, less than about 200 bp, less than about 150 bp, less than about 100 bp, or less than about 50 bp. In another embodiment, the amplicons that are generated by amplifying the miniSTRs are less than about 300 bp. In another embodiment, the amplicons that are generated by amplifying the miniSTRs are less than about 250 bp. In another embodiment, the amplicons that are generated by amplifying the miniSTRs are less than about 200 bp. Amplification of the informative allele includes using miniSTR primers, which allow for the amplification of reduced-size amplicons to detect STR alleles that are less than about 500 bp, less than about 450 bp, less than about 400 bp, less than about 350 bp, less than about 300 base pairs (bp), less than about 250 bp, less than about 200 bp, less than about 150 bp, less than about 100 bp, or less than about 50 bp. The reduced-size amplicons generated using the miniSTR primers are known as miniSTRs that are identified according to the marker name corresponding to the locus to which they have been mapped. In one embodiment, the miniSTR primers include mini STR primers that have permitted the maximum size reduction in amplicon size for all 13 CODIS STR loci in addition to the D2S1338, Penta D, and pentaE found in commercially available STR kits (Butler et al., J Forensic Sci 48:1054-1064 [2003]), miniSTR loci that are unlinked to the CODIS markers as described by Coble and Butler (Coble and Butler, J Forensic Sci 50:43-53 [2005]), and other minSTRs that have been characterized at NIST. Information regarding the miniSTRs characterized at NIST is accessible via the world wide web at cstl.nist.gov/biotech/strbase/newSTRs.htm. Any one pair or a combination of two or more pairs of miniSTR primers can be used to amplify at least one miniSTR.

In one embodiment, exemplary primer sets that can be used to amplify STRs in maternal cfDNA samples include the primer sets provided in Example 9 and disclosed as SEQ ID NOs:113-196.

Gender identification (sex-typing) in commonly performed in conjunction with STR typing using PCR products generated from the Amelogenin gene that occurs on both the X- and Y-chromosome. Amelogenin is not an STR locus, but it produces X and Y chromosome specific PCR products. A commonly used PCR primer set first published by Sullivan et al. (1993) (Sullivan et al., BioTechniques 15:637-641 [1993]) targets a 6 bp deletion that occurs on the X-chromosome, which enables amplicons generated from the X- and Y-chromosome to be distinguished from one another when electrophoretic separation is performed to separate STR alleles. Most commercial STR kits utilize the Sullivan et al. (1993) primers or minor modifications. Since females are X,X, only a single peak is observed when testing female DNA whereas males, which possess both X and Y chromosomes, exhibit two peaks with a standard Amelogenin test. In one embodiment, the method to determine the fraction of fetal nucleic acid in a maternal sample comprises coamplifying Ameleogenin with at least one miniSTR. In another embodiment, the method does not comprise coamplifying Amelogenin with miniSTR loci.

Amplification of the target nucleic acids in the mixture of fetal and maternal nucleic acid *e.g.* cfDNA, is accomplished any method that uses PCR or variations of the method including but not limited to digital PCR, real time PCR (RT-PCR), TaqMan PCR System (Applied Biosystems), SNPlex or GenPlex methods, asymmetric PCR, helicase-dependent amplification, hot-start PCR, qPCR, solid phase PCR, and touchdown PCR. Alternatively, replication of target nucleic acid sequences can be obtained by enzyme-independent methods *e.g.* chemical solid-phase synthesis using the phosphoramidites. Amplification of the target sequences is accomplished using primer pairs each capable of amplifying a target nucleic acid sequence comprising the polymorphic site e.g. SNP, in a multiplex PCR reaction. Multiplex PCR reactions include combining at least 2, at least three, at least 3, at least 5, at least 10, at least 15, at least 20, at least 25, at least 30, at least 40 or more sets of primers in the same reaction to quantify the amplified target nucleic acids comprising at least two, at least three, at least 5, at least 10, at least 15, at least 20, at least 25, at least 30, at least 40 or more polymorphic sites in the same sequencing reaction. Any panel of primer sets can be configured to amplify at least one informative polymorphic sequence.

In step **140** of method **100 (****Figure 1****),** a portion or all of the amplified polymorphic sequences are used to prepare a sequencing library for sequencing in a parallel fashion as described. In one embodiment, the library is prepared for sequencing-by-synthesis using Illumina's reversible terminator-based sequencing chemistry.

In step **140,** sequence information that is needed for determining fetal fraction is obtained using any of the known DNA sequencing methods. In one embodiment, the method described herein employs next generation sequencing technology (NGS) in which clonally amplified DNA templates or single DNA molecules are sequenced in a massively parallel fashion within a flow cell (*e.g.* as described in Volkerding et al. Clin Chem 55:641-658 [2009]; Metzker M Nature Rev 11:31-46 [2010]). In addition to high-throughput sequence information, NGS provides digital quantitative information, in that each sequence read is a countable "sequence tag" representing an individual clonal DNA template or a single DNA molecule. This quantification allows NGS to expand the digital PCR concept of counting cell-free DNA molecules (Fan et al., Proc Natl Acad Sci USA 105:16266-16271 [2008]; Chiu et al., Proc Natl Acad Sci U S A 2008;105:20458-20463 [2008]). The sequencing technologies of NGS include pyrosequencing, sequencing-by-synthesis with reversible dye terminators, sequencing by oligonucleotide probe ligation and real time sequencing.

Some of the sequencing technologies are available commercially, such as the sequencing-by-hybridization platform from Affymetrix Inc. (Sunnyvale, CA) and the sequencing-by-synthesis platforms from 454 Life Sciences (Bradford, CT), Illumina/Solexa (Hayward, CA) and Helicos Biosciences (Cambridge, MA), and the sequencing-by-ligation platform from Applied Biosystems (Foster City, CA), as described below. In addition to the single molecule sequencing performed using sequencing-by-synthesis of Helicos Biosciences, other single molecule sequencing technologies are encompassed by the method of the invention and include the SMRT™ technology of Pacific Biosciences, the Ion Torrent™ technology, and nanopore sequencing being developed for example, by Oxford Nanopore Technologies.

While the automated Sanger method is considered as a 'first generation' technology, Sanger sequencing including the automated Sanger sequencing, can also be employed by the method of the invention. Additional sequencing methods that comprise the use of developing nucleic acid imaging technologies *e.g.* atomic force microscopy (AFM) or transmission electron microspcopy (TEM), are also encompassed by the method of the invention. Exemplary sequencing technologies are described below.

In one embodiment, the DNA sequencing technology that is used in the method of the invention is the Helicos True Single Molecule Sequencing (tSMS) (*e.g.* as described in Harris T.D. et al., Science 320:106-109 [2008]). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm². The flow cell is then loaded into an instrument, e.g., HeliScope™ sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are discerned by imaging the flow cell surface. After imaging, a cleavage step removes the fluorescent label, and the process is repeated with other fluorescently labeled nucleotides until the desired read length is achieved. Sequence information is collected with each nucleotide addition step.

In one embodiment, the DNA sequencing technology that is used in the method of the invention is the 454 sequencing (Roche) (*e.g.* as described in Margulies, M. et al. Nature 437:376-380 [2005]). 454 sequencing involves two steps. In the first step, DNA is sheared into fragments of approximately 300-800 base pairs, and the fragments are blunt-ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is discerned and analyzed.

In one embodiment, the DNA sequencing technology that is used in the method of the invention is the SOLiD technology (Applied Biosystems). In SOLiD sequencing-by-ligation, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In one embodiment, the DNA sequencing technology that is used in the method of the invention is the single molecule, real-time (SMRT™) sequencing technology of Pacific Biosciences. In SMRT sequencing, the continuous incorporation of dye-labeled nucleotides is imaged during DNA synthesis. Single DNA polymerase molecules are attached to the bottom surface of individual zero-mode wavelength identifiers (ZMW identifiers) that obtain sequence information while phospolinked nucleotides are being incorporated into the growing primer strand. A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Identification of the corresponding fluorescence of the dye indicates which base was incorporated. The process is repeated.

In one embodiment, the DNA sequencing technology that is used in the method of the invention is nanopore sequencing (*e.g.* as described in Soni GV and Meller A. Clin Chem 53: 1996-2001 [2007]). Nanopore sequencing DNA analysis techniques are being industrially developed by a number of companies, including Oxford Nanopore Technologies (Oxford, United Kingdom). Nanopore sequencing is a single-molecule sequencing technology whereby a single molecule of DNA is sequenced directly as it passes through a nanopore. A nanopore is a small hole, of the order of 1 nanometer in diameter. Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence.

In one embodiment, the DNA sequencing technology that is used in the method of the invention is the chemical-sensitive field effect transistor (chemFET) array (e.g., as described in U.S. Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be discerned by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

In one embodiment, the DNA sequencing technology that is used in the method of the invention is the Halcyon Molecular's method that uses transmission electron microscopy (TEM). The method, termed Individual Molecule Placement Rapid Nano Transfer (IMPRNT), comprises utilizing single atom resolution transmission electron microscope imaging of high-molecular weight (150kb or greater) DNA selectively labeled with heavy atom markers and arranging these molecules on ultra-thin films in ultra-dense (3nm strand-to-strand) parallel arrays with consistent base-to-base spacing. The electron microscope is used to image the molecules on the films to determine the position of the heavy atom markers and to extract base sequence information from the DNA. The method is further described in PCT patent publication WO 2009/046445. The method allows for sequencing complete human genomes in less than ten minutes.

In one embodiment, the DNA sequencing technology is the Ion Torrent single molecule sequencing, which pairs semiconductor technology with a simple sequencing chemistry to directly translate chemically encoded information (A, C, G, T) into digital information (0, 1) on a semiconductor chip. In nature, when a nucleotide is incorporated into a strand of DNA by a polymerase, a hydrogen ion is released as a byproduct. Ion Torrent uses a high-density array of micro-machined wells to perform this biochemical process in a massively parallel way. Each well holds a different DNA molecule. Beneath the wells is an ion-sensitive layer and beneath that an ion sensor. When a nucleotide, for example a C, is added to a DNA template and is then incorporated into a strand of DNA, a hydrogen ion will be released. The charge from that ion will change the pH of the solution, which can be identified by Ion Torrent's ion sensor. The sequencer-essentially the world's smallest solid-state pH meter-calls the base, going directly from chemical information to digital information. The Ion personal Genome Machine (PGM™) sequencer then sequentially floods the chip with one nucleotide after another. If the next nucleotide that floods the chip is not a match. No voltage change will be recorded and no base will be called. If there are two identical bases on the DNA strand, the voltage will be double, and the chip will record two identical bases called. Direct identification allows recordation of nucleotide incorporation in seconds.

Other sequencing methods include digital PCR and sequencing by hybridization. Digital polymerase chain reaction (digital PCR or dPCR) can be used to directly identify and quantify nucleic acids in a sample. Digital PCR can be performed in an emulsion. Individual nucleic acids are separated, e.g., in a microfluidic chamber device, and each nucleic can is individually amplified by PCR. Nucleic acids can be separated such there is an average of approximately 0.5 nucleic acids/well, or not more than one nucleic acid/well. Different probes can be used to distinguish fetal alleles and maternal alleles. Alleles can be enumerated to determine copy number. In sequencing by hybridization, the hybridization comprises contacting the plurality of polynucleotide sequences with a plurality of polynucleotide probes, wherein each of the plurality of polynucleotide probes can be optionally tethered to a substrate. The substrate might be flat surface comprising an array of known nucleotide sequences. The pattern of hybridization to the array can be used to determine the polynucleotide sequences present in the sample. In other embodiments, each probe is tethered to a bead, e.g., a magnetic bead or the like. Hybridization to the beads can be identified and used to identify the plurality of polynucleotide sequences within the sample.

In one embodiment, the method employs massively parallel sequencing of millions of DNA fragments using Illumina's sequencing-by-synthesis and reversible terminator-based sequencing chemistry (*e.g.* as described in Bentley et al., Nature 6:53-59 [2009]). Template DNA can be genomic DNA *e.g.* cfDNA. In some embodiments, genomic DNA from isolated cells is used as the template, and it is fragmented into lengths of several hundred base pairs. In other embodiments, cfDNA is used as the template, and fragmentation is not required as cfDNA exists as short fragments. For example fetal cfDNA circulates in the bloodstream as fragments of <300 bp, and maternal cfDNA has been estimated to circulate as fragments of between about 0.5 and 1 Kb (Li et al., Clin Chem, 50: 1002-1011 [2004]). Illumina's sequencing technology relies on the attachment of fragmented genomic DNA to a planar, optically transparent surface on which oligonucleotide anchors are bound. Template DNA is end-repaired to generate 5'-phosphorylated blunt ends, and the polymerase activity of Klenow fragment is used to add a single A base to the 3' end of the blunt phosphorylated DNA fragments. This addition prepares the DNA fragments for ligation to oligonucleotide adapters, which have an overhang of a single T base at their 3' end to increase ligation efficiency. The adapter oligonucleotides are complementary to the flow-cell anchors. Under limiting-dilution conditions, adapter-modified, single-stranded template DNA is added to the flow cell and immobilized by hybridization to the anchors. Attached DNA fragments are extended and bridge amplified to create an ultra-high density sequencing flow cell with hundreds of millions of clusters, each containing ∼1,000 copies of the same template. The cluster amplified DNA molecules are sequenced using a robust four-color DNA sequencing-by-synthesis technology that employs reversible terminators with removable fluorescent dyes. High-sensitivity fluorescence identification is achieved using laser excitation and total internal reflection optics. Short sequence reads of about 20-40 bp *e.g.* 36 bp, are aligned against a repeat-masked reference genome and genetic differences are called using specially developed data analysis pipeline software. After completion of the first read, the templates can be regenerated in situ to enable a second read from the opposite end of the fragments. Thus, either single-end or paired end sequencing of the DNA fragments is used according to the method. Partial sequencing of DNA fragments present in the sample is performed, and sequence tags comprising reads of predetermined length *e.g.* 36 bp, that are mapped to a known reference genome are counted.

The length of the sequence read is associated with the particular sequencing technology. NGS methods provide sequence reads that vary in size from tens to hundreds of base pairs. In some embodiments of the method described herein, the sequence reads are about 20bp, about 25bp, about 30bp, about 35bp, about 40bp, about 45bp, about 50bp, about 55bp, about 60bp, about 65bp, about 70bp, about 75bp, about 80bp, about 85bp, about 90bp, about 95bp, about 100bp, about 110bp, about 120bp, about 130, about 140bp, about 150bp, about 200bp, about 250bp, about 300bp, about 350bp, about 400bp, about 450bp, about 500bp, about 550bp or about 600bp. It is expected that technological advances will enable single-end reads of greater than 500bp enabling for reads of greater than about 1000bp when paired end reads are generated. In one embodiment, the sequence reads are 36bp. Other sequencing methods that can be employed by the method of the invention include the single molecule sequencing methods that can sequence nucleic acids molecules >5000 bp. The massive quantity of sequence output is transferred by an analysis pipeline that transforms primary imaging output from the sequencer into strings of bases. A package of integrated algorithms performs the core primary data transformation steps: image analysis, intensity scoring, base calling, and alignment.

In one embodiment, partial sequencing of amplified target polymorphic nucleic acids is performed, and sequence tags comprising reads of predetermined length *e.g.* 36 bp, that map to a known reference genome are counted. Only sequence reads that uniquely align to a reference genome are counted as sequence tags. In one embodiment, the reference genome is an artificial target sequences genome that comprises the sequences of the polymorphic target nucleic acids *e.g.* SNPs. In one embodiment, the reference genome is an artificial SNP reference genome. In another r embodiment, the reference genome is an artificial STR reference genome. In yet another embodiment, the reference genome is an artificial tandem-STR reference genome. Artificial reference genomes can be compiled using the sequences of the target polymorphic nucleic acids. Artificial reference genomes can comprise polymorphic target sequence each comprising one or more different types of polymorphic sequences. For example, an artificial reference genome can comprise polymorphic sequences comprising SNP alleles and/or STRs. In one embodiment, the reference genome is the human reference genome NCBI36/hg18 sequence, which is available on the world wide web at genome.ucsc.edu/cgi-bin/hgGateway?org=Human&db=hg18&hgsid=166260105). Other sources of public sequence information include GenBank, dbEST, dbSTS, EMBL (the European Molecular Biology Laboratory), and the DDBJ (the DNA Databank of Japan). In another embodiment, the reference genome comprises the human reference genome NCBI36/hg18 sequence and an artificial target sequences genome, which includes the target polymorphic sequences *e.g.* a SNP genome. Mapping of the sequence tags is achieved by comparing the sequence of the mapped tag with the sequence of the reference genome to determine the chromosomal origin of the sequenced nucleic acid (*e.g.* cfDNA) molecule, and specific genetic sequence information is not needed. A number of computer algorithms are available for aligning sequences, including without limitation BLAST (Altschul et al., 1990), BLITZ (MPsrch) (Sturrock & Collins, 1993), FASTA (Person & Lipman, 1988), BOWTIE (Langmead et al., Genome Biology 10:R25.1-R25.10 [2009]), or ELAND (Illumina, Inc., San Diego, CA, USA). In one embodiment, one end of the clonally expanded copies of the plasma cfDNA molecules is sequenced and processed by bioinformatic alignment analysis for the Illumina Genome Analyzer, which uses the Efficient Large-Scale Alignment of Nucleotide Databases (ELAND) software. In embodiments of the method that comprise determining the presence or absence of an aneuploidy and fetal fraction using NGS sequencing methods, analysis of sequencing information for the determination of aneuploidy may allow for a small degree of mismatch (0-2 mismatches per sequence tag) to account for minor polymorphisms that may exist between the reference genome and the genomes in the mixed sample. Analysis of sequencing information for the determination of fetal fraction may allow for a small degree of mismatch depending on the polymorphic sequence. For example, a small degree of mismatch may be allowed if the polymorphic sequence is an STR. In cases when the polymorphic sequence is a SNP, all sequence that match exactly to either of the two alleles at the SNP site are counted first and filtered from the remaining reads, for which a small degree of mismatch may be allowed. Quantification of the number of sequence reads aligning to each chromosome for determining chromosomal aneuploidies can be determined as described herein, or using alternative analyses that employ normalizing the median number of sequence tags for a chromosome of interest to the median number of tags for each of the other autosomes (Fan et al., Proc Natl Acad Sci 105:16266-16271 [2008]), or that compare the number of unique reads aligning to each chromosome to the total number of reads aligning to all chromosomes to derive a percent genomic representation for each chromosome. A "z score" is generated to represent the difference between the percent genomic representation of the chromosome of interest and the mean percent representation for the same chromosome between a euploid control group, divided by the standard deviation (Chiu et al., Clin Chem 56:459-463 [2010]). In another embodiment, the sequencing information can be determined as described in U.S. Provisional Patent Application titled "Normalizing Biological Assays," docket no. 32047-768.101, filed January 19, 2010.

Analysis of sequencing information for the determination of fetal fraction may allow for a small degree of mismatch depending on the polymorphic sequence. For example, a small degree of mismatch may be allowed if the polymorphic sequence is an STR. In cases when the polymorphic sequence is a SNP, all sequences that match exactly to either of the two alleles at the SNP site are counted first and filtered from the remaining reads, for which a small degree of mismatch may be allowed. The present method for determining fetal fraction by sequencing of nucleic acids can be used in combination with other methods.

In step **160,** fetal fraction is determined based on the total number of tags that map to the first allele and the total number of tags that map to second allele at an informative polymorphic site *e.g.* a SNP, contained in a reference genome. For example, the reference genome is an artificial target sequence genome that encompasses the polymorphic sequences that comprise SNPs rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In one embodiment, the artificial reference genome includes the polymorphic target sequences of SEQ ID NOs:1-56 (see Example-3).

In another embodiment, the artificial genome is an artificial target sequence genome that encompasses polymorphic sequences that comprise tandem SNPs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959-rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

In another embodiment, the artificial target genome encompasses polymorphic sequences that comprise STRs selected from CSF1PO, FGA, TH01, TPOX, vWA, D3S1358,D5S818, D7S820, D8S1179, D13S317, D16S539, D18S51, D21S11, Penta D, Penta E, D2S1338, D1S1677, D2S441, D4S2364, D10S1248, D14S1434, D22S1045, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D5S2500, D4S2408, D4S2364, D3S4529, D3S3053, D2S1776, D2S441, D1S1677, D1S1627, and D1GATA113. The composition of the artificial target sequences genome will vary depending on the polymorphic sequences that are used for determining the fetal fraction. Accordingly, an artificial target sequences genome is not limited to the SNP, tandem SNP or STR sequences exemplified herein.

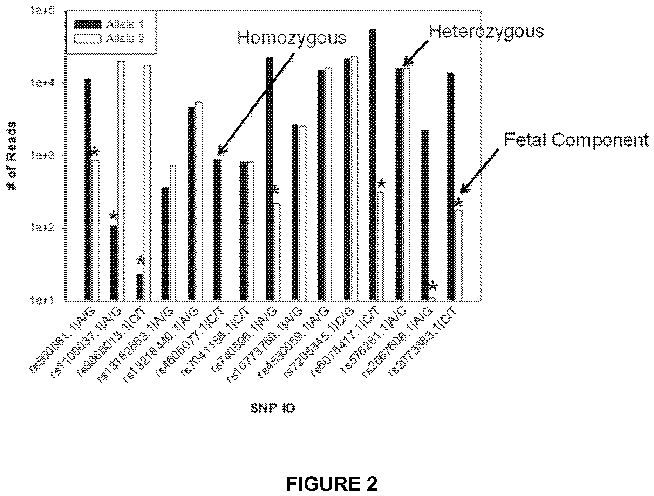

The informative polymorphic site *e.g*. SNP, is identified by the difference in the allelic sequences and the amount of each of the possible alleles. Fetal cfDNA is present at a concentration that is <10% of the maternal cfDNA. Thus, the presence of a minor contribution of an allele to the mixture of fetal and maternal nucleic acids relative to the major contribution of the maternal allele can be assigned to the fetus. Alleles that are derived from the maternal genome are herein referred to as major alleles, and alleles that are derived from the fetal genome are herein referred to as minor alleles. Alleles that are represented by similar levels of mapped sequence tags represent maternal alleles. The results of an exemplary multiplex amplification of target nucleic acids comprising SNPs and derived from a maternal plasma sample is shown in **Figure 2****.** Informative SNPs are discerned from the single nucleotide change at a polymorphic site, and fetal alleles are discerned by their relative minor contribution to the mixture of fetal and maternal nucleic acids in the sample when compared to the major contribution to the mixture by the maternal nucleic acids. Accordingly, the relative abundance of fetal cfDNA in the maternal sample is determined as a parameter of the total number of unique sequence tags mapped to the target nucleic acid sequence on a reference genome for each of the two alleles of the predetermined polymorphic site. In one embodiment, the fraction of fetal nucleic acids in the mixture of fetal and maternal nucleic acids is calculated for each of the informative allele (alleleₓ) as follows: ${% fetal fraction allele}_{x} = \left(\left(\sum {Fetal sequence tags for allele}_{x}\right) / \left(\sum {Maternal sequence tags for allele}_{x}\right)\right) \times 100$ and fetal fraction for the sample is calculated as the average of the fetal fraction of all of the informative alleles. Optionally, the fraction of fetal nucleic acids in the mixture of fetal and maternal nucleic acids is calculated for each of the informative allele (alleleₓ) as follows: ${% fetal fraction allele}_{x} = \left(\left(2 \times \sum {Fetal sequence tags for allele}_{x}\right) / \left(\sum {Maternal sequence tags for allele}_{x}\right)\right) \times 100,$ to compensate for the presence of 2 fetal alleles, one being masked by the maternal background.

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more informative alleles. In one embodiment, the fetal fraction is the average fetal fraction determined for at least 3 informative alleles.

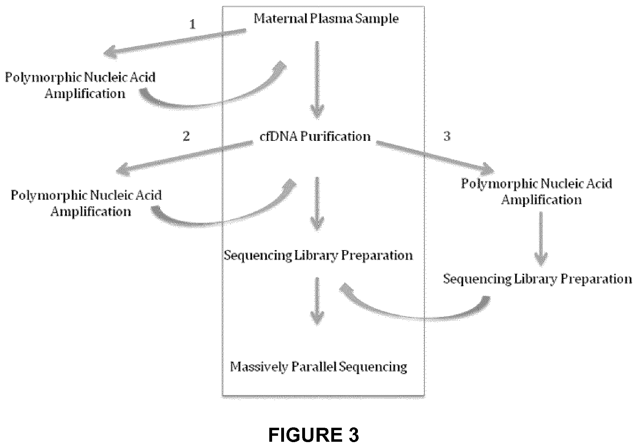

Figure 3, shows a flowchart of alternate methods whereby fetal fraction can be determined from amplified target nucleic acids that have been combined with unamplified fetal and maternal cfDNA sample to allow for the simultaneous determination of fetal fraction and the presence or absence of fetal aneuploidy by enriching the maternal sample comprising fetal and maternal nucleic acids for polymorphic target nucleic acids. In one embodiment, the sample that is enriched is the plasma fraction of a blood sample **(a).** For example, a portion of an original maternal plasma sample is used for amplifying target nucleic acid sequences. Subsequently, some or all of the amplified product is combined with the remaining unamplified original plasma sample thereby enriching it (see Example 7). In another embodiment, the sample that is enriched is the sample of purified cfDNA that is extracted from plasma **(b).** For example, enrichment comprises amplifying the target nucleic acids that are contained in a portion of an original sample of purified mixture of fetal and maternal nucleic acids *e.g*. cfDNA that has been purified from a maternal plasma sample, and subsequently combining some or all of the amplified product with the remaining unamplified original purified sample (see Example 6). In yet another embodiment, the sample that is enriched is a sequencing library sample prepared from a purified mixture of fetal and maternal nucleic acids **(c).** For example, enrichment comprises amplifying the target nucleic acids that are contained in a portion of an original sample of purified mixture of fetal and maternal nucleic acids *e.g*. cfDNA that has been purified from a maternal plasma sample, preparing a first sequencing library of unamplified nucleic acid sequences, preparing a second sequencing library of amplified polymorphic target nucleic acids, and subsequently combining some or all of the second sequencing library with some or all of the first sequencing library (see Example 5). The amount of amplified product that is used to enrich the original sample is selected to obtain sufficient sequencing information for determining both the presence or absence of aneuploidy and the fetal fraction from the same sequencing run. At least about 3%, at least about 5%, at least about 7%, at least about 10%, at least about 15%, at least about 20%, at least about 25%, at least about 30% or more of the total number of sequence tags obtained from sequencing are mapped to determine the fetal fraction. Sequencing of the library generated following any one of the methods depicted in **Figure 3****,** provides sequence tags derived from the amplified target nucleic acids and tags derived from the original unamplified maternal sample. Fetal fraction is calculated from the number of tags mapped to an artificial reference genome, and the presence or absence of aneuploidy is determined from the number of tags that map to the subject genome *e.g*. human genome.

An alternative method for determining fetal fraction from amplified polymorphic target nucleic acids at step **130** of **Figure 1****,** uses size separation of amplified polymorphic target nucleic acids comprising STRs (step **150** of **Figure 1**). As described above, the polymorphic character of a STR locus is due to variation in the number of tandemly repeated units between alleles. Because of the high polymorphism of the STRs most individuals will be heterozygous for STRs. Amplification of an STR will result in one or two PCR products in most samples. In samples obtained from pregnant women *e.g*. plasma samples, amplification of an STR will result in one or two major PCR products, which correspond to the one or two maternal alleles including one fetal maternally-inherited allele, and a third paternally-inherited fetal allele that is detected at an informative STR.

The STRs that are targeted for amplification are miniSTRs as described herein that are less than about 300 base pairs and that are amplified in a multiplex PCR reaction, which allows the simultaneous amplification of multiple loci in a single reaction. The primers are labeled with different fluorescent dyes each emitting fluorescence at a different wavelength *e.g*. 6FAM™, VIC™, NED™, and PET™, and the number of repeat units for each fluorescently tagged STR in the resulting PCR products is detected following their separation and accurate sizing that is achieved by slab or capillary electrophoresis. In one embodiment, capillary electrophoresis is used, and it can be performed in microfabricated channels or capillary arrays. Alternatively, methods utilizing mass spectrometry and microarray technology are used. Multiplex STR analysis can be performed to determine fetal fraction using commercially-available kits *e.g*. AmpFℓSTR® Identifiler® PCR Amplification Kit (**Figure 4**) and AmpFℓSTR® MiniFiler® PCR Amplification Kit (**Figure 5**) (Applied Biosystems, Foster City, Calif.). The AmpFℓSTR® MiniFiler® PCR Amplification Kit was designed to amplify as miniSTRs eight of the largest sized loci in the AmpFℓSTR® Identifiler® PCR Amplification Kit. Together with the gender-identification locus Amelogenin, the nine-locus multiplex enables simultaneous amplification of loci of cfDNA samples (see Example 10).

In one embodiment, multiplex STR analysis for determining fetal fraction is performed by amplifying polymorphic target nucleic acids present in a maternal plasma sample that each comprise a miniSTR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. In another embodiment, multiplex STR analysis for determining fetal fraction is performed by amplifying polymorphic target nucleic acids present in a maternal plasma sample for the panel of miniSTRs: CSF1PO, D13S317, D16S539, D18S51, D21S11, D2S1338, D7S820 and FGA. The miniSTRs can be located on the same or on different chromosomes. The method is a fetal gender-independent method. Therefore, in some embodiments, the miniSTRs are located on chromosomes other than the Y chromosome. In other embodiments, the miniSTRs are located on chromosomes other than chromosomes 13, 18, 21 or X *i.e.* chromosomes that might be involved in an aneuploidy.

Samples of maternal plasma often contain less than 100 pg of cfDNA. The low copy number DNA samples can fall below the sensitivity limitations of STR analysis methods. The intractable samples can be made amenable by increasing the number of starting cfDNA available for subsequent STR analysis by a whole genome amplification strategy. In one embodiment, the mixture of fetal and maternal nucleic acids can be preamplified before alleles are detected or quantified. For example, template cell-free DNA can be amplified by PCR. The nucleic acid can be amplified for about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 cycles. Nucleic acid can be amplified for about 1-10 cycles, about 1-20 cycles, about 1-30 cycles, about 1-40 cycles, about 5-15 cycles, about 5-20 cycles, about 5-30 cycles, about 5-40 cycles, about 10-15 cycles, about 10-20 cycles, about 10-30 cycles, about 10-40 cycles, about 20-30 cycles, about 20-40 cycles, or about 30-40 cycles. The amount of template nucleic acid that can be amplified can about 10-1000 pg, 25-1000 pg, 50-1000 pg, 100-1000, pg, 200-1000 pg, 300-1000 pg, 400-1000 pg, 500-1000 pg, 600-1000, pg, 700-1000 pg, or 800-1000 pg. Following preamplification, the nucleic acids can be diluted before alleles are detected or quantified. Preamplification can be used to increase the detection sensitivity of alleles in a sample, for example, a maternal sample (Example 11). In another embodiment, genotyping a polymorphism need not require a pre-amplification step. Any PCR-based amplification method can be used to preamplify the cfDNA. Amplification methods include but are not limited to whole genome amplification strategies including methods such as primer extension preamplification, degenerate oligonucleotide-primed PCR (DOP-PCR), low fragments from low quantities of DOP-PCR, improved primer extension preamplification PCR (IPEP PCR), and modified improved primer extension preamplification (mIPEP). Thus, in one embodiment, the method that is used for determining the fetal fraction in a maternal sample *e.g*. plasma sample, comprises preamplifying the mixture of fetal and maternal nucleic acids present in the plasma cfDNA sample using a whole genome amplification method, amplifying a plurality of polymorphic nucleic acids in said mixture of fetal and maternal nucleic acids, wherein each of said at least one polymorphic nucleic acid comprises an STR; determining the amount of fetal and maternal STR alleles at least one polymorphic nucleic acid; and calculating the fetal fraction from the amount of fetal and maternal STR alleles. Following preamplification, multiplex STR analysis for determining fetal fraction is performed by amplifying polymorphic target nucleic acids present in a maternal plasma sample that each comprise aminiSTR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Alternatively, multiplex STR analysis for determining fetal fraction is performed by amplifying polymorphic target nucleic acids for the panel of miniSTRs: CSF1PO, D13S317, D16S539, D18S51, D21S11, D2S1338, D7S820 and FGA.

### Applications

Methods described herein are applicable to diagnosis or prognosis of various disease conditions including, but not limited to, cancer, genetic disorders and infection. The fetal fraction of nucleic acid in a maternal sample can be used for determining a chromosomal abnormality. Examples of chromosomal abnormalities include, for example, aneuploidy, monosomy, trisomy, duplication, inversion, deletion, polyploidy, deletion of a part of a chromosome, addition, addition of a part of chromosome, insertion, a fragment of a chromosome, a region of a chromosome, chromosomal rearrangement, and translocation. For example, aneuploidy can refer to the occurrence of one or more extra or missing chromosomes in a sample.

Examples of fetal conditions that can be determined using the methods of the provided invention include, for example, Angleman syndrome (15q11.2-q13), cri-du-chat syndrome (5p-), DiGeorge syndrome and Velo-cardiofacial syndrome (22q11.2), Miller-Dieker syndrome (17 p13.3), Prader-Willi syndrome (15q11.2-q13), retinoblastoma (13q14), Smith-Magenis syndrome (17 p11.2), trisomy 13, trisomy 16, trisomy 18, trisomy 21 (Down's syndrome), triploidy, Williams syndrome (7q 11.23), and Wolf-Hirschhom syndrome (4p-). Examples of sex chromosome abnormalities that can be detected by methods described herein include, but are not limited to, Kallman syndrome (Xp22.3), steroid sulfate deficiency (STS) (Xp22.3), X-linked ichthyosis (Xp22.3), Klinefelter syndrome (XXY), fragile X syndrome, Turner syndrome metafemales or trisomy X, and monosomy X.

In one embodiment, fetal fraction information can be used to set thresholds and estimate minimum sample size in aneuploidy detection. Such use is described in Example 7 below. Fetal fraction information can be used in conjunction with sequencing information. For example, nucleic acids from a cell-free sample, for example a maternal plasma or serum sample, can be used to enumerate sequences in a sample. Sequences can be enumerated using any of the sequencing techniques described above. Knowledge of fetal fraction can be used to set "cutoff" thresholds to call "aneuploidy," "normal," or "marginal/no call" (uncertain) states. Then, calculations can be performed to estimate the minimum number of sequences required to achieve adequate sensitivity (i.e. probability of correctly identifying an aneuploidy state).

The determination of fetal fraction according to the method of the invention can be practiced in combination with any method used to determine the presence of absence of fetal aneuploidy in a maternal plasma sample. In addition to the method described herein for the determination of aneuploidy, the determination of fetal fraction by massively parallel sequencing can be used in conjunction with other methods for determining fetal aneuploidy, for example, according to the methods described in U.S. U.S. Patent Application Publication Nos. US 2007/0202525A1; US2010/0112575A1, US 2009/0087847A1; US2009/0029377A1; US 2008/0220422A1; US2008/0138809A1, US2008/0153090A1, and US Patent 7,645,576. The methods can also be combined with assays for determining other prenatal conditions associated with the mother and/or the fetus. For example, the method can be used in conjunction with prenatal analyses, for example, as described in U.S. Patent Application Publication Nos. US2010/0112590A1, US2009/0162842A1, US2007/0207466A1, and US2001/0051341A1.

The methods described can be applied to determine the fraction of any one population of nucleic acids in a mixture of nucleic acids contributed by different genomes. In addition to determining the fraction contributed to a sample by two individuals *e.g*. the different genomes are contributed by the fetus and the mother carrying the fetus, the methods can be used to determine the fraction of a genome in a mixture derived from two different cells of from one individual *e.g*. the genomes are contributed to the sample by aneuploid cancerous cells and normal euploid cells from the same subject.

### Example 1

### Determination of Fetal Fraction using Massively Parallel Sequencing: Sample Processing and cfDNA Extraction

Peripheral blood samples were collected from pregnant women in their first or second trimester of pregnancy and who were deemed at risk for fetal aneuploidy. Informed consent was obtained from each participant prior to the blood draw. Blood was collected before amniocentesis or chorionic villus sampling. Karyotype analysis was performed using the chorionic villus or amniocentesis samples to confirm fetal karyotype.

Peripheral blood drawn from each subject was collected in ACD tubes. One tube of blood sample (approximately 6-9 mL/tube) was transferred into one 15-mL low speed centrifuge tube. Blood was centrifuged at 2640 rpm, 4°C for 10 min using Beckman Allegra 6 R centrifuge and rotor model GA 3.8.

For cell-free plasma extraction, the upper plasma layer was transferred to a 15-ml high speed centrifuge tube and centrifuged at 16000 x g, 4°C for 10 min using Beckman Coulter Avanti J-E centrifuge, and JA-14 rotor. The two centrifugation steps were performed within 72 h after blood collection. Cell-free plasma comprising cfDNA was stored at -80°C and thawed only once before amplification of plasma cfDNA or for purification of cfDNA.

Purified cell-free DNA (cfDNA) was extracted from cell-free plasma using the QIAamp Blood DNA Mini kit (Qiagen) essentially according to the manufacturer's instruction. One milliliter of buffer AL and 100 µl of Protease solution were added to 1 ml of plasma. The mixture was incubated for 15 minutes at 56°C. One milliliter of 100% ethanol was added to the plasma digest. The resulting mixture was transferred to QIAamp mini columns that were assembled with VacValves and VacConnectors provided in the QIAvac 24 Plus column assembly (Qiagen). Vacuum was applied to the samples, and the cfDNA retained on the column filters was washed under vacuum with 750µl of buffer AW1, followed by a second wash with 750µl of buffer AW24. The column was centrifuged at 14,000 RPM for 5 minutes to remove any residual buffer from the filter. The cfDNA was eluted with buffer AE by centrifugation at 14,000 RPM, and the concentration determined using Qubit™ Quantitation Platform (Invitrogen).

### Example 2

### Determination of Fetal Fraction using Massively Parallel Sequencing: Preparation of Sequencing Libraries, Sequencing, and Analysis of Sequencing Data

### a. Preparation of Sequencing Libraries

All sequencing libraries *i.e.* target, primary and enriched libraries, were prepared from approximately 2 ng of purified cfDNA that was extracted from maternal plasma. Library preparation was performed using reagents of the NEBNext™ DNA Sample Prep DNA Reagent Set 1 (Part No. E6000L; New England Biolabs, Ipswich, MA) for Illumina® as follows. Because cell-free plasma DNA is fragmented in nature, no further fragmentation by nebulization or sonication was done on the plasma DNA samples. The overhangs of approximately 2 ng purified cfDNA fragments contained in 40µl were converted into phosphorylated blunt ends according to the NEBNext® End Repair Module by incubating in a 1.5ml microfuge tube the cfDNA with 5µl 10X phosphorylation buffer, 2µl deoxynucleotide solution mix (10 mM each dNTP), 1µl of a 1:5 dilution of DNA Polymerase I, 1µl T4 DNA Polymerase and 1µl T4 Polynucleotide Kinase provided in the NEBNext™ DNA Sample Prep DNA Reagent Set 1 for 15 minutes at 20°C. The enzymes were then heat inactivated by incubating the reaction mixture at 75°C for 5 minutes. The mixture was cooled to 4°C, and dA tailing of the blunt-ended DNA was accomplished using 10µl of the dA-tailing master mix containing the Klenow fragment (3' to 5' exo minus) (NEBNext™ DNA Sample Prep DNA Reagent Set 1), and incubating for 15 minutes at 37°C. Subsequently, the Klenow fragment was heat inactivated by incubating the reaction mixture at 75°C for 5 minutes. Following the inactivation of the Klenow fragment, 1µl of a 1:5 dilution of Illumina Genomic Adaptor Oligo Mix (Part No. 1000521; Illumina Inc., Hayward, CA) was used to ligate the Illumina adaptors (Non-Index Y-Adaptors) to the dA-tailed DNA using 4µl of the T4 DNA ligase provided in the NEBNext™ DNA Sample Prep DNA Reagent Set 1, by incubating the reaction mixture for 15 minutes at 25°C. The mixture was cooled to 4°C, and the adaptor-ligated cfDNA was purified from unligated adaptors, adaptor dimers, and other reagents using magnetic beads provided in the Agencourt AMPure XP PCR purification system (Part No. A63 881; Beckman Coulter Genomics, Danvers, MA). Eighteen cycles of PCR were performed to selectively enrich adaptor-ligated cfDNA using Phusion ® High-Fidelity Master Mix (Finnzymes, Woburn, MA) and Illumina's PCR primers complementary to the adaptors (Part No. 1000537 and 1000537). The adaptor-ligated DNA was subjected to PCR (98°C for 30 seconds; 18 cycles of 98°C for 10 seconds, 65°C for 30 seconds, and 72°C for 30 seconds; final extension at 72°C for 5 minutes, and hold at 4°C) using Illumina Genomic PCR Primers (Part Nos. 100537 and 1000538) and the Phusion HF PCR Master Mix provided in the NEBNext™ DNA Sample Prep DNA Reagent Set 1, according to the manufacturer's instructions. The amplified product was purified using the Agencourt AMPure XP PCR purification system (Agencourt Bioscience Corporation, Beverly, MA) according to the manufacturer's instructions available at www.beckmangenomics.com/products/AMPureXPProtocol_000387v001.pdf. The purified amplified product was eluted in 40µl of Qiagen EB Buffer, and the concentration and size distribution of the amplified libraries was analyzed using the Agilent DNA 1000 Kit for the 2100 Bioanalyzer (Agilent technologies Inc., Santa Clara, CA).

### b. Sequencing

Sequencing of library DNA was performed using the Genome Analyzer II (Illumina Inc., San Diego, CA, USA) according to standard manufacturer protocols. Copies of the protocol for whole genome sequencing using Illumina/Solexa technology may be found at BioTechniques.RTM. Protocol Guide 2007 Published December 2006: p 29, and on the world wide web at biotechniques.com/default.asp?page=protocol&subsection=article_display&id=112378. The DNA library was diluted to 1nM and denatured. Library DNA (5pM) was subjected to cluster amplification according to the procedure described in Illumina's Cluster Station User Guide and Cluster Station Operations Guide, available on the world wide web at illumina.com/systems/genome analyzer/cluster_station.ilmn. The amplified DNA was sequenced using Illumina's Genome Analyzer II to obtain single-end reads of 36bp. Only about 30 bp of random sequence information are needed to identify a sequence as belonging to a specific human chromosome. Longer sequences can uniquely identify more particular targets. In the present case, a large number of 36 bp reads were obtained, covering approximately 10% of the genome.

### c. Analysis of sequencing data for the determination of fetal fraction

Upon completion of sequencing of the sample, the Illumina "Sequencer Control Software" transferred image and base call files to a Unix server running the Illumina "Genome Analyzer Pipeline" software version 1.51. the 36bp reads were aligned to an artificial reference genome *e.g*. a SNP genome, using the BOWTIE program. The artificial reference genome was identified as the grouping of the polymorphic DNA sequences that encompass the alleles comprised in the polymorphic target sequences. For example, the artificial reference genome is a SNP genome comprising SEQ ID NOs: 1-56. Only reads that mapped uniquely to the artificial genome were used for the analysis of fetal fraction. Reads that matched perfectly to the SNP genome were counted as tags and filtered. Of the remaining reads, only reads having one or two mismatches were counted as tags and included in the analysis. Tags mapped to each of the polymorphic alleles were counted, and the fetal fraction was determined as a percent of the ratio of the number of tags mapped to the major allele *i.e.* maternal allele, and the number of tags mapped to the minor allele *i.e.* fetal allele.

### Example 3

### Selection of Autosomal SNPs for the Determination of Fetal Fraction

A set of 28 autosomal SNPs were selected from a list of 92 SNPs (Pakstis et al., Hum Genet 127:315-324 [2010]) and from Applied Biosystems by Life Technologies ™ (Carlsbad, CA) at world wide web address appliedbiosystems.com, and validated for use in multiplexed PCR amplification. Primers were designed to hybridize to a sequence close to the SNPs site on the cfDNA to ensure that it be included in the 36 bp read generated from the massively parallel sequencing on the Illumina Analyzer GII, and to generate amplicons of sufficient length to undergo bridge-amplification during cluster formation. Thus, primers were designed to generate amplicons that were at least 110 bp, which when combined with the universal adaptors (Illumina Inc., San Diego, CA) used for cluster amplification, resulted in DNA molecules of at least 200bp. Primer sequences were identified, and primer sets *i.e*. forward and reverse primers, were synthesized by Integrated DNA Technologies (San Diego, CA), and stored as a 1µM solution to be used for amplifying polymorphic target sequences as described in Examples 4-7. Table 1 provides the RefSNP (rs) accession ID numbers, the primers used for amplifying the target cfDNA sequence, and the sequences of the amplicons comprising the possible SNP alleles that would be generated using the primers. The SNPs given in Table 1 were used for the simultaneous amplification of 13 target sequences in a multiplexed assay. The panel provided in Table 1 is an exemplary SNP panel. Fewer or more SNPs can be employed to enrich the fetal and maternal DNA for polymorphic target nucleic acids. Additional SNPs that can be used include the SNPs given in Table 2. The SNP alleles are shown in bold and are underlined. Other additional SNPs that can be used to determine fetal fraction according to the present method include rs315791, rs3780962, rs1410059, rs279844, rs38882, rs9951171, rs214955, rs6444724, rs2503107, rs1019029, rs1413212, rs1031825, rs891700, rs1005533, rs2831700, rs354439, rs1979255, rs1454361, rs8037429, and rs1490413, which have been analyzed for determining fetal fraction by TaqMan PCR, and are disclosed in US Provisional applications 61/296,358 and 61/360,837.

**TABLE 1**

| **SNP Panel for the Determination of Fetal Fraction** | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs560681 | 1 | | | | |
| rs1109037 | 2 | | | | |
| rs9866013 | 3 | | | | |
| rs13182883 | 5 | | | | |
| | | | | | |
| rs13218440 | 6 | | | | |
| rs7041158 | 9 | | | | |
| rs740598 | 10 | | | | |
| rs10773760 | 12 | | | | |
| rs4530059 | 14 | | | | |
| rs7205345 | 16 | | | | |
| rs8078417 | 17 | | | | |
| rs576261 | 19 | | | | |
| | | | | | |
| rs2567608 | 20 | | | | |

**TABLE 2**

| **Additional SNPs for the Determination of Fetal Fraction** | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs430046 | 16 | | | | |
| rs9951171 | 18 | | | | |
| | | | | | |
| rs338882 | 5 | | | | |
| rs10776839 | 9 | | | | |
| rs9905977 | 17 | | | | |
| rs1277284 | 4 | | | | |
| | | | | | |
| rs258684 | 7 | | | | |
| rs1347696 | 8 | | | | |
| rs508485 | 11 | | | | |
| rs9788670 | 15 | | | | |
| rs8137254 | 22 | | | | |
| rs3143 | 19 | | | | |
| rs2182957 | 13 | | | | |
| | | | | | |
| rs3739005 | 2 | | | | |
| rs530022 | 1 | | | | |

### Example 4

### Determination of Fetal Fraction by Massively Parallel Sequencing of a Target Library

To determine the fraction of fetal cfDNA in a maternal sample, target polymorphic nucleic acid sequences each comprising a SNP were amplified and used for preparing a target library for sequencing in a massively parallel fashion.

cfDNA was extracted as described in Example 1. A target sequencing library was prepared as follows. cfDNA contained in 5µl of purified cfDNA was amplified in a reaction volume of 50µl containing 7.5µl of a 1µM primer mix (Table 1), 10µl of NEB 5X Mastermix and 27 µl water. Thermal cycling was performed with the Gene Amp9700 (Applied Biosystems) using the following cycling conditions: incubating at 95°C for 1 minute, followed by 20-30 cycles at 95°C for 20 seconds, 68°C for 1 minute, and 68°C for 30s, which was followed by a final incubation at 68°C for 5 minutes. A final hold at 4°C was added until the samples were removed for combining with the unamplified portion of the purified cfDNA sample. The amplified product was purified using the Agencourt AMPure XP PCR purification system (Part No. A63881; Beckman Coulter Genomics, Danvers, MA). A final hold at 4°C was added until the samples were removed for preparing the target library. The amplified product was analyzed with a 2100 Bioanalyzer (Agilent Technologies, Sunnyvale, CA), and the concentration of amplified product determined. A sequencing library of amplified target nucleic acids was prepared as described in Example 2, and was sequenced in a massively parallel fashion using sequencing-by-synthesis with reversible dye terminators and according to the Illumina protocol (BioTechniques.RTM. Protocol Guide 2007 Published December 2006: p 29, and on the world wide web at biotechniques.com/default.asp?page=protocol&subsection=article_display&id=112378). Analysis and counting of tags mapped to a reference genome consisting of 26 sequences (13 pairs each representing two alleles) comprising a SNP *i.e.* SEQ ID NO:1-26 was performed as described.

Table 3 provides the tag counts obtained from sequencing the target library, and the calculated fetal fraction derived from sequencing data.

**TABLE 3**

| **Determination of Fetal Fraction by Massively Parallel Sequencing of a Library of Polymorphic Nucleic Acids** | | |
|---|---|---|
| **SNP** | **SNP TAG COUNTS** | **Fetal Fraction (%)** |
| rs10773760.1\|Chr.12\|length=128\|allele=A | 236590 | 1.98 |
| rs10773760.2\|Chr.12\|length=128\|allele=G | 4680 | |
| rs13182883.1\|Chr.5\|length=111\|allele=A | 3607 | 4.99 |
| rs13182883.2\|Chr.5\|length=111\|allele=G | 72347 | |
| rs4530059.1\|Chr.14\|length=110\|allele=A | 3698 | 1.54 |
| rs4530059.1\|Chr.14\|length=110\|allele=G | 239801 | |
| rs8078417.1\|Chr.17\|length=110\|allele=C | 1E+06 | 3.66 |
| rs8078417.2\|Chr.17\|length=110\|allele=T | 50565 | |
| **Fetal Fraction** (**Mean±S**.**D**.) = **12.4±6.6** | | |

The results show that polymorphic nucleic acid sequences each comprising at least one SNP can be amplified from cfDNA derived from a maternal plasma sample to construct a library that can be sequenced in a massively parallel fashion to determine the fraction of fetal nucleic acids in the maternal sample.

### Example 5

### Determination of Fetal Fraction Following Enrichment of Fetal and Maternal Nucleic Acids in a cfDNA Sequencing Library Sample.

To enrich the fetal and maternal cfDNA contained in a primary sequencing library constructed using purified fetal and maternal cfDNA, a portion of a purified cfDNA sample was used for amplifying polymorphic target nucleic acid sequences, and for preparing a sequencing library of amplified polymorphic target nucleic acids, which was used to enrich the fetal and maternal nucleic acid sequences comprised in the primary library.

The method corresponds to workflow 3 diagrammed in Figure 3. A target sequencing library was prepared from a portion of the purified cfDNA as described in Example 2. A primary sequencing library was prepared using the remaining portion of the purified cfDNA as described in Example 2. Enrichment of the primary library for the amplified polymorphic nucleic acids comprised in the target library was obtained by diluting the primary and the target sequencing libraries to 10nM, and combining the target library with the primary library at a ratio of 1:9 to provide an enriched sequencing library. Sequencing of the enriched library and analysis of the sequencing data was performed as described in Example 2.

Table 4 provides the number of sequence tags that mapped to the SNP genome for the informative SNPs identified from sequencing an enriched library derived from plasma samples of pregnant women each carrying a T21, a T13, a T18 and a monosomy X fetus, respectively. Fetal fraction was calculated as follows:$% {fetal fraction allele}_{x} = \left(\left(\sum {Fetal sequence tags for allele}_{x}\right) / \left(\sum {Maternal sequence tags for allele}_{x}\right)\right) \times 100$

Table 4 also provides the number of the sequence tags mapped to the human reference genome. Tags mapped to the human reference genome were used to determine the presence or absence of aneuploidy using the same plasma sample that was utilized for determining the corresponding fetal fraction. Method for using sequence tags counts for determining aneuploidy are described in U.S. Provisional Applications 61/407,017 and 61/455,849778.

**TABLE 4**

| **Determination of Fetal Fraction by Massively Parallel Sequencing of an Enriched Library of Polymorphic Nucleic Acids** | | | |
|---|---|---|---|
| **Sample ID (karyotype)** | **SNP** | **SNP TAG COUNTS** | **FETAL FRACTION (%)** |
| 11409 (47, XY+21) | rs13182883.1\|Chr.5\|ength=111\|allele=A | 261 | 4.41 |
| | rs13182883.2\|Chr.5\|length=111\|allele=G | 5918 | |
| | rs740598.1\|Chr.10\|length=114\|allele=A | 5545 | 7.30 |
| | rs740598.2\|Chr.10\|length=114\|allele=G | 405 | |
| | rs8078417.1\|Chr.17\|length=101\|allele=C | 8189 | 6.74 |
| | rs8078417.2\|Chr.17\|length=110\|allele=T | 121470 | |
| | rs576261.1\|Chr.19\|length=114\|allele=A | 58342 | 7.62 |
| | rs576261.2\|Chr.19\|length=114\|allele=C | 4443 | |
| **Fetal Fraction** (**Mean±S**.**D**.) **= 6.5±1.5** | | | |
| **Sample ID** | | | |
| 95133 (47, XX+18) | rs1109037.1\|Chr.2\|length=126\|allele=A | 12229 | 2.15 |
| | rs1109037.2\|Chr.2\|length=126\|allele=G | 263 | |
| | rs13218440.1\|Chr.6\|length=139\|allele=A | 55949 | 3.09 |
| | rs13218440.2\|Chr.6\|length=139\|allele=G | 1729 | |
| | rs7041158.1\|Chr.9\|length=117\|allele=C | 7281 | 4.12 |
| | rs7041158.2\|Chr.9\|length=117\|allele=T | 300 | |
| | rs7205345.1\|Chr.16\|length=116\|allele=C | 53999 | 2.14 |
| | rs7205345.2\|Chr.16\|length=116\|allele=G | 1154 | |
| **Fetal Fraction** (**Mean±S**.**D**.) **= 2.9±0.9** | | | |
| **Sample ID** | | | |
| 51236 (46,XY+13) | rs13218440.1\|Chr.6\|length=139\|allele=A | 1119 | 1.65 |
| | rs13218440.2\|Chr.6\|length=139\|allele=G | 67756 | |
| | rs560681.1\|Chr.1\|length=111\|allele=A | 14123 | 5.18 |
| | rs560681.2\|Chr.1\|length=111\|allele=G | 732 | |
| | rs7205345.1\|Chr.16\|length=116\|allele=C | 18176 | 1.63 |
| | rs7205345.2\|Chr.16\|length=116\|allele=G | 296 | |
| | rs9866013.1\|Chr.3\|length=121\|allele=C | 117 | 2.33 |
| | rs9866013.21Chr.3\|length=121\|allele=T | 5024 | |
| **Fetal Fraction** (**Mean±S**.**D**.) **= 2**.**7±1**.**7** | | | |
| **Sample ID** | | | |
| 54430 (45,XO) | rs1109037.1\|Chr.2\|length=126\|allele=A | 19841 | 1.80 |
| | rs1109037.2\|Chr.2\|length=126\|allele=G | 357 | |
| | rs9866013.1\|Chr.3\|length=121\|allele=C | 12931 | 3.81 |
| | rs9866013.2\|Chr.3\|length=121\|allele=T | 493 | |
| | rs7041158.1\|Chr.9\|length=117\|allele=C | 2800 | 4.25 |
| | rs7041158.2\|Chr.9\|length=117\|allele=T | 119 | |
| | rs740598.1\|Chr.10\|length=114\|allele=A | 12903 | 4.87 |
| | rs740598.2\|Chr.10\|length=114\|allele=G | 628 | |
| | rs10773760.1\|Chr.12\|length=128\|allele=A | 46324 | 4.65 |
| | rs10773760.2\|Chr.12\|length=128\|allele=G | 2154 | |
| **Fetal Fraction** (**Mean±S**.**D**.) **= 3**.**9±1**.**2** | | | |

### Example 6

### Determination of Fetal Fraction by Massively Parallel Sequencing: Enrichment of Fetal and Maternal Nucleic Acids for Polymorphic Nucleic Acids in a Purified cfDNA Sample.

To enrich the fetal and maternal cfDNA contained in a purified sample of cfDNA extracted from a maternal plasma sample, a portion of the purified cfDNA was used for amplifying polymorphic target nucleic acid sequences each comprising one SNP chosen from the panel of SNPs given in Table 5.

The method corresponds to workflow 2 diagrammed in Figure 3. Cell-free plasma was obtained from a maternal blood sample, and cfDNA was purified from the plasma sample as described in Example 1. The final concentration was determined to be 92.8pg/µl. cfDNA contained in 5µl of purified cfDNA was amplified in a reaction volume of 50µl containing 7.5µl of a 1uM primer mix (Table 1), 10µl of NEB 5X Mastermix and 27 µl water. Thermal cycling was performed with the Gene Amp9700 (Applied Biosystems). Using the following cycling conditions: incubating at 95°C for 1 minute, followed by 30 cycles at 95°C for 20 seconds, 68°C for 1 minute, and 68°C for 30s, which was followed by a final incubation at 68°C for 5 minutes. A final hold at 4°C was added until the samples were removed for combining with the unamplified portion of the purified cfDNA sample. The amplified product was purified using the Agencourt AMPure XP PCR purification system (Part No. A63881; Beckman Coulter Genomics, Danvers, MA), and the concentration quantified using the Nanodrop 2000 (Thermo Scientific, Wilmington, DE). The purified amplification product was diluted 1:10 in water and 0.9 µl (371pg) added to 40µl of purified cfDNA sample to obtain a 10% spike. The enriched fetal and maternal cfDNA present in the purified cfDNA sample was used for preparing a sequencing library, and was sequenced as described in Example 2.

Table 5 provides the tag counts obtained for each of chromosomes 21, 18, 13, X and Y *i.e.* sequence tag density, and the tag counts obtained for the informative polymorphic sequences contained in the SNP reference genome *.i.e.* SNP tag density. The data show that sequencing information can be obtained from sequencing a single library constructed from a purified maternal cfDNA sample that has been enriched for sequences comprising SNPs to simultaneously determine the presence or absence of aneuploidy and the fetal fraction. The presence or absence of aneuploidy was determined using the number of tags mapped to chromosomes as described in U.S. Provisional Applications 61/407,017 and 61/455,849. In the example given, the data show that the fraction of fetal DNA in plasma sample AFR105 was quantifiable from the sequencing results of five informative SNPs and determined to be 3.84%. Sequence tag densities are provided for chromosomes 21, 13, 18, X and Y.

The example shows that the enrichment protocol provides the requisite tag counts for determining aneuploidy and fetal fraction from a single sequencing process.

**TABLE 5**

| **Determination of Fetal Fraction by Massively Parallel Sequencing: Enrichment of Fetal and Maternal Nucleic Acids for Polymorphic Nucleic Acids in a Purified cfDNA sample** | | | | | |
|---|---|---|---|---|---|
| **Aneuploidy** | | | | | |
| | Chromosome 21 | Chromosome 18 | Chromosome 13 | Chromosome X | Chromosome Y |
| Sequence Tag Density | 178763 | 359529 | 388204 | 572330 | 2219 |
| Karyotype | Unaffected | Unaffected | Unaffected | Unaffected | Unaffected |

| **Fetal Fraction** | | | | | |
|---|---|---|---|---|---|
| **SNP** | | **SNP TAG DENSITY** | | **FETAL FRACTION (%)** | |
| rs10773760.1\|Chr.12\|length=128\|allele=A | | 18903 | | 2.81 | |
| rs10773760.2\|Chr.12\|length=128\|allele=G | | 532 | | | |
| rs1109037.1\|Chr.2\|length=126\|allele=A | | 347 | | 5.43 | |
| rs1109037.2\|Chr.2\|length=126\|allele=G | | 6394 | | | |
| rs2567608.1\|Chr.20\|length=110\|allele=A | | 94503 | | 1.74 | |
| rs2567608.2\|Chr.20\|length=110\|allele=G | | 1649 | | | |
| rs7041158.1\|Chr.9\|length=117\|allele=C | | 107 | | 5.61 | |
| rs7041158.2\|Chr.9\|length=117\|allele=T | | 6 | | | |
| rs8078417.1\|Chr.17\|length=110\|allele=C | | 162668 | | 3.61 | |
| rs8078417.2\|Chr.17\|length=110\|allele=T | | 5877 | | | |
| **Fetal Fraction (Mean±S.D.) = 3**.**8±1**.**7** | | | | | |

### Example 7

### Determination of Fetal Fraction by Massively Parallel Sequencing: Enrichment of Fetal and Maternal Nucleic Acids for Polymorphic Nucleic Acids in a Plasma Sample.

To enrich the fetal and maternal cfDNA contained in an original plasma sample derived from a pregnant woman, a portion the original plasma sample was used for amplifying polymorphic target nucleic acid sequences each comprising one SNP chosen from the panel of SNPs given in Table 1, and a portion of the amplified product was combined with the remaining original plasma sample.

The method corresponds to workflow 2 diagrammed in Figure 3. cfDNA contained in 15µl of cell-free plasma was amplified in a reaction volume of 50µl containing 9ul of a 1 µM mixture of primers (15 plexTable 1), 1µl of Phusion blood DNA polymerase, 25ul of the 2X Phusion blood PCR buffer containing deoxynucleotide triphosphates (dNTPs: dATP, dCTP, dGTP and dTTP). Thermal cycling was performed with the Gene Amp9700 (Applied Biosystems) using the following cycling conditions: incubating at 95°C for 3 minutes, followed by 35 cycles at 95°C for 20 seconds, 55°C for 30s, and 70°C for 1 minute, which was followed by a final incubation at 68°C for 5 minutes. A final hold at 4°C was added until the samples were removed for combining with the unamplified portion of the cell-free plasma. The amplified product was diluted 1:2 with water and analyzed using the Bioanalyzer. An additional 3µl of amplified product was diluted with 11.85 µl of water to obtain a final concentration of 2ng/µl. 2.2µl of the diluted amplified product was combined with the remaining plasma sample. The enriched fetal and maternal cfDNA present in the plasma sample was purified as described in Example 1, and used for preparing a sequencing library. Sequencing and analysis of the sequencing data was performed as described in Example 2.

The results are given in Table 6. In the example given, the data show that the fraction of fetal DNA in plasma sample SAC2517 was quantifiable from the sequencing results of one informative SNP and determined to be 9.5%. In the example given, sample SAC2517 was shown by karyotyping to be unaffected for aneuploidies of chromosomes 21, 13, 18, X and Y. Sequence tag densities are provided for chromosomes 21, 13, 18, X and Y. The presence or absence of aneuploidy was determined using tag counts as described in U.S. Provisional Applications 61/407,017 and 61/455,849,.

The example demonstrates that enriching the mixture of fetal and maternal cfDNA present in a plasma sample for nucleic acid sequences that comprise at least one informative SNP can be used to provide the requisite sequence and SNP tag counts for determining aneuploidy and fetal fraction from a single sequencing process by massively parallel sequencing a library prepared from cfDNA contained in a plasma sample that is enriched for polymorphic nucleic acids.

**TABLE 6**

| **Determination of Fetal Fraction by Massively Parallel Sequencing: Enrichment of Fetal and Maternal Nucleic Acids for Polymorphic Nucleic Acids Comprising a SNP in a Plasma Sample** | | | | | |
|---|---|---|---|---|---|
| **Aneuploidy** | | | | | |
| | Chromosome 21 | Chromosome 18 | Chromosome 13 | Chromosome X | Chromosome Y |
| Sequence Tag Density | 183851 | 400582 | 470526 | 714055 | 2449 |
| Karyotype | Unaffected | Unaffected | Unaffected | Unaffected | Unaffected |

| **Fetal Fraction** | | | | | |
|---|---|---|---|---|---|
| **SNP** | | **TAG COUNTS** | | **FETAL FRACTION (%)** | |
| rs10773760.1\|Chr.12\|length=128\|allele=A | | 8536 | | **9.5** | |
| rs10773760.2\|Chr.12\|length=128\|allele=G | | 89924 | | | |

### Example 8

### Determination of Fetal Fraction by Massively Parallel Sequencing of Samples Comprising Amplified Polymorphic Sequences: Tandem SNPs

To determine the fraction of fetal cfDNA in a maternal sample, target polymorphic nucleic acid sequences each comprising a pair of tandem SNPs are amplified and used for preparing a target library for sequencing in a massively parallel fashion. Pairs of tandem SNPs can be selected from rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The primers used for amplifying the target sequences comprising the tandem SNPs are designed to encompass both SNP sites. For example, the forward primer is designed to encompass the first SNP, and the reverse primer is designed to encompass the second of the tandem SNP pair *i.e*. each of the SNP sites in the tandem pair is encompassed within the 36 bp generated by the sequencing method. Paired-end sequencing can be used to identify all sequences encompassing the tandem SNP sites. Exemplary sets of primers that are used to amplify the tandem SNPs disclosed herein are rs7277033-rs2110153_F: TCCTGGAAACAAAAGTATT (SEQ ID NO:197) and rs7277033-rs2110153_R: AACCTTACAACAAAGCTAGAA (SEQ ID NO:198), set rs2822654-rs1882882_F: ACTAAGCCTTGGGGATCCAG (SEQ ID NO:199) and rs2822654-rs1882882_R: TGCTGTGGAAATACTAAAAGG (SEQ ID NO:200), set rs368657-rs376635_F:CTCCAGAGGTAATCCTGTGA (SEQ ID NO:201) and rs368657-rs376635_R:TGGTGTGAGATGGTATCTAGG (SEQ ID NO:202), rs2822731-rs2822732_F:GTATAATCCATGAATCTTGTTT (SEQ ID NO:203) and rs2822731-rs2822732_R:TTCAAATTGTATATAAGAGAGT (SEQ ID NO:204), rs1475881-rs7275487_F:GCAGGAAAGTTATTTTTAAT (SEQ ID NO:205) and rs1475881-rs7275487_R:TGCTTGAGAAAGCTAACACTT (SEQ ID NO:206), rs1735976-rs2827016F:CAGTGTTTGGAAATTGTCTG (SEQ ID NO:207) and rs1735976-rs2827016_R:GGCACTGGGAGATTATTGTA (SEQ ID NO:208), rs447349-rs2824097_F:TCCTGTTGTTAAGTACACAT (SEQ ID NO:209) and rs447349-rs2824097_R:GGGCCGTAATTACTTTTG (SEQ ID NO:210), rs418989-rs13047336_F:ACTCAGTAGGCACTTTGTGTC (SEQ ID NO:211) and rs418989-rs13047336_R:TCTTCCACCACACCAATC (SEQ ID NO:212), rs987980-rs987981_F:TGGCTTTTCAAAGGTAAAA (SEQ ID NO:213) and rs987980- rs987981_R: GCAACGTTAACATCTGAATTT (SEQ ID NO:214), rs4143392- rs4143391_F: rs4143392- rs4143391 (SEQ ID NO:215) and rs4143392-rs4143391_R:ATTTTATATGTCATGATCTAAG (SEQ ID NO:216), rs1691324-rs13050434_F: AGAGATTACAGGTGTGAGC (SEQ ID NO:217) and rs1691324- rs13050434_R: ATGATCCTCAACTGCCTCT (SEQ ID NO:218), rs11909758-rs9980111_F: TGAAACTCAAAAGAGAAAAG (SEQ ID NO:219) and rs11909758-rs9980111_R: ACAGATTTCTACTTAAAATT (SEQ ID NO:220), rs2826842-rs232414_F: TGAAACTCAAAAGAGAAAAG (SEQ ID NO:221) and rs2826842-rs232414_R: ACAGATTTCTACTTAAAATT (SEQ ID NO:22), rs2826842-rs232414_F: GCAAAGGGGTACTCTATGTA (SEQ ID NO:223) and rs2826842-rs232414_R: TATCGGGTCATCTTGTTAAA (SEQ ID NO:224), rs1980969-rs1980970_F: TCTAACAAAGCTCTGTCCAAAA (SEQ ID NO:225) and rs1980969-rs1980970_R: CCACACTGAATAACTGGAACA (SEQ ID NO:226), rs9978999-rs9979175_F: GCAAGCAAGCTCTCTACCTTC (SEQ ID NO:227) and rs9978999-rs9979175_R: TGTTCTTCCAAAATTCACATGC (SEQ ID NO:228), rs1034346-rs12481852_F: ATTTCACTATTCCTTCATTTT (SEQ ID NO:229) and rs1034346-rs12481852_R: TAATTGTTGCACACTAAATTAC (SEQ ID NO:230), rs4817013-rs7277036_F: AAAAAGCCACAGAAATCAGTC (SEQ ID NO:231) and rs4817013-rs7277036_R: TTCTTATATCTCACTGGGCATT (SEQ ID NO:232), rs9981121-rs2829696_F: GGATGGTAGAAGAGAAGAAAGG (SEQ ID NO:233) and rs9981121-rs2829696_R: GGATGGTAGAAGAGAAGAAAGG (SEQ ID NO:234), rs455921-rs2898102_F: TGCAAAGATGCAGAACCAAC (SEQ ID NO:235) and rs455921-rs2898102_R: TTTTGTTCCTTGTCCTGGCTGA (SEQ ID NO:236), rs2898102- rs458848_F: TGCAAAGATGCAGAACCAAC (SEQ ID NO:237) and rs2898102- rs458848_R: GCCTCCAGCTCTATCCAAGTT (SEQ ID NO:238), rs961301-rs2830208_F: CCTTAATATCTTCCCATGTCCA (SEQ ID NO:239) and rs961301-rs2830208_R: ATTGTTAGTGCCTCTTCTGCTT (SEQ ID NO:240), rs2174536-rs458076_F: GAGAAGTGAGGTCAGCAGCT (SEQ ID NO:241) and rs2174536-rs458076_R: TTTCTAAATTTCCATTGAACAG (SEQ ID NO:242), rs1 1088023-rs11088024_F: GAAATTGGCAATCTGATTCT (SEQ ID NO:243) and rs11088023-rs11088024_R: CAACTTGTCCTTTATTGATGT (SEQ ID NO:244), rs1011734-rs1011733_F: CTATGTTGATAAAACATTGAAA (SEQ ID NO:245) and rs1011734-rs1011733_R: GCCTGTCTGGAATATAGTTT (SEQ ID NO:246), rs2831244-rs9789838_F: CAGGGCATATAATCTAAGCTGT (SEQ ID NO:247) and rs2831244-rs9789838_R: CAATGACTCTGAGTTGAGCAC (SEQ ID NO:248), rs8132769-rs2831440_F: ACTCTCTCCCTCCCCTCT (SEQ ID NO:249) and rs8132769-rs2831440_R: TATGGCCCCAAAACTATTCT (SEQ ID NO:250), rs8134080-rs2831524_F: ACAAGTACTGGGCAGATTGA (SEQ ID NO:251) and rs8134080-rs2831524_R: GCCAGGTTTAGCTTTCAAGT (SEQ ID NO:252), rs4817219-rs4817220_F: TTTTATATCAGGAGAAACACTG (SEQ ID NO:253) and rs4817219-rs4817220_R: CCAGAATTTTGGAGGTTTAAT (SEQ ID NO:254), rs2250911-rs2250997_F: TGTCATTCCTCCTTTATCTCCA (SEQ ID NO:255) and rs2250911-rs2250997_R: TTCTTTTGCCTCTCCCAAAG (SEQ ID NO:256), rs2831899-rs2831900_F: ACCCTGGCACAGTGTTGACT (SEQ ID NO:257) and rs2831899-rs2831900_R: TGGGCCTGAGTTGAGAAGAT (SEQ ID NO:258), rs2831902-rs2831903_F: AATTTGTAAGTATGTGCAACG (SEQ ID NO:259) and rs2831902-rs2831903_R: TTTTTCCCATTTCCAACTCT (SEQ ID NO:260), rs11088086-rs2251447_F: AAAAGATGAGACAGGCAGGT (SEQ ID NO:261) and rs11088086-rs2251447_R: ACCCCTGTGAATCTCAAAAT (SEQ ID NO:262), rs2832040-rs11088088_F: GCACTTGCTTCTATTGTTTGT (SEQ ID NO:263) and rs2832040-rs11088088_R: CCCTTCCTCTCTTCCATTCT (SEQ ID NO:264), rs2832141-rs2246777_F: AGCACTGCAGGTA (SEQ ID NO:265) and rs2832141-rs2246777_R: ACAGATACCAAAGAACTGCAA (SEQ ID NO:266), rs2832959 - rs9980934_F: TGGACACCTTTCAACTTAGA (SEQ ID NO:267) and rs2832959 -rs9980934_R: GAACAGTAATGTTGAACTTTTT (SEQ ID NO:268), rs2833734-rs2833735_F: TCTTGCAAAAAGCTTAGCACA (SEQ ID NO:269) and rs2833734-rs2833735_R: AAAAAGATCTCAAAGGGTCCA (SEQ ID NO:270), rs933121-rs933122_F: GCTTTTGCTGAACATCAAGT (SEQ ID NO:271) and rs933121-rs933122_R: CCTTCCAGCAGCATAGTCT (SEQ ID NO:272), rs2834140-rs12626953_F: AAATCCAGGATGTGCAGT (SEQ ID NO:273) and rs2834140-rs12626953_R: ATGATGAGGTCAGTGGTGT (SEQ ID NO:274), rs2834485-rs3453_F: CATCACAGATCATAGTAAATGG (SEQ ID NO:275) and rs2834485-rs3453_R: AATTATTATTTTGCAGGCAAT (SEQ ID NO:276), rs9974986-rs2834703_F: CATGAGGCAAACACCTTTCC (SEQ ID NO:277) and rs9974986-rs2834703_R: GCTGGACTCAGGATAAAGAACA (SEQ ID NO:278), rs2776266-rs2835001_F: TGGAAGCCTGAGCTGACTAA (SEQ ID NO:279) and rs2776266-rs2835001_R:CCTTCTTTTCCCCCAGAATC (SEQ ID NO:280), rs1984014-rs1984015_F:TAGGAGAACAGAAGATCAGAG (SEQ ID NO:281) and rs1984014-rs1984015_R:AAAGACTATTGCTAAATGCTTG (SEQ ID NO:282), rs7281674-rs2835316_F: TAAGCGTAGGGCTGTGTGTG (SEQ ID NO:283) and rs7281674-rs2835316_R: GGACGGATAGACTCCAGAAGG (SEQ ID NO:284), rs13047304-rs13047322_F: GAATGACCTTGGCACTTTTATCA (SEQ ID NO:285) and rs13047304-rs13047322_R: AAGGATAGAGATATACAGATGAATGGA (SEQ ID NO:286), rs2835735-rs2835736_F: CATGCACCGCGCAAATAC (SEQ ID NO:287) and rs2835735-rs2835736_R: ATGCCTCACCCACAAACAC (SEQ ID NO:288), rs13047608-rs2835826_F: TCCAAGCCCTTCTCACTCAC (SEQ ID NO:289) and rs13047608-rs2835826_R: CTGGGACGGTGACATTTTCT (SEQ ID NO:290), rs2836550-rs2212596_F: CCCAGGAAGAGTGGAAAGATT (SEQ ID NO:291) and rs2836550-rs2212596_R: TTAGCTTGCATGTACCTGTGT (SEQ ID NO:292), rs2836660-rs2836661_F: AGCTAGATGGGGTGAATTTT (SEQ ID NO:293) and _R: TGGGCTGAGGGGAGATTC (SEQ ID NO:294), rs465612-rs8131220_F: ATCAAGCTAATTAATGTTATCT (SEQ ID NO:295) and rs465612-rs8131220_R: AATGAATAAGGTCCTCAGAG (SEQ ID NO:296), rs9980072-rs8130031_F:TTTAATCTGATCATTGCCCTA (SEQ ID NO:297) and rs9980072-rs8130031_R: AGCTGTGGGTGACCTTGA (SEQ ID NO:298), rs418359-rs2836926_F: TGTCCCACCATTGTGTATTA (SEQ ID NO:299) and rs418359-rs2836926_R: TCAGACTTGAAGTCCAGGAT (SEQ ID NO:300), rs7278447-rs7278858_F: GCTTCAGGGGTGTTAGTTTT (SEQ ID NO:301) and rs7278447-rs7278858_R: CTTTGTGAAAAGTCGTCCAG (SEQ ID NO:302), rs385787-rs367001_F:CCATCATGGAAAGCATGG (SEQ ID NO:303) and rs385787-rs367001_R: TCATCTCCATGACTGCACTA (SEQ ID NO:304), rs367001-rs386095_F: GAGATGACGGAGTAGCTCAT (SEQ ID NO:305) and rs367001-rs386095_R: CCCAGCTGCACTGTCTAC (SEQ ID NO:306), rs2837296-rs2837297_F: TCTTGTTCCAATCACAGGAC (SEQ ID NO:307) and rs2837296-rs2837297_R: ATGCTGTTAGCTGAAGCTCT (SEQ ID NO:308), and rs2837381-rs4816672_F: TGAAAGCTCCTAAAGCAGAG (SEQ ID NO:309) andrs2837381-rs4816672_R:TTGAAGAGATGTGCTATCAT (SEQ ID NO:310). Polynucleotide sequences *e.g.* GC clamp sequences, can be included to ensure specific hybridization of AT-rich primers (Ghanta et al., PLOS ONE 5(10): doi10.1371/journal.pone.0013184 [2010], available on the world wide web at plosone.org). An example of a GC clamp sequence that can be included either 5' of the forward primer or 3' of the reverse primer is GCCGCCTGCAGCCCGCGCCCCCCGTGCCCCCGCCCCGCCGCCGGCCCGGGCGCC (SEQ ID NO:311).

Polymorphic sequences can be used alone or in combination with unamplified cfDNA to determine either fetal fraction or the presence or absence of aneuploidy and fetal fraction in a maternal sample as described for polymorphic SNP sequences. Sample preparation and enrichment of cfDNA sequencing library, a purified cfDNA sample, and a plasma sample is performed according to the method described in Examples 5, 6, and 7, respectively. All sequencing libraries are prepared as described in Example 2a., and sequencing is performed as described in Example 2b. Analysis of the sequencing data for the determination of fetal aneuploidy is performed as described in Example 5. Concomitant to the analysis for determining aneuploidy, the sequencing data is analyzed to determine the fetal fraction as follows. Following the transfer of the image and base call files to the Unix server running the Illumina "Genome Analyzer Pipeline" software version 1.51 as described in Example 3a., the 36bp reads are aligned to a 'tandem SNP genome' using the BOWTIE program. The tandem SNP genome is identified as the grouping of the DNA sequences that encompass the alleles of the 58 tandem SNP pairs disclosed above. Only reads that mapped uniquely to the tandem SNP genome are used for the analysis of fetal fraction. Reads that match perfectly to the tandem SNP genome are counted as tags and filtered. Of the remaining reads, only reads having one or two mismatches are counted as tags and included in the analysis. Tags mapped to each of the tandem SNP alleles are counted, and the fetal fraction is determined essentially as described in Example 6 above but accounting for tags mapped to the two tandem SNP alleles x and y present on each of the amplified polymorphic target nucleic acid sequences that are amplified to enrich the samples *i.e.* ${% fetal fraction allele}_{x + y} = \left(\left(\sum {Fetal sequence tags for allele}_{x + y}\right) / \left(\sum {Maternal sequence tags for allele}_{x + y}\right)\right) \times 100$ Only informative tandem SNPs are used to determine the fetal fraction.

Optionally, the fraction of fetal nucleic acids in the mixture of fetal and maternal nucleic acids is calculated for each of the informative allele (allele_{x+y}) as follows: ${% fetal fraction allele}_{x + y} = \left(\left(2 \times \sum {Fetal sequence tags for allele}_{x + y}\right) / \left(\sum {Maternal sequence tags for allele}_{x + y}\right)\right) \times 100,$ to compensate for the presence of 2 sets of tandem fetal alleles, one being masked by the maternal background.

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or more informative sets of tandem alleles. In one embodiment, the fetal fraction is the average fetal fraction determined for at least 3 informative sets of tandem alleles.

### Example 9

### Determination of Fetal Fraction by Massively Parallel Sequencing of Samples Comprising Amplified Polymorphic Sequences: STRs

To determine the fraction of fetal cfDNA in a maternal sample, target polymorphic nucleic acid sequences each comprising an STR are amplified and used for preparing a target library for sequencing in a massively parallel fashion.

Peripheral blood samples are obtained from pregnant subjects, and cfDNA is purified from the plasma fraction as described in Examples 1 and 2 STRs that are amplified are chosen from the codis and non-codis STRs disclosed in Table 7, and amplification of the polymorphic STR sequences is obtained using the corresponding sets of primers provided. For example, the STRs listed in Table 7 are amplified using the corresponding primers (SEQ ID NOs: 113-197), and the amplified product is used to generate a target sequencing library. The STR target sequencing library is prepared as described for the preparation of the SNP target library as described in Example 8. STRs CSF1PO, D13S317, D16S539, D18S51, D21S11, D2S1338D7S820, and FGA have been analyzed previously for determining fetal fraction, and are disclosed in US Provisional applications 61/296,358 and 61/360,837.

**TABLE 7**

| **CODIS and NON-CODIS miniSTRs** | | | | |
|---|---|---|---|---|
| **STR Locus (Marker Name)** | **Chromosome Location** | **Size Range (bp)** | **GenBank Accession** | **Primer Sequences (Forward/Reverse)** |
| **Codis miniSTR loci*** | | | | |
| CSF1PO | 5q33.1 | 89-129 | X14720 | ACAGTAACTGCCTTCATAGATAG (CSF1PO_F; SEQ ID NO:113) |
| | | | | GTGTCAGACCCTGTTCTAAGTA (CSF1PO_R; SEQ ID NO:114) |
| FGA | 4q31.3 | 125-281 | M64982 | AAATAAAATTAGGCATATTTACAAGC (FGA_F; SEQ ID NO:115) |
| | | | | GCTGAGTGATTTGTCTGTAATTG(FGA R; SEQ ID NO:116) |
| TH01 | 11p15.5 | 51-98 | D00269 | CCTGTTCCTCCCTTATTTCCC(TH01_F; SEQ ID NO:117) |
| | | | | GGGAACACAGACTCCATGGTG(TH01_R; SEQ ID NO:118) |
| TPOX | 2p25.3 | 65-101 | M68651 | CTTAGGGAACCCTCACTGAATG(TPOX_F ; SEQ ID NO:119) |
| | | | | GTCCTTGTCAGCGTTTATTTGC(TPOX_R; SEQ ID NO:120) |
| vWA | 12p13.31 | 88-148 | M25858 | AATAATCAGTATGTGACTTGGATTGA(v WA_F; SEQ ID NO:121) |
| | | | | ATAGGATGGATGGATAGATGGA(vWA_R ; SEQ ID NO:122) |
| D3S1358 | 3p21.31 | 72-120 | NT_005997 | CAGAGCAAGACCCTGTCTCAT(D3S1358_ F; SEQ ID NO:123) |
| | | | | TCAACAGAGGCTTGCATGTAT(D3S1358_ R; SEQ ID NO:124) |
| D5S818 | 5q23.2 | 81-117 | AC008512 | GGGTGATTTTCCTCTTTGGT(D5S818_F; SEQ ID NO:125) |
| | | | | |
| D7S820 | 7q21.11 | 136-176 | AC004848 | GAACACTTGTCATAGTTTAGAACGAAC( |
| | | | | D7S820_F; SEQ ID NO:127) |
| | | | | TCATTGACAGAATTGCACCA(D7S820_R; SEQ ID NO:128) |
| D8S1179 | 8q24.13 | 86-134 | AF216671 | TTTGTATTTCATGTGTACATTCGTATC(D 7S820_F; SEQ ID NO:129) |
| | | | | ACCTATCCTGTAGATTATTTTCACTGTG( D7S820_R; SEQ ID NO:130) |
| D13S317 | 13q31.1 | 88-132 | AL353628 | TCTGACCCATCTAACGCCTA(D13S317_F; SEQ ID NO:131) |
| | | | | CAGACAGAAAGATAGATAGATGATTGA( D13S317_R; SEQ ID NO:132) |
| D16S539 | 16q24.1 | 81-121 | AC024591 | ATACAGACAGACAGACAGGTG(D16S539 _F; SEQ ID NO:133) |
| | | | | GCATGTATCTATCATCCATCTCT(D16S53 9_R; SEQ ID NO:134) |
| D18S51 | 18q21.33 | 113-193 | AP001534 | TGAGTGACAAATTGAGACCTT(D18S51_F ; SEQ ID NO:135) |
| | | | | GTCTTACAATAACAGTTGCTACTATT(D1 8S51_R; SEQ ID NO:136) |
| D21S11 | 21q21.1 | 153-221 | AP000433 | ATTCCCCAAGTGAATTGC(D21S11_F; SEQ ID NO:137) |
| | | | | GGTAGATAGACTGGATAGATAGACGA(D 21S11_R; SEQ ID NO:138) |
| D2S1338 | 2q35 | 90-142 | AC01036 | TGGAAACAGAAATGGCTTGG(D2S1338_F ; SEQ ID NO:139) |
| | | | | GATTGCAGGAGGGAAGGAAG(D2S1338_ R; SEQ ID NO:140) |
| Penta D | 21q22.3 | 94-167 | AP001752 | GAGCAAGACACCATCTCAAGAA(Penta D_F; SEQ ID NO:141) |
| | | | | |
| Penta E | 15q26.2 | 80-175 | AC027004 | GGCGACTGAGCAAGACTC(Penta E_F; SEQ ID NO:143) |
| | | | | GGTTATTAATTGAGAAAACTCCTTACA( Penta E_R; SEQ ID NO:144) |

| **Non-Codis miniSTR loci*** | | | | |
|---|---|---|---|---|
| D22S1045 | 22q12.3 | 82 - 115 | AL022314 (17) | ATTTTCCCCGATGATAGTAGTCT(D22S10 45_F; SEQ ID NO:145) |
| | | | | GCGAATGTATGATTGGCAATATTTTT(D2 2S1045_R; SEQ ID NO:146) |
| D20S1082 | 20q13.2 | 73 - 101 | AL158015 | ACATGTATCCCAGAACTTAAAGTAAAC( D20S1082_F; SEQ ID NO:147) |
| | | | | GCAGAAGGGAAAATTGAAGCTG(D20S10 82_R; SEQ ID NO:148) |
| D20S482 | 20p13 | 85 - 126 | AL121781 (14) | CAGAGACACCGAACCAATAAGA(D20S48 2_F; SEQ ID NO:149) |
| | | | | GCCACATGAATCAATTCCTATAATAAA( D20S482_R; SEQ ID NO:150) |
| D18S853 | 18p11.31 | 82 - 104 | AP005130 (11) | GCACATGTACCCTAAAACTTAAAAT(D18 S853_F; SEQ ID NO:151) |
| | | | | GTCAACCAAAACTCAACAAGTAGTAA(D 18S853_R; SEQ ID NO:152) |
| D17S1301 | 17q25.1 | 114 - 139 | AC016888 (12) | AAGATGAAATTGCCATGTAAAAATA(D1 7S1301_F; SEQ ID NO:153) |
| | | | | GTGTGTATAACAAAATTCCTATGATGG( D17S1301_R; SEQ ID NO:154) |
| D17S974 | 17p13.1 | 114 - 139 | AC034303 (10) | GCACCCAAAACTGAATGTCATA(D17S97 4_F; SEQ ID NO:155) |
| | | | | GGTGAGAGTGAGACCCTGTC(D17S974_R ; SEQ ID NO:156) |
| D14S1434 | 14q32.13 | 70 - 98 | AL121612 (13) | TGTAATAACTCTACGACTGTCTGTCTG(D 14S1434_F; SEQ ID NO:157) |
| | | | | GAATAGGAGGTGGATGGATGG(D 14S 143 4_R; SEQ ID NO:158) |
| D12ATA63 | 12q23.3 | 76 - 106 | AC009771 (13) | GAGCGAGACCCTGTCTCAAG(D12ATA63 _F; SEQ ID NO:159) |
| | | | | GGAAAAGACATAGGATAGCAATTT(D12 ATA63_R; SEQ ID NO:160) |
| D11S4463 | 11q25 | 88 - 116 | AP002806 (14) | TCTGGATTGATCTGTCTGTCC(D11S4463_ F; SEQ ID NO:161) |
| | | | | GAATTAAATACCATCTGAGCACTGAA(D 11S4463_R; SEQ ID NO:162) |
| D10S1435 | 10p15.3 | 82 - 139 | AL354747 (11) | TGTTATAATGCATTGAGTTTTATTCTG(D 10S1435_F; SEQ ID NO:163) |
| | | | | |
| D10S1248 | 10q26.3 | 79 - 123 | AL391869 (13) | TTAATGAATTGAACAAATGAGTGAG(D1 0S1248_F; SEQ ID NO:165) |
| | | | | GCAACTCTGGTTGTATTGTCTTCAT(D10S 1248_R; SEQ ID NO:166) |
| D9S2157 | 9q34.2 | 71 - 107 | AL162417 (10) | CAAAGCGAGACTCTGTCTCAA(D9S2157_ F; SEQ ID NO:167) |
| | | | | GAAAATGCTATCCTCTTTGGTATAAAT( D9S2157_R; SEQ ID NO:168) |
| D9S1122 | 9q21.2 | 93 - 125 | AL161789 (12) | GGGTATTTCAAGATAACTGTAGATAGG( D9S1122_F; SEQ ID NO:168) |
| | | | | GCTTCTGAAAGCTTCTAGTTTACC(D9S11 22_R; SEQ ID NO:170) |
| D8S1115 | 8p11.21 | 63 - 96 | AC090739 (9) | TCCACATCCTCACCAACAC(D8S1115_F; SEQ ID NO:171) |
| | | | | GCCTAGGAAGGCTACTGTCAA(D8S1115_ R; SEQ ID NO:172) |
| D6S1017 | 6p21.1 | 81 - 110 | AL035588 (10) | CCACCCGTCCATTTAGGC(D6S1017_F; SEQ ID NO:173) |
| | | | | GTGAAAAAGTAGATATAATGGTTGGTG( D6S1017_R; SEQ ID NO:174) |
| D6S474 | 6q21 | 107 - 136 | AL357514 (17) | GGTTTTCCAAGAGATAGACCAATTA(D6S 474_F; SEQ ID NO:175) |
| | | | | GTCCTCTCATAAATCCCTACTCATATC(D 6S474_R; SEQ ID NO:176) |
| D5S2500 | 5q11.2 | 85 - 126 | AC008791 (17) | CTGTTGGTACATAATAGGTAGGTAGGT( D5S2500_F; SEQ ID NO:177) |
| | | | | GTCGTGGGCCCCATAAATC(D5S2500_R; SEQ ID NO:178) |
| D4S2408 | 4p15.1 | 85 - 109 | AC110763 (9) | AAGGTACATAACAGTTCAATAGAAAGC( D4S2408_F; SEQ ID NO:179) |
| | | | | GTGAAATGACTGAAAAATAGTAACCA(D 4S2408_R; SEQ ID NO:180) |
| D4S2364 | 4q22.3 | 67 - 83 | AC022317 (9) | CTAGGAGATCATGTGGGTATGATT(D4S2 364U_F; SEQ ID NO:181) |
| | | | | GCAGTGAATAAATGAACGAATGGA(D4S 2364_R; SEQ ID NO:182) |
| D3S4529 | 3p12.1 | 111 - 139 | AC117452 (13) | CCCAAAATTACTTGAGCCAAT(D3S452_F ; SEQ ID NO:183) |
| | | | | GAGACAAAATGAAGAAACAGACAG(D3 S 452_R; SEQ ID NO:184) |
| D3S3053 | 3q26.31 | 84 - 108 | AC069259 (9) | TCTTTGCTCTCATGAATAGATCAGT(D3S 3053_F; SEQ ID NO:185) |
| | | | | GTTTGTGATAATGAACCCACTCAG(D3 S3 053_R; SEQ ID NO:186) |
| D2S1776 | 2q24.3 | 127 - 161 | AC009475 (11) | TGAACACAGATGTTAAGTGTGTATATG( D2S1776_F; SEQ ID NO:187) |
| | | | | GTCTGAGGTGGACAGTTATGAAA(D2S17 76_R; SEQ ID NO:188) |
| D2S441 | 2p14 | 78 - 110 | AC079112 (12) | CTGTGGCTCATCTATGAAAACTT(D2S441 _F; SEQ ID NO:189) |
| | | | | GAAGTGGCTGTGGTGTTATGAT(D2S441_ R; SEQ ID NO:190) |
| D1S1677 | 1q23.3 | 81 - 117 | AL513307 (15) | TTCTGTTGGTATAGAGCAGTGTTT(D1S16 77_F; SEQ ID NO:191) |
| | | | | GTGACAGGAAGGACGGAATG(D1S1677_ R; SEQ ID NO:192) |
| D1S1627 | 1p21.1 | 81 - 100 | AC093119 (13) | CATGAGGTTTGCAAATACTATCTTAAC( D1S1627_F; SEQ ID NO:193) |
| | | | | GTTTTAATTTTCTCCAAATCTCCA(D 1 S 16 27_R; SEQ ID NO:194) |
| D1GATA113 | 1p36.23 | 81 - 105 | Z97987 (11) | TCTTAGCCTAGATAGATACTTGCTTCC(D 1GATA113_F; SEQ ID NO:195) |
| | | | | GTCAACCTTTGAGGCTATAGGAA(D1GA TA113_R; SEQ ID NO:196) |

| | | | | |
|---|---|---|---|---|
| *(Butler et al., J Forensic Sci 5:1054-1064; Hill et al., Poster #44- 17th International Symposium on Human Identification - 2006) | | | | |

Sequencing of the library enriched for polymorphic STR sequences is performed using a NGS technology *e.g*. sequencing by synthesis. Sequence reads of lengths that encompass the STRs *e.g*. miniSTRs of at least 100 bp, to a reference STR genome consisting of the polymorphic sequences which were amplified in the sample. Informative STR alleles are identified by differences in the length of the repeats, and the number of STR sequence tags are counted, and used to determine the fetal fraction. Optionally, amplification of the polymorphic STR sequences is performed to enrich a plasma sample, a purified cfDNA sample or a cfDNA sequencing library sample, as described in Examples 5, 6, and 7, respectively.

### Example 10

### Determination of Fetal Fraction by Capillary Electrophoresis of Polymorphic Sequences Comprising STRs

To determine fetal fraction in maternal samples comprising fetal and maternal cfDNA, peripheral blood samples were collected from volunteer pregnant women carrying either male or female fetuses. Peripheral blood samples were obtained and processed to provide purified cfDNA as described in Example 1

Ten microliters of cfDNA samples were analyzed using the AmpFlSTR® MiniFiler™ PCR amplification kit (Applied Biosystems, Foster City, CA) according to the manufacturer's instructions. Briefly, cfDNA contained in 10 µl was amplified in a reaction volume of 25 µl containing 5µL fluorescently labeled primers (AmpF*l*STR® MiniFiler™ Primer Set), and the AmpF*l*STR® MiniFiler™ Master Mix, which includes AmpliTaq Gold® DNA polymerase and associated buffer, salt (1.5 mM MgCl2), and 200 µM deoxynucleotide triphosphates (dNTPs: dATP, dCTP, dGTP and dTTP). The fluorescently labeled primers are forward primers that are labeled with 6FAM™, VIC™, NED™, and PET™ dyes. Thermal cycling was performed with the Gene Amp9700 (Applied Biosystems) using the following cycling conditions: incubating at 95°C for 10 minutes, followed by 30 cycles at 94°C for 20 seconds, 59°C for 2 minute, and 72°C for 1 minute, which was followed by a final incubation at 60°C for 45 minutes. A final hold at 4°C was added until the samples were removed for analysis. The amplified product was prepared by diluting 1ul of amplified product in 8.7ul Hi-DiTM formamide (Applied Biosystems) and 0.3 µl GeneScanTM-500 LIZ_internal size standard (Applied Biosystems), and analyzed with an ABI PRISM3130x1 Genetic Analyzer (Applied Biosystems) using Data Collection HID_G5_POP4 (Applied Biosystems), and a 36-cm capillary array. All genotyping was performed with GeneMapper_ID v3.2 software (Applied Biosystems) using manufacturer provided allelic ladders and bins and panels.

All genotyping measurement were performed on the Applied Biosystems 3130*xl* Genetic Analyzer, using a ±0.5-nt "window" around the size obtained for each allele to allow for detection and correct assignment of alleles. Any sample allele whose size was outside the ±0.5-nt window was determined to be OL *i.e.* "Off Ladder". OL alleles are alleles of a size that is not represented in the AmpF*l*STR® MiniFiler™ Allelic Ladder or an allele that does not correspond to an allelic ladder, but whose size is just outside a window because of measurement error. The minimum peak height threshold of >50 RFU was set based on validation experiments performed to avoid typing when stochastic effects are likely to interfere with accurate interpretation of mixtures. The calculation of fetal fraction is based on averaging all informative markers. Informative markers are identified by the presence of peaks on the electropherogram that fall within the parameters of preset bins for the STRs that are analyzed.

Calculations of fetal fraction were performed using the average peak height for major and minor alleles at every STR locus determined from triplicate injections. The rules applied to the calculation are:
1. off-ladder allele (OL) data for alleles are not included in the calculation; and
2. only peak heights derived from >50 RFU (relative fluorescence units) are included in the calculation
3. if only one bin is present the marker is deemed non-informative; and
4. if a second bin is called but the peaks of the first and second bins are within 50-70% of their relative fluorescence units (RFU) in peak height, the minority fraction is not measured and the marker is deemed not informative.

The fraction of the minor allele for any given informative marker is calculated by dividing the peak height of the minor component by the sum of the peak height for the major component, and expressed as a percent was first calculated for each informative locus as $fetal fraction = \left(\sum peak h e i g h t o f m i n o r a l l e l e / \sum peak height of major allele \left(s\right)\right) \times 100 ,$

The fetal fraction for a sample comprising two or more informative STRs, would be calculated as the average of the fetal fractions calculated for the two or more informative markers.

Table 8 provides the data obtained from analyzing cfDNA of a subject pregnant with a male fetus.

**TABLE 8**

| **Fetal Fraction Determined in cfDNA of a Pregnant Subject by Analysis of STRs** | | | | | | | | |
|---|---|---|---|---|---|---|---|---|
| STR | Allele 1 | Allele 2 | Allele 3 | Allelel Height | Allele 2 Height | Allele 3 Height | Fetal Fraction | Fetal Fraction (Mean/STR) |
| AMEL | X | Y | | 3599 | 106 | | 2.9 | |
| AMEL | X | Y | | 3602 | 110 | | 3.1 | |
| AMEL | X | Y | | 3652 | 109 | | 3.0 | 3.0 |
| CSF1PO | 11 | 12 | | 2870 | 2730 | | | |
| CSF1PO | 11 | 12 | | 2924 | 2762 | | | |
| CSF1PO | 11 | 12 | | 2953 | 2786 | | | |
| D13S317 | 11 | 12 | | 2621 | 2588 | | | |
| D13S317 | 11 | 12 | | 2680 | 2619 | | | |
| D13S317 | 11 | 12 | | 2717 | 2659 | | | |
| D16S539 | 9 | 11 | | 1056 | 1416 | | | |
| D16S539 | 9 | 11 | | 1038 | 1394 | | | |
| D16S539 | 9 | 11 | | 1072 | 1437 | | | |
| D18S51 | 13 | 15 | | 2026 | 1555 | | | |
| D18S51 | 13 | 15 | | 2006 | 1557 | | | |
| D18S51 | 13 | 15 | | 2050 | 1578 | | | |
| D21S11 | 28 | 31.2 | | 2450 | 61 | | 2.5 | |
| D21S11 | 28 | 31.2 | | 2472 | 62 | | 2.5 | |
| D21S11 | 28 | 31.2 | | 2508 | 67 | | 2.7 | 2.6 |
| D2S1338 | 20 | 23 | | 3417 | 3017 | | | |
| D2S1338 | 20 | 23 | | 3407 | 3020 | | | |
| D2S1338 | 20 | 23 | | 3493 | 3055 | | | |
| D7S820 | 9 | 12 | 13 | 2373 | 178 | 1123 | 5.1 | |
| D7S820 | 9 | 12 | 13 | 2411 | 181 | 1140 | 5.1 | |
| D7S820 | 9 | 12 | 13 | 2441 | 182 | 1156 | 5.1 | 5.1 |
| FGA | 17.2 | 22 | 25 | 68 | 1140 | 896 | 3.3 | |
| FGA | 17.2 | 22 | 25 | 68 | 1144 | 909 | 3.1 | |
| FGA | 17.2 | 22 | 25 | 68 | 1151 | 925 | 3.3 | 3.2 |
| **Fetal Fraction = 3.5** | | | | | | | | |

The results show that cfDNA can be used for determining the presence or absence of fetal DNA as indicated by the detection of a minor component at one or more STR alleles, for determining the percent fetal fraction, and for determining fetal gender as indicated by the presence or absence of the Amelogenin allele.

### Example 11

### Preamplification of cfDNA for Determining Fetal Fraction by Capillary Electrophoresis of Polymorphic Sequences Comprising STRs

To improve the sensitivity of the STR assay in detecting and quantifying the STR alleles in the minor contributor of the cfDNA sample, the number of starting genomes in the artificial samples was increased by a modified whole genome amplification strategy.

Peripheral blood samples were collected and processed as described in Example 2. Cell-free DNA was extracted from 1 ml cell-free plasma using the Roche MagNA Pure Compact Nucleic Acid Isolation Kit I- Large Volume (Roche Applied Science, IN) using the MagNA Pure Compact Instrument, and eluted in 50 µl of elution buffer. Ten microliters of the extracted cfDNA were used to quantify the cfDNA, and the remainder was stored (see storage instructions WI0035 Clinical Sample Storage). The concentration of the plasma extracted cfDNA was determined by fluorescence-based quantitation with UV absorbance measurements using the Qubit™ Quantitation Platform (Invitrogen).

The concentration of cfDNA quantified in plasma samples prepared using the MagnaPure Nucleic Acid Isolation Kit I from 16 pregnant subjects was determined to range between 20 and 100 pg/µl. As the fetal component of plasma cfDNA is known to contribute 3-10% of the total plasma cfDNA, artificial plasma samples were created by spiking aliquots of cfDNA derived from plasma of female volunteer subjects with cfDNA extracted from plasma of male volunteer subjects to mimic the ratios of fetal to maternal cfDNA found in the pregnant subjects. Artificial samples were created to contain 200-1000pg of extracted female cfDNA that was spiked with 45-150 pg of extracted male cfDNA in a total volume of 10 µl. Each artificial sample was spiked to contain 3%, 5% and 10% male cfDNA.

Artificial samples having concentrations of total cfDNA of less than approximately 50 pg/µl, were preamplified using the modified improved primer extension amplification PCR (mIPEP) amplification according to the method of Hanson and Ballantyne, (Hanson and Ballantyne, Analytical Biochem 346:246-257 [2005]) as follows. Ten microliters of spiked plasma cfDNA were amplified in a 25 µl reaction volume containing 1mM dNTPs, 2.5mM MgCl₂ (Applied Biosystems), 1X Expand High Fidelity Buffer (No.3), 10.5U Expand High Fidelity Enzyme Mix (Roche Diagnostics), and 40 µM PEP primer (5'-NNNNNNNNNNNNNNN-3', Qiagen). The amplification was performed in a GeneAmp PCR System 9700 Thermocycler under the following conditions: (1) 20 and 30 cycles of 94°C for 1 minute, 37°C for 2 minutes, and 0.1°C/s ramp to 55°C for 4 minutes. The amplification product was purified using a Qiagen column. The concentration of the amplification product was determined using the Qubit™ Quantitation Platform as described above. STR analysis was performed as described in Example 9 above, except that only peak heights>100 RFU were included in the calculations.

The results are shown in Tables 9, 10 and 11. The results provided in Table 9 show that the cfDNA contained in 10µl cfDNA of artificial samples ART23 and ART24 having a starting concentration of cfDNA of 46.2 and 50.2 pg/µl, respectively, was amplified by approximately 5 and 10 fold following 20 and 30 cycles of PCR amplification, respectively.

These data indicate that a pre-amplification of cfDNA using the mIPEP method provided enhanced levels of total cfDNA rendering the level of the minor component more amenable to the STR analysis.

**TABLE 9**

| **Preamplification with mIPEP** | | | |
|---|---|---|---|
| SAMPLE | cfDNA without mIPEP (pg/µl) | cfDNA with mIPEP:20 PCR cycles (pg/50µl) | cfDNA with mIPEP:30 PCR cycles (pg/50µl) |
| ART23 | 46.2 | 2265 | 4125 |
| ART24 | 50.2 | 2085 | 3875 |

Table 10 shows triplicate measurements profiling 9 loci of the cfDNA of spiked samples ART23 and ART24 following the mIPEP procedure with 20 and 30 cycles of amplification, as described above.

The data in Table 11 indicate that pre-amplification of cfDNA enables the detection and quantification of the minor component at most loci tested in artificially mixed samples having a starting cfDNA concentration that would otherwise not permit an accurate analysis of the minor STR alleles.

**TABLE 10**

| **mIPEP Preamplification and Detection of Minor Component** | | | | | | | | |
|---|---|---|---|---|---|---|---|---|
| | | **ART23 (453pg) mIPEP amplified 20 cycles** | **ART23 (82 5pg) mIPEP amplified 20 cycles** | **ART23 (462pg) Extracted unamplified cfDNA** | | **ART24 (417pg) mIPEP amplified 30 cycles** | **ART24 (775pg) mIPEP amplifie d 30 cycles** | **ART24 (502pg) Extracted unamplified cfDNA** |
| **STR Locus** | **Allele** | **Allele Height** | **Allele Height** | **Allele Height** | **Allele** | **Allele Height** | **Allele Height** | **Allele Height** |
| **AMEL** | X/Y | 291/95 | 397/170 | 535/832 | X/Y | 695/359 | 1878/1148 | 1564/1959 |
| **AMEL** | X/Y | 425/147 | 428/188 | 675/1048 | X/Y | 1216/619 | 1551/954 | 1573/1943 |
| **AMEL** | X/Y | 267/94 | 455/203 | 664/1043 | X/Y | 718/363 | 1479/924 | 1621/2024 |
| **CSF1PO** | 10/11 | 800/979 | 725/1009 | 1429/1325 | 11/12 | 2029/1317 | 4159/2317 | 2990/3083 |
| **CSF1PO** | 10/11 | 1147/1432 | 789/1102 | 1779/1650 | 11/12 | 3449/2223 | **3460/113 /1890** | 2996/3118 |
| **CSF1PO** | 10/11 | 729/906 | 831/1162 | 1783/1657 | 11/12 | 2006/1309 | 3362/1840 | 3072/3183 |
| **D13S317** | 12 | 743 | 515 | 1229 | 11 | 955 | 1490 | 3634 |
| **D13S317** | 12 | 1079 | 563 | 1534 | 11 | 1631 | 1198 | 3631 |
| **D13S317** | 12 | 668 | 583 | 1520 | 11 | 968 | 1170 | 3795 |
| **D16S539** | 9/10 | 239/140 | 370/466 | 835/676 | 10/11 | 513/512 | 1173/1472 | 1678/973 |
| **D16S539** | 9/10 | 347/203 | 64*(OL)/3 91/489 | 1046/864 | 10/11 | 859/870 | **973/1212** | 1730/999 |
| **D16S539** | 9/10 | 227/134 | 441/515 | 1055/860 | 10/11 | 530/513 | 960/1183 | 1784/1044 |
| **D18S51** | 14/15 | 359/464 | 363/220 | 785/541 | 12/18 | 1044/576 | 1840/786 | 2559/1507 |
| | | 512/645 | 391/226 | 999/672 | 12/18 | 1769/994 | 1511/643 | 2565/1469 |
| | | 313/402 | 409/245 | 994/685 | 12/18 | 1033/567 | 1496/631 | 2643/1523 |
| **D21S11** | 29/32 | 103/104 | 114/173 | 605/413 | 31.2 | 381 | 661 | 3276 |
| | | 149/153 | 130/182 | 759/523 | 31.2 | 650 | 536 | 3028 |
| | | 85/86 | 131/196 | 760/525 | 31.2 | 380 | 520 | 3282 |
| **D2S1338** | 18/20 | 572/383 | 428/363 | 1116/1013 | 19/20 | 1066/433 | 2315/1243 | 2962/2968 |
| | | 827/553 | 454/386 | 1428/1279 | 19/20 | 1821/757 | 1901/101 | 2942/2942 |
| | | 530/351 | 482/408 | 1431/1275 | 19/20 | 1063/444 | 1859/1012 | 3072/3067 |
| **D7S820** | 11/12 | 262/167 | 149/270 | 557/627 | 11/12 | 256/138 | 520/322 | 1550/1548 |
| | | 62/366/231 | 162/292 | 699/775 | 11/12 | 448/236 | 419/258 | 1484/1466 |
| | | 224/146 | 169/307 | 689/779 | 11/12 | 253/141 | 406/250 | 1579/1573 |
| **FGA** | 21/23 | 263/146 | 181/88 | 596/365 | 22/24 | 228/244 | 375/429 | 1272/1064 |
| | | 384/215 | 191/92 | 762/450 | 22/24 | 409/425 | 303/345 | 1221/1023 |
| | | 230/136 | 202/102 | 749/456 | 22/24 | 232/250 | 297/348 | 1298/1087 |

| | | | | | | | | |
|---|---|---|---|---|---|---|---|---|
| *"OL" means "Off Ladder measurement" | | | | | | | | |

**TABLE 11**

| **Fetal Fraction Determined in a Sample Following Preamplification Using mIPEP** | | | | | | |
|---|---|---|---|---|---|---|
| **STR marker** | **Allele 1/ Height** | **Allele 2/ Height** | **Allele 3/ Height** | **Allele 4/ Height** | **Percent minor fraction/STR -minor>100 RFU** | **Percent minor fraction/STR minor <100 RFU** |
| Amelogenin | X/2799 | Y/207 | | | | |
| Amelogenin | X/2751 | Y/198 | | | | |
| Amelogenin | X/3109 | Y/232 | | | | |
| | **X/2886** | **Y/212** | | | **7** | |
| CSF1PO | 10/2377 | 11/1869 | 12/508 | | | |
| CSF1PO | 10/2299 | 11/1814 | 12/498 | | | |
| CSF1PO | 10/2616 | 11/206 | 12/562 | | | |
| | **10/2431** | **11/1917** | **12/523** | | **12** | |
| D13S317 | 10/1232 | 11/1600 | 13/186 | | | |
| D13S317 | 10/1208 | 11/1548 | 13/182 | | | |
| D13S317 | 10/1386 | 11/1758 | 13/212 | | | |
| | **10/1275** | **11/1635** | **13/193** | | **12** | |
| D16S539 | 11/757 | 12/933 | | | | |
| D16S539 | 11/729 | 12/885 | | | | |
| D16S539 | 11/836 | 12/1031 | | | | |
| | **11/774** | **12/950** | | | 12 | |
| D18S51 | OL/80 | 14/3137 | 15/371 | | | |
| D18S51 | 11/73 | 14/3082 | 15/362 | | | |
| D18S51 | OL/83 | 14/3488 | 15/413 | | | |
| | **OL** | **14/3236** | **15/382** | | | |
| D21S11 | 29/953 | 30/941 | | | | |
| D21S11 | 29/921 | 30/908 | | | | |
| D21S11 | 29/1046 | 30/1045 | | | | |
| | **29/973** | **30/965** | | | | |
| D2S1338 | 17/461 | 18/366 | 20/2280 | 24/1760 | | |
| D2S1338 | 17/460 | 18/360 | 20/2240 | 24/1712 | | |
| D2S1338 | 17/508 | 18/409 | 20/2563 | 24/1971 | | |
| | **17/476** | **18/378** | **20/2361** | **24/1814** | **20** | |
| D7S820 | 8/1409 | 9/60 | 12/1059 | | | |
| D7S820 | 8/1380 | 9/60 | 12/1036 | | | |
| D7S820 | 8/1561 | 9/69 | 12/1166 | | | |
| | **8/1450** | **9/63** | **12/1087** | | | **2** |
| FGA | 19/825 | 21/850 | 25/279 | | | |
| FGA | 19/807 | 21/841 | 25/265 | | | |
| FGA | 19/913 | 21/958 | 25/306 | | | |
| | **19/848** | **21/883** | **25/283** | | **16** | |
| % fetal fraction>100RFU for minor allele **→** 12 | | | | | **12** | |
| % fetal fraction including <100RFU for minor allele **→** 11 | | | | | | **11** |

### Example 12

### Correlation of Fetal Fraction Determined by Analysis of Fetal and Maternal SNPs and STRs

To verify that the calculated fetal fraction *i*.*e*. fraction of minor nucleic acid component, determined using the SNP and STR assay as described in the preceding Examples provided an accurate measurement of the fetal fraction, the percent fetal fraction of cfDNA in the plasma from the same pregnant subjects was compared.

Peripheral blood samples were obtained from 48 volunteer subjects, 24 of the subjects were pregnant with male fetuses, and 24 were pregnant with female fetuses. cfDNA was prepared as described in Example 1, Fetal fraction using SNPs was determined by massively parallel sequencing by synthesis as described in Example 5, and fetal fraction using STRs was determined using capillary electrophoresis as described in Example 10

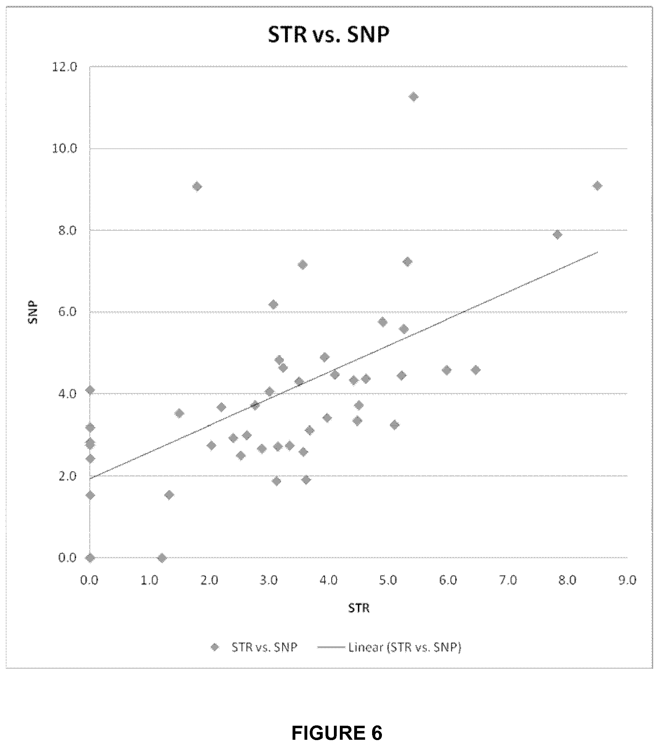

The results shown in **Figure 6** indicate that a positive correlation exists between the fraction determined using the STR assay and the fraction determined using the SNP sequencing. These data further validate the use of polymorphic sequences comprising STRs or SNPs for determining the fraction of fetal cfDNA in a plasma sample.

### Example 13

### Use of Fetal Fraction to Set Thresholds and Estimate Minimum Sample Size in Aneuploidy Detection

Counts of sequence matches to different chromosomes are manipulated to generate a score which will vary with chromosomal copy number that can be interpreted to identify chromosomal amplification or deletion. For example, such a score could be generated by comparing the relative amount of a sequence tags on a chromosome undergoing copy number changes to a chromosome known to be a euploid. Examples of scores that can be used to identify amplification or deletion include but are not limited to: counts for the chromosome of interest divided by counts of another chromosome from the same experimental run, the counts for the chromosome of interest divided by the total number of counts from the experimental run, comparison of counts from the sample of interest versus a separate control sample. Without loss of generality, it can be assumed that scores will increase as copy number increases. Knowledge of fetal fraction can be used to set "cutoff" thresholds to call "aneuploidy", "normal", or "marginal" (uncertain) states. Then, calculations are performed to estimate the minimum number of sequences required to achieve adequate sensitivity (i.e. probability of correctly identifying an aneuploidy state).

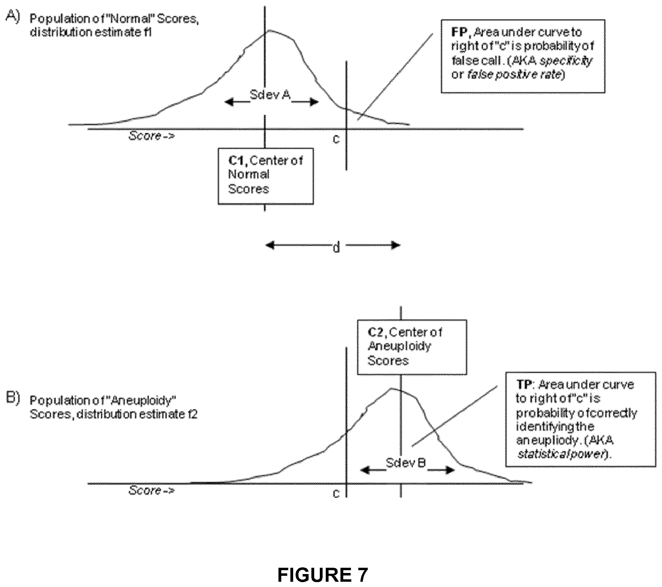

**Figure 7** is a plot of two different populations of scores. The x-axis is score and the y-axis is frequency. Scores on samples of chromosomes without aneuploidy can have a distribution shown in **Figure 7A. Figure 7B** illustrates a hypothetical distribution of a population of scores on samples with an amplified chromosome. Without loss of generality, the graphs and equations show the case of a univariate score where the aneuploidy condition represents an amplification of copy number. Multivariate cases and/or reduction/deletion abnormalities are simple extensions or rearrangements of the given descriptions and are intend to fall within the scope of this art.

The amount of "overlap" between the populations can determine how well normal and aneuploidy cases can be discriminated. In general, increasing fetal fraction, ff, increases discrimination power by separating the two population centers (by moving "C2," the "Center of Aneuploidy Scores", and increasing "d," causing the populations to overlap less. Furthermore, an increase in the absolute value of the magnitude, m, (for example having four copies of the chromosome instead of a trisomy) of the amplification will also increase separation of population centers leading to higher power (i.e. higher probability of correctly identifying aneuploidy states).

Increasing the number of sequences generated, N, reduces standard deviations "sdevA" and/or "sdevB," the spread of the two populations of scores, which also causes the populations to overlap less.

### Setting Thresholds and Estimating Sample Size

The following procedure can be used to set "c", the critical value for calling "aneuploidy", "normal", or "marginal" (uncertain) states. Without loss of generality, one sided statistical tests are used below.

First, an acceptable false positive rate, FP (sometimes also called "type I error" or "specificity"), is decided, which is the probability of a false positive or falsely calling aneuploidy. For example, FP can be at least, or about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, or 0.1.

Second, the value of "c" can be determined by solving the equation: FP = integral from c to infinity of (f1(x)dx). $FP = {\int }_{c}^{∞} f 1 \left(x\right) dx$

Once a critical value, c, has been determined, the minimum number sequences required to achieve a certain TP = True positive rate can be estimated. The true positive rate can be, for example, about 0.5, 0.6, 0.7, 0.8, or 0.9. In one embodiment, the true positive rate can be 0.8. In other words, N is the minimum number of sequences required to identify aneuploidy 100*TP percent of the time. N = minimum number such that TP = integral from c to infinity of f2(x,ff)dx > 0.8. N is determined by solving ${min}_{N} s . t . \left\{TB \geq {\int }_{c}^{∞} f 2 \left(x , N\right) dx\right\}$

In classical statistical tests f1 and f2 are often F, non-central F distributions (a special case of t and non-central t distributions) although that is not a necessary condition for this application.

### Setting "Levels" of Thresholds to Give More Control of Errors

Thresholds can also be set in stages using the above methods. For example, a threshold can be set for high confidence calling of "aneuploidy", say ca, using FP 0.001 and a "marginal" threshold, say cb, using FP 0.05. In this case if Score, S:

| | |
|---|---|
| (S > ca) | then call "Trisomy" |
| (cb > S <= ca) | then call "Marginal" |
| (S < cb) | then call "Normal" |

### Some Trivial Generalizations Falling Within Scope of this Art

Different values for thresholds such as TP, FP, etc can be used. Procedures can be run in any order. For example, one can start with N and solve for c, etc. Distributions can depend on ff so that f1(x,N,ff), f2(x,N,ff), and/or other variables. The above integral equations can be solved by reference to tables or by iterative computer methods. A non-centrality parameter can be estimated and power can be read from standard statistical tables. Statistical power and sample sizes may be derived from calculation or estimation of expected mean squares. Closed form theoretical distributions such as f, t, non-central t, normal, etc. or estimates (kernel or other) can be used to model the distributions f1, f2. Empirical threshold setting and parameter selection using Receiver Operator Characteristic Curves (ROC) can be used and collated with fetal fraction. Various estimates of distribution spread (variance, mean absolute deviation, inter quartile range, etc.) may be used. Various estimates of distribution center (mean, median, etc.) can be used. Two sided as opposed to one sided statistical tests can be used. The simple hypothesis test can be reformulated as linear or non-linear regression. Combinatorial methods, simulation (e.g., monte carlo), maximization (e.g., expectation maximization), iterative, or other methods can be used independently or in conjunction with the above to establish statistical power or thresholds.

## Claims

1. A method for determining the fraction of fetal nucleic acids in a maternal blood sample comprising a mixture of fetal and maternal genomic DNA, wherein said fetal and maternal genomic DNA is cell-free DNA (cfDNA), said method comprising:
(a) amplifying a plurality of polymorphic target nucleic acids in said mixture, to generate a panel of amplified polymorphic sites that contains a sufficient number of polymorphic sites such that at least one is an informative polymorphic site;
(b) performing massively parallel sequencing of at least a portion of the amplified product obtained in (a), wherein said sequencing comprises providing a plurality of sequence tags for said panel of amplified polymorphic sites; and
(c) based on said sequencing, determining said fraction of fetal nucleic acids, by using said plurality of sequence tags to:
(i) identify at least one informative polymorphic site in said panel of amplified polymorphic sites, wherein said at least one informative polymorphic site is identified by the difference in the allelic sequences at each polymorphic site and the number of sequence tags mapped to each of the possible alleles at each polymorphic site, and
(ii) determine said fraction of fetal nucleic acids by using the total number of tags that map to a first allele at an identified informative polymorphic site and the total number of tags that map to a second allele at the identified informative polymorphic site to calculate said fraction of fetal nucleic acids.

2. The method of Claim 1, wherein each of said plurality of polymorphic target nucleic acids comprises at least one single nucleotide polymorphism (SNP).

3. The method of Claim 2, wherein the at least one SNP is a single SNP.

4. The method of Claim 2, wherein the at least one SNP is a tandem SNP.

5. The method of Claim 1, wherein each of said plurality of polymorphic target nucleic acid comprises at least one short tandem repeat (STR).

6. The method of Claim 1, wherein said massively parallel sequencing is:
(a) sequencing-by-synthesis with reversible dye terminators; or
(b) sequencing-by-ligation.

7. The method of Claim 1, wherein said sequencing:
(a) is single molecule sequencing; or
(b) comprises an amplification.

8. The method of Claim 5, further comprising
(a) preamplifying said mixture of fetal and maternal nucleic acids; or
(b) resolving the size of the STRs using capillary electrophoresis.

9. The method of Claim 1, wherein said method is a fetal gender-independent method.

10. The method of Claim 1, wherein said plurality of polymorphic nucleic acids are located on a:
(a) plurality of different chromosomes; or
(b) chromosome other than chromosome 13, 18, 21, X or Y.

11. The method of Claim 1, wherein said plurality of polymorphic nucleic acids comprises at least 3, 4, 5, 6, 7, 8, 9, 10, 15, 20, 25, 30 or 40 polymorphic sites.

12. The method of Claim 1, wherein said panel of amplified polymorphic sites contains a sufficient number of polymorphic sites such that at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, or at least 40, are informative polymorphic sites.

13. The method of any preceding claim, wherein said method is combined with:
(a) a method used to determine the presence or absence of fetal aneuploidy in the maternal blood sample; or
(b) an assay for determining the presence or absence of a prenatal condition associated with the fetus.

14. The method of any preceding claim, further comprising:
(i) preparing a first sequencing library using at least a portion of said mixture of fetal and maternal genomic cfDNA obtained in step (a);
(ii) preparing a second sequencing library using at least a portion of the amplified product obtained in step (b); and/or
(ii) combining some or all of the second sequencing library with some or all of the first sequencing library prior to said massively parallel sequencing step (c), wherein said plurality of sequence tags allows for simultaneous determination of fraction of fetal nucleic acids and the presence or absence of fetal aneuploidy

15. The method of Claim 14, further comprising:
(i) using the calculated fraction of fetal nucleic acids in step (d) to set a cutoff threshold for calling an aneuploidy, a normal or a no call state; or
(ii) using the calculated fraction of fetal nucleic acids in step (d) to estimate the probability of correctly identifying an aneuploidy.

16. The method of any preceding claim, wherein the maternal sample comprising a mixture of fetal and maternal genomic DNA is a purified cfDNA sample extracted from a maternal plasma sample.

## Patentansprüche

1. Verfahren zur Bestimmung der Fraktion von fetalen Nukleinsäuren in einer maternalen Blutprobe, umfassend eine Mischung aus fetaler und maternaler genomer DNA, wobei die fetale und maternale genome DNA zellfreie DNA (cfDNA) ist, wobei das Verfahren umfasst:
(a) Amplifizieren einer Vielzahl von polymorphen Zielnukleinsäuren in der Mischung, um eine Auswahl von amplifizierten polymorphen Stellen zu generieren, die eine ausreichende Anzahl an polymorphen Stellen enthält, so dass mindestens eine eine informative polymorphe Stelle ist;
(b) Durchführen von massiver Parallelsequenzierung von mindestens einem Teil des in (a) erhaltenen amplifizierten Produkts, wobei das Sequenzieren das Bereitstellen einer Vielzahl von Sequenz-Tags für die Auswahl von amplifizierten polymorphen Stellen umfasst;
(c) auf Basis der Sequenzierung, Bestimmen der Fraktion von fetalen Nukleinsäuren unter Verwendung der Vielzahl von Sequenz-Tags zum:
(i) Identifizieren von mindestens einer informativen polymorphen Stelle in der Auswahl der amplifizierten polymorphen Stellen, wobei die mindestens eine informative polymorphe Stelle durch den Unterschied in den allelen Sequenzen von jeder polymorphen Stelle und die Anzahl der Sequenz-Tags, die jedem der möglichen Allelen von jeder polymorphen Stelle zugeordnet sind, identifiziert wird, und
(ii) Bestimmen der Fraktion der fetalen Nukleinsäuren, indem die Gesamtanzahl der Tags, die einem ersten Allel an einer identifizierten informativen polymorphen Stelle zugeordnet sind, und die Gesamtanzahl der Tags, die einem zweiten Allel an der identifizierten informativen polymorphen Stelle zugeordnet sind, verwendet werden, um die Fraktion der fetalen Nukleinsäuren zu berechnen.

2. Verfahren nach Anspruch 1, wobei jede der Vielzahl von polymorphen Zielnukleinsäuren mindestens einen Einzelnucleotidpolymorphismus (SNP) umfasst.

3. Verfahren nach Anspruch 2, wobei der mindestens eine SNP ein einzelner SNP ist.

4. Verfahren nach Anspruch 2, wobei der mindestens eine SNP ein Tandem-SNP ist.

5. Verfahren nach Anspruch 1, wobei jede der Vielzahl von polymorphen Zielnukleinsäuren mindestens ein Short Tandem-Repeat (STR) umfasst.

6. Verfahren nach Anspruch 1, wobei die massive Parallelsequenzierung
(a) Sequenzierung-durch-Synthese mit reversiblen Farbstoffterminatoren oder
(b) Sequenzierung-durch-Ligation ist.

7. Verfahren nach Anspruch 1, wobei die Sequenzierung
(a) Einzelmolekülsequenzierung ist oder
(b) eine Amplifizierung umfasst.

8. Verfahren nach Anspruch 5, ferner umfassend
(a) Präamplifizieren der Mischung von fetalen und maternalen Nukleinsäuren oder
(b) Aufspalten nach Größe der STRs unter Verwendung von Kapillarelektrophorese.

9. Verfahren nach Anspruch 1, wobei das Verfahren ein vom Geschlecht des Fetus unabhängiges Verfahren ist.

10. Verfahren nach Anspruch 1, wobei die Vielzahl von polymorphen Nukleinsäuren
(a) auf einer Vielzahl von unterschiedlichen Chromosomen oder
(b) auf einem anderen Chromosom als Chromosom 13, 18, 21, X oder Y lokalisiert ist.

11. Verfahren nach Anspruch 1, wobei die Vielzahl der polymorphen Nukleinsäuren mindestens 3, 4, 5, 6, 7, 8, 9, 10, 15, 20, 25, 30 oder 40 polymorphe Stellen umfasst.

12. Verfahren nach Anspruch 1, wobei die Auswahl an amplifizierten polymorphen Stellen eine ausreichende Anzahl an polymorphen Stellen enthält, so dass mindestens 2, mindestens 3, mindestens 4, mindestens 5, mindestens 6, mindestens 7, mindestens 8, mindestens 9, mindestens 10, mindestens 11, mindestens 12, mindestens 13, mindestens 14, mindestens 15, mindestens 16, mindestens 17, mindestens 18, mindestens 19, mindestens 20, mindestens 25, mindestens 30 oder mindestens 40 informative polymorphe Stellen sind.

13. Verfahren nach einem der vorhergehenden Ansprüche, wobei das Verfahren kombiniert wird mit
(a) einem Verfahren, das zur Bestimmung der Anwesenheit oder Abwesenheit von fetaler Aneuploidie in der maternalen Blutprobe verwendet wird, oder
(b) einem Assay zur Bestimmung der Anwesenheit oder Abwesenheit eines pränatalen Zustands, der mit dem Fetus im Zusammenhang steht.

14. Verfahren nach einem der vorhergehenden Ansprüche, ferner umfassend:
(i) Herstellen einer ersten Sequenzierungsbibliothek unter Verwendung von mindestens einem Teil der in Schritt (a) erhaltenen Mischung aus fetaler und maternaler genomer cfDNA;
(ii)Herstellen einer zweiten Sequenzierungsbibliothek unter Verwendung von mindestens einem Teil des in Schritt (b) erhaltenen amplifizierten Produkts; und/oder (iii) Kombinieren von einem Teil oder der gesamten zweiten Sequenzierungsbibliothek mit einem Teil oder der gesamten ersten Sequenzierungsbibliothek vor dem massiven Parallelsequenzierungsschritt (c), wobei die Vielzahl von Sequenz-Tags die gleichzeitige Bestimmung der Fraktion von fetalen Nukleinsäuren und der Anwesenheit oder Abwesenheit von fetaler Aneuploidie zulässt.

15. Verfahren nach Anspruch 14, ferner umfassend:
(i) Verwenden der berechneten Fraktion von fetalen Nukleinsäuren in Schritt (d) zum Festlegen eines Grenzschwellenwerts zum Angeben eines Aneuploidie-, eines normalen oder eines nicht eindeutigen Zustands; oder
(ii)Verwenden der berechneten Fraktion von fetalen Nukleinsäuren in Schritt (d) zur Abschätzung der Wahrscheinlichkeit des korrekten Identifizierens einer Aneuploidie.

16. Verfahren nach einem der vorhergehenden Ansprüche, wobei die maternale Probe, die eine Mischung aus fetaler und maternaler genomer DNA umfasst, eine aufgereinigte cfDNA-Probe ist, die aus einer maternalen Plasmaprobe extrahiert wurde.

## Revendications

1. Procédé de détermination de la fraction d'acides nucléiques foetaux dans un échantillon de sang maternel comprenant un mélange d'ADN génomique foetal et maternel, dans lequel ledit ADN génomique foetal et maternel est de l'ADN sans cellules (ADNcf), ledit procédé comprenant :
(a) l'amplification d'une pluralité d'acides nucléiques cibles polymorphes dans ledit mélange, afin de générer un panel de sites polymorphes amplifiés qui contient un nombre suffisant de sites polymorphes tel qu'au moins un est un site polymorphe informatif ;
(b) la conduite d'un séquençage massif en parallèle d'au moins une partie du produit amplifié obtenu dans (a), ledit séquençage comprenant la fourniture d'une pluralité d'étiquettes de séquence pour ledit panel de sites polymorphes amplifiés ; et
(c) sur la base dudit séquençage, la détermination de ladite fraction d'acides nucléiques foetaux, par de ladite pluralité d'étiquettes de séquence pour :
(i) identifier au moins un site polymorphe informatif dans ledit panel de sites polymorphes amplifiés, ledit au moins un site polymorphe informatif étant identifié par la différence des séquences alléliques à chaque site polymorphe et du nombre d'étiquettes de séquence associée à chacun des allèles possibles à chaque site polymorphe, et
(ii) déterminer ladite fraction d'acides nucléiques foetaux au moyen du nombre total d'étiquettes qui correspondent à un premier allèle à un site polymorphe informatif identifié et le nombre total d'étiquettes qui correspondent à un deuxième allèle au site polymorphe informatif identifié pour calculer ladite fraction d'acides nucléiques foetaux.

2. Procédé de la revendication 1, dans lequel chacun de ladite pluralité d'acides nucléiques cibles polymorphes comprend au moins un polymorphisme de simple nucléotide (SNP).

3. Procédé de la revendication 2, dans lequel l'au moins un SNP est un SNP unique.

4. Procédé de la revendication 2, dans lequel l'au moins un SNP est un SNP en tandem.

5. Procédé de la revendication 1, dans lequel chacun de ladite pluralité d'acides nucléiques cibles polymorphes comprend au moins une répétition en tandem courte (STR).

6. Procédé de la revendication 1, dans lequel ledit séquençage massif en parallèle est :
(a) un séquençage par synthèse avec des terminateurs de colorant réversible ; ou
(b) un séquençage par ligature.

7. Procédé de la revendication 1, dans lequel ledit séquençage :
(a) est un séquençage de molécule unique ; ou
(b) comprend une amplification.

8. Procédé de la revendication 5, comprenant en outre
(a) la préamplification dudit mélange d'acides nucléiques foetaux et maternels ; ou
(b) la résolution de la taille des STR par électrophorèse capillaire.

9. Procédé de la revendication 1, **caractérisé en ce que** ledit procédé est un procédé indépendant du sexe foetal.

10. Procédé de la revendication 1, dans lequel ladite pluralité d'acides nucléiques polymorphes sont situés sur :
(a) une pluralité de chromosomes différents ; ou
(b) un chromosome autre que le chromosome 13, 18, 21, X ou Y.

11. Procédé de la revendication 1, dans lequel ladite pluralité d'acides nucléiques polymorphes comprend au moins 3, 4, 5, 6, 7, 8, 9, 10, 15, 20, 25, 30 ou 40 sites polymorphes.

12. Procédé de la revendication 1, dans lequel ledit panel de sites polymorphes amplifiés contient un nombre suffisant de sites polymorphes tels qu'au moins 2, au moins 3, au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 25, au moins 30 ou au moins 40, qui sont des sites polymorphes informatifs.

13. Procédé de l'une quelconque des revendications précédentes, **caractérisé en ce que** ledit procédé est combiné avec :
(a) un procédé utilisé pour déterminer la présence ou l'absence d'aneuploïdie foetale dans l'échantillon de sang maternel ; ou
(b) un essai pour déterminer la présence ou l'absence d'une affection prénatale associée au foetus.

14. Procédé de l'une quelconque des revendications précédentes, comprenant en outre :
(i) la préparation d'une première banque de séquençage au moyen d'au moins une partie dudit mélange d'ADNcf génomique foetal et maternel obtenu dans l'étape (a) ;
(ii) la préparation d'une deuxième banque de séquençage au moyen d'au moins une partie du produit amplifié obtenu dans l'étape (b) ; et/ou
(iii) la combinaison d'une partie ou de la totalité de la deuxième banque de séquençage avec une partie ou la totalité de la première banque de séquençage avant ladite étape de séquençage massif en parallèle (c), dans lequel ladite pluralité d'étiquettes de séquence permet la détermination simultanée d'une fraction d'acides nucléiques foetaux et la présence ou l'absence d'aneuploïdie foetale.

15. Procédé de la revendication 14, comprenant en outre :
(i) l'utilisation de la fraction calculée d'acides nucléiques foetaux dans l'étape (d) pour définir un seuil de coupure pour indiquer une aneuploïdie, un état normal ou un état sans indication ; ou
(ii) l'utilisation de la fraction calculée d'acides nucléiques foetaux dans l'étape (d) pour estimer la probabilité d'identifier correctement une aneuploïdie.

16. Procédé de l'une quelconque des revendications précédentes, dans lequel l'échantillon maternel comprenant un mélange d'ADN génomique foetal et maternel est un échantillon d'ADNcf purifié extrait d'un échantillon de plasma maternel.
